# EUROPEAN PATENT APPLICATION

(11) **EP 3 735 990 A1**
(43) Date of publication of application: **11.11.2020**
(21) Application number: 19217139.5
(22) Date of filing: 09.12.2015
(51) Int. Cl.: A61K 47/68, A61K 31/4545, A61P 35/00, A61P 43/00

(54) **ANTIBODY DRUG CONJUGATES WITH CELL PERMEABLE BCL-XL INHIBITORS**

(30) Priority: 09.12.2014 US 201462089766 P
(62) Divisional of application: 15819955.4
(71) Applicant: Abbvie Inc., North Chicago, IL 60064 (US)
(72) Inventor: Boghaert, Erwin R., Wisconsin, WI Wisconsin 53158 (US); Ackler, Scott L., Gurnee, IL Illinois 60031 (US); Tao, Zhi-Fu, Vernon Hills, IL Illinois 60061 (US); Wang, Xilu, Libertyville, IL Illinois 60048 (US); Doherty, George, Libertyville, IL Illinois 60048 (US); Marin, Violeta L., Chicago, IL Illinois 60641 (US); Sullivan, Gerard M., Libertyville, IL 60048-5212 (US); Song, Xiaohong, Grayslake, IL Illinois 60030 (US); Kunzer, Aaron R., Arlington Heights, IL Illinois (US); Welch, Dennie S., Gurnee, IL Illinois 60031 (US); Bruncko, Milan, Green Oaks, IL Illinois 60048 (US); Judd, Andrew S., Grayslake, IL Illinois 60030 (US); Souers, Andrew J., Libertyville, IL Illinois 60048 (US)
(74) Representative: Schlich, George

(57) **Abstract**

Small molecule Bcl-xL inhibitors and Antibody Drug Conjugates (ADCs) comprising small molecule Bcl-xL inhibitors are disclosed herein. The Bcl-xL inhibitors and ADCs of the disclosure are useful for, among other things, inhibiting anti-apoptotic Bcl-xL proteins as a therapeutic approach towards the treatment of diseases that involve a dysregulated apoptosis pathway.

## Description

### 1. FIELD

The present disclosure pertains to compounds that inhibit the activity of Bcl-xL anti-apoptotic proteins, antibody drug conjugates comprising these inhibitors, methods useful for synthesizing these inhibitors and antibody drug conjugates, compositions comprising the inhibitors and antibody drug conjugates, and methods of treating diseases in which anti-apoptotic Bcl-xL proteins are expressed.

### 2. BACKGROUND

Apoptosis is recognized as an essential biological process for tissue homeostasis of all living species. In mammals in particular, it has been shown to regulate early embryonic development. Later in life, cell death is a default mechanism by which potentially dangerous cells (e.g., cells carrying cancerous defects) are removed. Several apoptotic pathways have been uncovered, and one of the most important involves the Bcl-2 family of proteins, which are key regulators of the mitochondrial (also called "intrinsic") pathway of apoptosis. See, Danial & Korsmeyer, 2004, Cell 116:205-219.

Dysregulated apoptotic pathways have been implicated in the pathology of many significant diseases such as neurodegenerative conditions (up-regulated apoptosis), such as for example, Alzheimer's disease; and proliferative diseases (down-regulated apoptosis) such as for example, cancer, autoimmune diseases and pro-thrombotic conditions.

In one aspect, the implication that down-regulated apoptosis (and more particularly the Bcl-2 family of proteins) is involved in the onset of cancerous malignancy has revealed a novel way of targeting this still elusive disease. Research has shown, for example, the anti-apoptotic proteins, Bcl-2 and Bcl-xL, are over-expressed in many cancer cell types. See, Zhang, 2002, Nature Reviews/Drug Discovery 1:101; Kirkin et al., 2004, Biochimica Biophysica Acta 1644:229-249; and Amundson et al., 2000, Cancer Research 60:6101-6110. The effect of this deregulation is the survival of altered cells which would otherwise have undergone apoptosis under normal conditions. The repetition of these defects associated with unregulated proliferation is thought to be the starting point of cancerous evolution.

These findings as well as numerous others have made possible the emergence of new strategies in drug discovery for targeting cancer. If a small molecule were able to enter the cell and overcome the anti-apoptotic protein over-expression, then it could be possible to reset the apoptotic process. This strategy can have the advantage that it can alleviate the problem of drug resistance which is usually a consequence of apoptotic deregulation (abnormal survival).

Researchers also have demonstrated that platelets also contain the necessary apoptotic machinery (e.g., Bax, Bak, Bcl-xL, Bcl-2, cytochrome c, caspase-9, caspase-3 and APAF-1) to execute programmed cell death through the intrinsic apoptotic pathway. Although circulating platelet production is a normal physiological process, a number of diseases are caused or exacerbated by excess of, or undesired activation of, platelets. The above suggests that therapeutic agents capable of inhibiting anti-apoptotic proteins in platelets and reducing the number of platelets in mammals may be useful in treating pro-thrombotic conditions and diseases that are characterized by an excess of, or undesired activation of, platelets.

Numerous Bcl-xL inhibitors have been developed for treatment of diseases (e.g., cancer) that involve dysregulated apoptotic pathways. However, Bcl-xL inhibitors can act on cells other than the target cells (e.g., cancer cells). For instance, pre-clinical studies have shown that pharmacological inactivation of Bcl-xL reduces platelet half-life and causes thrombocytopenia (see Mason et al., 2007, Cell 128:1173-1186).

Given the importance of Bcl-xL in regulating apoptosis, there remains a need in the art for agents that inhibit Bcl-xL activity, either selectively or non-selectively, as an approach towards the treatment of diseases in which apoptosis is dysregulated via expression or over-expression of anti-apoptotic Bcl-2 family proteins, such as Bcl-xL. Accordingly, new Bcl-xL inhibitors with reduced dose-limiting toxicity are needed.

Additionally, new methods of delivering Bcl-xL inhibitors that limit toxicity are needed. One potential means of delivering a drug to a cell which has not been explored for Bcl-xL inhibitors is delivery through the use of antibody drug conjugates (ADCs). ADCs are formed by chemically linking a cytotoxic drug to a monoclonal antibody through a linker. The monoclonal antibody of an ADC selectively binds to a target antigen of a cell (e.g., cancer cell) and releases the drug into the cell. ADCs have therapeutic potential because they combine the specificity of the antibody and the cytotoxic potential of the drug. Nonetheless, developing ADCs as therapeutic agents has thus far met with limited success owing to a variety of factors such as unfavorable toxicity profiles, low efficacies and poor pharmacological parameters. Accordingly, the development of new ADCs that overcomes these problems and can selectively deliver Bcl-xL to target cancer cells would be a significant discovery.

### 3. SUMMARY

It has now been discovered that small molecule inhibitors of Bcl-xL are efficacious when administered in the form of antibody drug conjugates (ADCs; also called immunoconjugates) that bind to antigens expressed on the surface of cells where inhibition of Bcl-xL and consequent induction of apoptosis would be beneficial. This discovery provides, for the first time, the ability to target Bcl-xL inhibitory therapies to specific cells and/or tissues of interest, potentially lowering serum levels necessary to achieve desired therapeutic benefit and/or avoiding and/or ameliorating potential side effects associated with systemic administration of the small molecule Bcl-xL inhibitors *per se.*

Accordingly, in one aspect, the present disclosure provides ADCs comprising inhibitors of Bcl-xL useful for, among other things, inhibiting anti-apoptotic Bcl-xL proteins as a therapeutic approach towards the treatment of diseases that involve a dysregulated apoptosis pathway. The ADCs generally comprise small molecule inhibitors of Bcl-xL linked by way of linkers to an antibody that specifically binds an antigen expressed on a target cell of interest.

The antibody of an ADC may be any antibody that binds, typically but not necessarily specifically, to an antigen expressed on the surface of a target cell of interest. Target cells of interest will generally include cells where induction of apoptosis via inhibition of anti-apoptotic Bcl-xL proteins is desirable, including, by way of example and not limitation, tumor cells that express or over-express Bcl-xL. Target antigens may be any protein expressed on the target cell of interest, but will typically be proteins that are either uniquely expressed on the target cell and not on normal or healthy cells, or that are over-expressed on the target cell as compared to normal or healthy cells, such that the ADCs selectively target specific cells of interest, such as, for example, tumor cells. As is well-known in the art, ADCs bound to certain cell-surface antigens that internalize a bound ADC have certain advantages. Accordingly, in some embodiments, the antigen targeted by the antibody is an antigen that has the ability to internalize an ADC bound thereto. However, the antigen targeted by the ADC need not be one that internalizes the bound ADC. Bcl-xL inhibitors released outside the target cell or tissue may enter the cell via passive diffusion or other mechanisms to inhibit Bcl-xL.

As will be appreciated by skilled artisans, the specific antigen, and hence antibody, selected will depend upon the identity of the desired target cell of interest. In certain specific therapeutic embodiments, the target antigen for the antibody of the ADC is an antigen that is not expressed on a normal or healthy cell type known or suspected of being dependent, at least in part, on Bcl-xL for survival. In other certain specific therapeutic embodiments, the antibody of the ADC is an antibody suitable for administration to humans.

A vast array of cell-specific antigens useful as therapeutic targets, as well as antibodies that bind these antigens, are known in the art, as are techniques for obtaining additional antibodies suitable for targeting known cell-specific antigens or later-discovered cell-specific antigens. Any of these various different antibodies may be included in the ADCs described herein.

The linkers linking the Bcl-xL inhibitors to the antibody of an ADC may be long, short, flexible, rigid, hydrophilic or hydrophobic in nature, or may comprise segments have different characteristics, such as segments of flexibility, segments of rigidity, *etc.* The linker may be chemically stable to extracellular environments, for example, chemically stable in the blood stream, or may include linkages that are not stable and release the Bcl-xL inhibitor in the extracellular millieu. In some embodiments, the linker includes linkages that are designed to release the Bcl-xL inhibitor upon internalization of the ADC within the cell. In some specific embodiments, the linker includes linkages designed to cleave and/or immolate or otherwise breakdown specifically or non-specifically inside cells. A wide variety of linkers useful for linking drugs to antibodies in the context of ADCs are known in the art. Any of these linkers, as well as other linkers, may be used to link the Bcl-xL inhibitors to the antibody of the ADCs described herein.

The number of Bcl-xL inhibitors linked to the antibody of an ADC can vary (called the "drug-to-antibody ratio," or "DAR"), and will be limited only by the number of available attachment sites on the antibody and the number of inhibitors linked to a single linker. Typically, a linker will link a single Bcl-xL inhibitor to the antibody of an ADC. As long as the ADC does not exhibit unacceptable levels of aggregation under the conditions of use and/or storage, ADCs with DARs of twenty, or even higher, are contemplated. In some embodiments, the ADCs described herein may have a DAR in the range of about 1-10, 1-8, 1-6, or 1-4. In certain specific embodiments, the ADCs may have a DAR of 2, 3 or 4. In some embodiments, Bcl-xL inhibitors, linkers and DAR combinations are selected such that the resultant ADC does not aggregate excessively under conditions of use and/or storage.

The Bcl-xL inhibitors comprising the ADCs described herein are generally compounds according to structural formula (IIa), below, and/or pharmaceutically acceptable salts thereof, where the various substituents Ar, Z¹, Z², R¹, R², R⁴, R^{10a}, R^{10b}, R^{10c}, R^{11a}, R^{11b}, and *n* are as defined in the Detailed Description section:

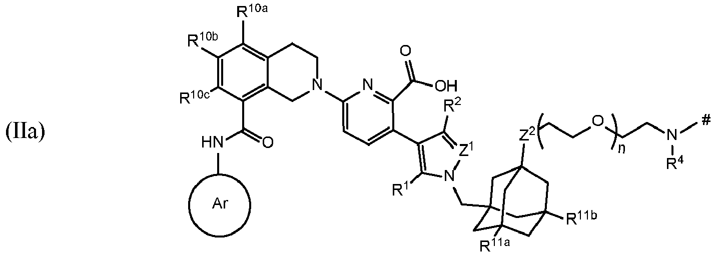

In formula (IIa), # represents the point of attachment to the linker. In an inhibitor that is not part of an ADC, # would represent a hydrogen atom.

In some embodiments, the ADCs described herein are generally compounds according to structural formula (I): 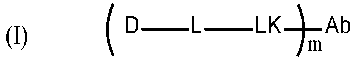 where Ab represents the antibody, D represents the drug (here, a Bcl-xL inhibitor), L represents the linker linking the drug D to the antibody Ab, LK represents a linkage formed between a functional group on linker L and a complementary functional group on antibody Ab, and m represents the number of linker-drug units linked to the antibody.

In certain specific embodiments, the ADCs are compounds according to structural formula (la), below, where the various substituents Ar, Z¹, Z², R¹, R², R⁴, R^{10a}, R^{10b}, R^{10c}, R^{11a}, R^{11b}, and *n* are as previously defined for formula (IIa), respectively, Ab and L are as defined for structural formula (I), and m is an integer ranging from 1 to 20, and in some embodiments from 2 to 8, and in some embodiments 1 to 8, and in some embodiments 2, 3, or 4:

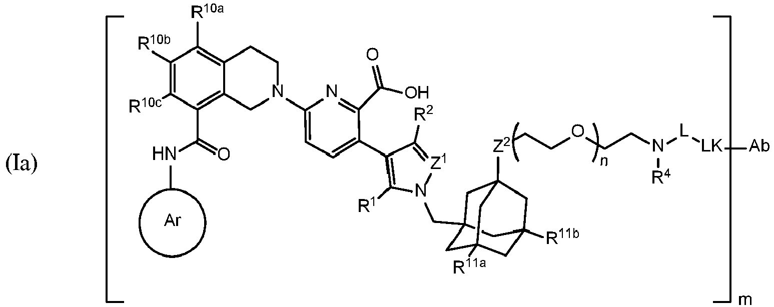

In another aspect, the present disclosure provides intermediate synthons useful for synthesizing the ADCs described herein, as well as methods for synthesizing the ADCs. The intermediate synthons generally comprise Bcl-xL inhibitors linked to a linker moiety that includes a functional group capable of linking the synthon to an antibody. The synthons are generally compounds according to structural formula (III), below, or salts thereof, where D is a Bcl-xL inhibitor as previously described herein, L is a linker as previously described and R*^{x}* comprises a functional group capable of conjugating the synthon to a complementary functional group on an antibody:

(III) D-L-R^{x}

In certain specific embodiments, the intermediate synthons are compounds according to structural formula (IIIa), below, or salts thereof, where the various substituents Ar, Z¹, Z², R¹, R², R⁴, R^{10a}, R^{10b}, R^{10c}, R^{11a}, R^{11b}, and *n* are as previously defined for structural formula (IIa), and R*^{x}* comprises a functional group as described above:

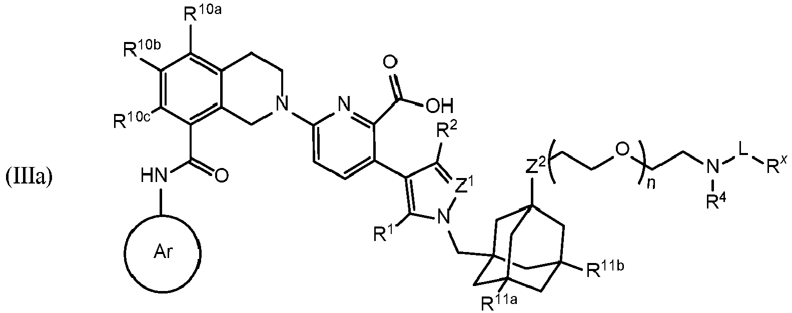

To synthesize an ADC, intermediate synthons according to structural formulae (III) or (IIIa), or salts thereof, are contacted with an antibody of interest under conditions in which functional group R*^{x}* reacts with a complementary functional group on the antibody to form a covalent linkage. The identity of group R^{x} will depend upon the desired coupling chemistry and the complementary groups on the antibody to which the synthons will be attached. Numerous groups suitable for conjugating molecules to antibodies are known in the art. Any of these groups may be suitable for R^{x}. Non-limiting exemplary functional groups (R*^{x}*) include NHS-esters, maleimides, haloacetyls, isothiocyanates, vinyl sulfones and vinyl sulfonamides.

In another aspect, the present disclosure provides compositions including the ADCs described herein. The compositions generally comprise one or more ADCs as described herein, and/or salts thereof, and one or more excipients, carriers or diluents. The compositions may be formulated for pharmaceutical use, or other uses. In a specific embodiment, the composition is formulated for pharmaceutical use and comprises an ADC according to structural formula (la), or a pharmaceutically acceptable salt thereof, and one or more pharmaceutically acceptable excipients, carriers or diluents.

Compositions formulated for pharmaceutical use may be packaged in bulk form suitable for multiple administrations, or may be packaged in the form of unit doses suitable for a single administration. Whether packaged in bulk or in the form of unit doses, the composition may be a dry composition, such as a lyophilate, or a liquid composition. Unit dosage liquid compositions may be conveniently packaged in the form of syringes pre-filled with an amount of ADC suitable for a single administration.

In still another aspect, the present disclosure provides methods of inhibiting anti-apoptotic Bcl-xL proteins. The method generally involves contacting an ADC as described herein, for example, an ADC according to structural formula (Ia), with a target cell that expresses or overexpresses Bcl-xL and an antigen for the antibody of the ADC under conditions in which the antibody binds the antigen on the target cell. Depending upon the antigen, the ADC may become internalized into the target cell. The method may be carried out *in vitro* in a cellular assay to inhibit Bcl-xL activity, or *in vivo* as a therapeutic approach towards the treatment of diseases in which inhibition of Bcl-xL activity is desirable.

In still another aspect, the present disclosure provides methods of inducing apoptosis in cells. The method generally involves contacting an ADC as described herein, for example, an ADC according to structural formula (Ia), with a target cell that expresses or overexpresses Bcl-xL and an antigen for the antibody of the ADC under conditions in which the antibody binds the antigen on the target cell. Depending upon the antigen, the ADC may become internalized into the target cell. The method may be carried out *in vitro* in a cellular assay to induce apoptosis, or *in vivo* as a therapeutic approach towards the treatment of diseases in which induction of apoptosis in specific cells would be beneficial. In one embodiment, the antibody of the ADC described herein binds a cell surface receptor or a tumor associated antigen expressed on a tumor cell. In another embodiment, the antibody of the ADC described herein binds one of the cell surface receptors or tumor associated antigens selected from EGFR, EpCAM and NCAM1. In another embodiment, the antibody of the ADC described herein binds EGFR, EpCAM or NCAM1. In another embodiment, the antibody of the ADC described herein binds EpCAM or NCAM1. In another embodiment, the antibody of the ADC described herein binds EpCAM. In another embodiment, the antibody of the ADC described herein binds NCAM1. In another embodiment, the antibody of the ADC described herein binds EGFR.

In yet another aspect, the present disclosure provides methods of treating disease in which inhibition of Bcl-xL and/or induction of apoptosis would be desirable. As will be discussed more thoroughly in the Detailed Description section, a wide variety of diseases are mediated, at least in part, by dysregulated apoptosis stemming, at least in part, by expression and/or overexpression of anti-apoptotic Bcl-xL proteins. Any of these diseases may be treated or ameliorated with the ADCs described herein.

The methods generally involve administering to a subject suffering from a disease mediated, at least in part, by expression or overexpression of Bcl-xL, an amount of an ADC effective to provide therapeutic benefit. The identity of the antibody of the ADC administered will depend upon the disease being treated. The therapeutic benefit achieved with the ADCs described herein will also depend upon the disease being treated. In certain instances, the ADC may treat or ameliorate the specific disease when administered as monotherapy. In other instances, the ADC may be part of an overall treatment regimen including other agents that, together with the ADC, treat or ameliorate the disease.

For example, elevated expression levels of Bcl-xL have been associated with resistance to chemotherapy and radiation therapy in cancers (Datta et al., 1995, Cell Growth Differ 6:363-370; Amundson et al., 2000, Cancer Res 60:6101-6110; Haura et al., 2004, Clin Lung Cancer 6:113-122). In the context of treating cancers, data disclosed herein establish that ADCs may be effective as monotherapy or may be effective when administered adjunctive to, or with, other targeted or non-targeted chemotherapeutic agents and/or radiation therapy. While not intending to be bound by any theory of operation, it is believed that inhibition of Bcl-xL activity with the ADCs described herein in tumors that have become resistant to targeted or non-targeted chemo- and/or radiation therapies will "sensitize" the tumors such that they are again susceptible to the chemotherapeutic agents and/or radiation treatment.

Accordingly, in the context of treating cancers, "therapeutic benefit" includes administration of the ADCs described herein adjunctive to, or with, targeted or non-targeted chemotherapeutic agents and/or radiation therapy, either in patients that have not yet begun the chemo- and/or radiation therapeutic regimens, or in patients that have exhibited resistance (or are suspected or becoming resistant) to the chemo- and/or radiation therapeutic regimens, as a means of sensitizing the tumors to the chemo- and/or radiation therapy. One embodiment pertains to a method of sensitizing a tumor to standard cytotoxic agents and/or radiation, comprising contacting the tumor with an ADC described herein that is capable of binding the tumor, in an amount effective to sensitize the tumor cell to a standard cytotoxic agent and/or radiation. Another embodiment pertains to a method of sensitizing a tumor to standard cytotoxic agents and/or radiation, comprising contacting the tumor with an ADC described herein that is capable of binding the tumor, in an amount effective to sensitize the tumor cell to a standard cytotoxic agent and/or radiation in which the tumor has become resistant to treatment with standard cytotoxic agents and/or radiation. Another embodiment pertains to a method of sensitizing a tumor to standard cytotoxic agents and/or radiation, comprising contacting the tumor with an ADC described herein that is capable of binding the tumor, in an amount effective to sensitize the tumor cell to a standard cytotoxic agent and/or radiation in which the tumor has not been previously exposed to standard cytotoxic agents and/or radiation therapy.

### 4. BRIEF DESCRIPTION OF THE FIGURES

**FIG. 1** depicts the inhibition of H1650 xenograft tumor growth after treatment with EGFR-targeting Bcl-xL inhibitory ADCs (Bcl-xLi ADCs) or in combination with docetaxel (DTX).
**FIG. 2** depicts the inhibition of H1650 xenograft tumor growth after treatment with various doses of EGFR-targeting Bcl-xLi ADCs.
**FIG. 3** depicts the inhibition of EBC-1 xenograft tumor growth after treatment with EGFR-targeting Bcl-xLi ADCs as single agent or in combination with docetaxel (DTX).
**FIG. 4** depicts inhibition of H146 xenograft tumor growth after treatment with a α-NCAM1 targeting Bcl-xLi ADC administered as single agent or in combination with the selective Bcl-2 inhibitor, ABT-199.
**FIG. 5** depicts the inhibition of H1963.FP5 xenograft tumor growth after treatment with a α-NCAM1 targeting Bcl-xLi ADC administered as single agent or in combination with the selective Bcl-2 inhibitor, ABT-199.
**FIG. 6** depicts the influence of Bcl-xLi ADCs with cell permeating Bcl-xL inhibitors on the number of circulating platelets.

### 5. DETAILED DESCRIPTION

The present disclosure concerns ADCs, synthons useful for synthesizing the ADCs, compositions comprising the ADCs and various methods of using the ADCs.

As will be appreciated by skilled artisans, the ADCs disclosed herein are "modular" in nature. Throughout the instant disclosure, various specific embodiments of the various "modules" comprising the ADCs, as well as the synthons useful for synthesizing the ADCs, are described. As specific non-limiting examples, specific embodiments of antibodies, linkers, and Bcl-xL inhibitors that may comprise the ADCs and synthons are described. It is intended that all of the specific embodiments described may be combined with each other as though each specific combination were explicitly described individually.

It will also be appreciated by skilled artisans that the various Bcl-xL inhibitors, ADCs and/or ADC synthons described herein may be in the form of salts, and in certain embodiments, particularly pharmaceutically acceptable salts. The compounds of the present disclosure that possess a sufficiently acidic, a sufficiently basic, or both functional groups, can react with any of a number of inorganic bases, and inorganic and organic acids, to form a salt. Alternatively, compounds that are inherently charged, such as those with a quaternary nitrogen, can form a salt with an appropriate counterion, e.g., a halide such as a bromide, chloride, or fluoride.

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenylsulfonic acid, carbonic acid, succinic acid, citric acid, *etc.* Base addition salts include those derived from inorganic bases, such as ammonium and alkali or alkaline earth metal hydroxides, carbonates, bicarbonates, and the like.

In the disclosure below, if both structural diagrams and nomenclature are included and if the nomenclature conflicts with the structural diagram, the structural diagram controls.

### 5.1. DEFINITIONS

Unless otherwise defined herein, scientific and technical terms used in connection with the present disclosure shall have the meanings that are commonly understood by those of ordinary skill in the art.

Various chemical substituents are defined below. In some instances, the number of carbon atoms in a substituent (*e.g.,* alkyl, alkanyl, alkenyl, alkynyl, cycloalkyl, heterocyclyl, heteroaryl, and aryl) is indicated by the prefix "Cₓ-C_{y}," wherein x is the minimum and y is the maximum number of carbon atoms. Thus, for example, "C₁-C₆ alkyl" refers to an alkyl containing from 1 to 6 carbon atoms. Illustrating further, "C₃-C₈ cycloalkyl" means a saturated hydrocarbyl ring containing from 3 to 8 carbon ring atoms. If a substituent is described as being "substituted," a hydrogen atom on a carbon or nitrogen is replaced with a non-hydrogen group. For example, a substituted alkyl substituent is an alkyl substituent in which at least one hydrogen atom on the alkyl is replaced with a non-hydrogen group. To illustrate, monofluoroalkyl is alkyl substituted with a fluoro radical, and difluoroalkyl is alkyl substituted with two fluoro radicals. It should be recognized that if there is more than one substitution on a substituent, each substitution may be identical or different (unless otherwise stated). If a substituent is described as being "optionally substituted", the substituent may be either (1) not substituted or (2) substituted. Possible substituents include, but are not limited to, C₁-C₆ alkyl, C₂-C₆ alkenyl, C₂-C₆ alkynyl, aryl, cycloalkyl, heterocyclyl, heteroaryl, halogen, C₁-C₆ haloalkyl, oxo, -CN, NO₂, -OR^{xa}, -OC(O)R^{z}, -OC(O)N(R^{xa})₂, -SR^{xa}, -S(O)₂R^{xa}, -S(O)₂N(R^{xa})₂, -C(O)R^{xa}, -C(O)OR^{xa}, -C(O)N(R^{xa})₂, -C(O)N(R^{xa})S(O)₂R^{z}, -N(R^{xa})₂, -N(R^{xa})C(O)R^{z}, -N(R^{xa})S(O)₂R^{z}, -N(R^{xa})C(O)O(R^{z}), -N(R^{xa})C(O)N(R^{xa})₂, -N(R^{xa})S(O)₂N(R^{xa})₂, -(C₁-C₆ alkylenyl)-CN, -(C₁-C₆ alkylenyl)-OR^{xa}, -(C₁-C₆ alkylenyl)-OC(O)R^{z}, -(C₁-C₆ alkylenyl)-OC(O)N(R^{xa})₂, -(C₁-C₆ alkylenyl)-SR^{xa}, -(C₁-C₆ alkylenyl)-S(O)₂R^{xa}, -(C₁-C₆ alkylenyl)-S(O)₂N(R^{xa})₂, -(C₁-C₆ alkylenyl)-C(O)R^{xa}, -(C₁-C₆ alkylenyl)-C(O)OR^{xa}, -(C₁-C₆ alkylenyl)-C(O)N(R^{xa})₂, -(C₁-C₆ alkylenyl)-C(O)N(R^{xa})S(O)₂R^{z}, -(C₁-C₆ alkylenyl)-N(R^{xa})₂, -(C₁-C₆ alkylenyl)-N(R^{xa})C(O)R^{z}, -(C₁-C₆ alkylenyl)-N(R^{xa})S(O)₂R^{z}, -(C₁-C₆ alkylenyl)-N(R^{xa})C(O)O(R^{z}), -(C₁-C₆ alkylenyl)-N(R^{xa})C(O)N(R^{xa})₂, or -(C₁-C₆ alkylenyl)-N(R^{xa})S(O)₂N(R^{xa})₂; wherein R^{xa}, at each occurrence, is independently hydrogen, aryl, cycloalkyl, heterocyclyl, heteroaryl, C₁-C₆ alkyl, or C₁-C₆ haloalkyl; and R^{z}, at each occurrence, is independently aryl, cycloalkyl, heterocyclyl, heteroaryl, C₁-C₆ alkyl or C₁-C₆ haloalkyl.

Various ADCs, synthons and Bcl-xL inhibitors comprising the ADCs and/or synthons are described in some embodiments herein by reference to structural formulae including substitments, for example substituents Ar, Z¹, Z², R¹, R², R⁴, R^{10a}, R^{10b}, R^{10c}, R^{11a}, R^{11b}, L, R^{x}, F^{x}, LK, Ab, *n,* and/or *m.* It is to be understood that the various groups comprising substituents may be combined as valence and stability permit. Combinations of substituents and variables envisioned by this disclosure are only those that result in the formation of stable compounds. As used herein, the term "stable" refers to compounds that possess stability sufficient to allow manufacture and that maintain the integrity of the compound for a sufficient period of time to be useful for the purpose detailed herein.

As used herein, the following terms are intended to have the following meanings:

The term "alkoxy" refers to a group of the formula -OR^{a}, where R^{a} is an alkyl group. Representative alkoxy groups include methoxy, ethoxy, propoxy, tert-butoxy and the like.

The term "alkoxyalkyl" refers to an alkyl group substituted with an alkoxy group and may be represented by the general formula -R^{b}OR^{a} where R^{b} is an alkylene group and R^{a} is an alkyl group.

The term "alkyl" by itself or as part of another substituent refers to a saturated or unsaturated branched, straight-chain or cyclic monovalent hydrocarbon radical that is derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl, cycloprop-1-en-1-yl; cycloprop-2-en-1-yl, prop-1-yn-1-yl, prop-2-yn-1-yl, *etc.;* butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, *etc.;* and the like. Where specific levels of saturation are intended, the nomenclature "alkanyl," "alkenyl" and/or "alkynyl" are used, as defined below. The term "lower alkyl" refers to alkyl groups with 1 to 6 carbons.

The term "alkanyl" by itself or as part of another substituent refers to a saturated branched, straight-chain or cyclic alkyl derived by the removal of one hydrogen atom from a single carbon atom of a parent alkane. Typical alkanyl groups include, but are not limited to, methyl; ethanyl; propanyls such as propan-1-yl, propan-2-yl (isopropyl), cyclopropan-1-yl, *etc.;* butanyls such as butan-1-yl, butan-2-yl (*sec*-butyl), 2-methyl-propan-1-yl (isobutyl), 2-methyl-propan-2-yl (*t*-butyl), cyclobutan-1-yl, *etc.;* and the like.

The term "alkenyl" by itself or as part of another substituent refers to an unsaturated branched, straight-chain or cyclic alkyl having at least one carbon-carbon double bond derived by the removal of one hydrogen atom from a single carbon atom of a parent alkene. Typical alkenyl groups include, but are not limited to, ethenyl; propenyls such as prop-1-en-1-yl, prop-1-en-2-yl, prop-2-en-1-yl, prop-2-en-2-yl, cycloprop-1-en-1-yl; cycloprop-2-en-1-yl ; butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, *etc.;* and the like.

The term "alkynyl" by itself or as part of another substituent refers to an unsaturated branched, straight-chain or cyclic alkyl having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, *etc.;* butynyls such as but-1-yn-1-yl, but-1-yn-3-yl, but-3-yn-1-yl, *etc.;* and the like.

The term "alkylamine" refers to a group of the formula -NHR^{a} and "dialkylamine" refers to a group of the formula -NR^{a}R^{a}, where each R^{a} is, independently of the others, an alkyl group.

The term "alkylene" refers to an alkane, alkene or alkyne group having two terminal monovalent radical centers derived by the removal of one hydrogen atom from each of the two terminal carbon atoms. Typical alkylene groups include, but are not limited to, methylene; and saturated or unsaturated ethylene; propylene; butylene; and the like. The term "lower alkylene" refers to alkylene groups with 1 to 6 carbons.

The term "aryl" means an aromatic carbocyclyl containing from 6 to 14 carbon ring atoms. An aryl may be monocyclic or polycyclic (*i.e.,* may contain more than one ring). In the case of polycyclic aromatic rings, only one ring in the polycyclic system is required to be aromatic while the remaining ring(s) may be saturated, partially saturated or unsaturated. Examples of aryls include phenyl, naphthalenyl, indenyl, indanyl, and tetrahydronaphthyl.

The prefix "halo" indicates that the substituent which includes the prefix is substituted with one or more independently selected halogen radicals. For example, haloalkyl means an alkyl substituent in which at least one hydrogen radical is replaced with a halogen radical. Typical halogen radicals include chloro, fluoro, bromo and iodo. Examples of haloalkyls include chloromethyl, 1-bromoethyl, fluoromethyl, difluoromethyl, trifluoromethyl, and 1,1,1-trifluoroethyl. It should be recognized that if a substituent is substituted by more than one halogen radical, those halogen radicals may be identical or different (unless otherwise stated).

The term "haloalkoxy" refers to a group of the formula -OR^{c}, where R^{c} is a haloalkyl.

The terms "heteroalkyl," "heteroalkanyl," "heteroalkenyl," "heteroalkynyl," and "heteroalkylene" refer to alkyl, alkanyl, alkenyl, alkynyl, and alkylene groups, respectively, in which one or more of the carbon atoms, *e.g.,* 1, 2 or 3 carbon atoms, are each independently replaced with the same or different heteratoms or heteroatomic groups. Typical heteroatoms and/or heteroatomic groups which can replace the carbon atoms include, but are not limited to, O, S, SO, NR^{c}, PH, S(O), S(O)₂, S(O)NR^{c}, S(O)₂NR^{c}, and the like, including combinations thereof, where each R^{c} is independently hydrogen or C₁-C₆ alkyl.

The terms "cycloalkyl" and "heterocyclyl" refer to cyclic versions of "alkyl" and "heteroalkyl" groups, respectively. For heterocyclyl groups, a heteroatom can occupy the position that is attached to the remainder of the molecule. A cycloalkyl or heterocyclyl ring may be a single-ring (monocyclic) or have two or more rings (bicyclic or polycyclic).

Monocyclic cycloalkyl and heterocyclyl groups will typically contain from 3 to 7 ring atoms, more typically from 3 to 6 ring atoms, and even more typically 5 to 6 ring atoms. Examples of cycloalkyl groups include, but are not limited to, cyclopropyl; cyclobutyls such as cyclobutanyl and cyclobutenyl; cyclopentyls such as cyclopentanyl and cyclopentenyl; cyclohexyls such as cyclohexanyl and cyclohexenyl; and the like. Examples of monocyclic heterocyclyls include, but are not limited to, oxetane, furanyl, dihydrofuranyl, tetrahydrofuranyl, tetrahydropyranyl, thiophenyl (thiofuranyl), dihydrothiophenyl, tetrahydrothiophenyl, pyrrolyl, pyrrolinyl, pyrrolidinyl, imidazolyl, imidazolinyl, imidazolidinyl, pyrazolyl, pyrazolinyl, pyrazolidinyl, triazolyl, tetrazolyl, oxazolyl, oxazolidinyl, isoxazolidinyl, isoxazolyl, thiazolyl, isothiazolyl, thiazolinyl, isothiazolinyl, thiazolidinyl, isothiazolidinyl, thiodiazolyl, oxadiazolyl (including 1,2,3-oxadiazolyl, 1,2,4-oxadiazolyl, 1,2,5-oxadiazolyl (furazanyl), or 1,3,4-oxadiazolyl), oxatriazolyl (including 1,2,3,4-oxatriazolyl or 1,2,3,5-oxatriazolyl), dioxazolyl (including 1,2,3-dioxazolyl, 1,2,4-dioxazolyl, 1,3,2-dioxazolyl, or 1,3,4-dioxazolyl), 1,4-dioxanyl, dioxothiomorpholinyl, oxathiazolyl, oxathiolyl, oxathiolanyl, pyranyl, dihydropyranyl, thiopyranyl, tetrahydrothiopyranyl, pyridinyl (azinyl), piperidinyl, diazinyl (including pyridazinyl (1,2-diazinyl), pyrimidinyl (1,3-diazinyl), or pyrazinyl (1,4-diazinyl)), piperazinyl, triazinyl (including 1,3,5-triazinyl, 1,2,4-triazinyl, and 1,2,3-triazinyl)), oxazinyl (including 1,2-oxazinyl, 1,3-oxazinyl, or 1,4-oxazinyl)), oxathiazinyl (including 1,2,3-oxathiazinyl, 1,2,4-oxathiazinyl, 1,2,5-oxathiazinyl, or 1,2,6-oxathiazinyl)), oxadiazinyl (including 1,2,3-oxadiazinyl, 1,2,4-oxadiazinyl, 1,4,2-oxadiazinyl, or 1,3,5-oxadiazinyl)), morpholinyl, azepinyl, oxepinyl, thiepinyl, diazepinyl, pyridonyl (including pyrid-2(1H)-onyl and pyrid-4(1H)-onyl), furan-2(5H)-onyl, pyrimidonyl (including pyramid-2(1H)-onyl and pyramid-4(3H)-onyl), oxazol-2(3H)-onyl, 1H-imidazol-2(3H)-onyl, pyridazin-3(2H)-onyl, and pyrazin-2(1H)-onyl.

Polycyclic cycloalkyl and hetrocyclyl groups contain more than one ring, and bicyclic cycloalkyl and heterocyclyl groups contain two rings. The rings may be in a bridged, fused or spiro orientation. Polycyclic cycloalkyl and heterocyclyl groups may include combinations of bridged, fused and/or spiro rings. In a spirocyclic cycloalkyl or heterocyclyl, one atom is common to two different rings. An example of a spirocycloalkyl is spiro[4.5]decane and an example of a spiroheterocyclyls is a spiropyrazoline.

In a bridged cycloalkyl or heterocyclyl, the rings share at least two common non-adjacent atoms. Examples of bridged cycloalkyls include, but are not limited to, adamantyl and norbornanyl rings. Examples of bridged heterocyclyls include, but are not limited to, 2-oxatricyclo[3.3.1.1^{3,7}]decanyl.

In a fused-ring cycloalkyl or heterocyclyl, two or more rings are fused together, such that two rings share one common bond. Examples of fused-ring cycloalkyls include decalin, naphthylene, tetralin, and anthracene. Examples of fused-ring heterocyclyls containing two or three rings include imidazopyrazinyl (including imidazo[1,2-a]pyrazinyl), imidazopyridinyl (including imidazo[1,2-a]pyridinyl), imidazopyridazinyl (including imidazo[1,2-b]pyridazinyl), thiazolopyridinyl (including thiazolo[5,4-c]pyridinyl, thiazolo[5,4-b]pyridinyl, thiazolo[4,5-b]pyridinyl, and thiazolo[4,5-c]pyridinyl), indolizinyl, pyranopyrrolyl, 4H-quinolizinyl, purinyl, naphthyridinyl, pyridopyridinyl (including pyrido[3,4-b]-pyridinyl, pyrido[3,2-b]-pyridinyl, or pyrido[4,3-b]-pyridinyl), and pteridinyl. Other examples of fused-ring heterocyclyls include benzo-fused heterocyclyls, such as dihydrochromenyl, tetrahydroisoquinolinyl, indolyl, isoindolyl (isobenzazolyl, pseudoisoindolyl), indoleninyl (pseudoindolyl), isoindazolyl (benzpyrazolyl), benzazinyl (including quinolinyl (1-benzazinyl) or isoquinolinyl (2-benzazinyl)), phthalazinyl, quinoxalinyl, quinazolinyl, benzodiazinyl (including cinnolinyl (1,2-benzodiazinyl) or quinazolinyl (1,3-benzodiazinyl)), benzopyranyl (including chromanyl or isochromanyl), benzoxazinyl (including 1,3,2-benzoxazinyl, 1,4,2-benzoxazinyl, 2,3,1-benzoxazinyl, or 3,1,4-benzoxazinyl), benzo[d]thiazolyl, and benzisoxazinyl (including 1,2-benzisoxazinyl or 1,4-benzisoxazinyl).

The term "heteroaryl" refers to an aromatic heterocyclyl containing from 5 to 14 ring atoms. A heteroaryl may be a single ring or 2 or 3 fused rings. Examples of heteroaryls include 6-membered rings such as pyridyl, pyrazyl, pyrimidinyl, pyridazinyl, and 1,3,5-, 1,2,4- or 1,2,3-triazinyl; 5-membered ring substituents such as triazolyl, pyrrolyl, imidazyl, furanyl, thiophenyl, pyrazolyl, oxazolyl, isoxazolyl, thiazolyl, 1,2,3-, 1,2,4-, 1,2,5-, or 1,3,4-oxadiazolyl and isothiazolyl; 6/5-membered fused ring substituents such as imidazopyrazinyl (including imidazo[1,2-a]pyrazinyl)imidazopyridinyl (including imidazo[1,2-a]pyridinyl), imidazopyridazinyl (including imidazo[1,2-b]pyridazinyl), thiazolopyridinyl (including thiazolo[5,4-c]pyridinyl, thiazolo[5,4-b]pyridinyl, thiazolo[4,5-b]pyridinyl, and thiazolo[4,5-c]pyridinyl), benzo[d]thiazolyl, benzothiofuranyl, benzisoxazolyl, benzoxazolyl, purinyl, and anthranilyl; and 6/6-membered fused rings such as benzopyranyl, quinolinyl, isoquinolinyl, cinnolinyl, quinazolinyl, and benzoxazinyl. Heteroaryls may also be heterocycles having aromatic (4N+2 pi electron) resonance contributors such as pyridonyl (including pyrid-2(1H)-onyl and pyrid-4(1H)-onyl), pyrimidonyl (including pyramid-2(1H)-onyl and pyramid-4(3H)-onyl), pyridazin-3(2H)-onyl and pyrazin-2(1H)-onyl.

The term "sulfonate" as used herein means a salt or ester of a sulfonic acid.

The term "methyl sulfonate" as used herein means a methyl ester of a sulfonic acid group.

The term "carboxylate" as used herein means a salt or ester of a caboxylic acid.

The term "sugar" as used herein in the context of linkers means an O-glycoside or *N-*glycoside carbohydrate derivatives of the monosaccharide class and may originate from naturally-occurring sources or may be synthetic in origin. For example "sugar" includes derivatives such as but not limited to those derived from beta-glucuronic acid and beta-galactose. Suitable sugar substitutions include but are not limited to hydroxyl, amine, carboxylic acid, esters, and ethers.

The term "NHS ester" means the N-hydroxysuccinimide ester derivative of a carboxylic acid.

The term salt when used in context of "or salt thereof" includes salts commonly used to form alkali metal salts and to form addition salts of free acids or free bases. In general, these salts typically may be prepared by conventional means by reacting, for example, the appropriate acid or base with a compound of the invention.

Where a salt is intended to be administered to a patient (as opposed to, for example, being in use in an *in vitro* context), the salt preferably is pharmaceutically acceptable and/or physiologically compatible. The term "pharmaceutically acceptable" is used adjectivally in this patent application to mean that the modified noun is appropriate for use as a pharmaceutical product or as a part of a pharmaceutical product. The term "pharmaceutically acceptable salt" includes salts commonly used to form alkali metal salts and to form addition salts of free acids or free bases. In general, these salts typically may be prepared by conventional means by reacting, for example, the appropriate acid or base with a compound of the invention.

### 5.2 EXEMPLARY EMBODIMENTS

As noted in the Summary, one aspect of the instant disclosure concerns ADCs comprising Bcl-xL inhibitors linked to antibodies by way of linkers. In specific embodiments, the ADCs are compounds according to structural formula (I), below, or salts thereof, wherein Ab represents the antibody, D represents a Bcl-xL inhibitor (drug), L represents a linker, LK represents a linkage formed between a reactive functional group on linker L and a complementary functional group on antibody Ab and m represents the number of D-L-LK units linked to the antibody:

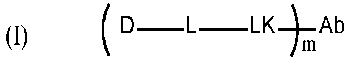

Specific embodiments of the various Bcl-xL inhibitors (D), linkers (L) and antibodies (Ab) that can comprise the ADCs described herein, as well as the number of Bcl-xL inhibitors linked to the ADCs, are described in more detail below

### 5.2.1 BCL-XL INHIBITORS

The ADCs comprise one or more Bcl-xL inhibitors, which may be the same or different, but are typically the same. In some embodiments, the Bcl-xL inhibitors comprising the ADCs, and in certain specific embodiments D of structural formula (I), above, are compounds according to structural formula (IIa): 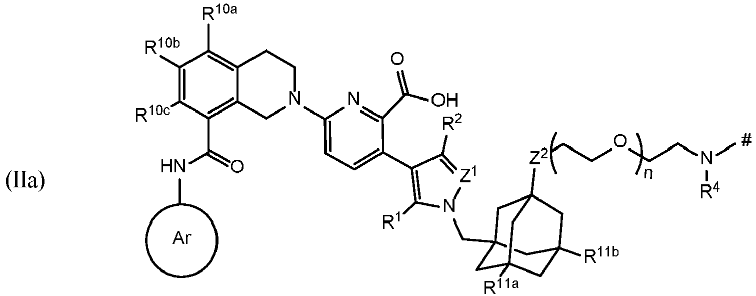 or salts thereof, wherein:
Ar is selected from 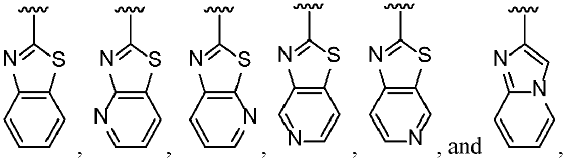 which is optionally substituted with one or more substituents independently selected from halo, cyano, methyl, and halomethyl;
Z¹ is selected from N, CH and C-CN;
Z² is selected from NH, CH₂, O, S, S(O), and S(O₂);
R¹ is selected from methyl, chloro, and cyano;
R² is selected from hydrogen, methyl, chloro, and cyano;
R⁴ is hydrogen, C₁₋₄ alkanyl, C₂₋₄ alkenyl, C₂₋₄ alkynyl, C₁₋₄ haloalkyl or C₁₋₄ hydroxyalkyl, wherein the R⁴ C₁₋₄ alkanyl, C₂₋₄ alkenyl, C₂₋₄ alkynyl, C₁₋₄ haloalkyl and C₁₋₄ hydroxyalkyl are optionally substituted with one or more substituents independently selected from OCH₃, OCH₂CH₂OCH₃, and OCH₂CH₂NHCH₃;
R^{10a}, R^{10b}, and R^{10c} are each, independently of one another, selected from hydrogen, halo, C₁₋₆ alkanyl, C₂₋₆ alkenyl, C₂₋₆ alkynyl, and C₁₋₆ haloalkyl;
R^{11a} and R^{11b} are each, independently of one another, selected from hydrogen, methyl, ethyl, halomethyl, hydroxyl, methoxy, halo, CN and SCH₃;
n is 0, 1, 2 or 3; and
# represents the point of attachment to linker L.

In certain embodiments, Ar of formula (IIa) is selected from 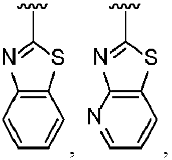 and 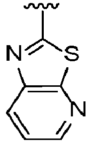 and is optionally substituted with one or more substituents independently selected from halo, cyano, methyl, and halomethyl. In particular embodiments, Ar is

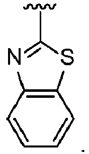

In certain embodiments, Z¹ of formula (IIa) is N.

In certain embodiments, Z¹ of formula (IIa) is CH.

In certain embodiments, Z² of formula (IIa) is O.

In certain embodiments, R¹ of formula (IIa) is selected from methyl and chloro.

In certain embodiments, R² of formula (IIa) is selected from hydrogen and methyl. In particular embodiments, R² is hydrogen.

In certain embodiments, R¹ in formula (IIa) is methyl, R² is hydrogen and Z¹ is N.

In certain embodiments, R^{10a} in formula (IIa) is halo and R^{10b} and R^{10c} are each hydrogen. In particular embodiments, R^{10a} is fluoro.

In certain embodiments, R^{10b} in formula (IIa) is halo and R^{10a} and R^{10c} are each hydrogen. In particular embodiments, R^{10b} is fluoro.

In certain embodiments, R^{10c} in formula (IIa) is halo and R^{10a} and R^{10b} are each hydrogen. In particular embodiments, R^{10c} is fluoro.

In certain embodiments, R^{10a}, R^{10b} and R^{10c} in formula (IIa) are each hydrogen.

In certain embodiments, R^{11a} and R^{11b} in formula (IIa) are the same. In particular embodiments, R^{11a} and R^{11b} are each methyl.

In certain embodiments, n of formula (IIa) is 0 or 1.

Exemplary Bcl-xL inhibitors and/or salts thereof that may be included in the ADCs described herein include compounds W1.01-W1.08, described in Examples 1.1-1.8, respectively.

The Bcl-xL inhibitors comprising the ADCs, when not included in an ADC, bind to and inhibit anti-apoptotic Bcl-xL proteins, inducing apoptosis. The ability of a specific Bcl-xL inhibitor according to structural formula (IIa) to bind and inhibit Bcl-xL activity when not included in an ADC (*i.e.,* a compound or salt according to structural formula (IIa) in which # represents a hydrogen atom), may be confirmed in standard binding and activity assays, including, for example, the TR-FRET Bcl-xL binding assays described in Tao et al., 2014, ACS Med. Chem. Lett., 5:1088-1093. A specific TR-FRET Bcl-xL binding assay that can be used to confirm Bcl-xL binding is provided in Example 4, below. Typically, Bcl-xL inhibitors useful in the ADCs described herein will exhibit a Kᵢ in the binding assay of Example 4 of less than about 10 nM, but may exhibit a significantly lower Kᵢ, for example a Kᵢ of less than about 1, 0.1, or even 0.01 nM.

Bcl-xL inhibitory activity may also be confirmed in standard cell-based cytotoxicity assays, such as the FL5.12 cellular and Molt-4 cytotoxicity assays described in Tao et al., 2014, ACS Med. Chem. Lett., 5:1088-1093. A specific Molt-4 cellular cytoxicity assay that may be used to confirm Bcl-xL inhibitory activity of specific Bcl-xL inhibitors is provided in Example 5, below. Typically, Bcl-xL inhibitors useful in the ADCs described herein will exhibit an EC₅₀ of less than about 500 nM in the Molt-4 cytotoxicity assay of Example 5, but may exhibit a significantly lower EC₅₀, for example an EC₅₀ of less than about 250, 100, 50, 20, 10 or even 5 nM.

Although the Bcl-xL inhibitors defined by structural formula (IIa) are expected to be cell permeable and penetrate cells when not included in an ADC, the Bcl-xL inhibitory activity of compounds that do not freely traverse cell membranes may be confirmed in cellular assays with permeabilized cells. As discussed in the Background section, the process of mitochondrial outer-membrane permeabilization (MOMP) is controlled by the Bcl-2 family proteins. Specifically, MOMP is promoted by the pro-apoptotic Bcl-2 family proteins Bax and Bak which, upon activation oligomerize on the outer mitochondrial membrane and form pores, leading to release of cytochrome c (cyt c). The release of cyt c triggers formulation of the apoptosome which, in turn, results in caspase activation and other events that commit the cell to undergo programmed cell death (*see,* Goldstein et al., 2005, Cell Death and Differentiation 12:453-462). The oligomerization action of Bax and Bak is antagonized by the anti-apoptotic Bcl-2 family members, including Bcl-2 and Bcl-xL. Bcl-xL inhibitors, in cells that depend upon Bcl-xL for survival, can cause activation of Bax and/or Bak, MOMP, release of cyt c and downstream events leading to apoptosis. The process of cyt c release can be assessed via western blot of both mitochondrial and cytosolic fractions of cytochrome c in cells and used as a proxy measurement of apoptosis in cells.

As a means of detecting Bcl-xL inhibitory activity and consequent release of cyt c for molecules with low cell permeability, the cells can be treated with an agent that causes selective pore formation in the plasma, but not mitochondrial, membrane. Specifically, the cholesterol/phospholipid ratio is much higher in the plasma membrane than the mitochondrial membrane. As a result, short incubation with low concentrations of the cholesterol-directed detergent digitonin selectively permeabilizes the plasma membrane without significantly affecting the mitochondrial membrane. This agent forms insoluble complexes with cholesterol leading to the segregation of cholesterol from its normal phospholipid binding sites. This action, in turn, leads to the formation of holes about 40-50 Å wide in the lipid bilayer. Once the plasma membrane is permeabilized, cytosolic components able to pass over digitonin-formed holes can be washed out, including the cytochrome C that was released from mitochondria to cytosol in the apoptotic cells (Campos, 2006, Cytometry A 69(6):515-523).

Typically, Bcl-xL inhibitors will yield an EC₅₀ of less than about 10 nM in the Molt-4 cell permeabilized cyt c assay of Example 5, although the compounds may exhibit significantly lower EC₅₀s, for example, less than about 5, 1, or even 0.5 nM.

Although many of the Bcl-xL inhibitors of structural formula (IIa) selectively or specifically inhibit Bcl-xL over other anti-apoptotic Bcl-2 family proteins, selective and/or specific inhibition of Bcl-xL is not necessary. The Bcl-xL inhibitors comprising the ADCs may also, in addition to inhibiting Bcl-xL, inhibit one or more other anti-apoptotic Bcl-2 family proteins, such as, for example, Bcl-2. In some embodiments, the Bcl-xL inhibitors comprising the ADC are selective and/or specific for Bcl-xL. By specific or selective is meant that the particular Bcl-xL inhibitor binds or inhibits Bcl-xL to a greater extent than Bcl-2 under equivalent assay conditions. In specific embodiments, the Bcl-xL inhibitors comprising the ADCs exhibit in the range of 10-fold, 100-fold, or even greater specificity for Bcl-xL than Bcl-2 in a Bcl-xL binding assay.

### 5.2.2 LINKERS

In the ADCs described herein, the Bcl-xL inhibitors are linked to the antibody by way of linkers. The linker linking a Bcl-xL inhibitor to the antibody of an ADC may be short, long, hydrophobic, hydrophilic, flexible or rigid, or may be composed of segments that each independently have one or more of the above-mentioned properties such that the linker may include segments having different properties. The linkers may be polyvalent such that they covalently link more than one Bcl-xL inhibitor to a single site on the antibody, or monovalent such that covalently they link a single Bcl-xL inhibitor to a single site on the antibody.

As will be appreciated by skilled artisans, the linkers link the Bcl-xL inhibitors to the antibody by forming a covalent linkage to the Bcl-xL inhibitor at one location and a covalent linkage to antibody at another. The covalent linkages are formed by reaction between functional groups on the linker and functinal groups on the inhibitors and antibody. As used herein, the expression "linker" is intended to include (i) unconjugated forms of the linker that include a functional group capable of covalently linking the linker to a Bcl-xL inhibitor and a functional group capable of covalently linking the linker to an antibody; (ii) partially conjugated forms of the linker that include a functional group capable of covalently linking the linker to an antibody and that is covalently linked to a Bcl-xL inhibitor, or *vice versa;* and (iii) fully conjugated forms of the linker that are covalently linked to both a Bcl-xL inhibitor and an antibody. In some specific embodiments of intermediate synthons and ADCs described herein, moieties comprising the functional groups on the linker and covalent linkages formed between the linker and antibody are specifically illustrated as R*^{x}* and LK, respectively. One embodiment pertains to an ADC formed by contacting an antibody that binds a cell surface receptor or tumor associated antigen expressed on a tumor cell with a synthon described herein under conditions in which the synthon covalently links to the antibody. One embodiment pertains to a method of making an ADC formed by contacting a synthon described herein under conditions in which the synthon covalently links to the antibody. One embodiment pertains to a method of inhibiting Bcl-xL activity in a cell that expresses Bcl-xL, comprising contacting the cell with an ADC described herein that is capable of binding the cell, under conditions in which the ADC binds the cell.

The linkers are preferably, but need not be, chemically stable to conditions outside the cell, and may be designed to cleave, immolate and/or otherwise specifically degrade inside the cell. Alternatively, linkers that are not designed to specifically cleave or degrade inside the cell may be used. A wide variety of linkers useful for linking drugs to antibodies in the context of ADCs are known in the art. Any of these linkers, as well as other linkers, may be used to link the Bcl-xL inhibitors to the antibody of the ADCs described herein. Exemplary polyvalent linkers that may be used to link many Bcl-xL inhibitors to an antibody are described, for example, in U.S. Patent No 8,399,512; U.S. Published Application No. 2010/0152725; U.S. Patent No. 8,524,214; U.S. Patent No. 8,349,308; U.S. Published Application No. 2013/189218; U.S. Published Application No. 2014/017265; WO 2014/093379; WO 2014/093394; WO 2014/093640, the contents of which are incorporated herein by reference in their entireties. For example, the Fleximer® linker technology developed by Mersana *et al.* has the potential to enable high-DAR ADCs with good physicochemical properties. As shown below, the Fleximer® linker technology is based on incorporating drug molecules into a solubilizing poly-acetal backbone via a sequence of ester bonds. The methodology renders highly-loaded ADCs (DAR up to 20) whilst maintaining good physicochemical properties. This methodology could be utilized with Bcl-xL inhibitors as shown in the Scheme below.

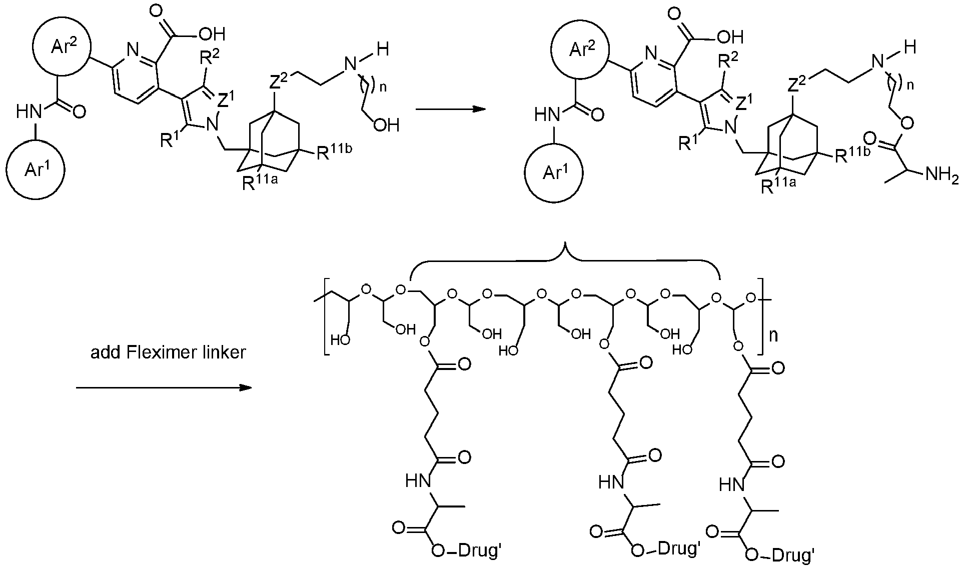

To utilize the Fleximer® linker technology depicted in the scheme above, an aliphatic alcohol must be present or introduced into the Bcl-xL inhibitor. The alcohol moiety is then conjugated to an alanine moiety, which is then synthetically incorporated into the Fleximer® linker. Liposomal processing of the ADC *in vitro* releases the parent alcohol-containing drug.

Additional examples of dendritic type linkers can be found in US 2006/116422; US 2005/271615; de Groot et al., (2003) Angew. Chem. Int. Ed. 42:4490-4494; Amir et al., (2003) Angew. Chem. Int. Ed. 42:4494-4499; Shamis et al., (2004) J. Am. Chem. Soc. 126:1726-1731 ; Sun et al., (2002) Bioorganic & Medicinal Chemistry Letters 12:2213-2215; Sun et al., (2003) Bioorganic & Medicinal Chemistry 11:1761-1768; and King et al., (2002) Tetrahedron Letters 43:1987-1990.

Exemplary monovalent linkers that may be used are described, for example, in Nolting, 2013, Antibody-Drug Conjugates, Methods in Molecular Biology 1045:71-100; Kitson et al., 2013, CROs/CMOs - Chemica Oggi - Chemistry Today 31(4): 30-36; Ducry et al., 2010, Bioconjugate Chem. 21:5-13; Zhao et al., 2011, J. Med. Chem. 54:3606-3623; U.S. Patent No. 7,223,837; U.S. Patent No. 8,568,728; U.S. Patent No. 8,535,678; and WO2004010957, the content of each of which is incorporated herein by reference in their entireties.

By way of example and not limitation, some cleavable and noncleavable linkers that may be included in the ADCs described herein are described below.

### 5.2.2.1 CLEAVABLE LINKERS

In certain embodiments, the linker selected is cleavable *in vitro* and *in vivo.* Cleavable linkers may include chemically or enzymatically unstable or degradable linkages. Cleavable linkers generally rely on processes inside the cell to liberate the drug, such as reduction in the cytoplasm, exposure to acidic conditions in the lysosome, or cleavage by specific proteases or other enzymes within the cell. Cleavable linkers generally incorporate one or more chemical bonds that are either chemically or enzymatically cleavable while the remainder of the linker is noncleavable.

In certain embodiments, a linker comprises a chemically labile group such as hydrazone and/or disulfide groups. Linkers comprising chemically labile groups exploit differential properties between the plasma and some cytoplasmic compartments. The intracellular conditions to facilitate drug release for hydrazone containing linkers are the acidic environment of endosomes and lysosomes, while the disulfide containing linkers are reduced in the cytosol, which contains high thiol concentrations, e.g., glutathione. In certain embodiments, the plasma stability of a linker comprising a chemically labile group may be increased by introducing steric hindrance using substituents near the chemically labile group.

Acid-labile groups, such as hydrazone, remain intact during systemic circulation in the blood's neutral pH environment (pH 7.3-7.5) and undergo hydrolysis and release the drug once the ADC is internalized into mildly acidic endosomal (pH 5.0-6.5) and lysosomal (pH 4.5-5.0) compartments of the cell. This pH dependent release mechanism has been associated with nonspecific release of the drug. To increase the stability of the hydrazone group of the linker, the linker may be varied by chemical modification, e.g., substitution, allowing tuning to achieve more efficient release in the lysosome with a minimized loss in circulation.

Hydrazone-containing linkers may contain additional cleavage sites, such as additional acid-labile cleavage sites and/or enzymatically labile cleavage sites. ADCs including exemplary hydrazone-containing linkers include the following structures: 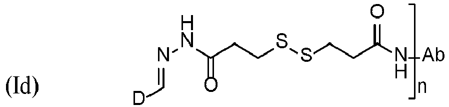 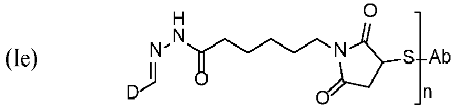 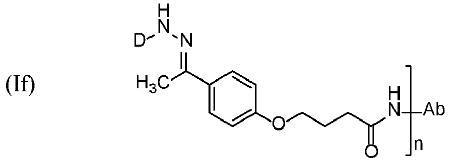 wherein D and Ab represent the drug and Ab, respectively, and n represents the number of drug-linkers linked to the antibody. In certain linkers such as linker (Id), the linker comprises two cleavable groups - a disulfide and a hydrazone moiety. For such linkers, effective release of the unmodified free drug requires acidic pH or disulfide reduction and acidic pH. Linkers such as (Ie) and (If) have been shown to be effective with a single hydrazone cleavage site.

Other acid-labile groups that may be included in linkers include *cis*-aconityl-containing linkers. *cis*-Aconityl chemistry uses a carboxylic acid juxtaposed to an amide bond to accelerate amide hydrolysis under acidic conditions.

Cleavable linkers may also include a disulfide group. Disulfides are thermodynamically stable at physiological pH and are designed to release the drug upon internalization inside cells, wherein the cytosol provides a significantly more reducing environment compared to the extracellular environment. Scission of disulfide bonds generally requires the presence of a cytoplasmic thiol cofactor, such as (reduced) glutathione (GSH), such that disulfide-containing linkers are reasonable stable in circulation, selectively releasing the drug in the cytosol. The intracellular enzyme protein disulfide isomerase, or similar enzymes capable of cleaving disulfide bonds, may also contribute to the preferential cleavage of disulfide bonds inside cells. GSH is reported to be present in cells in the concentration range of 0.5-10 mM compared with a significantly lower concentration of GSH or cysteine, the most abundant low-molecular weight thiol, in circulation at approximately 5 µM. Tumor cells, where irregular blood flow leads to a hypoxic state, result in enhanced activity of reductive enzymes and therefore even higher glutathione concentrations. In certain embodiments, the *in vivo* stability of a disulfide-containing linker may be enhanced by chemical modification of the linker, *e.g.,* use of steric hindrance adjacent to the disulfide bond.

ADCs including exemplary disulfide-containing linkers include the following structures: 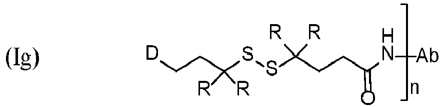 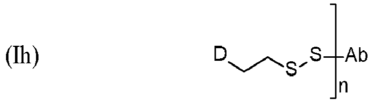 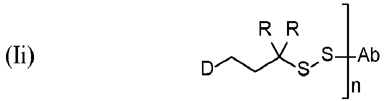 wherein D and Ab represent the drug and antibody, respectively, n represents the number of drug-linkers linked to the antibody and R is independently selected at each occurrence from hydrogen or alkyl, for example. In certain embodiments, increasing steric hindrance adjacent to the disulfide bond increases the stability of the linker. Structures such as (Ig) and (Ii) show increased *in vivo* stability when one or more R groups are selected from a lower alkyl such as methyl.

Another type of linker that may be used is a linker that is specifically cleaved by an enzyme. In one embodiment, the linker is cleavable by a lysosomal enzyme. Such linkers are typically peptide-based or include peptidic regions that act as substrates for enzymes. Peptide based linkers tend to be more stable in plasma and extracellular milleu than chemically labile linkers. Peptide bonds generally have good serum stability, as lysosomal proteolytic enzymes have very low activity in blood due to endogenous inhibitors and the unfavorably high pH value of blood compared to lysosomes. Release of a drug from an antibody occurs specifically due to the action of lysosomal proteases, *e.g.,* cathepsin and plasmin. These proteases may be present at elevated levels in certain tumor tissues. In one embodiment, the linker is cleavable by the lysosomal enzyme is Cathepsin B. In certain embodiments, the linker is cleavable by a lysosomal enzyme, and the lysosomal enzyme is β-glucuronidase or β-galactosidase. In certain embodiments, the linker is cleavable by a lysosomal enzyme, and the lysosomal enzyme is β-glucuronidase. In certain embodiments, the linker is cleavable by a lysosomal enzyme, and the lysosomal enzyme is β-galactosidase.

In exemplary embodiments, the cleavable peptide is selected from tetrapeptides such as Gly-Phe-Leu-Gly, Ala-Leu-Ala-Leu or dipeptides such as Val-Cit, Val-Ala, and Phe-Lys. In certain embodiments, dipeptides are preferred over longer polypeptides due to hydrophobicity of the longer peptides.

A variety of dipeptide-based cleavable linkers useful for linking drugs such as doxorubicin, mitomycin, campotothecin, tallysomycin and auristatin/auristatin family members to antibodies have been described *(see,* Dubowchik et al., 1998, J. Org. Chem. 67:1866-1872; Dubowchik et al., 1998, Bioorg. Med. Chem. Lett. 8:3341-3346; Walker et al., 2002, Bioorg. Med. Chem. Lett. 12:217-219; Walker et al., 2004, Bioorg. Med. Chem. Lett. 14:4323-4327; and Francisco et al., 2003, Blood 102:1458-1465, the contents of each of which are incorporated herein by reference). All of these dipeptide linkers, or modified versions of these dipeptide linkers, may be used in the ADCs described herein. Other dipeptide linkers that may be used include those found in ADCs such as Seattle Genetics' Brentuximab Vendotin SGN-35 (Adcetris™), Seattle Genetics SGN-75 (anti-CD-70, MC-monomethyl auristatin F(MMAF), Celldex Therapeutics glembatumumab (CDX-011) (anti-NMB, Val-Cit- monomethyl auristatin E(MMAE), and Cytogen PSMA-ADC (PSMA-ADC-1301) (anti-PSMA, Val-Cit-MMAE).

Enzymatically cleavable linkers may include a self-immolative spacer to spatially separate the drug from the site of enzymatic cleavage. The direct attachment of a drug to a peptide linker can result in proteolytic release of an amino acid adduct of the drug, thereby impairing its activity. The use of a self-immolative spacer allows for the elimination of the fully active, chemically unmodified drug upon amide bond hydrolysis.

One self-immolative spacer is the bifunctional *para*-aminobenzyl alcohol group, which is linked to the peptide through the amino group, forming an amide bond, while amine containing drugs may be attached through carbamate functionalities to the benzylic hydroxyl group of the linker (to give a *p*-amidobenzylcarbamate, PABC). The resulting prodrugs are activated upon protease-mediated cleavage, leading to a 1,6-elimination reaction releasing the unmodified drug, carbon dioxide, and remnants of the linker group. The following scheme depicts the fragmentation of *p-*amidobenzyl carbamate and release of the drug: 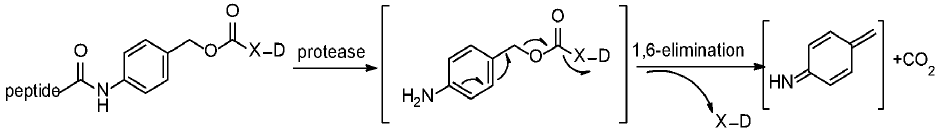 wherein X-D represents the unmodified drug.

Heterocyclic variants of this self-immolative group have also been described. *See* U.S. Patent No. 7,989,434.

In certain embodiments, the enzymatically cleavable linker is a β-glucuronic acid-based linker. Facile release of the drug may be realized through cleavage of the β-glucuronide glycosidic bond by the lysosomal enzyme β-glucuronidase. This enzyme is present abundantly within lysosomes and is overexpressed in some tumor types, while the enzyme activity outside cells is low. β-Glucuronic acid-based linkers may be used to circumvent the tendency of an ADC to undergo aggregation due to the hydrophilic nature of β-glucuronides. In certain embodiments, β-glucuronic acid-based linkers are preferred as linkers for ADCs linked to hydrophobic drugs. The following scheme depicts the release of the drug from an ADC containing a β-glucuronic acid-based linker:

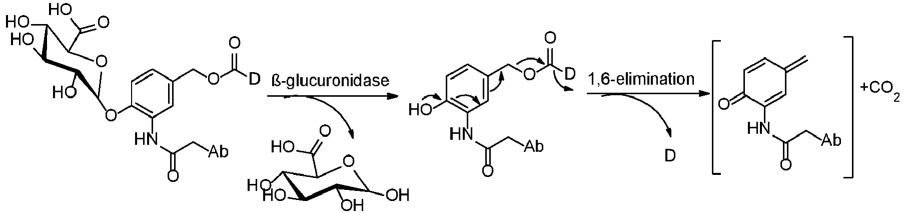

A variety of cleavable β-glucuronic acid-based linkers useful for linking drugs such as auristatins, camptothecin and doxorubicin analogues, CBI minor-groove binders, and psymberin to antibodies have been described *(see,* Jeffrey et al., 2006, Bioconjug. Chem. 17:831-840; Jeffrey et al., 2007, Bioorg. Med. Chem. Lett. 17:2278-2280; and Jiang et al., 2005, J. Am. Chem. Soc. 127:11254-11255, the contents of each of which are incorporated herein by reference). All of these β-glucuronic acid-based linkers may be used in the ADCs described herein. In certain embodiments, the enzymatically cleavable linker is a β-galactoside-based linker. β-galactoside is present abundantly within lysosomes, while the enzyme activity outside cells is low.

Additionally, Bcl-xL inhibitors containing a phenol group can be covalently bonded to a linker through the phenolic oxygen. One such linker, described in U.S. Published App. No. 2009/0318668, relies on a methodology in which a diamino-ethane "SpaceLink" is used in conjunction with traditional "PABO"-based self-immolative groups to deliver phenols. The cleavage of the linker is depicted schematically below using a Bcl-xL inhibitor of the disclosure.

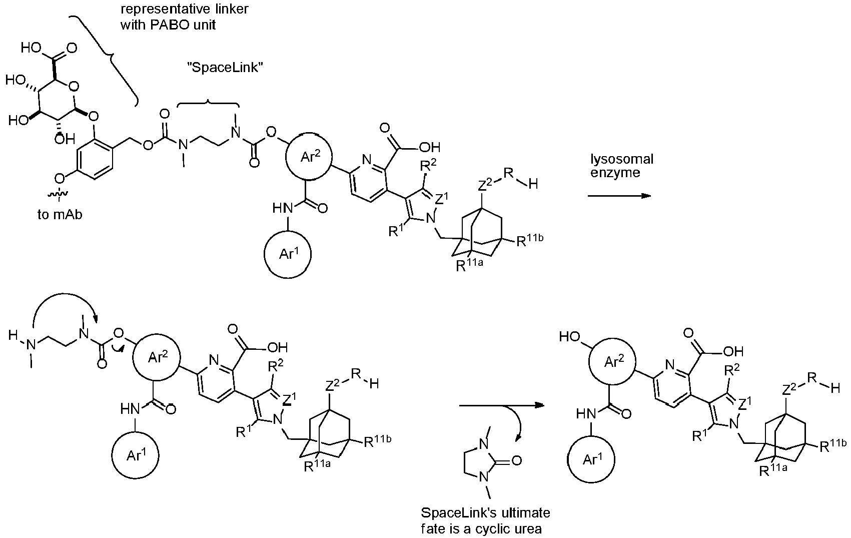

Cleavable linkers may include noncleavable portions or segments, and/or cleavable segments or portions may be included in an otherwise non-cleavable linker to render it cleavable. By way of example only, polyethylene glycol (PEG) and related polymers may include cleavable groups in the polymer backbone. For example, a polyethylene glycol or polymer linker may include one or more cleavable groups such as a disulfide, a hydrazone or a dipeptide.

Other degradable linkages that may be included in linkers include ester linkages formed by the reaction of PEG carboxylic acids or activated PEG carboxylic acids with alcohol groups on a biologically active agent, wherein such ester groups generally hydrolyze under physiological conditions to release the biologically active agent. Hydrolytically degradable linkages include, but are not limited to, carbonate linkages; imine linkages resulting from reaction of an amine and an aldehyde; phosphate ester linkages formed by reacting an alcohol with a phosphate group; acetal linkages that are the reaction product of an aldehyde and an alcohol; orthoester linkages that are the reaction product of a formate and an alcohol; and oligonucleotide linkages formed by a phosphoramidite group, including but not limited to, at the end of a polymer, and a 5' hydroxyl group of an oligonucleotide.

In certain embodiments, the linker comprises an enzymatically cleavable peptide moiety, for example, a linker comprising structural formula (IVa), (IVb) or (IVc): 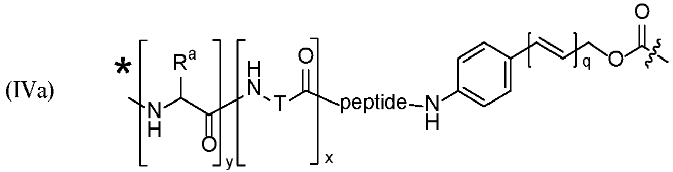 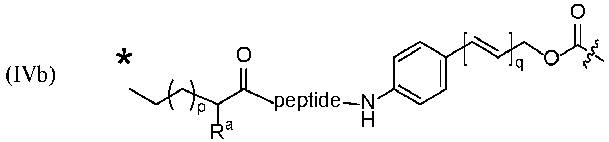 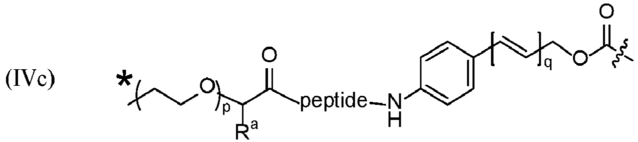 or a salt thereof, wherein:
peptide represents a peptide (illustrated N→C, wherein peptide includes the amino and carboxy "termini") a cleavable by a lysosomal enzyme;
T represents a polymer comprising one or more ethylene glycol units or an alkylene chain, or combinations thereof;
R^{a} is selected from hydrogen, alkyl, sulfonate and methyl sulfonate;
p is an integer ranging from 0 to 5;
q is 0 or 1;
x is 0 or 1;
y is 0 or 1;
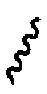 represents the point of attachment of the linker to the Bcl-xL inhibitor; and
* represents the point of attachment to the remainder of the linker.

In certain embodiments, the linker comprises an enzymatically cleavable peptide moiety, for example, a linker comprising structural formula (IVa), (IVb), or (IVc), or salts thereof.

In certain embodiments, the peptide is selected from a tripeptide or a dipeptide. In particular embodiments, the dipeptide is selected from: Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; Cit-Asp; Ala-Val; Val-Ala; Phe-Lys; Lys-Phe; Val-Lys; Lys-Val; Ala-Lys; Lys-Ala; Phe-Cit; Cit-Phe; Leu-Cit; Cit-Leu; Ile-Cit; Cit-Ile; Phe-Arg; Arg-Phe; Cit-Trp; and Trp-Cit, or salts thereof.

Specific exemplary embodiments of linkers according to structural formula (IVa) that may be included in the ADCs described herein include the linkers illustrated below (as illustrated, the linkers include a group suitable for covalently linking the linker to an antibody):

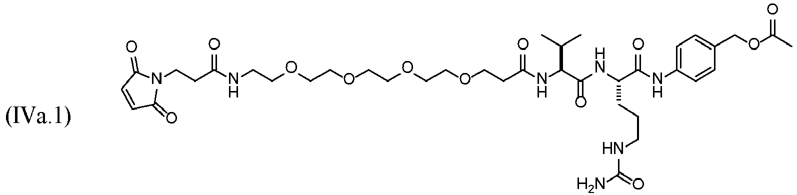

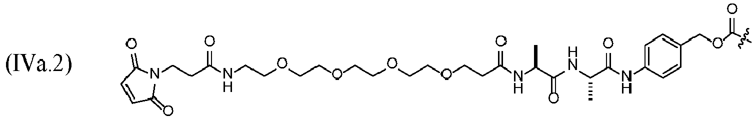

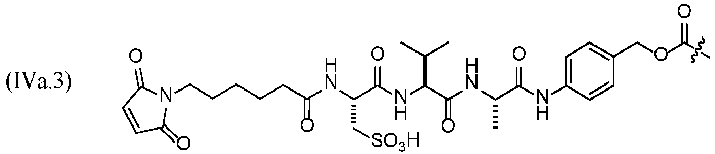

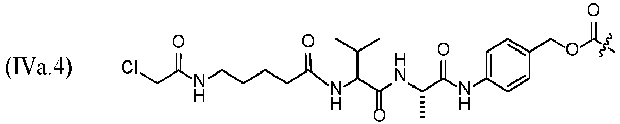

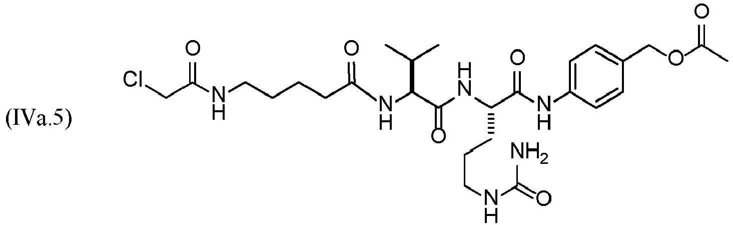

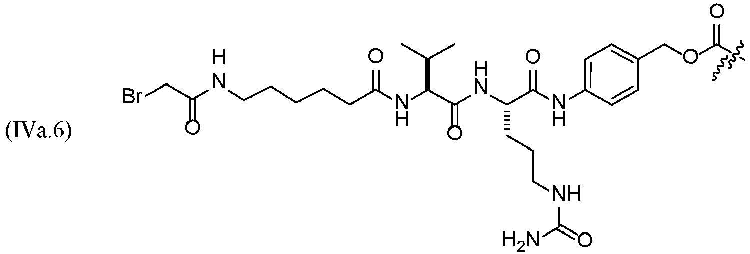

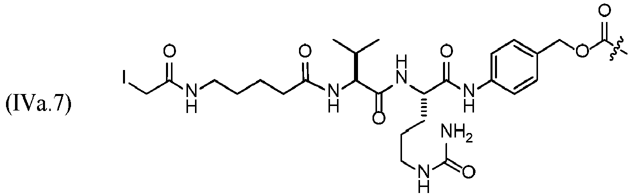

Specific exemplary embodiments of linkers according to structural formula (IVb) that may be included in the ADCs described herein include the linkers illustrated below (as illustrated, the linkers include a group suitable for covalently linking the linker to an antibody):

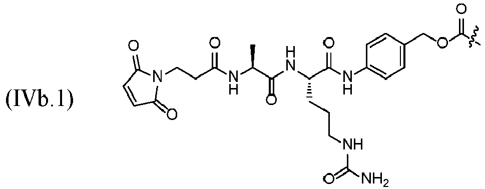

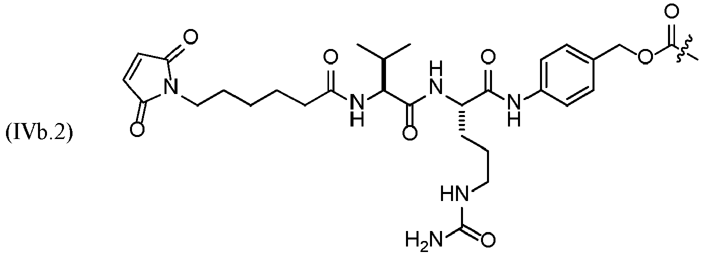

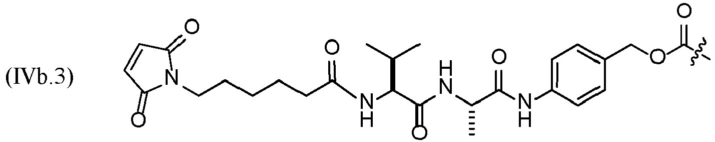

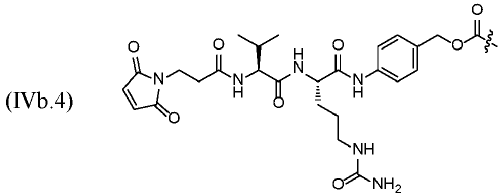

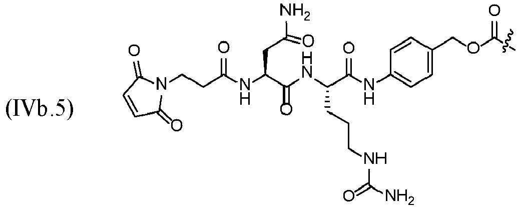

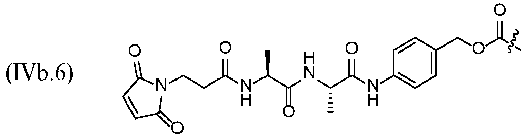

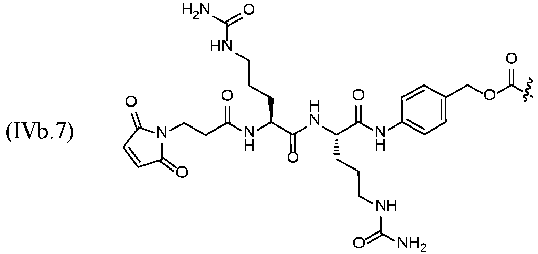

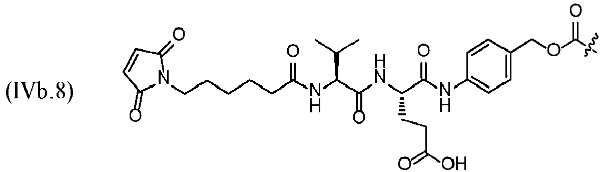

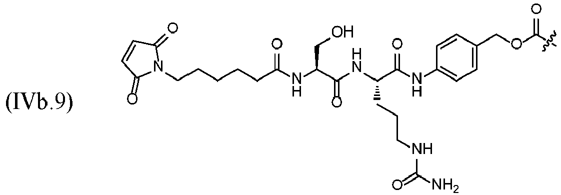

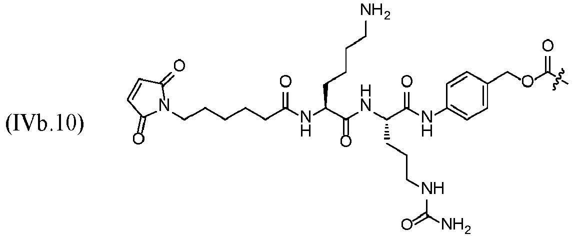

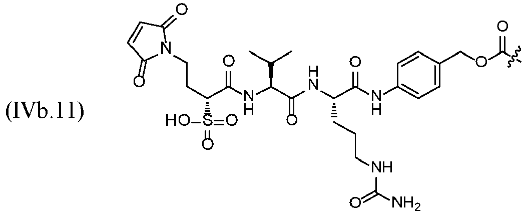

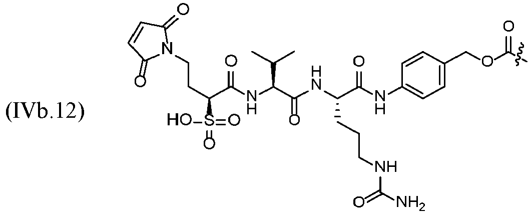

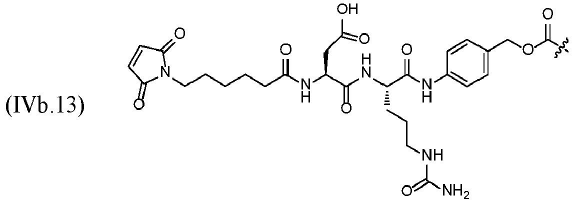

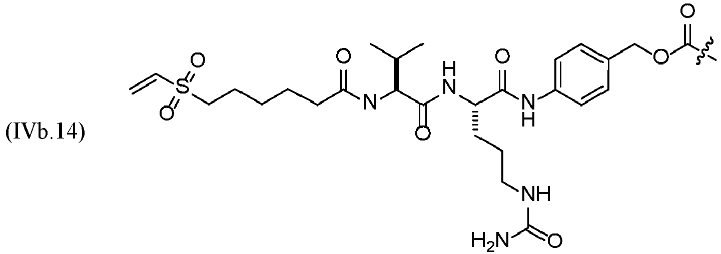

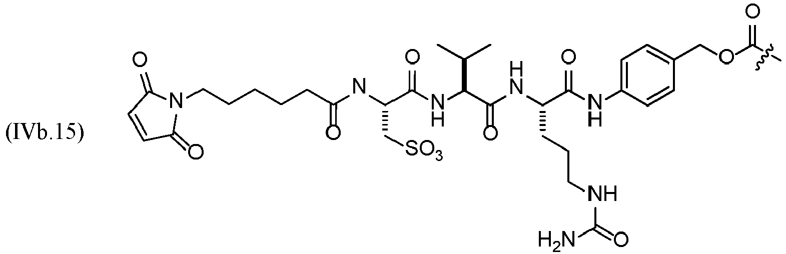

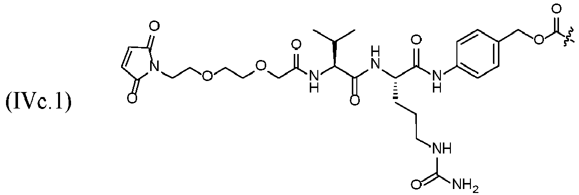

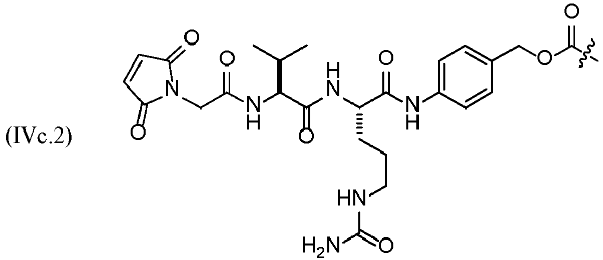

In certain embodiments, the linker comprises an enzymatically cleavable sugar moiety, for example, a linker comprising structural formula (Va), (Vb), (Vc), or (Vd): 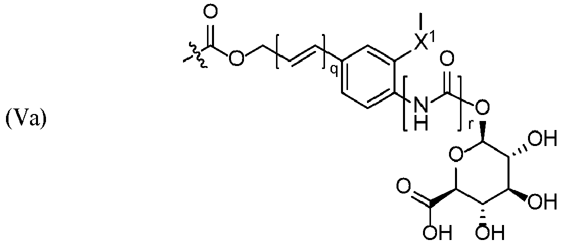 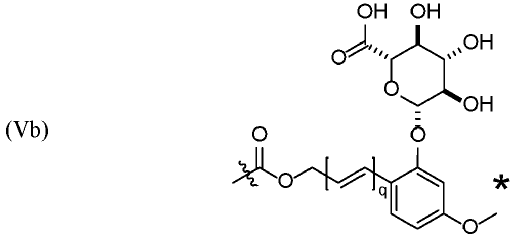 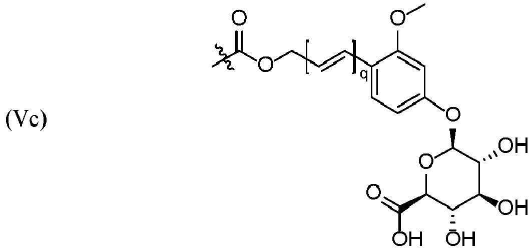 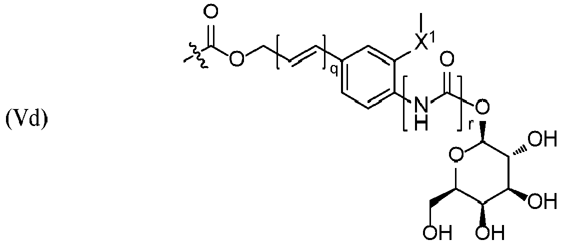 or a salt thereof, wherein:
q is 0 or 1;
r is 0 or 1;
X¹ is O or NH;
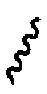 represents the point of attachment of the linker to the drug; and
* represents the point of attachment to the remainder of the linker.

Specific exemplary embodiments of linkers according to structural formula (Va) that may be included in the ADCs described herein include the linkers illustrated below (as illustrated, the linkers include a group suitable for covalently linking the linker to an antibody):

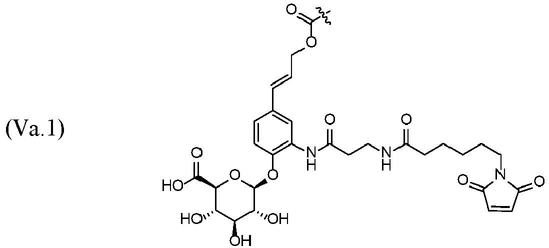

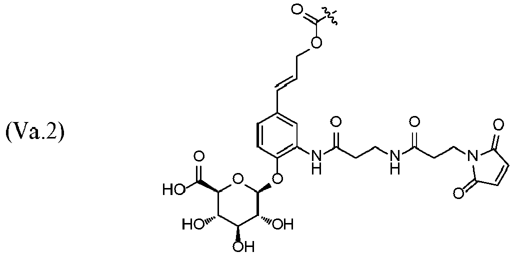

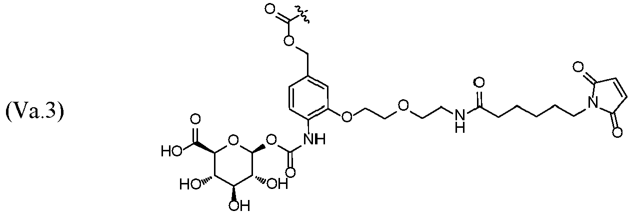

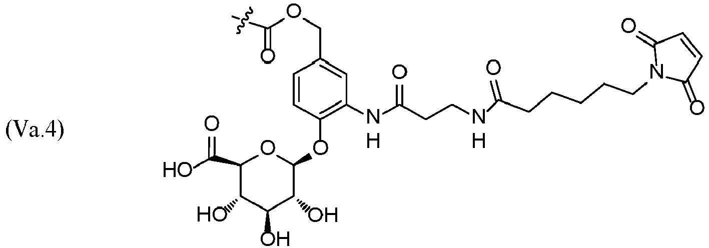

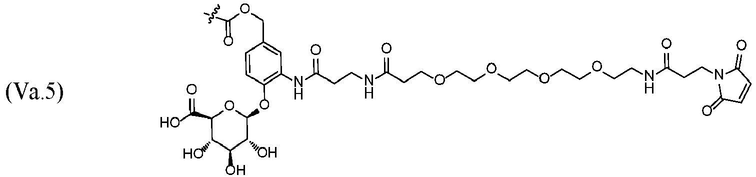

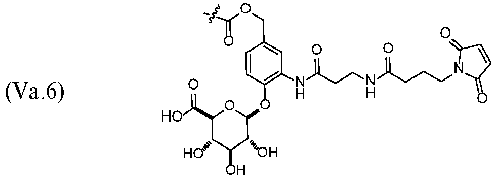

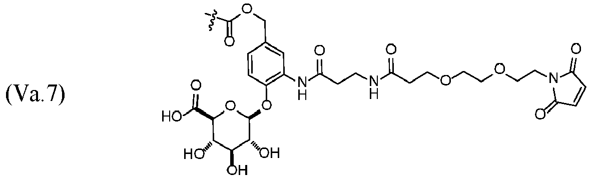

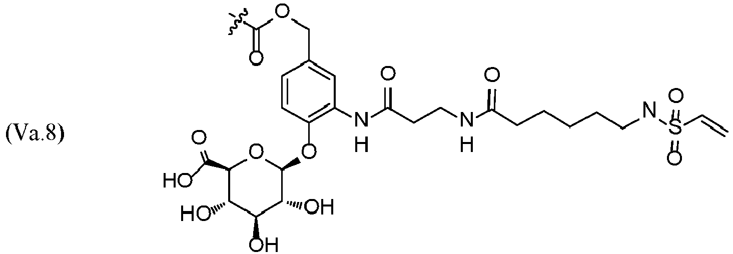

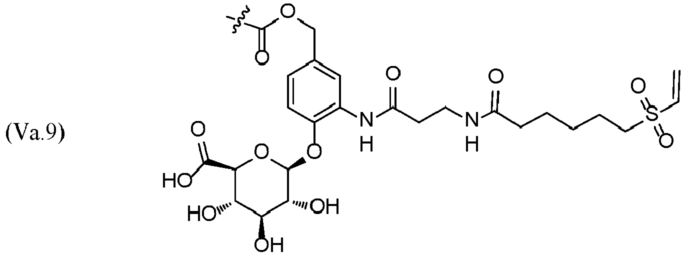

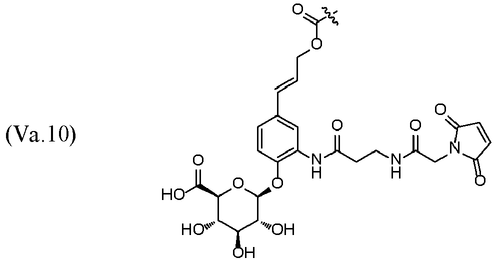

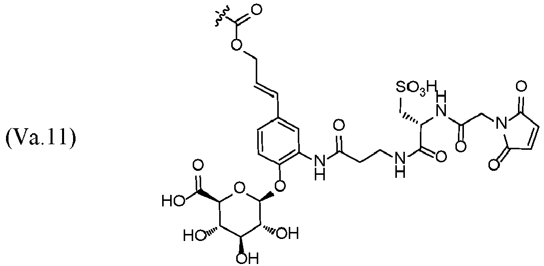

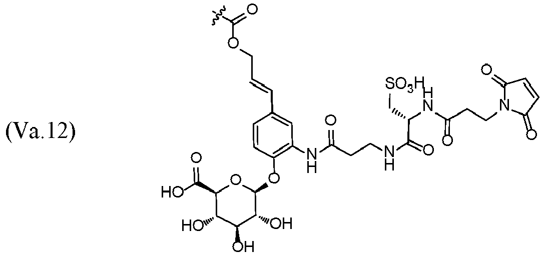

Specific exemplary embodiments of linkers according to structural formula (Vb) that may be included in the ADCs described herein include the linkers illustrated below (as illustrated, the linkers include a group suitable for covalently linking the linker to an antibody):

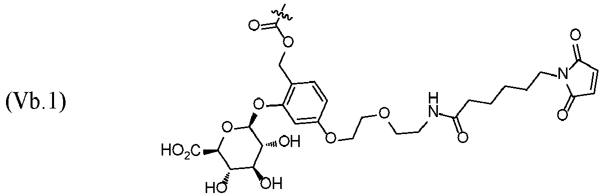

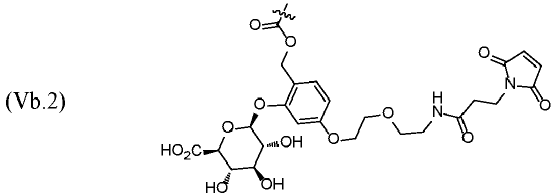

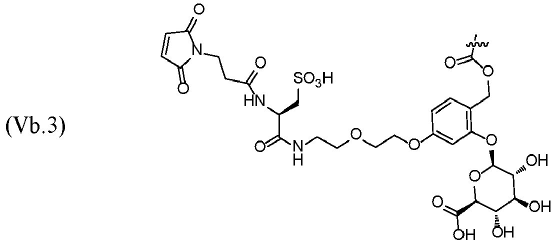

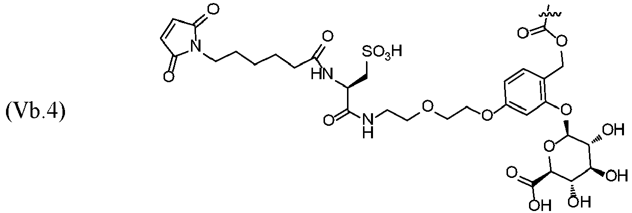

Specific exemplary embodiments of linkers according to structural formula (Vc) that may be included in the ADCs described herein include the linkers illustrated below (as illustrated, the linkers include a group suitable for covalently linking the linker to an antibody):

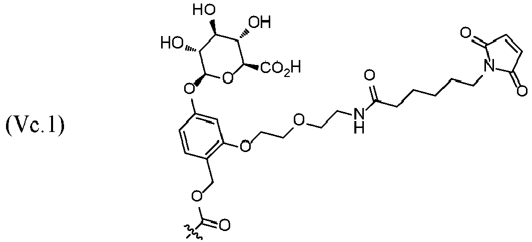

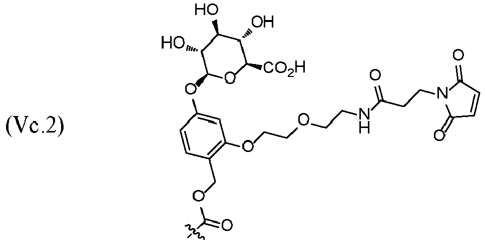

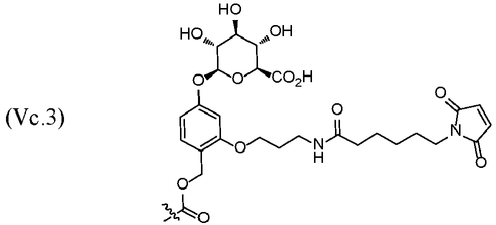

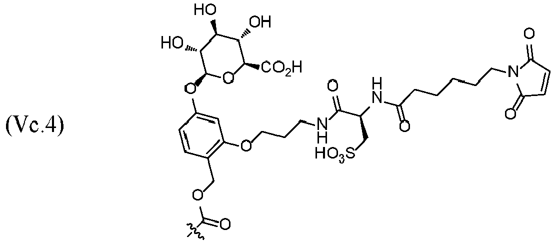

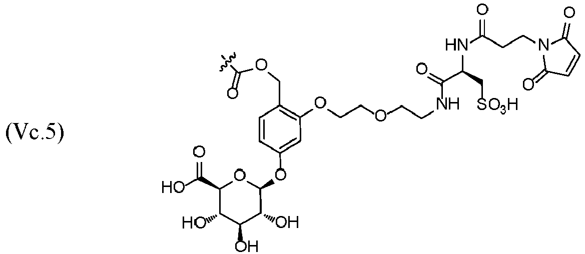

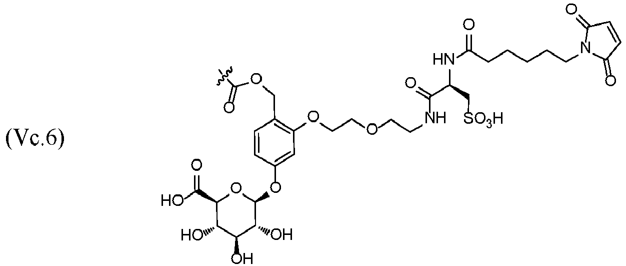

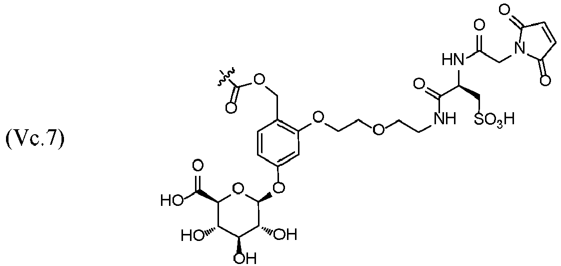

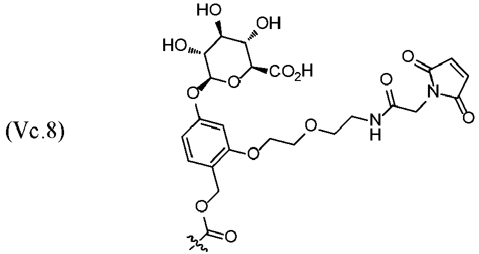

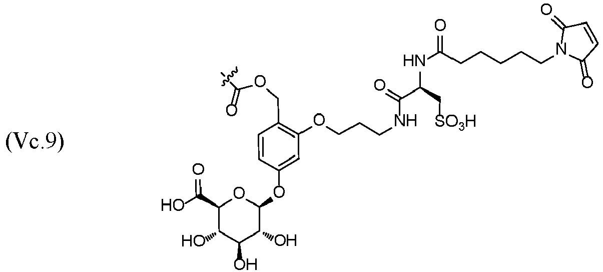

Specific exemplary embodiments of linkers according to structural formula (Vd) that may be included in the ADCs described herein include the linkers illustrated below (as illustrated, the linkers include a group suitable for covalently linking the linker to an antibody):

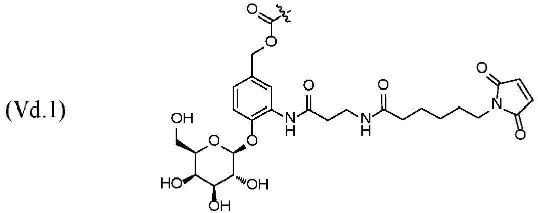

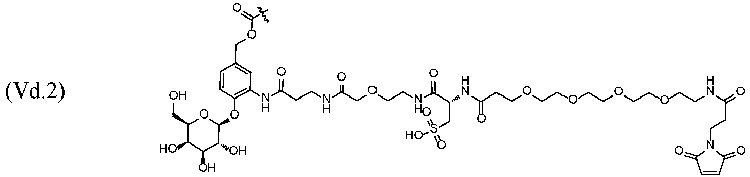

### 5.2.2.2 NON-CLEAVABLE LINKERS

Although cleavable linkers may provide certain advantages, the linkers comprising the ADC described herein need not be cleavable. For noncleavable linkers, the drug release does not depend on the differential properties between the plasma and some cytoplasmic compartments. The release of the drug is postulated to occur after internalization of the ADC via antigen-mediated endocytosis and delivery to lysosomal compartment, where the antibody is degraded to the level of amino acids through intracellular proteolytic degradation. This process releases a drug derivative, which is formed by the drug, the linker, and the amino acid residue to which the linker was covalently attached. The amino-acid drug metabolites from conjugates with noncleavable linkers are more hydrophilic and generally less membrane permeable, which leads to less bystander effects and less nonspecific toxicities compared to conjugates with a cleavable linker. In general, ADCs with noncleavable linkers have greater stability in circulation than ADCs with cleavable linkers. Non-cleavable linkers may be alkylene chains, or maybe polymeric in natures, such as, for example, based upon polyalkylene glycol polymers, amide polymers, or may include segments of alkylene chains, polyalkylene glycols and/or amide polymers. In certain embodiments, the linker comprises a polyethylene glycol segment having from 1 to 6 ethylene glycol units.

A variety of non-cleavable linkers used to link drugs to antibodies have been described. (*See,* Jeffrey et al., 2006, Bioconjug. Chem. 17:831-840; Jeffrey et al., 2007, Bioorg. Med. Chem. Lett. 17:2278-2280; and Jiang et al., 2005, J. Am. Chem. Soc. 127:11254-11255, the contents of which are incorporated herein by reference). All of these linkers may be included in the ADCs described herein.

In certain embodiments, the linker is non-cleavable *in vivo,* for example a linker according to structural formula (VIa), (VIb), (VIc) or (VId) as illustrated, the linkers include a group suitable for covalently linking the linker to an antibody: 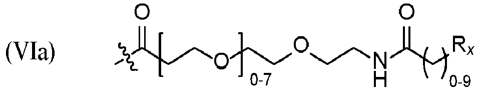 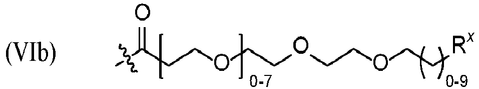 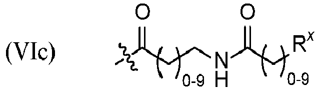 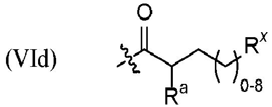 or salts thereof, wherein:
R^{a} is selected from hydrogen, alkyl, sulfonate and methyl sulfonate;
R*^{x}* is a moiety including a functional group capable of covalently linking the linker to an antibody; and
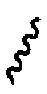 represents the point of attachment of the linker to the Bcl-xL inhibitor.

Specific exemplary embodiments of linkers according to structural formula (VIa)-(VId) that may be included in the ADCs described herein include the linkers illustrated below (as illustrated, the linkers include a group suitable for covalently linking the linker to an antibody, and " 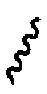 " represents the point of attachment a Bcl-xL inhibitor):

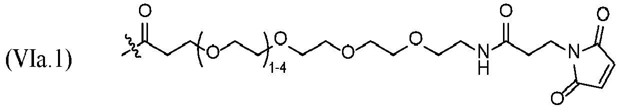

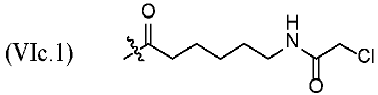

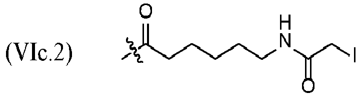

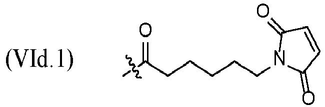

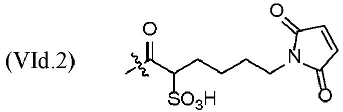

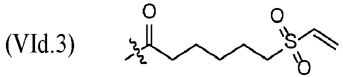

### 5.2.2.3 GROUPS USED TO ATTACH LINKERS TO ANTIBODIES

Attachment groups can be electrophilic in nature and include: maleimide groups, activated disulfides, active esters such as NHS esters and HOBt esters, haloformates, acid halides, alkyl and benzyl halides such as haloacetamides. As discussed below, there are also emerging technologies related to "self-stabilizing" maleimides and "bridging disulfides" that can be used in accordance with the disclosure.

Loss of the drug-linker from the ADC has been observed as a result of a maleimide exchange process with albumin, cysteine or glutathione (Alley et al., 2008, Bioconjugate Chem. 19: 759-769). This is particularly prevalent from highly solvent-accessible sites of conjugation while sites that are partially accessible and have a positively charged environment promote maleimide ring hydrolysis (Junutula et al., 2008, Nat. Biotechnol. 26: 925-932). A recognized solution is to hydrolyze the succinimide formed from conjugation as this is resistant to deconjugation from the antibody, thereby making the ADC stable in serum. It has been reported previously that the succinimide ring will undergo hydrolysis under alkaline conditions (Kalia et al., 2007, Bioorg. Med. Chem. Lett. 17: 6286-6289). One example of a "self-stabilizing" maleimide group that hydrolyzes spontaneously under antibody conjugation conditions to give an ADC species with improved stability is depicted in the schematic below. *See* U.S. Published Application No. 2013/0309256.

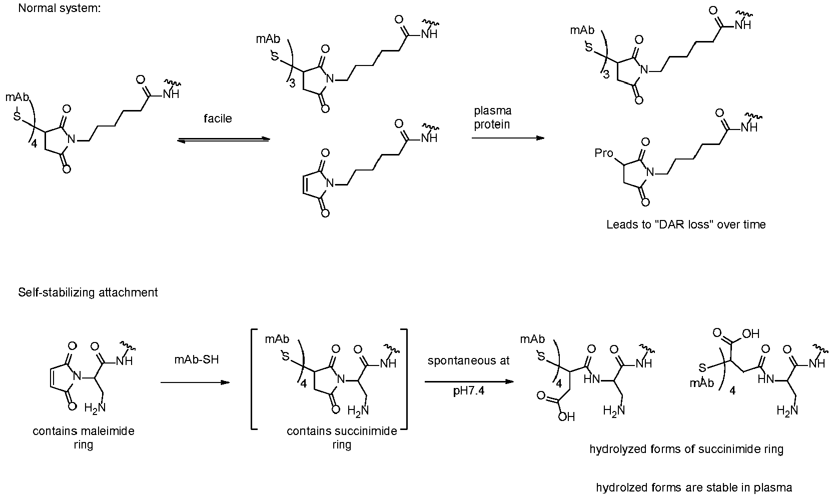

Polytherics has disclosed a method for bridging a pair of sulfhydryl groups derived from reduction of a native hinge disulfide bond. *See,* Badescu et al., 2014, Bioconjugate Chem. 25:1124-1136. The reaction is depicted in the schematic below. An advantage of this methodology is the ability to synthesize homogenous DAR4 ADCs by full reduction of IgGs (to give 4 pairs of sulfhydryls) followed by reaction with 4 equivalents of the alkylating agent. ADCs containing "bridged disulfides" are also claimed to have increased stability.

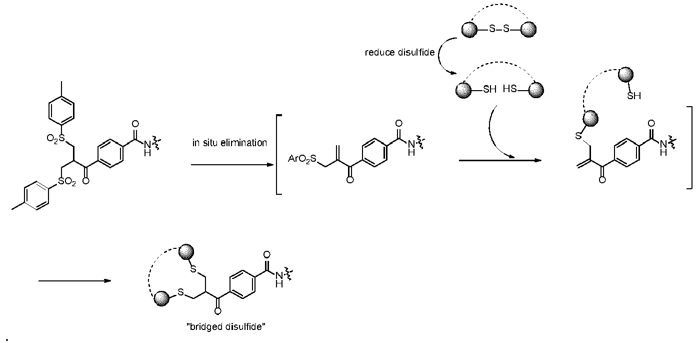

Similarly, as depicted below, a maleimide derivative that is capable of bridging a pair of sulfhydryl groups has been developed. *See* U.S. Published Application No. 2013/0224228.

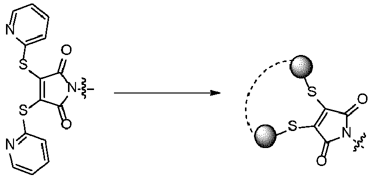

### 5.2.2.4 LINKER SELECTION CONSIDERATIONS

As is known by skilled artisans, the linker selected for a particular ADC may be influenced by a variety of factors, including but not limited to, the site of attachment to the antibody (*e.g.,* lys, cys or other amino acid residues), structural constraints of the drug pharmacophore and the lipophilicity of the drug. The specific linker selected for an ADC should seek to balance these different factors for the specific antibody/drug combination. For a review of the factors that are influenced by choice of linkers in ADCs, *see* Nolting, Chapter 5 "Linker Technology in Antibody-Drug Conjugates," In: Antibody-Drug Conjugates: Methods in Molecular Biology, vol. 1045, pp. 71-100, Laurent Ducry (Ed.), Springer Science & Business Medica, LLC, 2013.

For example, ADCs have been observed to effect killing of bystander antigen-negative cells present in the vicinity of the antigen-positive tumor cells. The mechanism of bystander cell killing by ADCs has indicated that metabolic products formed during intracellular processing of the ADCs may play a role. Neutral cytotoxic metabolites generated by metabolism of the ADCs in antigen-positive cells appear to play a role in bystander cell killing while charged metabolites may be prevented from diffusing across the membrane into the medium and therefore cannot affect bystander killing. In certain embodiments, the linker is selected to attenuate the bystander killing effect caused by cellular metabolites of the ADC. In certain embodiments, the linker is selected to increase the bystander killing effect.

The properties of the linker may also impact aggregation of the ADC under conditions of use and/or storage. Typically, ADCs reported in the literature contain no more than 3-4 drug molecules per antibody molecule (*see, e.g.,* Chari, 2008, Acc Chem Res 41:98-107). Attempts to obtain higher drug-to-antibody ratios ("DAR") often failed, particularly if both the drug and the linker were hydrophobic, due to aggregation of the ADC (King et al., 2002, J Med Chem 45:4336-4343; Hollander et al., 2008, Bioconjugate Chem 19:358-361; Burke et al., 2009 Bioconjugate Chem 20:1242-1250). In many instances, DARs higher than 3-4 could be beneficial as a means of increasing potency. In instances where the Bcl-xL inhibitor is hydrophobic in nature, it may be desirable to select linkers that are relatively hydrophilic as a means of reducing ADC aggregation, especially in instances where DARS greater than 3-4 are desired. Thus, in certain embodiments, the linker incorporates chemical moieties that reduce aggregation of the ADCs during storage and/or use. A linker may incorporate polar or hydrophilic groups such as charged groups or groups that become charged under physiological pH to reduce the aggregation of the ADCs. For example, a linker may incorporate charged groups such as salts or groups that deprotonate, *e.g.,* carboxylates, or protonate, *e.g.,* amines, at physiological pH.

Exemplary polyvalent linkers that have been reported to yield DARs as high as 20 that may be used to link numerous Bcl-xL inhibitors to an antibody are described in in U.S. Patent No 8,399,512; U.S. Published Application No. 2010/0152725; U.S. Patent No. 8,524,214; U.S. Patent No. 8,349,308; U.S. Published Application No. 2013/189218; U.S. Published Application No. 2014/017265; WO 2014/093379; WO 2014/093394; WO 2014/093640, the content of which are incorporated herein by reference in their entireties.

In particular embodiments, the aggregation of the ADCs during storage or use is less than about 40% as determined by size-exclusion chromatography (SEC). In particular embodiments, the aggregation of the ADCs during storage or use is less than 35%, such as less than about 30%, such as less than about 25%, such as less than about 20%, such as less than about 15%, such as less than about 10%, such as less than about 5%, such as less than about 4%, or even less, as determined by size-exclusion chromatography (SEC).

One embodiment pertains to ADCs or synthons in which linker L is selected from the group consisting of linkers IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c2, V1d.1-V1d.3, and salts thereof.

### 5.3 ANTIBODIES

The antibody of an ADC may be any antibody that binds, typically but not necessarily specifically, an antigen expressed on the surface of a target cell of interest. The antigen need not, but in some embodiments, is capable of internalizing an ADC bound thereto into the cell. Target cells of interest will generally include cells where induction of apoptosis via inhibition of anti-apoptotic Bcl-xL proteins is desirable, including, by way of example and not limitation, tumor cells that express or over-express Bcl-xL. Target antigens may be any protein, glycoprotein, polysaccharide, lipoprotein, *etc.* expressed on the target cell of interest, but will typically be proteins that are either uniquely expressed on the target cell and not on normal or healthy cells, or that are over-expressed on the target cell as compared to normal or healthy cells, such that the ADCs selectively target specific cells of interest, such as, for example, tumor cells. As will be appreciated by skilled artisans, the specific antigen, and hence antibody, selected will depend upon the identity of the desired target cell of interest. In specific embodiments, the antibody of the ADC is an antibody suitable for administration to humans.

Antibodies (Abs) and immunoglobulins (Igs) are glycoproteins having the same structural characteristics. While antibodies exhibit binding specificity to a specific target, immunoglobulins include both antibodies and other antibody-like molecules which lack target specificity. Native antibodies and immunoglobulins are usually heterotetrameric glycoproteins of about 150,000 daltons, composed of two identical light (L) chains and two identical heavy (H) chains. Each heavy chain has at one end a variable domain (VH) followed by a number of constant domains. Each light chain has a variable domain at one end (VL) and a constant domain at its other end.

References to "VH" refer to the variable region of an immunoglobulin heavy chain of an antibody, including the heavy chain of an Fv, scFv, or Fab. References to "VL" refer to the variable region of an immunoglobulin light chain, including the light chain of an Fv, scFv, dsFv or Fab.

The term "antibody" herein is used in the broadest sense and refers to an immunoglobulin molecule that specifically binds to, or is immunologically reactive with, a particular antigen, and includes polyclonal, monoclonal, genetically engineered and otherwise modified forms of antibodies, including but not limited to murine, chimeric antibodies, humanized antibodies, heteroconjugate antibodies (*e.g.,* bispecific antibodies, diabodies, triabodies, and tetrabodies), and antigen binding fragments of antibodies, including *e.g.,* Fab', F(ab')₂, Fab, Fv, rIgG, and scFv fragments. The term "scFv" refers to a single chain Fv antibody in which the variable domains of the heavy chain and the light chain from a traditional antibody have been joined to form one chain.

Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, *i.e.,* a molecule that contains an antigen binding site that immunospecifically binds an antigen of a target of interest or part thereof, such targets including but not limited to, cancer cell or cells that produce autoimmune antibodies associated with an autoimmune disease. The immunoglobulin disclosed herein can be of any type (*e.g.,* IgG, IgE, IgM, IgD, and IgA), class (*e.g.,* IgG1, IgG2, IgG3, IgG4, IgA1 and IgA2) or subclass of immunoglobulin molecule. The immunoglobulins can be derived from any species. In one aspect, however, the immunoglobulin is of human, murine, or rabbit origin.

The term "antibody fragment" refers to a portion of a full-length antibody, generally the target binding or variable region. Examples of antibody fragments include Fab, Fab', F(ab')₂ and Fv fragments. An "Fv" fragment is the minimum antibody fragment which contains a complete target recognition and binding site. This region consists of a dimer of one heavy and one light chain variable domain in a tight, non-covalent association (VH -VL dimer). It is in this configuration that the three CDRs of each variable domain interact to define a target binding site on the surface of the VH -VL dimer. Often, the six CDRs confer target binding specificity to the antibody. However, in some instances even a single variable domain (or half of an Fv comprising only three CDRs specific for a target) can have the ability to recognize and bind target. "Single-chain Fv" or "scFv" antibody fragments comprise the VH and VL domains of an antibody in a single polypeptide chain. Generally, the Fv polypeptide further comprises a polypeptide linker between the VH and VL domains which enables the scFv to form the desired structure for target binding. "Single domain antibodies" are composed of a single VH or VL domains which exhibit sufficient affinity to the target. In a specific embodiment, the single domain antibody is a camelized antibody (*see, e.g.,* Riechmann, 1999, Journal of Immunological Methods 231:25-38).

The Fab fragment contains the constant domain of the light chain and the first constant domain (CH₁) of the heavy chain. Fab' fragments differ from Fab fragments by the addition of a few residues at the carboxyl terminus of the heavy chain CH₁ domain including one or more cysteines from the antibody hinge region. F(ab') fragments are produced by cleavage of the disulfide bond at the hinge cysteines of the F(ab')₂ pepsin digestion product. Additional chemical couplings of antibody fragments are known to those of ordinary skill in the art.

Both the light chain and the heavy chain variable domains have complementarity determining regions (CDRs), also known as hypervariable regions. The more highly conserved portions of variable domains are called the framework (FR). As is known in the art, the amino acid position/boundary delineating a hypervariable region of an antibody can vary, depending on the context and the various definitions known in the art. Some positions within a variable domain may be viewed as hybrid hypervariable positions in that these positions can be deemed to be within a hypervariable region under one set of criteria while being deemed to be outside a hypervariable region under a different set of criteria. One or more of these positions can also be found in extended hypervariable regions. The CDRs in each chain are held together in close proximity by the FR regions and, with the CDRs from the other chain, contribute to the formation of the target binding site of antibodies (*see* Kabat et al., Sequences of Proteins of Immunological Interest (National Institute of Health, Bethesda, Md. 1987). As used herein, numbering of immunoglobulin amino acid residues is done according to the immunoglobulin amino acid residue numbering system of Kabat *et al.,* unless otherwise indicated.

In certain embodiments, the antibodies of the ADCs the disclosure are monoclonal antibodies. The term "monoclonal antibody" (mAb) refers to an antibody that is derived from a single copy or clone, including *e.g.,* any eukaryotic, prokaryotic, or phage clone, and not the method by which it is produced. Preferably, a monoclonal antibody of the disclosure exists in a homogeneous or substantially homogeneous population. Monoclonal antibody includes both intact molecules, as well as, antibody fragments (such as, for example, Fab and F(ab')₂ fragments) which are capable of specifically binding to a protein. Fab and F(ab')₂ fragments lack the Fc fragment of intact antibody, clear more rapidly from the circulation of the animal, and may have less non-specific tissue binding than an intact antibody (Wahl et al., 1983, J. Nucl. Med. 24:316). Monoclonal antibodies useful with the present disclosure can be prepared using a wide variety of techniques known in the art including the use of hybridoma, recombinant, and phage display technologies, or a combination thereof. The antibodies of the disclosure include chimeric, primatized, humanized, or human antibodies.

While in most instances antibodies are composed of only the genetically-encoded amino acids, in some embodiments non-encoded amino acids may be incorporated at specific locations to control the number of Bcl-xL inhibitors linked to the antibody, as well as their locations. Examples of non-encoded amino acids that may be incorporated into antibodies for use in controlling stoichiometry and attachment location, as well as methods for making such modified antibodies are discussed in Tian et al., 2014, Proc Nat'l Acad Sci USA 111(5):1766-1771 and Axup et al., 2012, Proc Nat'l Acad Sci USA 109(40): 16101-16106 the entire contents of which are incorporated herein by reference. In certain embodiments, the non-encoded amino acids limit the number of Bcl-xL inhibitors per antibody to about 1-8 or about 2-4.

In certain embodiments, the antibody of the ADCs described herein is a chimeric antibody. The term "chimeric" antibody as used herein refers to an antibody having variable sequences derived from a non-human immunoglobulin, such as rat or mouse antibody, and human immunoglobulin constant regions, typically chosen from a human immunoglobulin template. Methods for producing chimeric antibodies are known in the art. *See, e.g.,* Morrison, 1985, Science 229(4719):1202-7; Oi et al., 1986, BioTechniques 4:214-221; Gillies et al., 1985, J. Immunol. Methods 125:191-202; U.S. Pat. Nos. 5,807,715; 4,816,567; and 4,816397, which are incorporated herein by reference in their entireties.

In certain embodiments, the antibody of the ADCs described herein is a humanized antibody. "Humanized" forms of non-human (*e.g.,* murine) antibodies are chimeric immunoglobulins, immunoglobulin chains or fragments thereof (such as Fv, Fab, Fab', F(ab')₂ or other target-binding subdomains of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody can also comprise at least a portion of an immunoglobulin constant region (Fc), typically that of a human immunoglobulin consensus sequence. Methods of antibody humanization are known in the art. *See, e.g.,* Riechmann et al., 1988, Nature 332:323-7; U.S. Patent Nos: 5,530,101; 5,585,089; 5,693,761; 5,693,762; and 6,180,370 to Queen et al.*;* EP239400; PCT publication WO 91/09967; U.S. Patent No. 5,225,539; EP592106; EP519596; Padlan, 1991, Mol. Immunol., 28:489-498; Studnicka et al., 1994, Prot. Eng. 7:805-814; Roguska et al., 1994, Proc. Natl. Acad. Sci. 91:969-973; and U.S. Patent No. 5,565,332, all of which are hereby incorporated by reference in their entireties.

In certain embodiments, the antibody of the ADCs described herein is a human antibody. Completely "human" antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods using antibody libraries derived from human immunoglobulin sequences. *See* U.S. Patent Nos. 4,444,887 4,716,111, 6,114,598, 6,207,418, 6,235,883, 7,227,002, 8,809,151 and U.S. Published Application No. 2013/189218, the contents of which are incorporated herein by reference in their entireties. Human antibodies can also be produced using transgenic mice which are incapable of expressing functional endogenous immunoglobulins, but which can express human immunoglobulin genes. *See, e.g.,* U.S. Patent Nos. 5,413,923; 5,625,126; 5,633,425; 5,569,825; 5,661,016; 5,545,806; 5,814,318; 5,885,793; 5,916,771; 5,939,598; 7,723,270; 8,809,051 and U.S. Published Application No. 2013/117871, which are incorporated by reference herein in their entireties. In addition, companies such as Medarex (Princeton, NJ), Astellas Pharma (Deerfield, IL), and Regeneron (Tarrytown, NY) can be engaged to provide human antibodies directed against a selected antigen using technology similar to that described above. Completely human antibodies that recognize a selected epitope can be generated using a technique referred to as "guided selection." In this approach a selected non-human monoclonal antibody, *e.g.,* a mouse antibody, is used to guide the selection of a completely human antibody recognizing the same epitope (Jespers et al., 1988, Biotechnology 12:899-903).

In certain embodiments, the antibody of the ADCs described herein is a primatized antibody. The term "primatized antibody" refers to an antibody comprising monkey variable regions and human constant regions. Methods for producing primatized antibodies are known in the art. *See, e.g.,* U.S. Patent Nos. 5,658,570; 5,681,722; and 5,693,780, which are incorporated herein by reference in their entireties.

In certain embodiments, the antibody of the ADCs described herein is a bispecific antibody or a dual variable domain antibody (DVD). Bispecific and DVD antibodies are monoclonal, often human or humanized, antibodies that have binding specificities for at least two different antigens. DVDs are described, for example, in U.S. Patent No. 7,612,181, the disclosure of which is incorporated herein by reference.

In certain embodiments, the antibody of the ADCs described herein is a derivatized antibody. For example, but not by way of limitation, derivatized antibodies are typically modified by glycosylation, acetylation, pegylation, phosphorylation, amidation, derivatization by known protecting/blocking groups, proteolytic cleavage, linkage to a cellular ligand or other protein, *etc.* Any of numerous chemical modifications can be carried out by known techniques, including, but not limited to, specific chemical cleavage, acetylation, formylation, metabolic synthesis of tunicamycin, *etc.* Additionally, the derivative can contain one or more non-natural amino acids, *e.g.,* using ambrx technology (*see, e.g.,* Wolfson, 2006, Chem. Biol. 13(10):1011-2).

In certain embodiments, the antibody of the ADCs described herein has a sequence that has been modified to alter at least one constant region-mediated biological effector function relative to the corresponding wild type sequence. For example, in some embodiments, the antibody can be modified to reduce at least one constant region-mediated biological effector function relative to an unmodified antibody, e.g., reduced binding to the Fc receptor (FcR). FcR binding can be reduced by mutating the immunoglobulin constant region segment of the antibody at particular regions necessary for FcR interactions (*see e.g.,* Canfield and Morrison, 1991, J. Exp. Med. 173:1483-1491; and Lund et al., 1991, J. Immunol. 147:2657-2662).

In certain embodiments, the antibody of the ADCs described herein is modified to acquire or improve at least one constant region-mediated biological effector function relative to an unmodified antibody, *e.g.,* to enhance FcγR interactions (*See, e.g.,* US 2006/0134709). For example, an antibody with a constant region that binds FcγRIIA, FcγRIIB and/or FcγRIIIA with greater affinity than the corresponding wild type constant region can be produced according to the methods described herein.

In certain specific embodiments, the antibody of the ADCs described herein is an antibody that binds tumor cells, such as an antibody against a cell surface receptor or a tumor-associated antigen (TAA). In attempts to discover effective cellular targets for cancer diagnosis and therapy, researchers have sought to identify transmembrane or otherwise tumor-associated polypeptides that are specifically expressed on the surface of one or more particular type(s) of cancer cell as compared to on one or more normal non-cancerous cell(s). Often, such tumor-associated polypeptides are more abundantly expressed on the surface of the cancer cells as compared to the surface of the non-cancerous cells. Such cell surface receptor and tumor-associated antigens are known in the art, and can prepared for use in generating antibodies using methods and information which are well known in the art.

### 5.3.1 EXEMPLARY CELL SURFACE RECEPTORS AND TAAS

Examples of cell surface receptor and TAAs to which the antibody of the ADCs described herein may be targeted include, but are not limited to, the various receptors and TAAs listed below. For convenience, information relating to these antigens, all of which are known in the art, is listed below and includes names, alternative names, Genbank accession numbers and primary reference(s), following nucleic acid and protein sequence identification conventions of the National Center for Biotechnology Information (NCBI). Nucleic acid and protein sequences corresponding to the listed cell surface receptors and TAAs are available in public databases such as GenBank.
4-1BB
5AC
5T4
Alpha-fetoprotein
angiopoietin 2
ASLG659
TCL1
BMPR1B
Brevican (BCAN, BEHAB)
C242 antigen
C5
CA-125
CA-125 (imitation)
CA-IX (Carbonic anhydrase 9)
CCR4
CD140a
CD152
CD19
CD20
CD200
CD21 (C3DR) 1)
CD22 (B-cell receptor CD22-B isoform)
CD221
CD23 (gE receptor)
CD28
CD30 (TNFRSF8)
CD33
CD37
CD38(cyclic ADP ribose hydrolase)
CD4
CD40
CD44 v6
CD51
CD52
CD56
CD70
CD72 (Lyb-2, B-cell differentiation antigen CD72)
CD74
CD79a (CD79A, CD79α, immunoglobulin-associated alpha) Genbank accession No. NP_001774.10)
CD79b (CD79B, CD79β, B29)
CD80
CEA
CEA-related antigen
ch4D5
CLDN18.2
CRIPTO (CR, CR1, CRGF, TDGF1 teratocarcinoma-derived growth factor)
CTLA-4
CXCR5
DLL4
DR5
E16 (LAT1, SLC7A5) EGFL7
EGFR
EpCAM
EphB2R (DRT, ERK, Hek5, EPHT3, Tyro5)
Episialin
ERBB3
ETBR (Endothelin type B receptor)
FCRH1 (Fc receptor-like protein 1)
FcRH2 (IFGP4, IRTA4, SPAP1, SPAP1B, SPAP1C, SH2 domain containing phosphatase anchor protein
Fibronectin extra domain-B
Folate receptor 1
Frizzled receptor
GD2
GD3 ganglioside
GEDA
GPNMB
HER1
HER2 (ErbB2)
HER2/neu
HER3
HGF
HLA-DOB
HLA-DR
Human scatter factor receptor kinase
IGF-1 receptor
IgG4
IL-13
IL20Rα (IL20Ra, ZCYTOR7)
IL-6
ILGF2
ILFR1R
integrin α
integrin α₅β₁
Integrin αᵥβ₃
IRTA2 (Immunoglobulin superfamily receptor translocation associated 2, Gene Chromosome 1q21)
Lewis-Y antigen
LY64 (RP105)
MCP-1
MDP (DPEP1)
MPF (MSLN, SMR, mesothelin, megakaryocyte potentiating factor)
MS4A1
MSG783 (RNF124, hypothetical protein FLJ20315)
MUC1
Mucin CanAg
Napi3 (NAPI-3B, NPTIIb, SLC34A2, type II sodium-dependent phosphate transporter 3b)
NCA (CEACAM6)
P2X5 (Purinergic receptor P2X ligand-gated ion channel 5)
PD-1
PDCD1
PDGF-R α
Prostate specific membrane antigen
PSCA (Prostate stem cell antigen precursor)
PSCA hlg
RANKL
RON
SDC1
Sema 5b
SLAMF7 (CS-1)
STEAP1
STEAP2 (HGNC_8639, PCANAP1, STAMP1, STEAP2, STMP, prostate cancer associated gene 1)
TAG-72
TEM1
Tenascin C
TENB2, (TMEFF2, tomoregulin, TPEF, HPP1, TR)
TGF-β
TRAIL-E2
TRAIL-R1
TRAIL-R2
TrpM4 (BR22450, FLJ20041, TRPM4, TRPM4B, transient receptor potential cation channel subfamily M, member 4)
TA CTAA16.88
TWEAK-R
TYRP1 (glycoprotein 75)
VEGF
VEGF-A
EGFR-1
VEGFR-2
Vimentin

### 5.3.2 EXEMPLARY ANTIBODIES

Exemplary antibodies to be used with ADCs of the disclosure include but are not limited to 3F8 (GD2), Abagovomab (CA-125 (imitation)), Adecatumumab (EpCAM), Afutuzumab (CD20), Alacizumab pegol (VEGFR2), ALD518 (IL-6), Alemtuzumab (CD52), Altumomab pentetate (CEA), Amatuximab (Mesothelin), Anatumomab mafenatox (TAG-72), Apolizumab (HLA-DR), Arcitumomab (CEA), Bavituximab (Phosphatidylserine), Bectumomab (CD22), Belimumab (BAFF), Besilesomab (CEA-related antigen), Bevacizumab (VEGF-A), Bivatuzumab mertansine (CD44 v6), Blinatumomab (CD19), Brentuximab vedotin ((CD30 (TNFRSF8)), Cantuzumab mertansine (Mucin CanAg), Cantuzumab ravtansine (MUC1), Capromab pendetide (Prostatic carcinoma cells), Carlumab (MCP-1), Catumaxomab (EpCAM, CD3), CC49 (Tag-72), cBR96-DOX ADC (Lewis-Y antigen), Cetuximab (EGFR), Citatuzumab bogatox (EpCAM), Cixutumumab (IGF-1 receptor), Clivatuzumab tetraxetan(MUC1), Conatumumab (TRAIL-E2), Dacetuzumab (CD40), Dalotuzumab (Insulin-like growth factor I receptor), Daratumumab ((CD38 (cyclic ADP ribose hydrolase)), Demcizumab (DLL4), Denosumab (RANKL), Detumomab (B-lymphoma cell), Drozitumab (DR5), Dusigitumab (ILGF2), Ecromeximab (GD3 ganglioside), Eculizumab (C5), Edrecolomab (EpCAM), Elotuzumab (SLAMF7), Elsilimomab (IL-6), Enavatuzumab (TWEAK receptor), Enoticumab (DLL4), Ensituximab (5AC), Epitumomab cituxetan (Episialin), Epratuzumab (CD22), Ertumaxomab ((HER2/neu, CD3)), Etaracizumab (Integrin αᵥβ₃), Farletuzumab (Folate receptor 1), FBTA05 (CD20), Ficlatuzumab (HGF), Figitumumab (IGF-1 receptor), Flanvotumab ((TYRP1 (glycoprotein 75)), Fresolimumab (TGF-β), Galiximab (CD80), Ganitumab (IGF-I), Gemtuzumab ozogamicin (CD33), Girentuximab ((Carbonic anhydrase 9 (CA-IX)), Glembatumumab vedotin (GPNMB), Ibritumomab tiuxetan (CD20), Icrucumab (VEGFR-1), Ig-ovomab (CA-125), IMAB362 (CLDN18.2), Imgatuzumab (EGFR), Indatuximab ravtansine (SDC1), Intetumumab (CD51), Inotuzumab ozogamicin (CD22), Ipilimumab (CD152), Iratumumab ((CD30 (TNFRSF8)), Labetuzumab (CEA), Lambrolizumab (PDCD1), Lexatumumab (TRAIL-R2), Lintuzumab (CD33), Lorvotuzumab mertansine (CD56), Lucatumumab (CD40), Lumiliximab ((CD23 (IgE receptor)), Mapatumumab (TRAIL-R1), Margetuximab (ch4D5), Matuzumab (EGFR), Milatuzumab (CD74), Mitumomab (GD3 ganglioside), Mogamulizumab (CCR4), Moxetumomab pasudotox (CD22), Nacolomab tafenatox (C242 antigen), Naptumomab estafenatox (5T4), Narnatumab (RON), Natalizumab (integrin α₄). Necitumumab (EGFR), Nesvacumab (angiopoietin 2), Nimotuzumab (EGFR), Nivolumab (IgG4), Ocaratuzumab (CD20), Ofatumumab (CD20), Olaratumab (PDGF-R α), Onartuzumab (Human scatter factor receptor kinase), Ontuxizumab (TEM1), Oportuzumab monato (EpCAM), Oregovomab (CA-125), Otlertuzumab (CD37), Panitumumab (EGFR), Pankomab (Tumor specific glycosylation of MUC1), Parsatuzumab (EGFL7), Patritumab (HER3), Pemtumomab (MUC1), Pertuzumab (HER2/neu), Pidilizumab (PD-1), Pinatuzumab vedotin (CD22), Pritumumab (Vimentin), Racotumomab (N-glycolylneuraminic acid), Radretumab (Fibronectin extra domain-B), Ramucirumab (VEGFR2), Rilotumumab (HGF), Rituximab (CD20), Robatumumab (IGF-1 receptor), Samalizumab (CD200), Satumomab pendetide (TAG-72), Seribantumab (ERBB3), Sibrotuzumab (FAP), SGN-CD19A (CD19), SGN-CD33A (CD33), Siltuximab (IL-6), Solitomab (EpCAM), Sonepcizumab (Sphingosine-1- phosphate), Tabalumb (BAFF), Tacatuzumab tetraxetan (Alpha-fetoprotein), Taplitumomab paptox (CD19), Tenatumomab (Tenascin C), Teprotumumab (CD221), TGN1412 (CD28), Ticilimumab (CTLA-4), Tigatuzumab (TRAIL-R2), TNX-650 (IL-13), Tovetumab (CD140a), Trastuzumab (HER2/neu), TRBS07 (GD2), Tremelimumab (CTLA-4), Tucotuzumab celmoleukin (EpCAM), Ublituximab (MS4A1), Urelumab (4-1BB), Vandetanib (VEGF), Vantictumab (Frizzled receptor), Volociximab (integrin α₅β₁), Vorsetuzumab mafodotin (CD70), Votumumab (Tumor antigen CTAA16.88), Zalutumumab (EGFR), Zanolimumab (CD4), and Zatuximab (HER1).

In certain embodiments, the antibody of the ADC binds EGFR, NCAM1 or EpCAM.

### 5.4 METHODS OF MAKING ANTIBODIES

The antibody of an ADC can be prepared by recombinant expression of immunoglobulin light and heavy chain genes in a host cell. For example, to express an antibody recombinantly, a host cell is transfected with one or more recombinant expression vectors carrying DNA fragments encoding the immunoglobulin light and heavy chains of the antibody such that the light and heavy chains are expressed in the host cell and, optionally, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Molecular Cloning; A Laboratory Manual, Second Edition (Sambrook, Fritsch and Maniatis (eds), Cold Spring Harbor, N. Y., 1989), Current Protocols in Molecular Biology (Ausubel, F.M. et al., eds., Greene Publishing Associates, 1989) and in U.S. Patent No. 4,816,397.

In one embodiment, the Fc variant antibodies are similar to their wild-type equivalents but for changes in their Fc domains. To generate nucleic acids encoding such Fc variant antibodies, a DNA fragment encoding the Fc domain or a portion of the Fc domain of the wild-type antibody (referred to as the "wild-type Fc domain") can be synthesized and used as a template for mutagenesis to generate an antibody as described herein using routine mutagenesis techniques; alternatively, a DNA fragment encoding the antibody can be directly synthesized.

Once DNA fragments encoding wild-type Fc domains are obtained, these DNA fragments can be further manipulated by standard recombinant DNA techniques, for example, to convert the constant region genes to full-length antibody chain genes. In these manipulations, a CH-encoding DNA fragment is operatively linked to another DNA fragment encoding another protein, such as an antibody variable region or a flexible linker. The term "operatively linked," as used in this context, is intended to mean that the two DNA fragments are joined such that the amino acid sequences encoded by the two DNA fragments remain in-frame.

To express the Fc variant antibodies, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. A variant antibody light chain gene and the antibody heavy chain gene can be inserted into separate vectors or, more typically, both genes are inserted into the same expression vector.

The antibody genes are inserted into the expression vector by standard methods (e.g., ligation of complementary restriction sites on the antibody gene fragment and vector, or blunt end ligation if no restriction sites are present). Prior to insertion of the variant Fc domain sequences, the expression vector can already carry antibody variable region sequences. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

In addition to the antibody chain genes, the recombinant expression vectors carry regulatory sequences that control the expression of the antibody chain genes in a host cell. The term "regulatory sequence" is intended to include promoters, enhancers and other expression control elements (*e.g.,* polyadenylation signals) that control the transcription or translation of the antibody chain genes. Such regulatory sequences are described, for example, in Goeddel, Gene Expression Technology: Methods in Enzymology 185 (Academic Press, San Diego, CA, 1990). It will be appreciated by those skilled in the art that the design of the expression vector, including the selection of regulatory sequences may depend on such factors as the choice of the host cell to be transformed, the level of expression of protein desired, *etc.* Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. For further description of viral regulatory elements, and sequences thereof, *see, e.g.,* U.S. Patent No. 5,168,062 by Stinski, U.S. Patent No. 4,510,245 by Bell et al.*,* and U.S. Patent No. 4,968,615 by Schaffner et al.

In addition to the antibody chain genes and regulatory sequences, the recombinant expression vectors can carry additional sequences, such as sequences that regulate replication of the vector in host cells (*e.g.,* origins of replication) and selectable marker genes. The selectable marker gene facilitates selection of host cells into which the vector has been introduced (*See, e.g.,* U.S. Patents Nos. 4,399,216, 4,634,665 and 5,179,017, all by Axel et al.). For example, typically the selectable marker gene confers resistance to drugs, such as G418, puromycin, blasticidin, hygromycin or methotrexate, on a host cell into which the vector has been introduced. Suitable selectable marker genes include the dihydrofolate reductase (DHFR) gene (for use in DHFR⁻ host cells with methotrexate selection/amplification) and the neo gene (for G418 selection). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, lipofection, calcium-phosphate precipitation, DEAE- dextran transfection and the like.

It is possible to express the antibodies in either prokaryotic or eukaryotic host cells. In certain embodiments, expression of antibodies is performed in eukaryotic cells, e.g., mammalian host cells, for optimal secretion of a properly folded and immunologically active antibody. Exemplary mammalian host cells for expressing the recombinant antibodies include Chinese Hamster Ovary (CHO cells) (including DHFR⁻ CHO cells, described in Urlaub and Chasin, 1980, Proc. Natl. Acad. Sci. USA 77:4216-4220, used with a DHFR selectable marker, *e.g.,* as described in Kaufman and Sharp, 1982, Mol. Biol. 159:601-621), NS0 myeloma cells, COS cells, 293 cells and SP2/0 cells. When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods. Host cells can also be used to produce portions of intact antibodies, such as Fab fragments or scFv molecules.

In some embodiments, the antibody of an ADC can be a bifunctional antibody. Such antibodies, in which one heavy and one light chain are specific for one antigen and the other heavy and light chain are specific for a second antigen, can be produced by crosslinking an antibody to a second antibody by standard chemical crosslinking methods. Bifunctional antibodies can also be made by expressing a nucleic acid engineered to encode a bifunctional antibody.

In certain embodiments, dual specific antibodies, *i.e.* antibodies that bind one antigen and a second, unrelated antigen using the same binding site, can be produced by mutating amino acid residues in the light chain and/or heavy chain CDRs. Exemplary second antigens include a pro-inflammatory cytokine (such as, for example, lymphotoxin, interferon-γ, or interleukin-1). Dual specific antibodies can be produced, *e.g.,* by mutating amino acid residues in the periphery of the antigen binding site (*See, e.g.,* Bostrom et al., 2009, Science 323:1610-1614). Dual functional antibodies can be made by expressing a nucleic acid engineered to encode a dual specific antibody.

Antibodies can also be produced by chemical synthesis (*e.g.,* by the methods described in Solid Phase Peptide Synthesis, 2nd ed., 1984 The Pierce Chemical Co., Rockford, Ill.). Antibodies can also be generated using a cell-free platform (*see, e.g.,* Chu et al., Biochemia No. 2, 2001 (Roche Molecular Biologicals)).

Methods for recombinant expression of Fc fusion proteins are described in Flanagan et al., Methods in Molecular Biology, vol. 378: Monoclonal Antibodies: Methods and Protocols.

Once an antibody has been produced by recombinant expression, it can be purified by any method known in the art for purification of an immunoglobulin molecule, for example, by chromatography (e.g., ion exchange, affinity, particularly by affinity for antigen after Protein A or Protein G selection, and sizing column chromatography), centrifugation, differential solubility, or by any other standard technique for the purification of proteins.

Once isolated, an antibody can, if desired, be further purified, e.g., by high performance liquid chromatography (*See, e.g.,* Fisher, Laboratory Techniques In Biochemistry And Molecular Biology (Work and Burdon, eds., Elsevier, 1980)), or by gel filtration chromatography on a Superdex™ 75 column (Pharmacia Biotech AB, Uppsala, Sweden).

### 5.5 ANTIBODY-DRUG CONJUGATE SYNTHONS

Antibody-Drug Conjugate synthons are synthetic intermediates used to form ADCs. The synthons are generally compounds according to structural formula (III):

(III) D-L-R*^{x}*

or salts thereof, wherein D is a Bcl-xL inhibitor as previously described, L is a linker as previously described, and R*^{x}* is a moiety that comprises a functional group suitable for covalently linking the synthon to an antibody. In specific embodiments, the synthons are compounds according to structural formula (IIIa) or salts thereof, where Ar, R¹, R², R⁴, R^{10a}, R^{10b}, R^{10c}, R^{11a}, R^{11b}, Z¹, Z², and *n* are as previously defined for structural formula (IIa), and L and R*^{x}* are as defined for structural formula (III):

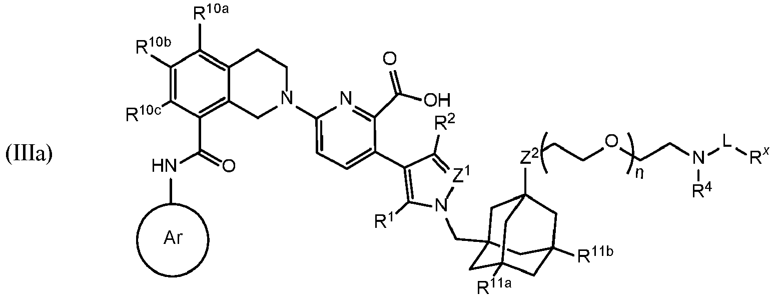

To synthesize an ADC, an intermediate synthon according to structural formula (III), or a salt thereof, is contacted with an antibody of interest under conditions in which functional group R*^{x}* reacts with a "complementary" functional group on the antibody, F^{x}, to form a covalent linkage.

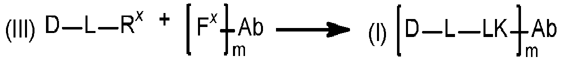

The identities of groups R*^{x}* and F*^{x}* will depend upon the chemistry used to link the synthon to the antibody. Generally, the chemistry used should not alter the integrity of the antibody, for example its ability to bind its target. Preferably, the binding properties of the conjugated antibody will closely resemble those of the unconjugated antibody. A variety of chemistries and techniques for conjugating molecules to biological molecules such as antibodies are known in the art and in particular to antibodies, are well-known. *See, e.g.,* Amon et al., "Monoclonal Antibodies For Immunotargeting Of Drugs In Cancer Therapy," in Monoclonal Antibodies And Cancer Therapy, Reisfeld et al. eds., Alan R. Liss, Inc., 1985; Hellstrom et al., "Antibodies For Drug Delivery," in Controlled Drug Delivery (Robinson et al. eds., Marcel Dekker, Inc., 2nd ed. 1987; Thorpe, "Antibody Carriers Of Cytotoxic Agents In Cancer Therapy: A Review," in Monoclonal Antibodies '84: Biological And Clinical Applications, Pinchera et al., eds., 1985; "Analysis, Results, and Future Prospective of the Therapeutic Use of Radiolabeled Antibody In Cancer Therapy," in Monoclonal Antibodies ForCancer Detection And Therapy, Baldwin et al., eds., Academic Press, 1985; and Thorpe et al., 1982, Immunol. Rev. 62:119-58; and WO 89/12624. Any of these chemistries may be used to link the synthons to an antibody.

In one embodiment, **R***^{x}* comprises a functional group capable of linking the synthon to an amino group on an antibody. In another embodiment, **R***^{x}* comprises an NHS-ester or an isothiocyanate. In another embodiment, **R***^{x}* comprises a functional group capable of linking the synthon to a sulfhydryl group on an antibody. In another embodiment, **R***^{x}* comprises a haloacetyl or a maleimide.

Typically the synthons are linked to the side chains of amino acid residues of the antibody, including, for example, the primary amino group of accessible lysine residues or the sulfhydryl group of accessible cysteine residues. Free sulfhydryl groups may be obtained by reducing interchain disulfide bonds.

In one embodiment, **LK** is a linkage formed with an amino group on antibody **Ab.** In another embodiment, **LK** is an amide or a thiourea. In another embodiment, **LK** is a linkage formed with a sulfhydryl group on antibody **Ab.** In another embodiment, **LK** is a thioether.

In one embodiment, **D** is selected from the group consisting of W1.01, W1.02, W1.03, W1.04, W1.05, W1.06, W1.07, and W1.08 and salts thereof; **L** is selected from the group consisting of linkers IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c.2, V1d.1-V1d.3, and salts thereof; **LK** is selected from the group consisting of amide, thiourea and thioether; and m is an integer ranging from 1 to 8.

A number of functional groups R*^{x}* and chemistries useful for linking synthons to accessible lysine residues are known, and include by way of example and not limitation NHS-esters and isothiocyanates.

A number of functional groups R*^{x}* and chemistries useful for linking synthons to accessible free sulfhydryl groups of cysteine residues are known, and include by way of example and not limitation haloacetyls and maleimides.

In one embodiment, **D** is selected from the group consisting of W1.01, W1.02, W1.03, W1.04, W1.05, W1.06, W1.07, and W1.08 and salts thereof; **L** is selected from the group consisting of linkers IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c.2, V1d.1-V1d.3, and salts thereof; **R*^{x}*** comprises a functional group selected from the group consisting of NHS-ester, isothiocyanate, haloacetyl and maleimide.

However, conjugation chemistries are not limited to available side chain groups. Side chains such as amines may be converted to other useful groups, such as hydroxyls, by linking an appropriate small molecule to the amine. This strategy can be used to increase the number of available linking sites in the antibody by conjugating multifunctional small molecules to side chains of accessible amino acid residues of the antibody.

The antibody may also be engineered to include amino acid residues for conjugation. An approach for engineering antibodies to include non-genetically encoded amino acid residues useful for conjugating drugs in the context of ADCs is described in Axup et al., 2003, Proc Natl Acad Sci 109:16101-16106 and Tian et al., 2014, Proc Natl Acad Sci 111:1776-1771.

Exemplary synthons useful for making ADCs described herein include, but are not limited to, the following synthons:

| **Example No.** | **Synthon** | **Synthon structure** |
|---|---|---|
| 2.1 | E | 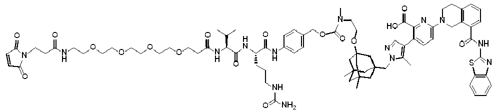 |
| 2.2 | D | 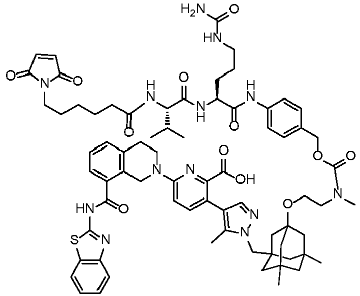 |
| 2.3 | J | 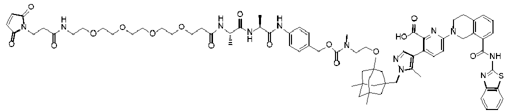 |
| 2.4 | K | 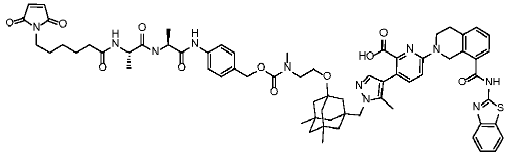 |
| 2.5 | L | 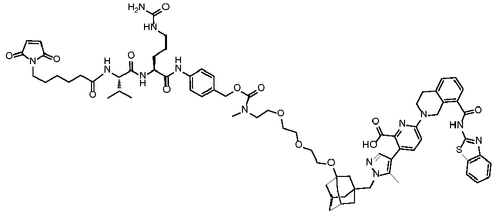 |
| 2.6 | M | 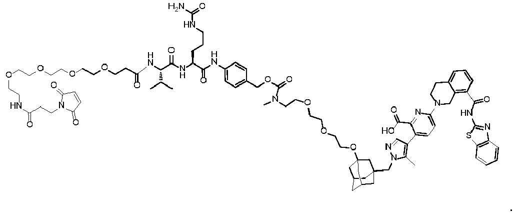 |
| 2.7 | V | 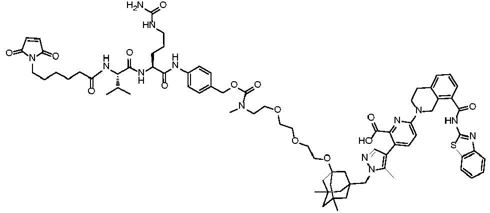 |
| 2.8 | DS | 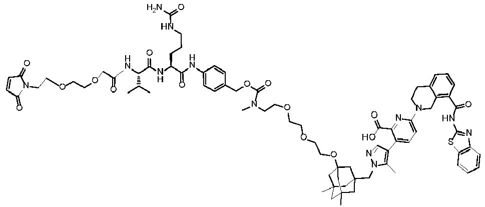 |
| 2.10 | BG | 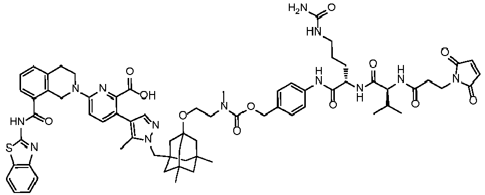 |
| 2.12 | BI | 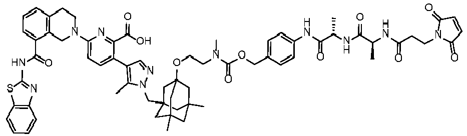 |
| 2.17 | BO | 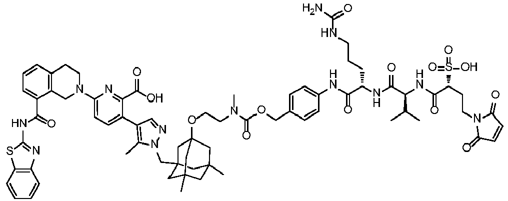 |
| 2.18 | BP | 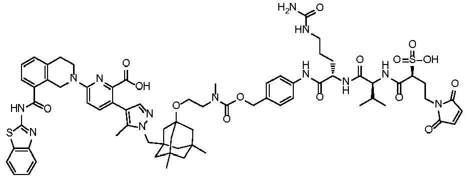 |
| 2.21 | IQ | 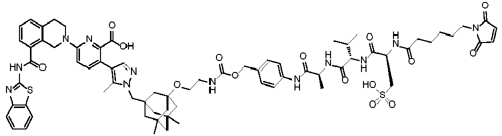 |
| 2.22 | DB | 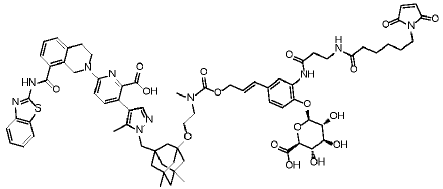 |
| 2.23 | DM | 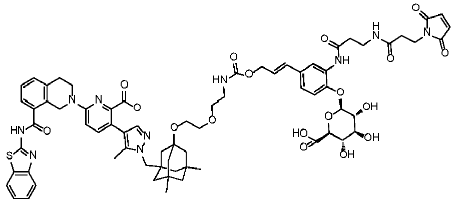 |
| 2.24 | DL | 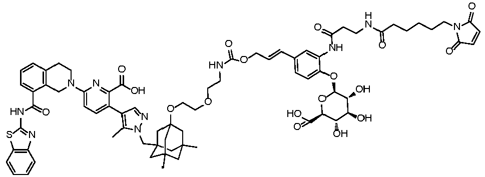 |
| 2.25 | DR | 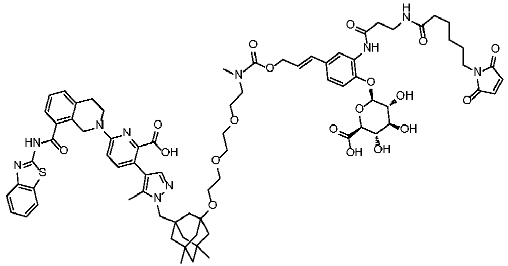 |
| 2.26 | DZ | 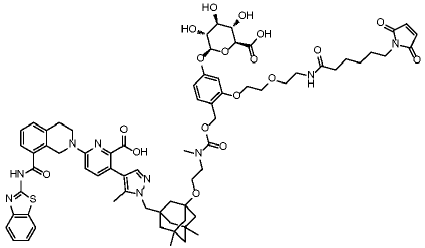 |
| 2.27 | EA | 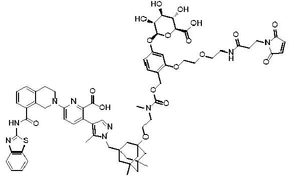 |
| 2.28 | EO | 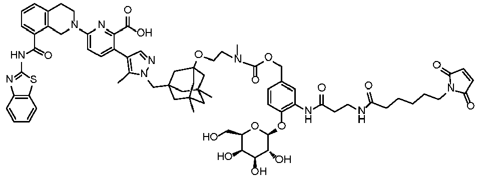 |
| 2.29 | FB | 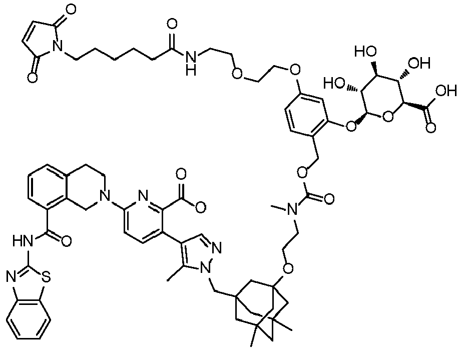 |
| 2.30 | KX | 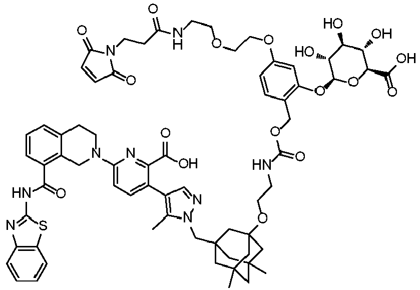 |
| 2.31 | FF | 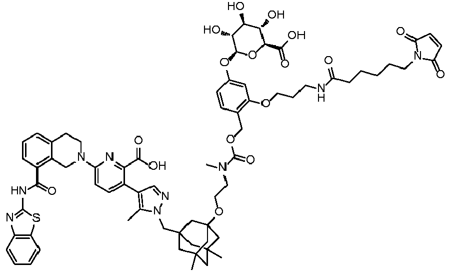 |
| 2.32 | FU | 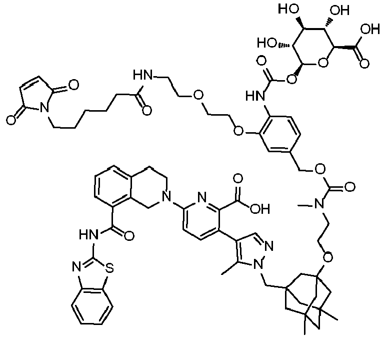 |
| 2.33 | GH | 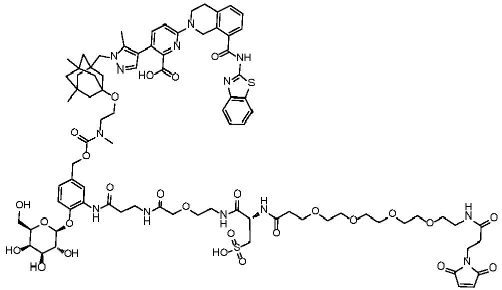 |
| 2.34 | FX | 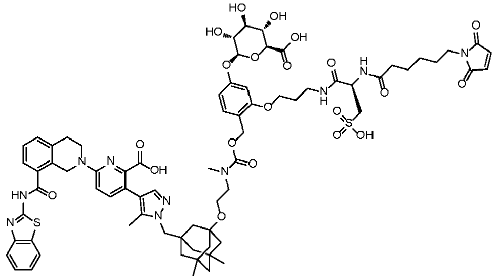 |
| 2.35 | H | 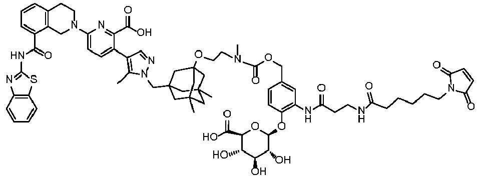 |
| 2.36 | I | 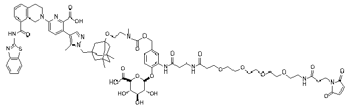 |
| 2.37 | KQ | 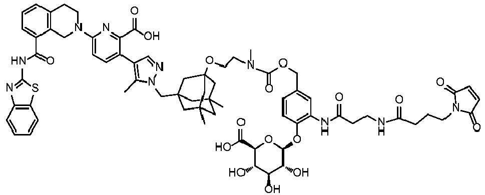 |
| 2.37 | KP | 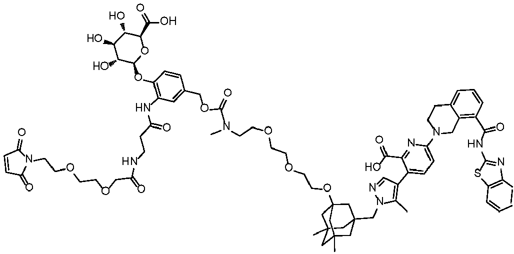 |
| 2.39 | HA | 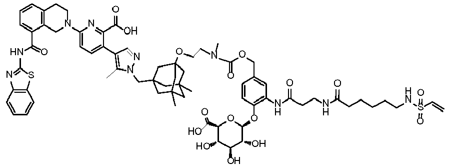 |
| 2.40 | HB | 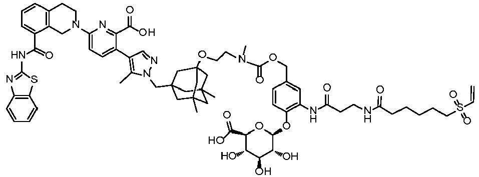 |
| 2.41 | LB | 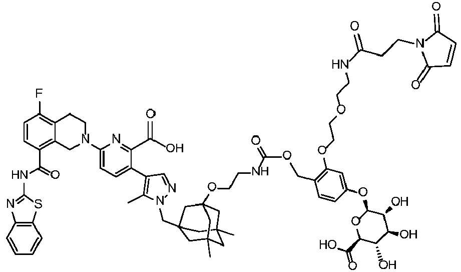 |
| 2.42 | NF | 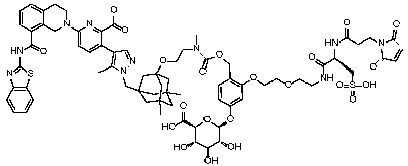 |
| 2.43 | NG | 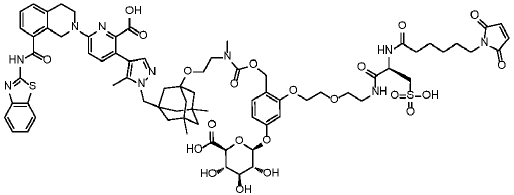 |
| 2.44 | AS | 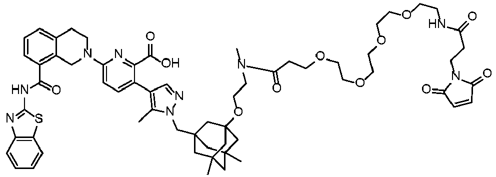 |
| 2.45 | AT | 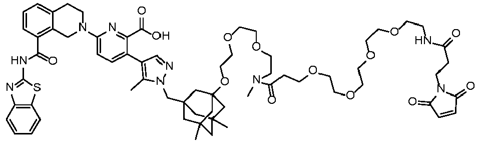 |
| 2.46 | AU | 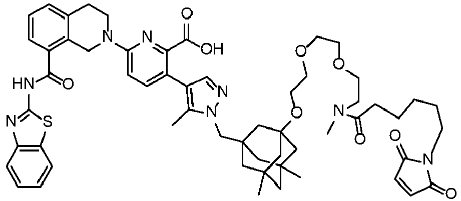 |
| 2.47 | BK | 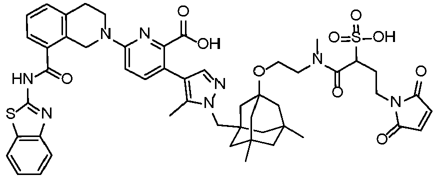 |
| 2.48 | BQ | 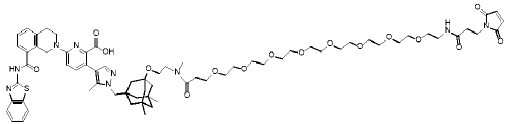 |
| 2.49 | BR | 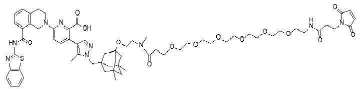 |
| 2.50 | OI | 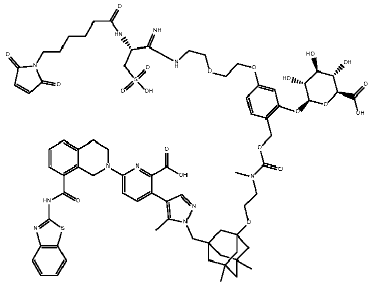 |
| 2.51 | NX | 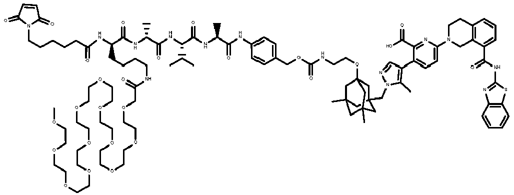 |
| 2.52 | OJ | 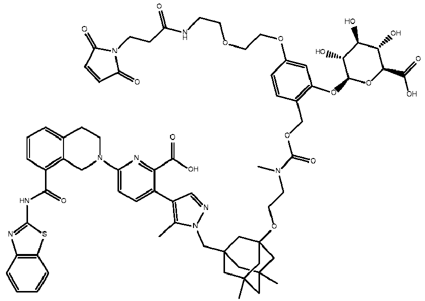 |
| 2.53 | XY | 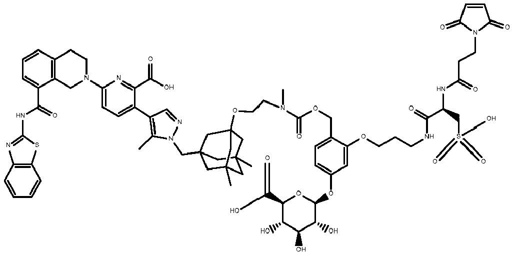 |

### 5.6 ANTIBODY DRUG CONJUGATES

Bcl-xL inhibitory activity of ADCs described herein may be confirmed in cellular assays with appropriate target cells and/or *in vivo* assays. Specific assays that may be used to confirm activity of ADCs that target EGFR, EpCAM or NCAM1 are provided in Examples 7 and 8. Generally, ADCs will exhibit an EC₅₀ of less than about 5000 nM in such a cellular assay, although the ADCs may exhibit significantly lower EC₅₀s, for example, less than about 500, 300, or even 100 nM. Similar cellular assays with cells expressing specific target antigens may be used to confirm the Bcl-xL inhibitory activity of ADCs targeting other antigens.

### 5.7 METHODS OF SYNTHESIS

The Bcl-xL inhibitors and synthons described herein may be synthesized using standard, known techniques of organic chemistry. General schemes for synthesizing Bcl-xL inhibitors and synthons that may be used as-is or modified to synthesize the full scope of Bcl-xL inhibitors and synthons described herein are provided below. Specific methods for synthesizing exemplary Bcl-xL inhibitors and synthons that may be useful for guidance are provided in the Examples section.

ADCs may likewise be prepared by standard methods, such as methods analogous to those described in Hamblett et al., 2004, "Effects of Drug Loading on the Antitumor Activity of a Monoclonal Antibody Drug Conjugate," Clin. Cancer Res. 10:7063-7070; Doronina et al., 2003, "Development of potent and highly efficacious monoclonal antibody auristatin conjugates for cancer therapy," Nat. Biotechnol. 21(7):778-784; and Francisco et al., 2003, "cAClO-vcMMAE, an anti-CD30-monomethylauristatin E conjugate with potent and selective antitumor activity," Blood 102:1458-1465. For example, ADCs with four drugs per antibody may be prepared by partial reduction of the antibody with an excess of a reducing reagent such as DTT or TCEP at 37 °C for 30 minutes, then the buffer exchanged by elution through SEPHADEX® G-25 resin with 1 mM DTPA in DPBS. The eluent is diluted with further DPBS, and the thiol concentration of the antibody may be measured using 5,5'-dithiobis(2-nitrobenzoic acid) [Ellman's reagent]. An excess, for example 5-fold, of a linker-drug synthon is added at 4 °C for 1 hour, and the conjugation reaction may be quenched by addition of a substantial excess, for example 20-fold, of cysteine. The resulting ADC mixture may be purified on SEPHADEX G-25 equilibrated in PBS to remove unreacted synthons, desalted if desired, and purified by size-exclusion chromatography. The resulting ADC may then be then sterile filtered, for example, through a 0.2 µm filter, and lyophilized if desired for storage. In certain embodiments, all of the interchain cysteine disulfide bonds are replaced by linker-drug conjugates.

Specific methods for synthesizing exemplary ADCs that may be used to synthesize the full range of ADCs described herein are provided in the Examples section.

### 5.7.1 GENERAL METHODS FOR SYNTHESIZING BCL-XL INHIBITORS

### 5.7.1.1 SYNTHESIS OF COMPOUND (9)

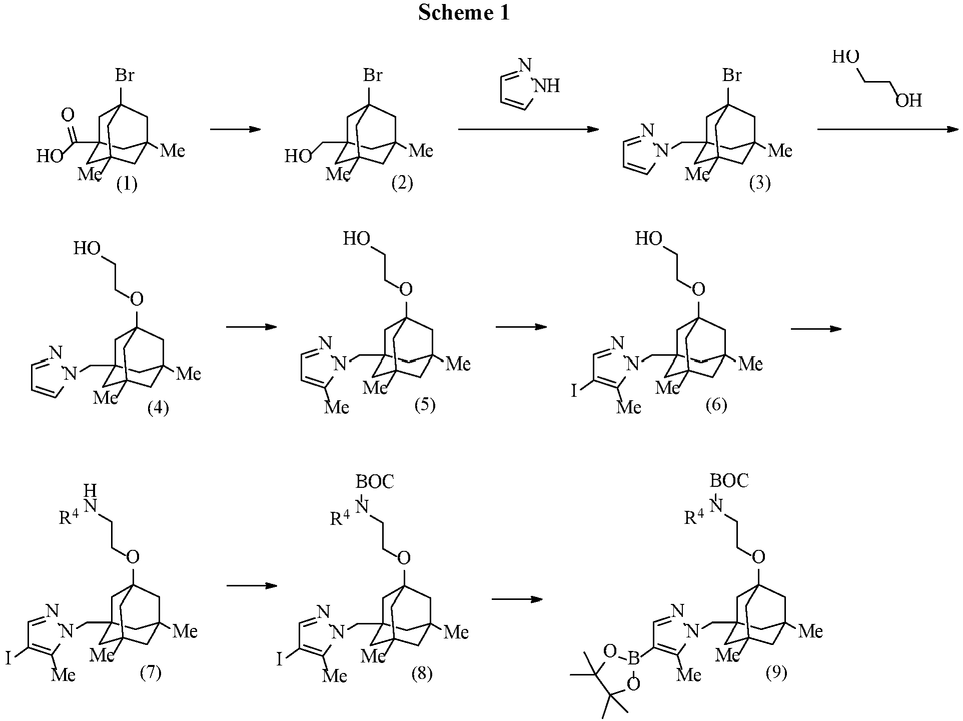

The synthesis of pyrazole intermediate, formula (9), is described in Scheme 1. 3-Bromo-5,7-dimethyladamantanecarboxylic acid (1) can be treated with BH₃·THF to provide 3-bromo-5,7-dimethyladamantanemethanol (2). The reaction is typically performed at ambient temperature in a solvent, such as, but not limited to, tetrahydrofuran. 1-((3-Bromo-5,7- dimethyltricy-clo[3.3.1.1^{3,7}]dec-1-yl)methyl)-1H-pyrazole (3) can be prepared by treating 3-bromo-5,7-dimethyladamantanemethanol (2) with 1H-pyrazole in the presence of cyanomethylenetributylphosphorane. The reaction is typically performed at an elevated temperature in a solvent such as, but not limited to, toluene. 1-((3-Bromo-5,7-dimethyltricyclo[3.3.1.1^{3,7}]dec-1-yl)methyl)-1H-pyrazole (3) can be treated with ethane-1,2-diol in the presence of a base such as, but not limited to, triethylamine, to provide 2-{[3,5-dimethyl-7-(1*H*-pyrazol-1-ylmethyl)tricyelo[3.3.1.1^{3,7}]dec-1-yl]oxy}ethanol (4). The reaction is typically performed at an elevated temperature, and the reaction may be performed under microwave conditions. 2-1[3,5-Dimethyl-7-(1*H*-pyrazol-1-ylmethyl)tricyclo[3.3.1.1^{3,7}]dec-1-yl]oxy}ethanol (4) can be treated with a strong base, such as, but not limited to, n-butyllithium, followed by the addition of iodomethane, to provide 2-({3,5-dimethyl-7-[(5-methyl-1*H*-pyrazol-1-yl)methyl]tricyelo[3.3.1.1^{3,7}]dec-1-yl}oxy)ethanol (5). The addition and reaction is typically performed in a solvent such as, but not limited to, tetrahydrofuran, at a reduced temperature before warming up to ambient temperature for work up. 2-({3,5-Dimethyl-7-[(5-methyl-1*H*-pyrazol-1-yl)methyl]tricyclo[3.3.1.1^{3,7}]dec-1-yl}oxy)ethanol (5) can be treated with N-iodosuccinimide to provide 1-({3,5-dimethyl-7-[2-(hydroxy)ethoxy]tricyclo[3.3.1.1^{1,7}]dec-1-yl}methyl)-4-iodo-5-methyl-1H-pyrazole (6). The reaction is typically performed at ambient temperature is a solvent such as, but not limited to, N,N-dimethylformamide. Compounds of formula (7) can be prepared by reacting 1-({3,5-dimethyl-7-[2-(hydroxy)ethoxy]tricyclo[3.3.1.1^{3,7}]dec-1-yl}methyl)-4-iodo-5-methyl-1H-pyrazole (6) with methanesulfonyl chloride, in the presence of a base such as, but not limited to, triethylamine, followed by the addition of amine, H₂NR⁴. The reaction with methanesulfonyl chloride is typically performed at low temperature, before increasing the temperature for the reaction with the amine, and the reaction is typically performed in a solvent such as, but not limited to tetrahydrofuran. Compounds of formula (7) can be reacted with di-tert-butyl dicarbonate in the presence of 4-dimethylaminopyridine to provide compounds of formula (8). The reaction is typically performed at ambient temperature in a solvent such as, but not limited to tetrahydrofuran. The borylation of compounds of formula (8) to provide compounds of formula (9) can be performed under conditions described herein and readily available in the literature.

### 5.7.1.2 SYNTHESIS OF COMPOUND (14)

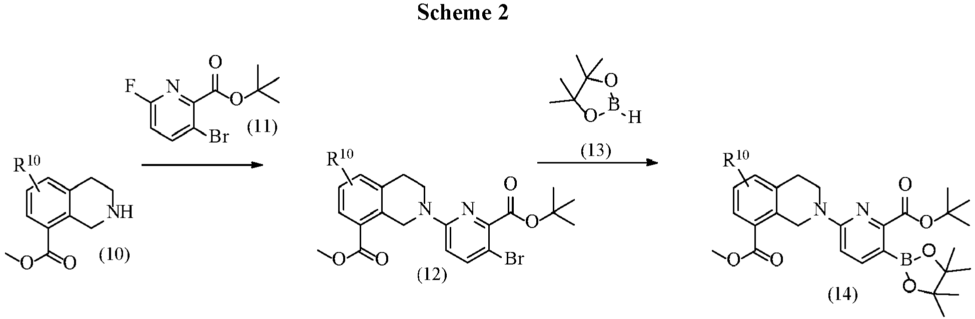

The synthesis of intermediate, formula (14), is described in Scheme 2. Compounds of formula (12) can be prepared by reacting compounds of formula (10), with *tert*-butyl 3-bromo-6-fluoropicolinate (11) in the presence of a base, such as, but not limited to, N,N-diisopropylethylamine, or triethylamine. The reaction is typically performed under an inert atmosphere at an elevated temperature in a solvent, such as, but not limited to, dimethyl sulfoxide. Compounds of formula (12) can be reacted with 4,4,5,5-tetramethyl-1,3,2-dioxaborolane (13), under borylation conditions described herein or in the literature to provide compounds of formula (14).

### 5.7.1.3 SYNTHESIS OF COMPOUND (24)

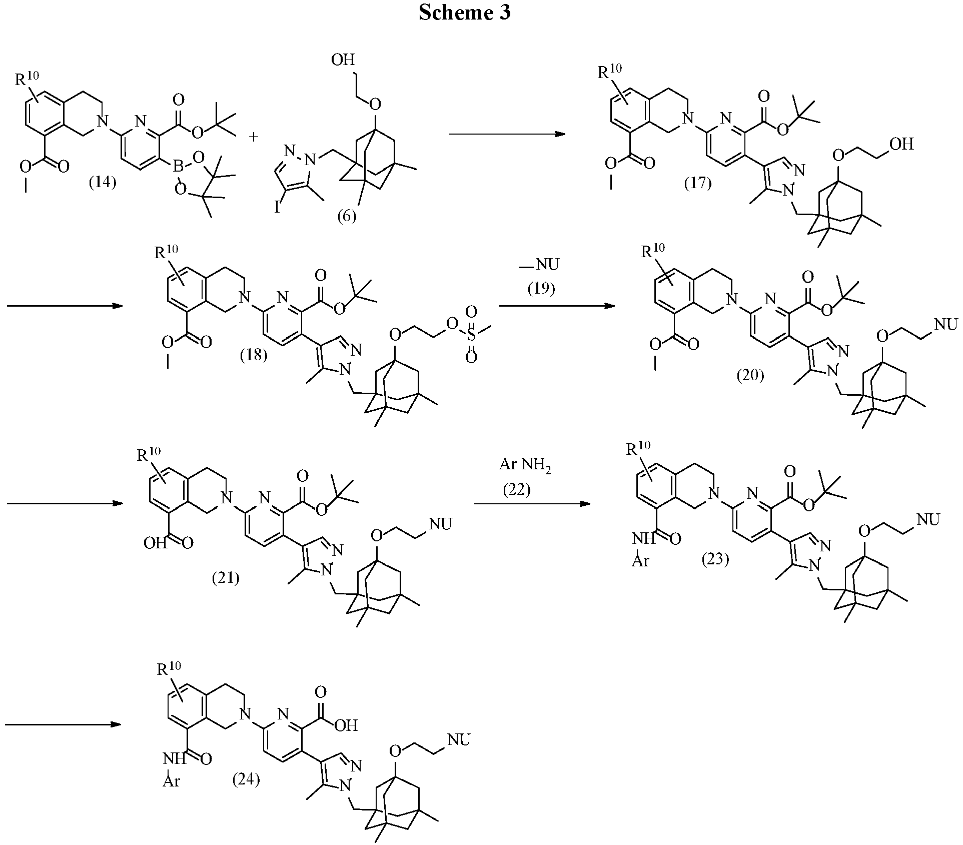

Scheme 3 describes a method to make intermediates that contain -Nu (nucleophile) tethered to an adamantane and picolinate protected as a t-butyl ester. Methyl 2-(6-(*tert-*butoxycarbonyl)-5-(4,4,5,5-tetramethyl-1,3,2-dioxaborolan-2-yl)pyridin-2-yl)-1,2,3,4-tetrahydroisoquinoline-8-carboxylate (14) can be reacted with 1-({3,5-dimethyl-7-[2-(hydroxy)ethoxy]tricyclo[3.3.1.1^{3,7}]dec-1-yl}methyl)-4-iodo-5-methyl-1H-pyrazole (6) under Suzuki Coupling conditions described herein or in the literature to provide methyl 2-(6-(*tert-*butoxycarbonyl)-5-(1-((3-(2-hydroxyethoxy)-5,7-dimethyladamantan-1-yl)methyl)-5-methyl-1H-pyrazol-4-yl)pyridin-2-yl)-1,2,3,4-tetrahydroisoquinoline-8-carboxylate (17). Methyl 2-(6-(*tert-*butoxycarbonyl)-5-(1-((3-(2-hydroxyethoxy)-5,7-dimethyladamantan-1-yl)methyl)-5-methyl-1H-pyrazol-4-yl)pyridin-2-yl)-1,2,3,4-tetrahydroisoquinoline-8-carboxylate (17) can be treated with a base such as but not limited to triethylamine, followed by methanesulfonyl chloride to provide methyl 2-(6-(*tert*-butoxycarbonyl)-5-(1-((3,5-dimethyl-7-(2-((methylsulfonyl)oxy)ethoxy)adamantan-1-yl)methyl)-5-methyl-1H-pyrazol-4-yl)pyridin-2-yl)-1,2,3,4-tetrahydroisoquinoline-8-carboxylate (18). The addition is typically performed at low temperature before warming up to ambient temperature in a solvent, such as, but not limited to, dichloromethane. Methyl 2-(6-(*tert*-butoxycarbonyl)-5-(1-((3,5-dimethyl-7-(2-((methylsulfonyl)oxy)ethoxy)adamantan-1-yl)methyl)-5-methyl-1H-pyrazol-4-yl)pyridin-2-yl)-1,2,3,4-tetrahydroisoquinoline-8-carboxylate (18) can be reacted with a nucleophile (Nu) of formula (19) to provide compounds of formula (20). Examples of nucleophiles include, but are not limited to, sodium azide, methylamine, ammonia and di-tert-butyl iminodicarbonate. Compounds of formula (20) can be reacted with lithium hydroxide to provide compounds of formula (21). The reaction is typically performed at ambient temperature in a solvent such as but not limited to tetrahydrofuran, methanol, water, or mixtures thereof. Compounds of formula (21) can be reacted with compounds of formula (22), wherein Ar is as described herein, under amidation conditions described herein or readily available in the literature to provide compounds of formula (23). Compounds of the formula (23) can be treated with acids, such as trifluoroacetic acid or HCl, in solvents, such as but not limited to dichloromethane or dioxane, to provide compounds of the formula (24).

### 5.7.1.4 SYNTHESIS OF COMPOUND (34)

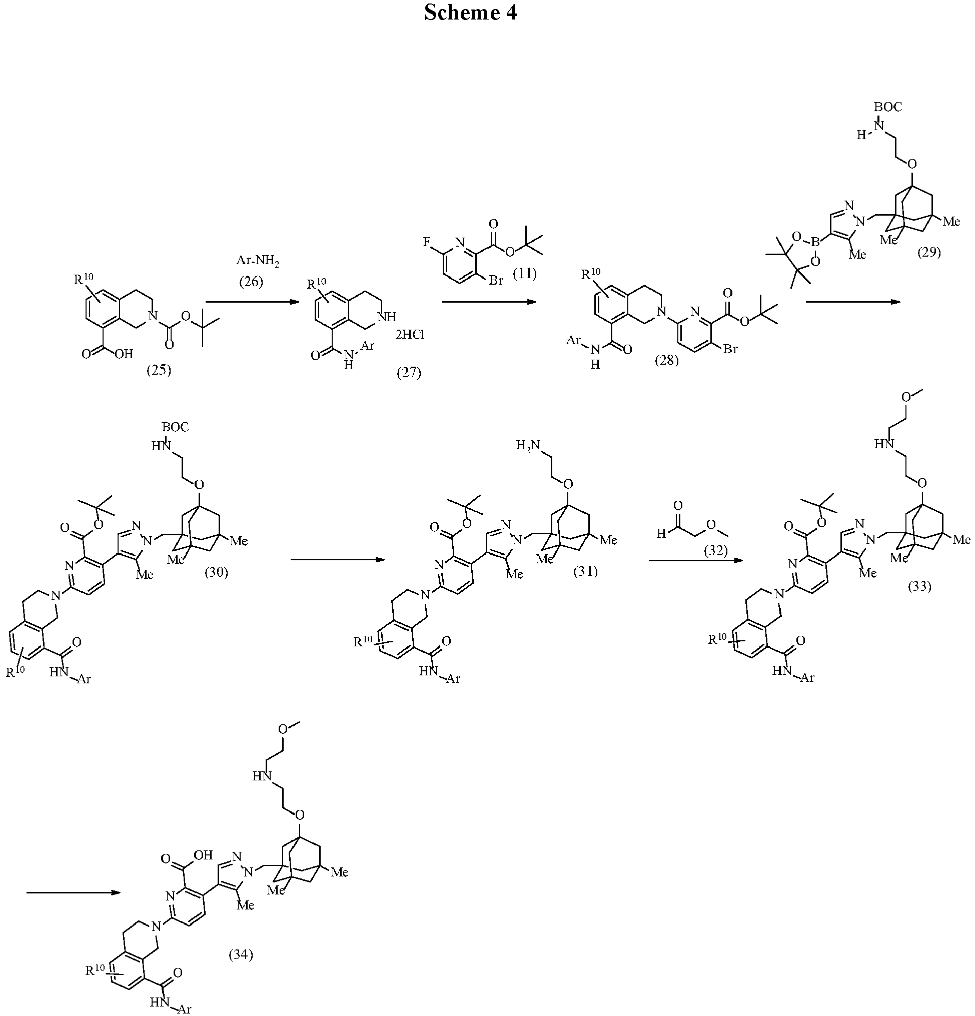

The synthesis of compound (34) is described in Scheme 4. Compounds of formula (25) can be reacted with compounds of formula (26), wherein Ar is as described herein, under amidation conditions described herein or readily available in the literature to provide compounds of formula (27). Compounds of formula (27) can be reacted with *tert*-butyl 3-bromo-6-fluoropicolinate (11) in the presence of a base such as, but not limited to, cesium carbonate, to provide compounds of formula (28). The reaction is typically performed at elevated temperature in a solvent such as, but not limited to, N,N-dimethylacetamide. Compounds of formula (30) can be prepared by reacting compounds of formula (28) with a boronate ester (or the equivalent boronic acid) of formula (29) under Suzuki Coupling conditions described herein or in the literature. Compounds of formula (31) can be prepared by treating compounds of formula (30) with trifluoroacetic acid. The reaction is typically performed at ambient temperature in a solvent such as but not limited to dichloromethane. Compounds of formula (31) can be reacted with 2-methoxyacetaldehyde (32) followed by a reducing agent such as, but not limited to, sodium borohydride, to provide compounds of formula (33). The reaction is typically performed at ambient temperature in a solvent such as, but not limited to, dichloromethane, methanol, or mixtures thereof. Compounds of the formula (33) can be treated with acids, such as trifluoroacetic acid or HCl, in solvents, such as but not limited to dichloromethane or dioxane, to provide compounds of the formula (34).

### 5.7.2 GENERAL METHODS FOR SYNTHESIZING SYNTHONS

In the following schemes, the variable Ar² represents 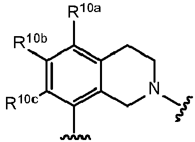 in the compound of formula (IIa) and the variable Ar¹ represents 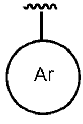 in the compound of formula (iia).

### 5.7.2.1 Synthesis of Compound (89)

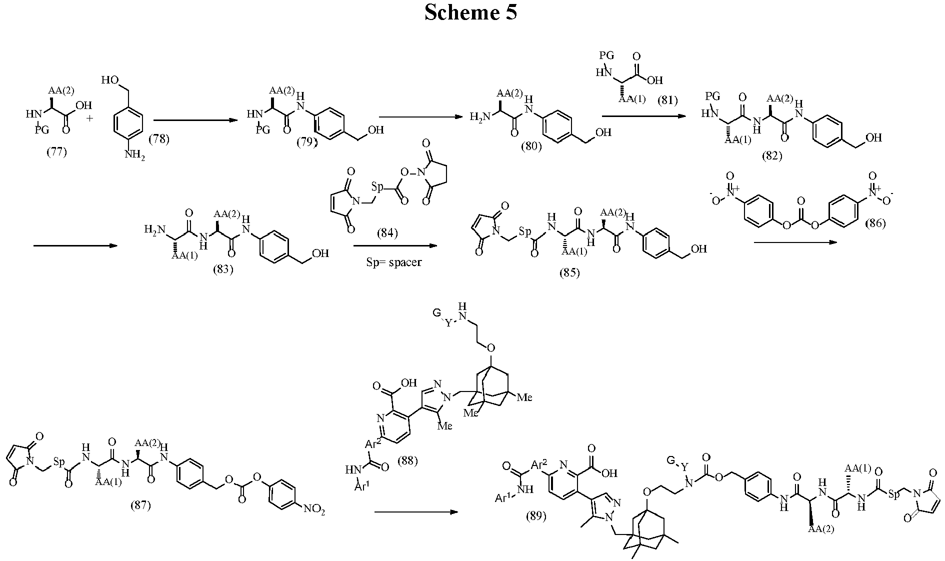

As shown in scheme 5, compounds of formula (77), wherein PG is an appropriate base labile protecting group and AA(2) is Cit, Ala, or Lys, can be reacted with 4-(aminophenyl)methanol (78), under amidation conditions described herein or readily available in the literature to provide compound (79). Compound (80) can be prepared by reacting compound (79) with a base such as, but not limited to, diethylamine. The reaction is typically performed at ambient temperature in a solvent such as but not limited to N,N-dimethylformamide. Compound (81), wherein PG is an appropriate base or acid labile protecting group and AA(1) is Val or Phe, can be reacted with compound (80), under amidation conditions described herein or readily available in the literature to provide compound (82). Compound (83) can be prepared by treating compound (82) with diethylamine or trifluoroacetic acid, as appropriate. The reaction is typically performed at ambient temperature in a solvent such as but not limited to dichloromethane. Compound (84), wherein Sp is a spacer, can be reacted with compound (83) to provide compound (85). The reaction is typically performed at ambient temperature in a solvent such as but not limited to N,N-dimethylformamide. Compound (85) can be reacted with bis(4-nitrophenyl) carbonate (86) in the presence of a base such as, but not limited to N,N-diisopropylethylamine, to provide compounds (87). The reaction is typically performed at ambient temperature in a solvent such as but not limited to N,N-dimethylformamide. Compounds (87) can be reacted with compound (88) in the presence of a base such as, but not limited to, N,N-diisopropylethylamine, to provide compound (89). The reaction is typically performed at ambient temperature in a solvent such as, but not limited to, N,N-dimethylformamide.

### 5.7.2.2 Synthesis of Compounds (94) and (96)

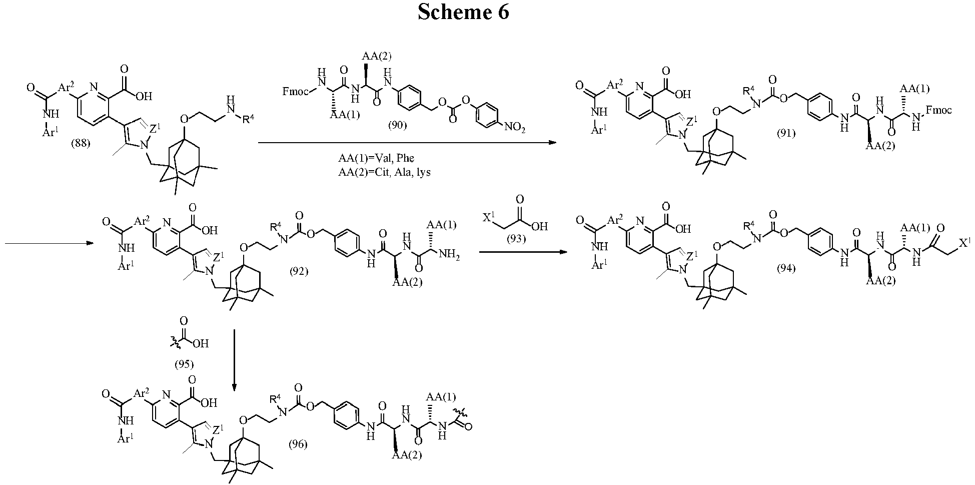

Scheme 6 describes the installment of alternative mAb-linker attachments to dipeptide synthons. Compound (88) can be reacted with compound (90) in the presence of a base such as, but not limited to, N,N-diisopropylethylamine, to provide compound (91). The reaction is typically performed at ambient temperature in a solvent such as but not limited to N,N-dimethylformamide. Compound (92) can be prepared by reacting compound (91) with diethylamine. The reaction is typically performed at ambient temperature in a solvent such as but not limited to N,N-dimethylformamide. Compound (93), wherein X¹ is Cl, Br, or I, can be reacted with compound (92), under amidation conditions described herein or readily available in the literature to provide compound (94). Compound (92) can be reacted with compounds of formula (95) under amidation conditions described herein or readily available in the literature to provide compound (96).

### 5.7.2.3 Synthesis of Compound (106)

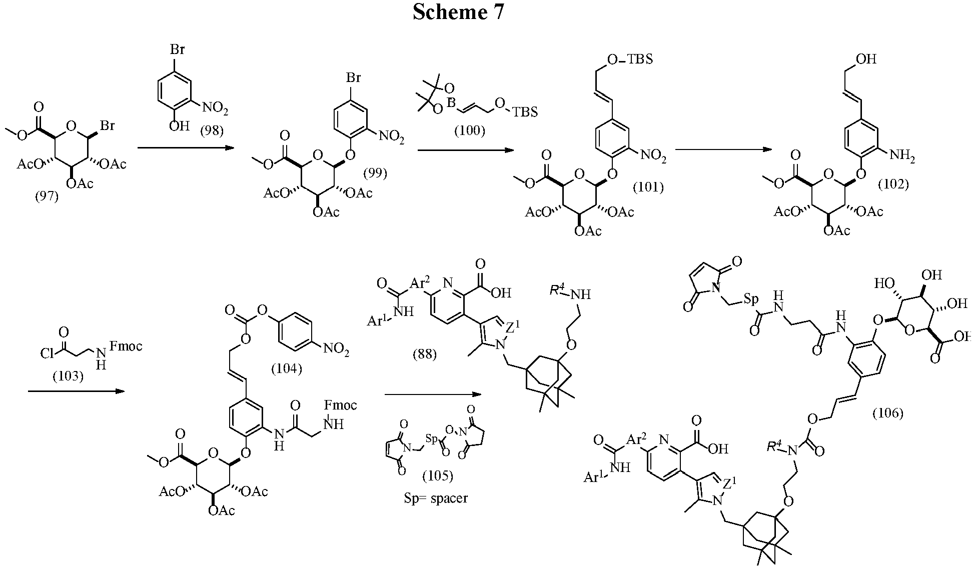

Scheme 7 describes the synthesis of vinyl glucuronide linker intermediates and synthons. (2R,3R,4S,5S,6S)-2-Bromo-6-(methoxycarbonyl)tetrahydro-2H-pyran-3,4,5-triyl triacetate (97) can be treated with silver oxide, followed by 4-bromo-2-nitrophenol (98) to provide (2S,3R,4S,5S,6S)-2-(4-bromo-2-nitrophenoxy)-6-(methoxycarbonyl)tetrahydro-2H-pyran-3,4,5-triyl triacetate (99). The reaction is typically performed at ambient temperature in a solvent, such as, but not limited to, acetonitrile. (2S,3R,4S,5S,6S)-2-(4-Bromo-2-nitrophenoxy)-6-(methoxycarbonyl)tetrahydro-2H-pyran-3,4,5-triyl triacetate (99) can be reacted with (E)-*tert*-butyldimethyl((3-(4,4,5,5-tetramethyl-1,3,2-dioxaborolan-2-yl)allyl)oxy)silane (100) in the presence of a base such as, but not limited to, sodium carbonate, and a catalyst such as but not limited to tris(dibenzylideneacetone)dipalladium (Pd₂(dba)₃), to provide (2S,3R,4S,5S,6S)-2-(4-((E)-3-((*tert*-butyldimethylsilyl)oxy)prop-1-en-1-yl)-2-nitrophenoxy)-6-(methoxycarbonyl)tetrahydro-2H-pyran-3,4,5-triyl triacetate (101). The reaction is typically performed at an elevated temperature in a solvent, such as, but not limited to, tetrahydrofuran. (2S,3R,4S,5S,6S)-2-(2-amino-4-((E)-3-hydroxyprop-1-en-1-yl)phenoxy)-6-(methoxycarbonyl)tetrahydro-2H-pyran-3,4,5-triyl triacetate (102) can be prepared by reacting (2S,3R,4S,5 S,6S)-2-(4-((E)-3-((*tert*-butyldimethylsilyl)oxy)prop-1-en-1-yl)-2-nitrophenoxy)-6-(methoxycarbonyl)tetrahydro-2H-pyran-3,4,5-triyl triacetate (101) with zinc in the presence of an acid such as, but not limited to, hydrochloric acid. The addition is typically performed at low temperature before warming to ambient temperature in a solvent such as, but not limited to, tetrahydrofuran, water, or mixtures thereof. (2S,3R,4S,5S,6S)-2-(2-amino-4-((E)-3-hydroxyprop-1-en-1-yl)phenoxy)-6-(methoxycarbonyl)tetrahydro-2H-pyran-3,4,5-triyl triacetate (102) can be reacted with (9H-fluoren-9-yl)methyl (3-chloro-3-oxopropyl)carbamate (103), in the presence of a base such as, but not limited to, N,N-diisopropylethylamine, to provide (2S,3R,4S,5S,6S)-2-(2-(3-((((9H-fluoren-9-yl)methoxy)carbonyl)amino)propanamido)-4-((E)-3-hydroxyprop-1-en-1-yl)phenoxy)-6-(methoxycarbonyl)tetrahydro-2H-pyran-3,4,5-triyl triacetate (104). The addition is typically performed at low temperature before warming to ambient temperature in a solvent such as, but not limited to, dichloromethane. Compound (88) can be reacted with (2S,3R,4S,5S,6S)-2-(2-(3-((((9H-fluoren-9-yl)methoxy)carbonyl)amino)propanamido)-4-((E)-3-hydroxyprop-1-en-1-yl)phenoxy)-6-(methoxycarbonyl)tetrahydro-2H-pyran-3,4,5-triyl triacetate (104) in the presence of a base such as, but not limited to, N,N-diisopropylethylamine, followed by work up and reaction with compound of formula (105) in the presence of a base such as, but not limited to, N,N-diisopropylethylamine to provide compound (106). The reactions are typically performed at ambient temperature in a solvent such as, but not limited to N,N- dimethylformamide.

### 5.7.2.4 Synthesis of Compound (115)

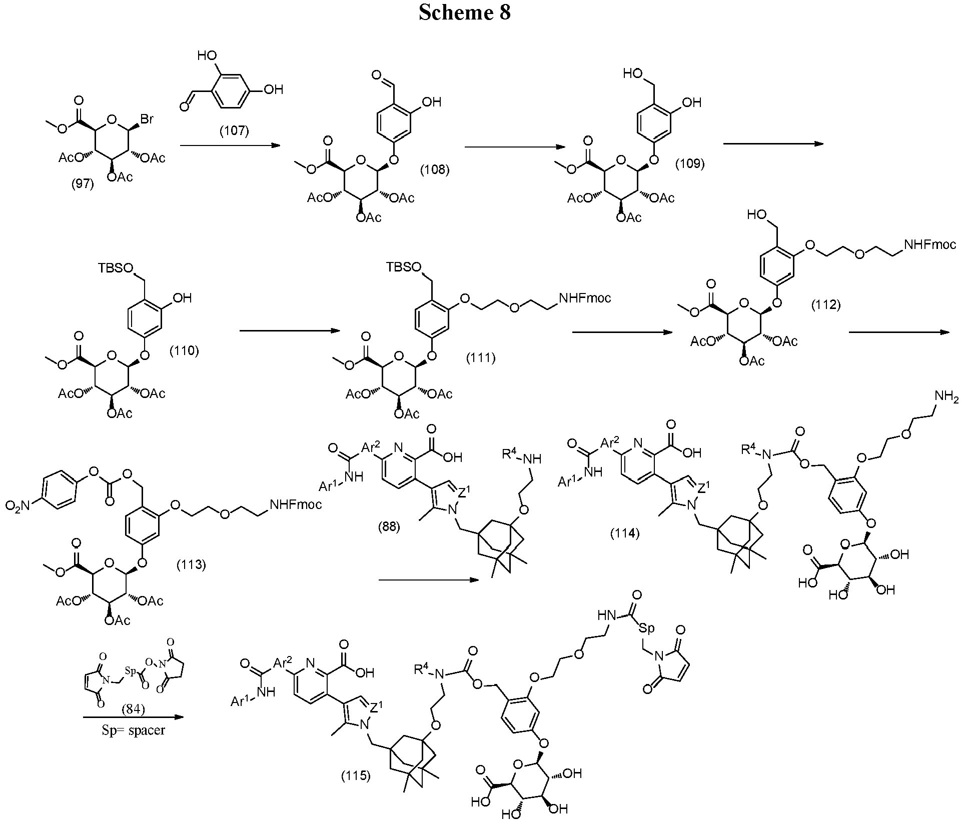

Scheme 8 describes the synthesis of a representative 2-ether glucuronide linker intermediate and synthon. (2S,3R,4S,5S,6S)-2-Bromo-6-(methoxycarbonyl)tetrahydro-2H-pyran-3,4,5-triyl triacetate (97) can be reacted with 2,4-dihydroxybenzaldehyde (107) in the presence of silver carbonate to provide (2S,3R,4S,5S,6S)-2-(4-formyl-3-hydroxyphenoxy)-6-(methoxycarbonyl)tetrahydro-2H-pyran-3,4,5-triyl triacetate (108). The reaction is typically performed at an elevated temperature in a solvent, such as, but not limited to, acetonitrile. (2S,3R,4S,5S,6S)-2-(4-Formyl-3-hydroxyphenoxy)-6-(methoxycarbonyl)tetrahydro-2H-pyran-3,4,5-triyl triacetate (108) can be treated with sodium borohydride to provide (2S,3R,4S,5S,6S)-2-(3-hydroxy-4-(hydroxymethyl)phenoxy)-6-(methoxycarbonyl)tetrahydro-2H-pyran-3,4,5-triyl triacetate (109). The addition is typically performed at low temperature before warming to ambient temperature in a solvent such as but not limited to tetrahydrofuran, methanol, or mixtures thereof. (2S,3R,4S,5S,6S)-2-(4-(((*tert*-butyldimethylsilyl)oxy)methyl)-3-hydroxyphenoxy)-6-(methoxycarbonyl)tetrahydro-2H-pyran-3,4,5-triyl triacetate (110) can be prepared by reacting (2S,3R,4S,5S,6S)-2-(3-hydroxy-4-(hydroxymethyl)phenoxy)-6-(methoxycarbonyl)tetrahydro-2H-pyran-3,4,5-triyl triacetate (109) with *tert*-butyldimethylsilyl chloride in the presence of imidazole. The reaction is typically performed at low temperature in a solvent, such as, but not limited to, dichloromethane. (2S,3R,4S,5S,6S)-2-(3-(2-(2-((((9H-Fluoren-9-yl)methoxy)carbonyl)amino)ethoxy)ethoxy)-4-(((*tert*-butyldimethylsilyl)oxy)methyl)phenoxy)-6-(methoxycarbonyl)tetrahydro-2H-pyran-3,4,5-triyl triacetate (111) can be prepared by reacting (2S,3R,4S,5S,6S)-2-(4-(((*tert*-butyldimethylsilyl)oxy)methyl)-3-hydroxyphenoxy)-6-(methoxycarbonyl)tetrahydro-2H-pyran-3,4,5-triyl triacetate (110) with (9H-fluoren-9-yl)methyl (2-(2-hydroxyethoxy)ethyl)carbamate in in the presence of triphenylphosphine and a azodicarboxylate such as, but not limited to, di-*tert*-butyl diazene-1,2-dicarboxylate. The reaction is typically performed at ambient temperature in a solvent such as but not limited to toluene. (2S,3R,4S,5S,6S)-2-(3-(2-(2-((((9H-Fluoren-9-yl)methoxy)carbonyl)amino)ethoxy)ethoxy)-4-(((*tert-*butyldimethylsilyl)oxy)methyl)phenoxy)-6-(methoxycarbonyl)tetrahydro-2H-pyran-3,4,5-triyl triacetate (111) can be treated with acetic acid to provide (2S,3F,4S,5S,6S)-2-(3-(2-(2-((((9H-fluoren-9-yl)methoxy)carbonyl)amino)ethoxy)ethoxy)-4-(hydroxymethyl)phenoxy)-6-(methoxycarbonyl)tetrahydro-2H-pyran-3,4,5-triyl triacetate (112). The reaction is typically performed at ambient temperature in a solvent such as but not limited to water, tetrahydrofuran, or mixtures thereof. (2S,3R,4S,5S,6S)-2-(3-(2-(2-((((9H-Fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyl triacetate (113) can be prepared by reacting (2S,3R,4S,5S,6S)-2-(3-(2-(2-((((9H-fluoren-9-yl)methoxy)carbonyl)amino)ethoxy)ethoxy)-4-(hydroxymethyl)phenoxy)-6-(methoxycarbonyl)tetrahydro-2H-pyran-3,4,5-triyl triacetate (112) with bis(4-nitrophenyl) carbonate in the presence of a base such as but not limited to N,N-diisopropylethylamine. The reaction is typically performed at ambient temperature in a solvent such as but not limited to N,N-dimethylformamide. (2S,3R,4S,5S,6S)-2-(3-(2-(2-((((9H-Fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyl triacetate (113) can be treated with compound (88) in the presence of a base such as but not limited to N,N-diisopropylethylamine, followed by treatment with lithium hydroxide to provide a compound (114). The reaction is typically performed at ambient temperature in a solvent such as but not limited to N,N-dimethylformamide, tetrahydrofuran, methanol, or mixtures thereof. Compound (115) can be prepared by reacting compound (114) with compound (84) in the presence of a base such as but not limited to N,N-diisopropylethylamine. The reaction is typically performed at ambient temperature in a solvent such as but not limited to N,N-dimethylformamide.

### 5.7.2.5 Synthesis of Compound (119)

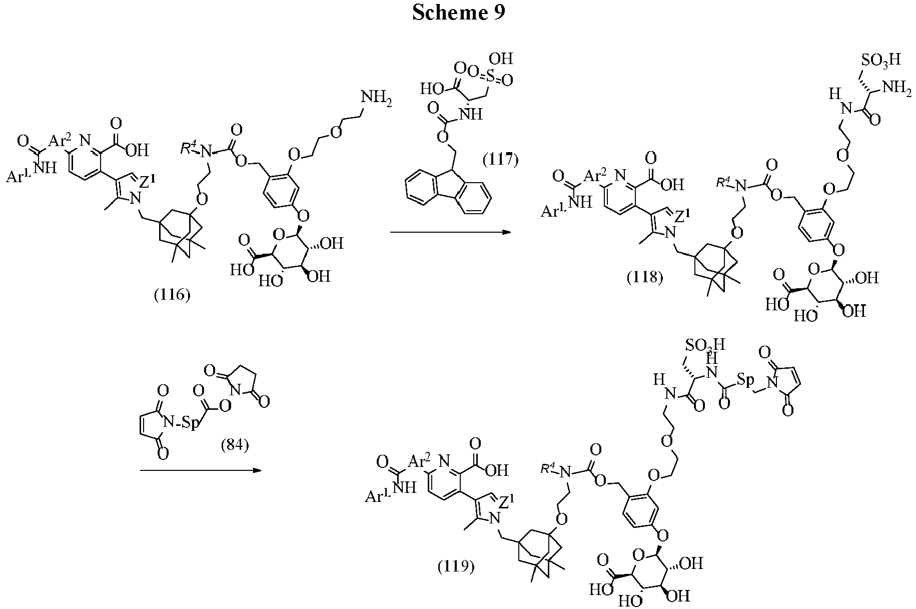

Scheme 9 describes the introduction of a second solubilizing group to a sugar linker. Compound (116) can be reacted with (R)-2-((((9H-fluoren-9-yl)methoxy)carbonyl)amino)-3-sulfopropanoic acid (117), under amidation conditions described herein or readily available in the literature, followed by treatment with a base such as but not limited to diethylamine, to provide compound (118). Compound (118) can be reacted with compound (84), wherein Sp is a spacer, under amidation conditions described herein or readily available in the literature, to provide compound (119).

### 5.7.2.6 Synthesis of Compound (129)

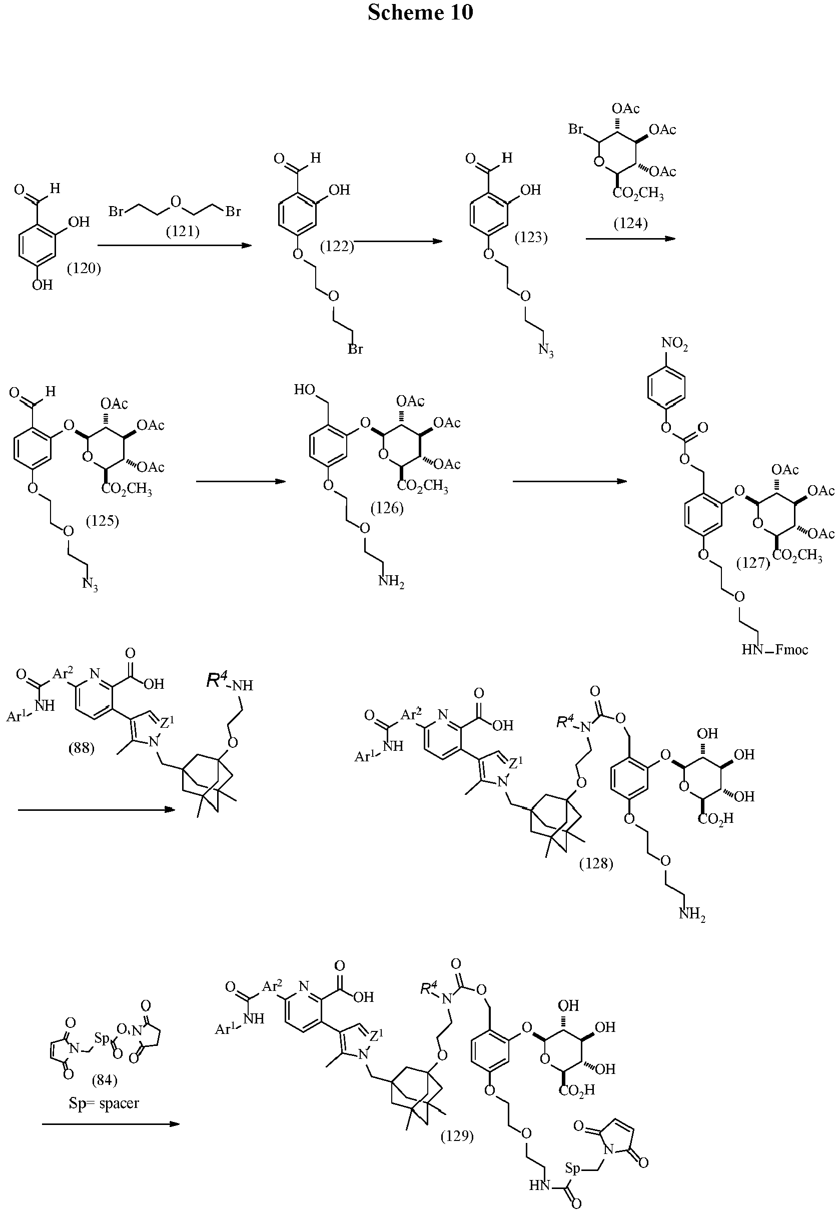

Scheme 10 describes the synthesis of 4-ether glucuronide linker intermediates and synthons. 4-(2-(2-Bromoethoxy)ethoxy)-2-hydroxybenzaldehyde (122) can be prepared by reacting 2,4-dihydroxybenzaldehyde (120) with 1-bromo-2-(2-bromoethoxy)ethane (121) in the presence of a base such as, but not limited to, potassium carbonate. The reaction is typically performed at an elevated temperature in a solvent such as but not limited to acetonitrile. 4-(2-(2-Bromoethoxy)ethoxy)-2-hydroxybenzaldehyde (122) can be treated with sodium azide to provide 4-(2-(2-azidoethoxy)ethoxy)-2-hydroxybenzaldehyde (123). The reaction is typically performed at ambient temperature in a solvent such as but not limited to N,N-dimethylformamide. (2S,3R,4S,5S,6S)-2-(5-(2-(2-Azidoethoxy)ethoxy)-2-formylphenoxy)-6-(methoxycarbonyl)tetrahydro-2H-pyran-3,4,5-triyl triacetate (125) can be prepared by reacting 4-(2-(2-azidoethoxy)ethoxy)-2-hydroxybenzaldehyde (123) with (3R,4S,5S,6S)-2-bromo-6-(methoxycarbonyl)tetrahydro-2H-pyran-3,4,5-triyl triacetate (124) in the presence of silver oxide. The reaction is typically performed at ambient temperature in a solvent such as, but not limited to, acetonitrile. Hydrogenation of (2S,3R,4S,5S,6S)-2-(5-(2-(2-azidoethoxy)ethoxy)-2-formylphenoxy)-6-(methoxycarbonyl)tetrahydro-2H-pyran-3,4,5-triyl triacetate (125) in the presence of Pd/C will provide (2S,3R,4S,5S,6S)-2-(5-(2-(2-aminoethoxy)ethoxy)-2-(hydroxymethyl)phenoxy)-6-(methoxycarbonyl)tetrahydro-2H-pyran-3,4,5-triyl triacetate (126). The reaction is typically performed at ambient temperature in a solvent such as, but not limited to, tetrahydrofuran. (2S,3R,4S,5S,6S)-2-(5-(2-(2-((((9H-Fluoren-9-yl)methoxy)carbonyl)amino)ethoxy)ethoxy)-2-(hydroxymethyl)phenoxy)-6-(methoxycarbonyl)tetrahydro-2H-pyran-3,4,5-triyl triacetate (127) can be prepared by treating (2S,3R,4S,5S,6S)-2-(5-(2-(2-aminoethoxy)ethoxy)-2-(hydroxymethyl)phenoxy)-6-(methoxycarbonyl)tetrahydro-2H-pyran-3,4,5-triyl triacetate (126) with (9H-fluoren-9-yl)methyl carbonochloridate in the presence of a base, such as, but not limited to, N,N-diisopropylethylamine. The reaction is typically performed at low temperature in a solvent such as, but not limited to, dichloromethane. Compound (88) can be reacted with (2S,3R,4S,5S,6S)-2-(5-(2-(2-((((9H-Fluoren-9-yl)methoxy)carbonyl)amino)ethoxy)ethoxy)-2-(hydroxymethyl)phenoxy)-6-(methoxycarbonyl)tetrahydro-2H-pyran-3,4,5-triyl triacetate (127) in the presence of a base, such as, but not limited to, N,N-diisopropylethylamine, followed by treatment with lithium hydroxide to provide compound (128). The reaction is typically performed at low temperature in a solvent such as, but not limited to, N,N-dimethylformamide. Compound (129) can be prepared by reacting compound (128) with compound (84) in the presence of a base such as, but not limited to, N,N-diisopropylethylamine. The reaction is typically performed at ambient temperature in a solvent such as but not limited to N,N-dimethylformamide.

### 5.7.2.7 Synthesis of Compound (139)

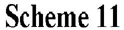

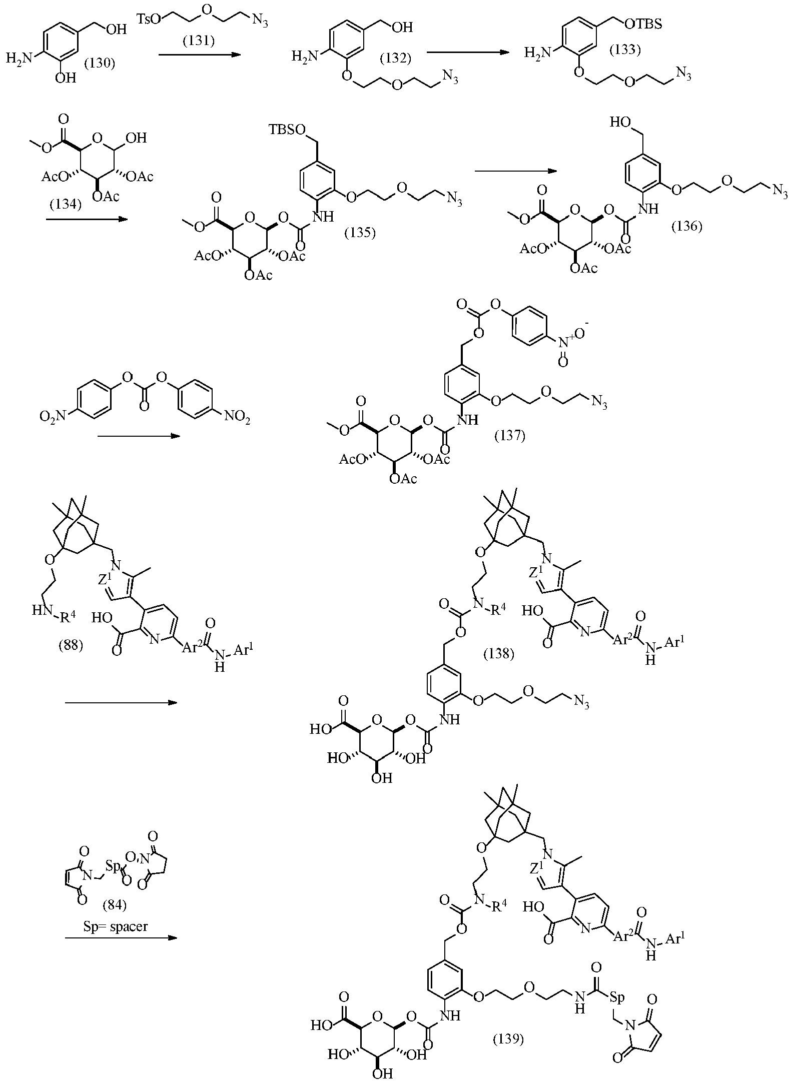

Scheme 11 describes the synthesis of carbamate glucuronide intermediates and synthons. 2-Amino-5-(hydroxymethyl)phenol (130) can be treated with sodium hydride and then reacted with 2-(2-Azidoethoxy)ethyl 4-methylbenzenesulfonate (131) to provide (4-amino-3-(2-(2-azidoethoxy)ethoxy)phenyl)methanol (132). The reaction is typically performed at an elevated temperature in a solvent such as, but not limited to N,N-dimethylformamide. 2-(2-(2-Azidoethoxy)ethoxy)-4-(((tert-butyldimethylsilyl)oxy)methyl)aniline (133) can be prepared by reacting (4-amino-3-(2-(2-azidoethoxy)ethoxy)phenyl)methanol (132) with tert-butyldimethylchlorosilane in the presence of imidazole. The reaction is typically performed at ambient temperature in a solvent such as, but not limited to tetrahydrofuran. 2-(2-(2-Azidoethoxy)ethoxy)-4-(((tert-butyldimethylsilyl)oxy)methyl)aniline (133) can be treated with phosgene, in the presence of a base such as but not limited to triethylamine, followed by reaction with (3R,4S,5S,6S)-2-hydroxy-6-(methoxycarbonyl)tetrahydro-2H-pyran-3,4,5-triyl triacetate (134) in the presence of a base such as but not limited to triethylamine, to provide (2S,3R,4S,5S,6S)-2-(((2-(2-(2-azidoethoxy)ethoxy)-4-(((tert-butyldimethylsilyl)oxy)methyl)phenyl)carbamoyl)oxy)-6-(methoxycarbonyl)tetrahydro-2H-pyran-3,4,5-triyl triacetate (135). The reaction is typically performed in a solvent such as, but not limited to, toluene, and the additions are typically performed at low temperature, before warming up to ambient temperature after the phosgene addition and heating at an elevated temperature after the (3R,4S,SS,6S)-2-hydroxy-6-(methoxycarbonyl)tetrahydro-2H-pyran-3,4,5-triyl triacetate (134) addition. (2S,3R,4S,5S,6S)-2-(((2-(2-(2-Azidoethoxy)ethoxy)-4-(hydroxymethyl)phenyl)carbamoyl)oxy)-6-(methoxycarbonyl)tetrahydro-2H-pyran-3,4,5-triyl triacetate (136) can be prepared by reacting 2S,3R,4S,5S,6S)-2-(((2-(2-(2-azidoethoxy)ethoxy)-4-(((tert-butyldimethylsilyl)oxy)methyl)phenyl)carbamoyl)oxy)-6-(methoxycarbonyl)tetrahydro-2H-pyran-3,4,5-triyl triacetate (135) with p-toluenesulfonic acid monohydrate. The reaction is typically performed at ambient temperature in a solvent such as, but not limited to methanol. (2S,3R,4S,5S,6S)-2-(((2-(2-(2-Azidoethoxy)ethoxy)-4-(hydroxymethyl)phenyl)carbamoyl)oxy)-6-(methoxycarbonyl)tetrahydro-2H-pyran-3,4,5-triyl triacetate (136) can be reacted with bis(4-nitrophenyl)carbonate in the presence of a base such as, but not limited to, N,N-diisopropylethylamine, to provide (2S,3R,4S,5S,6S)-2-(((2-(2-(2-azidoethoxy)ethoxy)-4-((((4-nitrophenoxy)carbonyl)oxy)methyl)phenyl)carbamoyl)oxy)-6-(methoxycarbonyl)tetrahydro-2H-pyran-3,4,5-triyl triacetate (137). The reaction is typically performed at ambient temperature in a solvent such as, but not limited to, N,N-dimethylformamide. (2S,3R,4S,5S,6S)-2-(((2-(2-(2-Azidoethoxy)ethoxy)-4-((((4-nitrophenoxy)carbonyl)oxy)methyl)phenyl)carbamoyl)oxy)-6-(methoxycarbonyl)tetrahydro-2H-pyran-3,4,5-triyl triacetate (137) can be reacted with compound in the presence of a base such as, but not limited to, N,N-diisopropylethylamine, followed by treatment with aqueous lithium hydroxide, to provide compound (138). The first step is typically conducted at ambient temperature in a solvent such as, but not limited to N,N-dimethylformamide, and the second step is typically conducted at low temperature in a solvent such as but not limited to methanol. Compound (138) can be treated with tris(2-carboxyethyl))phosphine hydrochloride, followed by reaction with compound (84) in the presence of a base such as, but not limited to, N,N-diisopropylethylamine, to provide compound (139). The reaction with tris(2-carboxyethyl))phosphine hydrochloride is typically performed at ambient temperature in a solvent such as, but not limited to, tetrahydrofuran, water, or mixtures thereof, and the reaction with N-succinimidyl 6-maleimidohexanoate is typically performed at ambient temperature in a solvent such as, but not limited to, N,N-dimethylformamide.

### 5.7.2.8 Synthesis of Compound (149)

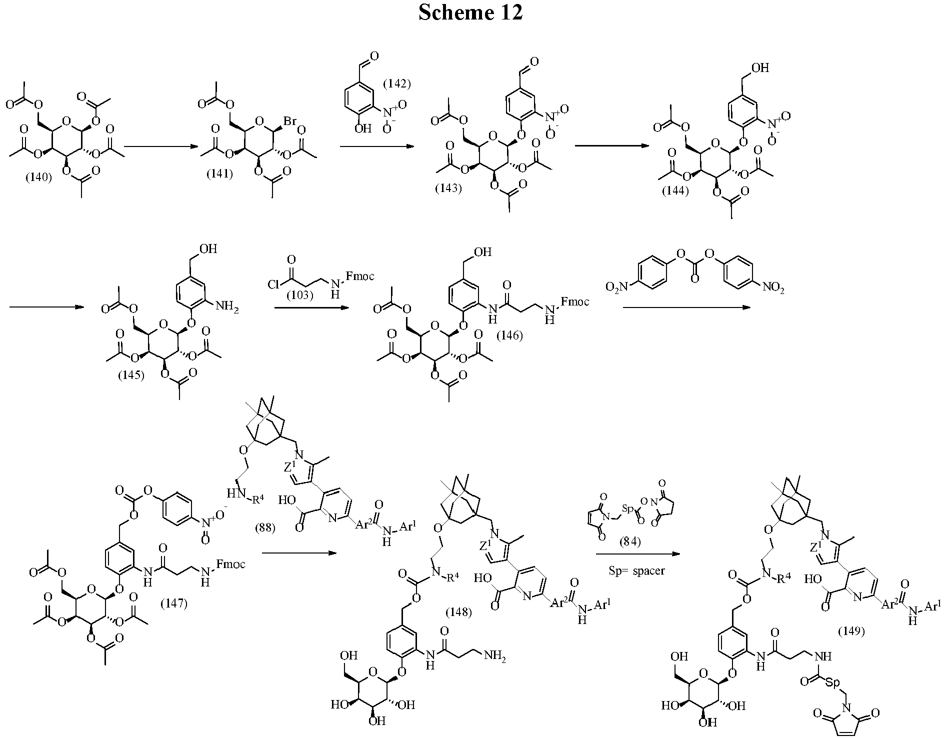

Scheme 20 describes the synthesis of galactoside linker intermediates and synthons. (2S,3R,4S,5S,6R)-6-(Acetoxymethyl)tetrahydro-2H-pyran-2,3,4,5-tetrayl tetraacetate (140) can be treated with HBr in acetic acid to provide (2R,3S,4S,SR,6S)-2-(acetoxymethyl)-6-bromotetrahydro-2H-pyran-3,4,5-triyl triacetate (141). The reaction is typically performed at ambient temperature under a nitrogen atmosphere. (2R,3S,4S,5R,6S)-2-(Acetoxymethyl)-6-(4-formyl-2-nitrophenoxy)tetrahydro-2H-pyran-3,4,5-triyl triacetate (143) can be prepared by treating (2R,3S,4S,5R,6S)-2-(acetoxymethyl)-6-bromotetrahydro-2H-pyran-3,4,5-triyl triacetate (141) with silver(I) oxide in the presence of 4-hydroxy-3-nitrobenzaldehyde (142). The reaction is typically performed at ambient temperature in a solvent such as, but not limited to, acetonitrile. (2R,3S,4S,5R,6S)-2-(Acetoxymethyl)-6-(4-formyl-2-nitrophenoxy)tetrahydro-2H-pyran-3,4,5-triyl triacetate (143) can be treated with sodium borohydride to provide (2R,3S,4S,5R,6S)-2-(acetoxymethyl)-6-(4-(hydroxymethyl)-2-nitrophenoxy)tetrahydro-2H-pyran-3,4,5-triyl triacetate (144). The reaction is typically performed at low temperature in a solvent such as but not limited to tetrahydrofuran, methanol, or mixtures thereof. (2R,3S,4S,SR,6S)-2-(Acetoxymethyl)-6-(2-amino-4-(hydroxymethyl)phenoxy)tetrahydro-2H-pyran-3,4,5-triyl triacetate (145) can be prepared by treating (2R,3S,4S,5R,6S)-2-(acetoxymethyl)-6-(4-(hydroxymethyl)-2-nitrophenoxy)tetrahydro-2H-pyran-3,4,5-triyl triacetate (144) with zinc in the presence of hydrochloric acid. The reaction is typically performed at low temperature, under a nitrogen atmosphere, in a solvent such as, but not limited to, tetrahydrofuran. (2S,3R,4S,5S,6R)-2-(2-(3-((((9H-Fluoren-9-yl)methoxy)carbonyl)amino)propanamido)-4-(hydroxymethyl)phenoxy)-6-(acetoxymethyl)tetrahydro-2H-pyran-3,4,5-triyl triacetate (146) can be prepared by reacting (2R,3S,4S,5R,6S)-2-(acetoxymethyl)-6-(2-amino-4-(hydroxymethyl)phenoxy)tetrahydro-2H-pyran-3,4,5-triyl triacetate (145) with (9H-fluoren-9-yl)methyl (3-chloro-3-oxopropyl)carbamate (103) in the presence of a base such as, but not limited to, N,N-diisopropylethylamine. The reaction is typically performed at low temperature, in a solvent such as, but not limited to, dichloromethane. (2S,3R,4S,5S,6R)-2-(2-(3-((((9H-Fluoren-9-yl)methoxy)carbonyl)amino)propanamido)-4-(hydroxymethyl)phenoxy)-6-(acetoxymethyl)tetrahydro-2H-pyran-3,4,5-triyl triacetate (146) can be reacted with bis(4-nitrophenyl)carbonate in the presence of a base such as, but not limited to, N,N-diisopropylethylamine, to provide (2S,3R,4S,5S,6R)-2-(2-(3-((((9H-fluoren-9-yl)methoxy)carbonyl)amino)propanamido)-4-((((4-nitrophenoxy)carbonyl)oxy)methyl)phenoxy)-6-(acetoxymethyl)tetrahydro-2H-pyran-3,4,5-triyl triacetate (147). The reaction is typically performed at low temperature, in a solvent such as, but not limited to, N,N-dimethylformamide. (2S,3R,4S,5S,6R)-2-(2-(3-((((9H-Fluoren-9-yl)methoxy)carbonyl)amino)propanamido)-4-((((4-nitrophenoxy)carbonyl)oxy)methyl)phenoxy)-6-(acetoxymethyl)tetrahydro-2H-pyran-3,4,5-triyl triacetate (147) can be reacted with compound (88) in the presence of a base such as, but not limited to N,N-diisopropylethylamine, followed by treatment with lithium hydroxide, to provide compound (148). The first step is typically performed at low temperature, in a solvent such as, but not limited to, N,N-dimethylformamide, and the second step is typically performed at ambient temperature, in a solvent such as, but not limited to, methanol. Compound (148) can be treated with compound (84), wherein Sp is a spacer, in the presence of a base, such as, but not limited to N,N-diisopropylethylamine, to provide compound (149). The reaction is typically performed at ambient temperature, in a solvent such as, but not limited to, N,N-dimethylformamide.

### 5.8 COMPOSITIONS

The Bcl-xL inhibitors and/or ADCs described herein may be in the form of compositions comprising the inhibitor or ADC and one or more carriers, excipients and/or diluents. The compositions may be formulated for specific uses, such as for veterinary uses or pharmaceutical uses in humans. The form of the composition (*e.g.,* dry powder, liquid formulation, *etc.*) and the excipients, diluents and/or carriers used will depend upon the intended uses of the inhibitors and/or ADCs and, for therapeutic uses, the mode of administration.

For therapeutic uses, the Bcl-xL inhibitor and/or ADC compositions may be supplied as part of a sterile, pharmaceutical composition that includes a pharmaceutically acceptable carrier. This composition can be in any suitable form (depending upon the desired method of administering it to a patient). The pharmaceutical composition can be administered to a patient by a variety of routes such as orally, transdermally, subcutaneously, intranasally, intravenously, intramuscularly, intrathecally, topically or locally. The most suitable route for administration in any given case will depend on the particular Bcl-xL inhibitor or ADC, the subject, and the nature and severity of the disease and the physical condition of the subject. Typically, the Bcl-xL inhibitors will be administered orally or parenterally, and ADC pharmaceutical composition will be administered intravenously or subcutaneously.

Pharmaceutical compositions can be conveniently presented in unit dosage forms containing a predetermined amount of Bcl-xL inhibitor or an ADC described herein per dose. The quantity of inhibitor or ADC included in a unit dose will depend on the disease being treated, as well as other factors as are well known in the art. For Bcl-xL inhibitors, such unit dosages may be in the form of tablets, capsules, lozenges, *etc.* containing an amount of Bcl-xL inhibitor suitable for a single administration. For ADCs, such unit dosages may be in the form of a lyophilized dry powder containing an amount of ADC suitable for a single administration, or in the form of a liquid. Dry powder unit dosage forms may be packaged in a kit with a syringe, a suitable quantity of diluent and/or other components useful for administration. Unit dosages in liquid form may be conveniently supplied in the form of a syringe pre-filled with a quantity of ADC suitable for a single administration.

The pharmaceutical compositions may also be supplied in bulk form containing quantities of ADC suitable for multiple administrations

Pharmaceutical compositions of ADCs may be prepared for storage as lyophilized formulations or aqueous solutions by mixing an ADC having the desired degree of purity with optional pharmaceutically-acceptable carriers, excipients or stabilizers typically employed in the art (all of which are referred to herein as "carriers"), *i.e.,* buffering agents, stabilizing agents, preservatives, isotonifiers, non-ionic detergents, antioxidants, and other miscellaneous additives. *See,* Remington's Pharmaceutical Sciences, 16th edition (Osol, ed. 1980). Such additives should be nontoxic to the recipients at the dosages and concentrations employed.

Buffering agents help to maintain the pH in the range which approximates physiological conditions. They may be present at concentrations ranging from about 2 mM to about 50 mM. Suitable buffering agents for use with the present disclosure include both organic and inorganic acids and salts thereof such as citrate buffers (*e.g.,* monosodium citrate-disodium citrate mixture, citric acid-trisodium citrate mixture, citric acid-monosodium citrate mixture, *etc.*), succinate buffers (*e.g.,* succinic acid-monosodium succinate mixture, succinic acid-sodium hydroxide mixture, succinic acid-disodium succinate mixture, *etc*.), tartrate buffers (*e.g.,* tartaric acid-sodium tartrate mixture, tartaric acid-potassium tartrate mixture, tartaric acid-sodium hydroxide mixture, *etc*.), fumarate buffers (*e.g.,* fumaric acid-monosodium fumarate mixture, fumaric acid-disodium fumarate mixture, monosodium fumarate-disodium fumarate mixture, *etc*.), gluconate buffers (*e.g.,* gluconic acid-sodium gluconate mixture, gluconic acid-sodium hydroxide mixture, gluconic acid-potassium gluconate mixture, *etc.*), oxalate buffer (*e.g.,* oxalic acid-sodium oxalate mixture, oxalic acid-sodium hydroxide mixture, oxalic acid-potassium oxalate mixture, *etc.*)*,* lactate buffers (*e.g.,* lactic acid-sodium lactate mixture, lactic acid-sodium hydroxide mixture, lactic acid-potassium lactate mixture, *etc*.) and acetate buffers (*e.g.,* acetic acid-sodium acetate mixture, acetic acid-sodium hydroxide mixture, *etc.*)*.* Additionally, phosphate buffers, histidine buffers and trimethylamine salts such as Tris can be used.

Preservatives may be added to retard microbial growth, and can be added in amounts ranging from about 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (*e.g.,* chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polyhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc.,* organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (*e.g.,* peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran.

Non-ionic surfactants or detergents (also known as "wetting agents") may be added to help solubilize the glycoprotein as well as to protect the glycoprotein against agitation-induced aggregation, which also permits the formulation to be exposed to shear surface stressed without causing denaturation of the protein. Suitable non-ionic surfactants include polysorbates (20, 80, *etc*.), polyoxamers (184, 188 *etc*.), Pluronic polyols, polyoxyethylene sorbitan monoethers (TWEEN®-20, TWEEN®-80, *etc*.). Non-ionic surfactants may be present in a range of about 0.05 mg/ml to about 1.0 mg/ml, for example about 0.07 mg/ml to about 0.2 mg/ml.

Additional miscellaneous excipients include bulking agents (*e.g.,* starch), chelating agents (*e.g.,* EDTA), antioxidants (*e.g.,* ascorbic acid, methionine, vitamin E), and cosolvents.

### 5.9 METHODS OF USE

The Bcl-xL inhibitors included in the ADCs, as well as the synthons delivered by the ADCs, inhibit Bcl-xL activity and induce apoptosis in cells expressing Bcl-xL. Accordingly, the Bcl-xL inhibitors and/or ADCs may be used in methods to inhibit Bcl-xL activity and/or induce apoptosis in cells.

For Bcl-xL inhibitors, the method generally involves contacting a cell whose survival depends, at least in part, upon Bcl-xL expression with an amount of a Bcl-xL inhibitor sufficient to inhibit Bcl-xL activity and/or induce apoptosis. For ADCs, the method generally involves contacting a cell whose survival depends, at least in part upon Bcl-xL expression, and that expresses a cell-surface antigen for the antibody of the ADC with an ADC under conditions in which the ADC binds the antigen.

In certain embodiments, especially those in which the Bcl-xL inhibitor comprises the ADC has been low or very low cell permeability, the antibody of the ADC binds a target capable of internalizing the ADC into the cell, where it can deliver its Bcl-xL inhibitory synthon. The method may be carried out *in vitro* in a cellular assay to inhibit Bcl-xL activity and/or inhibit apoptosis, or *in vivo* as a therapeutic approach towards treating diseases in which inhibition of apoptosis and/or induction of apoptosis would be desirable.

Dysregulated apoptosis has been implicated in a variety of diseases, including, for example, autoimmune disorders (*e.g.,* systemic lupus erythematosus, rheumatoid arthritis, graft-versus-host disease, myasthenia gravis, or Sjogren's syndrome), chronic inflammatory conditions (*e.g.,* psoriasis, asthma or Crohn's disease), hyperproliferative disorders (*e.g.,* breast cancer, lung cancer), viral infections (*e.g.,* herpes, papilloma, or HIV), and other conditions, such as osteoarthritis and atherosclerosis. The Bcl-xL inhibitor or ADCs described herein may be used to treat or ameliorate any of these diseases. Such treatments generally involve administering to a subject suffering from the disease an amount of a Bcl-xL inhibitor or ADC described herein sufficient to provide therapeutic benefit. For ADCs, the identity of the antibody of the ADC administered will depend upon the disease being treated - thus the antibody should bind a cell-surface antigen expressed in the cell type where inhibition of Bcl-xL activity would be beneficial. The therapeutic benefit achieved will also depend upon the specific disease being treated. In certain instances, the Bcl-xL inhibitor or ADC may treat or ameliorate the disease itself, or symptoms of the disease, when administered as monotherapy. In other instances, the Bcl-xL inhibitor or ADC may be part of an overall treatment regimen including other agents that, together with the inhibitor or ADC, treat or ameliorate the disease being treated, or symptoms of the disease. Agents useful to treat or ameliorate specific diseases that may be administered adjunctive to, or with, the Bcl-xL inhibitors and/or ADCs described herein will be apparent to those of skill in the art.

Although absolute cure is always desirable in any therapeutic regimen, achieving a cure is not required to provide therapeutic benefit. Therapeutic benefit may include halting or slowing the progression of the disease, regressing the disease without curing, and/or ameliorating or slowing the progression of symptoms of the disease. Prolonged survival as compared to statistical averages and/or improved quality of life may also be considered therapeutic benefit.

One particular class of diseases that involve dysregulated apoptosis and that are significant health burden world-wide are cancers. In a specific embodiment, the Bcl-xL inhibitors and/or ADCs described herein may be used to treat cancers. The cancer may be, for example, solid tumors or hematological tumors. Cancers that may be treated with ADCs described herein include, but are not limited to bladder cancer, brain cancer, breast cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, myeloma, prostate cancer, small cell lung cancer and spleen cancer. ADCs may be especially beneficial in the treatment of cancers because the antibody can be used to target the Bcl-xL inhibitory synthon specifically to tumor cells, thereby potentially avoiding or ameliorating undesirable side-effects and/or toxicities that may be associated with systemic administration of unconjugated inhibitors. One embodiment pertains to a method of treating a disease involving dysregulated intrinsic apoptosis, comprising administering to a subject having a disease involving dysregulated apotosis an amount of an ADC described herein effective to provide therapeutic benefit, wherein the antibody of the ADC binds a cell surface receptor on a cell whose intrinsic apoptosis is dysregulated.. One embodiment pertains to a method of treating cancer, comprising administering to a subject having cancer an ADC described herein that is capable of binding a cell surface receptor or a tumor associated antigen expressed on the surface of the cancer cells, in an amount effective to provide therapeutic benefit.

In the context of tumorigenic cancers, therapeutic benefit, in addition to including the effects discussed above, may also specifically include halting or slowing progression of tumor growth, regressing tumor growth, eradicating one or more tumors and/or increasing patient survival as compared to statistical averages for the type and stage of the cancer being treated. In one embodiment, the cancer being treated is a tumorigenic cancer.

The Bcl-xL inhibitors and/or ADCs may be administered as monotherapy to provide therapeutic benefit, or may be administered adjunctive to, or with, other chemotherapeutic agents and/or radiation therapy. Chemotherapeutic agents to which the inhibitors and/or ADCs described herein may be utilized as adjunctive therapy may be targeted (for example, other Bcl-xL inhibitors or ADCs, protein kinase inhibitors, *etc*.) or non-targeted (for example, non-specific cytotoxic agents such as radionucleotides, alkylating agents and intercalating agents). Non-targeted chemotherapeutic agents with which the inhibitors and/or ADCs described herein may be adjunctively administered include, but are not limited to, methotrexate, taxol, L-asparaginase, mercaptopurine, thioguanine, hydroxyurea, cytarabine, cyclophosphamide, ifosfamide, nitrosoureas, cisplatin, carboplatin, mitomycin, dacarbazine, procarbizine, topotecan, nitrogen mustards, Cytoxan, etoposide, 5-fluorouracil, BCNU, irinotecan, camptothecins, bleomycin, doxorubicin, idarubicin, daunorubicin, dactinomycin, plicamycin, mitoxantrone, asparaginase, vinblastine, vincristine, vinorelbine, paclitaxel, calicheamicin, and docetaxel.

Elevated Bcl-xL expression has been shown to correlate with resistance to chemotherapy and radiation therapy. Data herein demonstrate that Bcl-xL inhibitors and/or ADCs that may not be effective as monotherapy to treat cancer may be administered adjunctive to, or with, other chemotherapeutic agents or radiation therapy to provide therapeutic benefit. While not intending to be bound by any therapy of operation, it is believed that administration of the Bcl-xL inhibitors and/or ADCs described herein to tumors that have become resistant to standard of care chemotherapeutic agents and/or radiation therapy sensitizes the tumors such that they again respond to the chemo and/or radiation therapy. One embodiment pertains to a method in which the ADC described herein is administered in an amount effective to sensitize the tumor cells to standard chemotherapy and/or radiation therapy. Accordingly, in the context of treating cancers, "therapeutic benefit" includes administering the inhibitors and/or ADCs described herein adjunctive to, or with, chemotherapeutic agents and/or radiation therapy, either in patients who have not yet begin such therapy or who have but have not yet exhibited signs of resistance, or in patients who have begun to exhibit signs of resistance, as a means of sensitizing the tumors to the chemo and/or radiation therapy.

### 5.10 DOSAGES AND ADMINISTRATION REGIMENS

The amount of ADC administered will depend upon a variety of factors, including but not limited to, the particular disease being treated, the mode of administration, the desired therapeutic benefit, the stage or severity of the disease, the age, weight and other characteristics of the patient, *etc.* Determination of effective dosages is within the capabilities of those skilled in the art.

Effective dosages may be estimated initially from cellular assays. For example, an initial dose for use in humans may be formulated to achieve a circulating blood or serum concentration of ADC that is expected to achieve a cellular concentration of Bcl-xL inhibitor that is at or above an IC₅₀ or ED₅₀ of the particular inhibitory molecule measured in a cellular assay.

Initial dosages for use in humans may also be estimated from *in vivo* animal models. Suitable animal models for a wide variety of diseases are known in the art.

When administered adjunctive to, or with, other agents, such as other chemotherapeutic agents, the ADCs may be administered on the same schedule with the other agents, or on a different schedule. When administered on the same schedule, the ADC may be administered before, after, or concurrently with the other agent. In some embodiments where the ADC is administered adjunctive to, or with, standard chemo- and/or radiation therapy. The ADC may be initiated prior to commencement of the standard therapy, for example a day, several days, a week, several weeks, a month, or even several months before commencement of standard chemo- and/or radiation therapy.

When administered adjunctive to, or with, other agents, such as for example standard chemotherapeutic agents, the other agent will typically be administered according to its standard dosing schedule with respect to route, dosage and frequency. However, in some instances less than the standard amount may be necessary for efficacy when administered adjunctive to ADC therapy.

### 6. EXAMPLES

### Example 1. Synthesis of Exemplary Bcl-xL Inhibitors

This Example provides synthetic methods for exemplary Bcl-xL inhibitory compounds W1.01-W1.08. Bcl-xL inhibitors (W1.01-W1.08) and synthons (Examples 2.1-2.53) were named using ACD/Name 2012 release (Build 56084, 05 April 2012, Advanced Chemistry Development Inc., Toronto, Ontario). Bcl-xL inhibitor and synthon intermediates were named with ACD/Name 2012 release (Build 56084, 05 April 2012, Advanced Chemistry Development Inc., Toronto, Ontario), or ChemDraw® Ver. 9.0.7 (CambridgeSoft, Cambridge, MA), or ChemDraw® Ultra Ver. 12.0 (CambridgeSoft, Cambridge, MA).

### 1.1. Synthesis of 6-[8-(1,3-benzothiazol-2-ylcarbamoyl)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid (Compound W1.01)

### 1.1.1. 3-bromo-5,7-dimethyladamantanecarboxylic acid

In a 50 mL round-bottomed flask at 0°C was added bromine (16 mL). Iron powder (7 g) was then added, and the reaction was stirred at 0°C for 30 minutes. 3,5-Dimethyladamantane-1-carboxylic acid (12 g) was then added. The mixture was warmed up to room temperature and stirred for 3 days. A mixture of ice and concentrated HCl was poured into the reaction mixture. The resulting suspension was treated twice with Na₂SO₃ (50 g in 200 mL water) to destroy bromine and was extracted three times with dichloromethane. The combined organics were washed with IN aqueous HCl, dried over Na₂SO₄, filtered, and concentrated to give the crude title compound.

### 1.1.2. 3-bromo-5,7-dimethyladamantanemethanol

To a solution of Example 1.1.1 (15.4 g) in tetrahydrofuran (200 mL) was added BH₃ (1M in tetrahydrofuran, 150 mL). The mixture was stirred at room temperature overnight. The reaction mixture was then carefully quenched by adding methanol dropwise. The mixture was then concentrated under vacuum, and the residue was balanced between ethyl acetate (500 mL) and 2N aqueous HCl (100 mL). The aqueous layer was further extracted twice with ethyl acetate, and the combined organic extracts were washed with water and brine, dried over Na₂SO₄, and filtered. Evaporation of the solvent gave the title compound.

### 1.1.3. 1-((3-bromo-5,7-dimethyltricyclo[3.3.1.1^{3,7}]dec-1-yl)methyl)-1H-pyrazole

To a solution of Example 1.1.2 (8.0 g) in toluene (60 mL) was added 1H-pyrazole (1.55 g) and cyanomethylenetributylphosphorane (2.0 g). The mixture was stirred at 90°C overnight. The reaction mixture was then concentrated and the residue was purified by silica gel column chromatography (10:1 heptane:ethyl acetate) to give the title compound. MS (ESI) m/e 324.2 (M+H)⁺.

### 1.1.4. 2-{[3,5-dimethyl-7-(1H-pyrazol-1-ylmethyl)tricyclo[3.3.1.1^{3,7}]dec-1-yl]oxy}ethanol

To a solution of Example 1.1.3 (4.0 g) in ethane-1,2-diol (12 mL) was added triethylamine (3 mL). The mixture was stirred at 150°C under microwave conditions (Biotage Initiator) for 45 minutes. The mixture was poured into water (100 mL) and extracted three times with ethyl acetate. The combined organic extracts were washed with water and brine, dried over Na₂SO₄, and filtered. Evaporation of the solvent gave the crude product, which was purified by silica gel chromatography, eluting with 20% ethyl acetate in heptane, followed by 5% methanol in dichloromethane, to give the title compound. MS (ESI) m/e 305.2 (M+H)⁺.

### 1.1.5. 2-({3,5-dimethyl-7-[(5-methyl-1H-pyrazol-1-yl)methyl]tricyclo[3.3.1.1^{3,7}]dec-1-yl}oxy)ethanol

To a cooled (-78°C) solution of Example 1.1.4 (6.05 g) in tetrahydrofuran (100 mL) was added n-BuLi (40 mL, 2.5M in hexane). The mixture was stirred at -78°C for 1.5 hours. Iodomethane (10 mL) was added through a syringe, and the mixture was stirred at -78°C for 3 hours. The reaction mixture was then quenched with aqueous NH₄Cl and extracted twice with ethyl acetate, and the combined organic extracts were washed with water and brine. After drying over Na₂SO₄, the solution was filtered and concentrated, and the residue was purified by silica gel column chromatography, eluting with 5% methanol in dichloromethane, to give the title compound. MS (ESI) m/e 319.5 (M+H)⁺.

### 1.1.6. 1-({3,5-dimethyl-7-[2-(hydroxy)ethoxy]tricyclo[3.3.1.1^{3,7}]dec-1-yl}methyl)-4-iodo-5-methyl-1H-pyrazole

To a solution of Example 1.1.5 (3.5 g) in N,N-dimethylformamide (30 mL) was added N-iodosuccinimide (3.2 g). The mixture was stirred at room temperature for 1.5 hours. The reaction mixture was then diluted with ethyl acetate (600 mL) and washed with aqueous NaHSO₃, water, and brine. After drying over Na₂SO₄, the solution was filtered and concentrated and the residue was purified by silica gel chromatography (20% ethyl acetate in dichloromethane) to give the title compound. MS (ESI) m/e 445.3 (M+H)⁺.

### 1.1.7. 2-({3-[(4-iodo-5-methyl-1H-pyrazol-1-yl)methyl]-5,7-dimethyltricyclo[3.3.1.1^{1,7}]dec-1-yl}oxy)ethyl methanesulfonate

To a cooled solution of Example 1.1.6 (6.16 g) in dichloromethane (100 mL) was added triethylamine (4.21 g) followed by methanesulfonyl chloride (1.6 g). The mixture was stirred at room temperature for 1.5 hours. The reaction mixture was then diluted with ethyl acetate (600 mL) and washed with water and brine. After drying over Na₂SO₄, the solution was filtered and concentrated, and the residue was used in the next reaction without further purification. MS (ESI) m/e 523.4 (M+H)⁺.

### 1.1.8. 1-({3,5-dimethyl-7-[2-(methylamino)ethoxy]tricyclo[3.3.1.1^{1,7}]dec-1-yl}methyl)-4-iodo-5-methyl-1H-pyrazole

A solution of Example 1.1.7 (2.5 g) in 2M methylamine in methanol (15 mL) was stirred at 100°C for 20 minutes under microwave conditions (Biotage Initiator). The reaction mixture was concentrated under vacuum. The residue was then diluted with ethyl acetate (400 mL) and washed with aqueous NaHCO₃, water and brine. After drying over Na₂SO₄, the solution was filtered and concentrated, and the residue was used in the next reaction without further purification. MS (ESI) m/e 458.4 (M+H)⁺.

### 1.1.9. tert-butyl [2-({3-[(4-iodo-5-methyl-1H-pyrazol-1-yl)methyl]-5,7-dimethyltricyclo[3.3.1.1^{3,7}]dec-1-yl}oxy)ethyl]methylcarbamate

To a solution of Example 1.1.8 (2.2 g) in tetrahydrofuran (30 mL) was added di-*tert*-butyl dicarbonate (1.26 g) and a catalytic amount of 4-dimethylaminopyridine. The mixture was stirred at room temperature for 1.5 hours and diluted with ethyl acetate (300 mL). The solution was washed with saturated aqueous NaHCO₃, water (60 mL), and brine (60 mL). The organic layer was dried with Na₂SO₄, filtered, and concentrated. The residue was purified by silica gel chromatography, eluting with 20% ethyl acetate in dichloromethane, to give the title compound. MS (ESI) m/e 558.5 (M+H)⁺.

### 1.1.10. 6-fluoro-3-bromopicolinic acid

A slurry of 6-amino-3-bromopicolinic acid (25 g) in 400 mL 1:1 dichloromethane/chloroform was added to nitrosonium tetrafluoroborate (18.2 g) in dichloromethane (100 mL) at 5°C over 1 hour, and the resulting mixture was stirred for another 30 minutes, then warmed to 35°C and stirred overnight. The reaction was cooled to room temperature, and then adjusted to pH 4 with aqueous NaH₂PO₄ solution. The resulting solution was extracted three times with dichloromethane, and the combined extracts were washed with brine, dried over sodium sulfate, filtered and concentrated to provide the title compound.

### 1.1.11. Tert-butyl 3-bromo-6-fluoropicolinate

Para-toluenesulfonyl chloride (27.6 g) was added to a solution of Example 1.1.10 (14.5 g) and pyridine (26.7 mL) in dichloromethane (100 mL) and tert-butanol (80 mL) at 0°C. The reaction was stirred for 15 minutes, warmed to room temperature, and stirred overnight. The solution was concentrated and partitioned between ethyl acetate and aqueous Na₂CO₃ solution. The layers were separated, and the aqueous layer extracted with ethyl acetate. The organic layers were combined, rinsed with aqueous Na₂CO₃ solution and brine, dried over sodium sulfate, filtered, and concentrated to provide the title compound.

### 1.1.12. methyl 2-(5-bromo-6-(tert-butoxycarbonyl)pyridin-2-yl)-1,2,3,4-tetrahydroisoquinoline-8-carboxylate

To a solution of methyl 1,2,3,4-tetrahydroisoquinoline-8-carboxylate hydrochloride (12.37 g) and Example 1.1.11 (15 g) in dimethyl sulfoxide (100 mL) was added N,N-diisopropylethylamine (12 mL). The mixture was stirred at 50°C for 24 hours. The mixture was then diluted with ethyl acetate (500 mL), washed with water and brine, and dried over Na₂SO₄. Filtration and evaporation of the solvent gave a residue that was purified by silica gel chromatography, eluting with 20% ethyl acetate in heptane, to give the title compound. MS (ESI) m/e 448.4 (M+H)⁺.

### 1.1.13. methyl 2-(6-(tert-butoxycarbonyl)-5-(4,4,5,5-tetramethyl-1,3,2-dioxaborolan-2-yl)pyridin-2-yl)-1,2,3,4-tetrahydroisoquinoline-8-carboxylate

To a solution of Example 1.1.12 (2.25 g) and [1,1'-bis(diphenylphosphino)ferrocene]dichloropalladium(II) (205 mg) in acetonitrile (30 mL) was added triethylamine (3 mL) and pinacolborane (2 mL). The mixture was stirred at reflux for 3 hours. The mixture was diluted with ethyl acetate (200 mL) and washed with water and brine, and dried over Na₂SO₄. Filtration, evaporation of the solvent, and silica gel chromatography (eluted with 20% ethyl acetate in heptane) gave the title compound. MS (ESI) m/e 495.4 (M+H)⁺.

### 1.1.14. methyl 2-(6-(tert-butoxycarbonyl)-5-(1-((3-(2-((tert-butoxycarbonyl)(methyl)amino)ethoxy)-5,7-dimethyltricyclo[3.3.1.1^{3,7}]dec-1-yl)methyl)-5-methyl-1H-pyrazol-4-yl)pyridin-2-yl)-1,2,3,4-tetrahydroisoquinoline-8-carboxylate

To a solution of Example 1.1.13 (4.94 g) in tetrahydrofuran (60 mL) and water (20 mL) was added Example 1.1.9 (5.57 g), 1,3,5,7-tetramethyl-8-tetradecyl-2,4,6-trioxa-8-phosphaadamantane (412 mg), tris(dibenzylideneacetone)dipalladium(0) (457 mg), and K₃PO₄ (11 g). The mixture was stirred at reflux for 24 hours. The reaction mixture was cooled, diluted with ethyl acetate (500 mL), washed with water and brine, and dried over Na₂SO₄. Filtration and evaporation of the solvent gave a residue that was purified by silica gel chromatography, eluting with 20% ethyl acetate in heptane, to give the title compound. MS (ESI) m/e 799.1 (M+H)⁺.

### 1.1.15. 2-(6-(tert-butoxycarbonyl)-5-(1-((3-(2-((tert-butoxycarbonyl)(methyl)amino)ethoxy)-5,7-dimethyltricyclo[3.3.1.1^{3,7}]dec-1-yl)methyl)-5-methyl-1H-pyrazol-4-yl)pyridin-2-yl)-1,2,3,4-tetrahydroisoquinoline-8-carboxylic acid

To a solution of Example 1.1.14 (10 g) in tetrahydrofuran (60 mL), methanol (30 mL) and water (30 mL) was added lithium hydroxide monohydrate (1.2 g). The mixture was stirred at room temperature for 24 hours. The reaction mixture was neutralized with 2% aqueous HCl and concentrated under vacuum. The residue was diluted with ethyl acetate (800 mL) and washed with water and brine, and dried over Na₂SO₄. Filtration and evaporation of the solvent gave the title compound. MS (ESI) m/e 785.1 (M+H)⁺.

### 1.1.16. tert-butyl 6-[8-(1,3-benzothiazol-2-ylcarbamoyl)-3,4-dihydroisoquinolin-2(1H)-yl]-3-{1-[(3-{2-[(tert-butoxycarbonyl)(methyl)amino]ethoxy}-5,7-dimethyltricyclo[3.3.1.1^{3,7}]dec-1-yl)methyl]-5-methyl-1H-pyrazol-4-yl}pyridine-2-carboxylate

To a solution of Example 1.1.15 (10 g) in N,N-dimethylformamide (20 mL) was added benzo[d]thiazol-2-amine (3.24 g), fluoro-N,N,N',N'-tetramethylformamidinium hexafluorophosphate (5.69 g) and N,N-diisopropylethylamine (5.57 g). The mixture was stirred at 60°C for 3 hours. The reaction mixture was diluted with ethyl acetate (800 mL) and washed with water and brine, and dried over Na₂SO₄. Filtration and evaporation of the solvent gave a residue that was purified by silica gel chromatography, eluting with 20% ethyl acetate in dichloromethane, to give the title compound. MS (ESI) m/e 915.5 (M+H)⁺.

### 1.1.17. 6-[8-(1,3-benzothiazol-2-ylcarbamoyl)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid

To a solution of Example 1.1.16 (5 g) in dichloromethane (20 mL) was added trifluoroacetic acid (10 mL). The mixture was stirred overnight. The solvent was evaporated under vacuum, and the residue was dissolved in dimethyl sulfoxide/methanol (1:1, 10 mL), and chromatographed via reverse-phase using an Analogix system and a C18 cartridge (300 g), eluting with 10-85% acetonitrile and 0.1% trifluoroacetic acid in water, to give the title compound as a TFA salt. ¹H NMR (300 MHz, dimethyl sulfoxide d₆) δ ppm 12.85 (s, 1H), 8.13-8.30 (m, 2H), 8.03 (d, 1H), 7.79 (d, 1H), 7.62 (d, 1H), 7.32-7.54 (m, 3H), 7.28 (d, 1H), 6.96 (d, 1H), 4.96 (dd, 1H), 3.80-3.92 (m, 4H), 3.48-3.59 (m, 1H), 2.91-3.11 (m, 2H), 2.51-2.59 (m, 4H), 2.03-2.16 (m, 2H), 1.21-1.49 (m, 6H), 0.97-1.20 (m, 4H), 0.87 (s, 6H). MS (ESI) m/e 760.4 (M+H)⁺.

### 1.2. Synthesis of 6-[8-(1,3-benzothiazol-2-ylcarbamoyl)-3,4-dihydroisoquinolin-2(1H)-yl]-3-(1-{[(1r,3R,5S,7s)-3,5-dimethyl-7-(2-{2-[2-(methylamino)ethoxy]ethoxy}ethoxy)tricyclo[3.3.1.1^{3,7}]dec-1-yl]methyl}-5-methyl-1H-pyrazol-4-yl)pyridine-2-carboxylic acid (Compound W1.02)

### 1.2.1. 2-(2-(2-(((3-((1H-pyrazol-1-yl)methyl)-5,7-dimethyladamantan-1-yl)oxy)ethoxy)ethoxy)ethanol

To a solution of Example 1.1.3 (2.65 g) in 2,2'-(ethane-1,2-diylbis(oxy))diethanol (15 g) was added triethylamine (3 mL). The mixture was stirred at 180°C under microwave conditions (Biotage Initiator) for 120 minutes. The mixture was diluted with water and acetonitrile (1:1, 40 mL). The crude material was added to a reverse phase column (C18, SF65-800g) and was eluted with 10-100% acetonitrile in water with 0.1% trifluoroacetic acid to afford the title compound. MS (ESI) m/e 393.0 (M+H)⁺.

### 1.2.2. 2-(2-(2-((3,5-dimethyl-7-((5-methyl-1H-pyrazol-1-yl)methyl)adamantan-1-yl)oxy)ethoxy)ethoxy)ethanol

To a cooled (0°C) solution of Example 1.2.1 (2.69 g) in tetrahydrofuran (20 mL) was added n-BuLi (10 mL, 2.5M in hexane). The mixture was stirred at 0°C for 1.5 hours. Iodomethane (1 mL) was added through a syringe, and the mixture was stirred at 0°C for 1.5 hours. The reaction mixture was quenched with trifluoroacetic acid (1 mL). After evaporation of the solvents, the residue was used directly in the next step. MS (ESI) m/e 407.5 (M+H)⁺.

### 1.2.3. 2-(2-(2-((3-((4-iodo-5-methyl-1H-pyrazol-1-yl)methyl)-5,7-dimethyladamantan-1-yl)oxy)ethoxy)ethoxy)ethanol

To a cooled (0°C) solution of Example 1.2.2 (2.78 g) in N,N-dimethylformamide (30 mL) was added N-iodosuccinimide (1.65 g). The mixture was stirred at room temperature for 2 hours. The crude product was added to a reverse phase column (C-18, SF65-800g) and was eluted with 10-100% acetonitrile in water with 0.1% trifluoroacetic acid to afford the title compound. MS (ESI) m/e 533.0 (M+H)⁺.

### 1.2.4. 2-(2-(2-((3-((4-iodo-5-methyl-1H-pyrazol-1-yl)methyl)-5,7-dimethyladamantan-1-yl)oxy)ethoxy)ethoxy)- N-methylethanamine

To a cooled (0°C) solution of Example 1.2.3 (2.45 g) in tetrahydrofuran (10 mL) was added triethylamine (1 mL) followed by methanesulfonyl chloride (0.588 g). The mixture was stirred at room temperature for 2 hours. Methanamine (10 mL, 2M in methanol) was added to the reaction mixture and transferred to a 20 mL microwave tube. The mixture was heated under microwave conditions (Biotage Initiator) at 100 °C for 60 minutes. After cooling to room temperature, the solvent was removed under vacuum. The residue was added to a reverse phase column (C18, SF40-300g) and eluted with 40-100% acetonitrile in water with 0.1% trifluoroacetic acid to afford the title compound. MS (ESI) m/e 546.0 (M+H)⁺.

### 1.2.5. tert-butyl (2-(2-(2-((3-((4-iodo-5-methyl-1H-pyrazol-1-yl)methyl)-5,7-dimethyladamantan-1-yl)oxy)ethoxy)ethoxy)ethyl)(methyl)carbamate

To a solution of Example 1.2.4 (1.41 g) in tetrahydrofuran (20 mL) was added di-tert-butyl dicarbonate (1 g) and 4-dimethylaminopyridine (0.6 g). The mixture was stirred at room temperature for 3 hours, and the solvent was removed by vacuum. The residue was purified by silica gel chromatography, eluting with 10-100% ethyl acetate in hexane, to give the title compound. MS (ESI) m/e 645.8 (M+H)⁺.

### 1.2.6. tert-butyl (2-(2-(2-((3,5-dimethyl-7-((5-methyl-4-(4,4,5,5-tetramethyl-1,3,2-dioxaborolan-2-yl)-1H-pyrazol-1-yl)methyl)adamantan-1-yl)oxy)ethoxy)ethoxy)ethyl)(methyl)carbamate

To a solution of Example 1.2.5 (1.25 g), dicyclohexylphosphino-2',6'-dimethoxybiphenyl (0.09 g), pinacolborane (1.5 mL) and triethylamine (1.5 mL) in dioxane (20 mL) was added bis(benzonitrile)palladium(II) chloride (0.042 g). After degassing, the mixture was stirred at 90 °C overnight. Evaporation of the solvent and silica gel column purification (eluting with 20-100% ethyl acetate in hexane) gave the title compound. MS (ESI) m/e 646.1 (M+H)⁺.

### 1.2.7. tert-butyl 8-(benzo[d]thiazol-2-ylcarbamoyl)-3,4-dihydroisoquinoline-2(1H)-carboxylate

To a solution of 2-(*tert*-butoxycarbonyl)-1,2,3,4-tetrahydroisoquinoline-8-carboxylic acid (6.8 g) and benzo[*d*]thiazol-2-amine (5.52 g) in dichloromethane (80 mL) was added 1-ethyl-3-[3-(dimethylamino)propyl]-carbodiimide hydrochloride (9.4 g) and 4-dimethylaminopyridine (6 g). The mixture was stirred at room temperature overnight. The reaction mixture was diluted with dichloromethane (400 mL), washed with 5% aqueous HCl, water, and brine, and dried over Na₂SO₄. The mixture was filtered, and the filtrate was concentrated under reduced pressure to provide the title compound.

### 1.2.8. N-(benzo[d]thiazol-2-yl)-1,2,3,4-tetrahydroisoquinoline-8-carboxamide dihydrochloride

To a solution of Example 1.2.7 (8.5 g) in dichloromethane (80 mL) was added 2N HCl in diethyl ether (80 mL). The reaction mixture was stirred at room temperature overnight and concentrated under reduced pressure to provide the title compound.

### 1.2.9. tert-butyl 6-(8-(benzo[d]thiazol-2-ylcarbamoyl)-3,4-dihydroisoquinolin-2(1H)-yl)-3-bromopicolinate

Example 1.1.11 (0.736 g), Example 1.2.8 (1.62 g), and Cs₂CO₃ (4.1 g) were stirred in 12 mL of anhydrous N,N-dimethylacetamide at 120 °C for 12 hours. The cooled reaction mixture was then diluted with ethyl acetate and 10% citric acid. The organic phase was washed three times with citric acid, once with water and brine, and dried over Na₂SO₄. Filtration and concentration afforded crude material, which was chromatographed on silica gel using 0-40% ethyl acetate in hexanes to provide the title compound.

### 1.2.10. tert-butyl 6-(8-(benzo[d]thiazol-2-ylcarbamoyl)-3,4-dihydroisoquinolin-2(1H)-yl)-3-(1-((((1s,7s)-3,5-dimethyl-7-((2,2,5-trimethyl-4-oxo-3,8,11-trioxa-5-azatridecan-13-yl)oxy)adamantan-1-yl)methyl)-5-methyl-1H-pyrazol-4-yl)picolinate

To a solution of Example 1.2.6 (0.135 g) in tetrahydrofuran (1 mL) and water (1 mL) was added Example 1.2.9 (0.12 g), 1,3,5,7-tetramethyl-8-tetradecyl-2,4,6-trioxa-8-phosphaadamantane (0.023 g), tris(dibenzylideneacetone)dipalladium(0) (0.015 g), and K₃PO₄ (0.2 g). The mixture was stirred at 140 °C for 5 minutes under microwave conditions (Biotage Initiator). The reaction mixture was diluted with toluene (5 mL) and filtered. Evaporation of solvent and silica gel purification (20-100% ethyl acetate in heptane) gave the title compound. MS (ESI) m/e 1004.8 (M+H)⁺.

### 1.2.11. 6-[8-(1,3-benzothiazol-2-ylcarbamoyl)-3,4-dihydroisoquinolin-2(1H)-yl]-3-(1-{[3,5-dimethyl-7-(2-{2-[2-(methylamino)ethoxy]ethoxy}ethoxy)tricyclo[3.3.1.1^{3,7}]dec-1-yl]methyl}-5-methyl-1H-pyrazol-4-yl)pyridine-2-carboxylic acid

Example 1.2.10 (1.42 g) in dichloromethane (10 mL) was treated with trifluoroacetic acid (6 mL), and the reaction was stirred at room temperature for 24 hours. The volatiles were removed under reduced pressure. The residue was purified by reverse phase chromatography using a Gilson system (C18, SF40-300g) eluting with 30-100% acetonitrile in water containing 0.1% v/v trifluoroacetic acid. The desired fractions were combined and freeze-dried to provide the title compound as a TFA salt. ¹H NMR (300 MHz, dimethyl sulfoxide-d₆) δ ppm 12.85 (br.s, 1H), 8.33 (br.s, 2H), 8.03 (d, 1H), 7.79 (d, 1H), 7.62 (d, 1H), 7.41-7.54 (m, 3H), 7.32-7.40 (m, 2H), 7.28 (s, 1H), 6.95 (d, 1H), 4.95 (s, 2H), 3.85-3.93 (m, 2H), 3.81 (s, 2H), 3.60-3.66 (m, 2H), 3.52-3.58 (m, 4H), 3.45 (s, 3H), 2.97-3.12 (m, 4H), 2.56 (t, 2H), 2.10 (s, 3H), 1.34-1.41 (m, 2H), 1.18-1.31 (m, 4H), 0.95-1.18 (m, 6H), 0.85 (s, 6H). MS (ESI) m/e 848.2 (M+H)⁺.

### 1.3. Synthesis of 3-(1-1[3-(2-aminoethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-3,4-dihydroisoquinolin-2(1H)-yl]pyridine-2-carboxylic acid (Compound W1.03)

### 1.3.1. methyl 2-(6-(tert-butoxycarbonyl)-5-(1-((3-(2-hydroxyethoxy)-5,7-dimethyladamantan-1-yl)methyl)-5-methyl-1H-pyrazol-4-yl)pyridin-2-yl)-1,2,3,4-tetrahydroisoquinoline-8-carboxylate

To a solution of Example 1.1.13 (2.25 g) in tetrahydrofuran (30 mL) and water (10 mL) was added Example 1.1.6 (2.0 g), 1,3,5,7-tetramethyl-6-phenyl-2,4,8-trioxa-6-phosphaadmante (329 mg), tris(dibenzylideneacetone)dipalladium(0) (206 mg) and potassium phosphate tribasic (4.78 g). The mixture was refluxed overnight, cooled, and diluted with ethyl acetate (500 mL). The resulting mixture was washed with water and brine, and the organic layer was dried over Na₂SO₄, filtered, and concentrated. The residue was purified by flash chromatography, eluting with 20% ethyl acetate in heptanes and then with 5% methanol in dichloromethane to provide the title compound.

### 1.3.2. methyl 2-(6-(tert-butoxycarbonyl)-5-(1-((3,5-dimethyl-7-(2-((methylsulfonyl)oxy)ethoxy)adamantan-1-yl)methyl)-5-methyl-1H-pyrazol-4-yl)pyridin-2-yl)-1,2,3,4-tetrahydroisoquinoline-8-carboxylate

To a cold solution of Example 1.3.1 (3.32 g) in dichloromethane (100 mL) in an ice-bath was sequentially added triethylamine (3 mL) and methanesulfonyl chloride (1.1 g). The reaction mixture was stirred at room temperature for 1.5 hours, diluted with ethyl acetate, and washed with water and brine. The organic layer was dried over Na₂SO₄, filtered, and concentrated to provide the title compound.

### 1.3.3. methyl 2-(5-(1-((3-(2-azidoethoxy)-5,7-dimethyladamantan-1-yl)methyl)-5-methyl-1H-pyrazol-4-yl)-6-(tert-butoxycarbonyl)pyridin-2-yl)-1,2,3,4-tetrahydroisoquinoline-8-carboxylate

To a solution of Example 1.3.2 (16.5 g) in N,N-dimethylformamide (120 mL) was added sodium azide (4.22 g). The mixture was heated at 80°C for 3 hours, cooled, diluted with ethyl acetate and washed with water and brine. The organic layer was dried over Na₂SO₄, filtered, and concentrated. The residue was purified by flash chromatography, eluting with 20% ethyl acetate in heptanes to provide the title compound.

### 1.3.4. 2-(5-(1-((3-(2-azidoethoxy)-5,7-dimethyladamantan-1-yl)methyl)-5-methyl-1H-pyrazol-4-yl)-6-(tert-butoxycarbonyl)pyridin-2-yl)-1,2,3,4-tetrahydroisoquinoline-8-carboxylic acid

To a solution of Example 1.3.3 (10 g) in a mixture of tetrahydrofuran (60 mL), methanol (30 mL) and water (30 mL) was added lithium hydroxide monohydrate (1.2 g). The mixture was stirred at room temperature overnight and neutralized with 2% aqueous HCl. The resulting mixture was concentrated, and the residue was dissolved in ethyl acetate (800 mL), and washed with water and brine. The organic layer was dried over Na₂SO₄, filtered and concentrated to provide the title compound.

### 1.3.5. tert-butyl 3-(1-((3-(2-azidoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

The title compound was prepared by following the procedure described in 1.1.16, replacing Example 1.1.15 with Example 1.3.4.

### 1.3.6. tert-butyl 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

To a solution of Example 1.3.5 (2.0 g) in tetrahydrofuran (30 mL) was added Pd/C (10%, 200 mg). The mixture was stirred under hydrogen atmosphere overnight. The reaction was filtered, and the filtrate was concentrated to provide the title compound.

### 1.3.7. 3-(1-{[3-(2-aminoethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-3,4-dihydroisoquinolin-2(1H)-yl]pyridine-2-carboxylic acid

Example 1.3.6 (300 mg) in dichloromethane (3 mL) was treated with trifluoroacetic acid (3 mL) overnight. The reaction mixture was concentrated ,and the residue was purified by reverse phase chromatography using a Gilson system (300g C18 column), eluting with 10-70% acetonitrile in 0.1% trifluoroacetic acid water solution, to provide the title compound as a trifluoroacetic acid salt. ¹H NMR (400 MHz, dimethyl sulfoxide-d₆) δ ppm 12.85 (s, 1H) 8.03 (d, 1H) 7.79 (d, 1H) 7.59-7.73 (m, 4H) 7.41-7.53 (m, 3H) 7.32-7.40 (m, 2H) 7.29 (s, 1H) 6.96 (d, 1H) 4.96 (s, 2H) 3.89 (t, 2H) 3.83 (s, 2H) 3.50 (t, 2H) 3.02 (t, 2H) 2.84-2.94 (m, 2H) 2.11 (s, 3H) 1.41 (s, 2H) 1.21-1.36 (m, 4H) 1.08-1.19 (m, 4H) 0.96-1.09 (m, 2H) 0.87 (s, 6H). MS (ESI) m/e 744.3 (M-H)⁻.

### 1.4. Synthesis of 3-[1-({3-[2-(2-aminoethoxy)ethoxy]-5,7-dimethyltricyclo[3.3.1.1^{3,7}]dec-1-yl}methyl)-5-methyl-1H-pyrazol-4-yl]-6-[8-(1,3-benzothiazol-2-ylcarbamoyl)-3,4-dihydroisoquinolin-2(1H)-yl]pyridine-2-carboxylic acid (Compound W1.04)

### 1.4.1. 2-(2-((3-((1H-pyrazol-1-yl)methyl)-5,7-dimethyladamantan-1-yl)oxy)ethoxy)ethanol

The title compound was prepared as described in Example 1.1.4 by substituting ethane-1,2-diol with 2,2'-oxydiethanol. MS (ESI) m/e 349.2 (M+H)⁺.

### 1.4.2. 2-(2-((3,5-dimethyl-7-((5-methyl-1H-pyrazol-1-yl)methyl)adamantan-1-yl)oxy)ethoxy)ethanol

The title compound was prepared as described in Example 1.1.5 by substituting Example 1.1.4 with Example 1.4.1. MS (ESI) m/e 363.3 (M+H)⁺.

### 1.4.3. 2-(2-((3-((4-iodo-5-methyl-1H-pyrazol-1-yl)methyl)-5,7-dimethyladamantan-1-yl)oxy)ethoxy)ethanol

The title compound was prepared as described in Example 1.1.6 by substituting Example 1.1.5 with Example 1.4.2. MS (ESI) m/e 489.2 (M+H)⁺.

### 1.4.4. 2-(2-((3-((4-iodo-5-methyl-1H-pyrazol-1-yl)methyl)-5,7-dimethyladamantan-1-yl)oxy)ethoxy)ethyl methanesulfonate

The title compound was prepared as described in Example 1.1.7 by substituting Example 1.1.6 with Example 1.4.3. MS (ESI) m/e 567.2 (M+H)⁺.

### 1.4.5. 2-(2-((3-((4-iodo-5-methyl-1H-pyrazol-1-yl)methyl)-5,7-dimethyladamantan-1-yl)oxy)ethoxy)ethanamine

The title compound was prepared as described in Example 1.1.8 by substituting Example 1.1.7 with Example 1.4.4, and 2N methylamine in methanol with 7N ammonia in methanol. MS (ESI) m/e 488.2 (M+H)⁺.

### 1.4.6. tert-butyl (2-(2-((3-((4-iodo-5-methyl-1H-pyrazol-1-yl)methyl)-5,7-dimethyladamantan-1-yl)oxy)ethoxy)ethyl)carbamate

The title compound was prepared as described in Example 1.1.9 by substituting Example 1.1.8 with Example 1.4.5. MS (ESI) m/e 588.2 (M+H)⁺.

### 1.4.7. methyl 2-(6-(tert-butoxycarbonyl)-5-(1-((3-(2-(2-((tert-butoxycarbonyl)amino)ethoxy)ethoxy)-5,7-dimethyladamantan-1-yl)methyl)-5-methyl-1H-pyrazol-4-yl)pyridin-2-yl)-1,2,3,4-tetrahydroisoquinoline-8-carboxylate

The title compound was prepared as described in Example 1.1.14 by substituting Example 1.1.9 with Example 1.4.6. MS (ESI) m/e 828.5 (M+H)⁺.

### 1.4.8. 2-(6-(tert-butoxycarbonyl)-5-(1-((3-(2-(2-((tert-butoxycarbonyl)amino)ethoxy)ethoxy)-5,7-dimethyladamantan-1-yl)methyl)-5-methyl-1H-pyrazol-4-yl)pyridin-2-yl)-1,2,3,4-tetrahydroisoquinoline-8-carboxylic acid

The title compound was prepared as described in Example 1.1.15 by substituting Example 1.1.14 with Example 1.4.7. MS (ESI) m/e 814.5 (M+H)⁺.

### 1.4.9. tert-butyl 6-(8-(benzo[d]thiazol-2-ylcarbamoyl)-3,4-dihydroisoquinolin-2(1H)-yl)-3-(1-((3-(2-(2-((tert-butoxycarbonyl)amino)ethoxy)ethoxy)-5,7-dimethyladamantan-1-yl)methyl)-5-methyl-1H-pyrazol-4-yl)picolinate

The title compound was prepared as described in Example 1.1.16 by substituting Example 1.1.15 with Example 1.4.8. MS (ESI) m/e 946.2 (M+H)⁺.

### 1.4.10. 3-[1-({3-[2-(2-aminoethoxy)ethoxy]-5,7-dimethyltricyclo[3.3.1.1^{3,7}]dec-1-yl}methyl)-5-methyl-1H-pyrazol-4-yl]-6-[8-(1,3-benzothiazol-2-ylcarbamoyl)-3,4-dihydroisoquinolin-2(1H)-yl]pyridine-2-carboxylic acid

The title compound was prepared as described in Example 1.1.17 by substituting Example 1.1.16 with Example 1.4.9. ¹H NMR (400 MHz, dimethyl sulfoxide-d₆) δ ppm 12.85 (s, 1H), 7.99-8.08 (m, 1H), 7.60-7.82 (m, 4H), 7.20-7.52 (m, 5H), 6.93-6.99 (m, 1H), 4.96 (s, 2H), 3.45-3.60 (m, 6H), 2.09-2.14 (m, 4H), 0.95-1.47 (m, 19H), 0.81-0.91 (m, 6H). MS (ESI) m/e 790.2 (M+H)⁺ .

### 1.5. Synthesis of 6-[8-(1,3-benzothiazol-2-ylcarbamoyl)-3,4-dihydroisoquinolin-2(1H)-yl]-3-{1- [(3- {2- [(2-methoxyethyl)amino]ethoxy}-5,7-dimethyltricyelo[3.3.1.1^{3,7}]dec-1-yl)methyl]-5-methyl-1H-pyrazol-4-yl}pyridine-2-carboxylic acid (Compound W1.05)

### 1.5.1. tert-butyl 6-(8-(benzo[d]thiazol-2-ylcarbamoyl)-3,4-dihydroisoquinolin-2(1H)-yl)-3-(1-((((1r,3r)-3-(2-((2-methoxyethyl)amino)ethoxy)-5,7-dimethyladamantan-1-yl)methyl)-5-methyl-1H-pyrazol-4-yl)picolinate

A solution of Example 1.3.6 (0.050 g) and 2-methoxyacetaldehyde (6.93 mg) were stirred together in dichloromethane(0.5 mL) at room temperature for 1 hour. To the reaction was added a suspension of sodium borohydride (2 mg) in methanol (0.2 mL). After stirring for 30 minutes, the reaction was diluted with dichloromethane (2 mL) and quenched with saturated aqueous sodium bicarbonate (1 mL). The organic layer was separated, dried over magnesium sulfate, filtered, and concentrated to give the title compound. MS (ELSD) m/e 860.5 (M+H)⁺.

### 1.5.2. 6-[8-(1,3-benzothiazol-2-ylcarbamoyl)-3,4-dihydroisoquinolin-2(1H)-yl]-3-{1-[(3-{2-[(2-methoxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid

A solution of Example 1.5.1 in dichloromethane (1 mL) was treated with trifluoroacetic acid (0.5 mL). After stirring overnight, the reaction was concentrated, dissolved in N,N-dimethylformamide (1.5 mL) and water (0.5 mL) and was purified by Prep HPLC using a Gilson system eluting with 10-85% acetonitrile in water containing 0.1% v/v trifluoroacetic acid. The desired fractions were combined and freeze-dried to provide the title compound as a TFA salt. ¹H NMR (400 MHz, dimethyl sulfoxide-*d*₆) δ ppm 12.85 (s, 2H), 8.39 (s, 2H), 8.03 (d, 1H), 7.79 (d, 1H), 7.62 (d, 1H), 7.53-7.42 (m, 3H), 7.40-7.33 (m, 2H), 7.29 (s, 1H), 6.96 (d, 1H), 4.96 (s, 2H), 3.89 (t, 2H), 3.83 (s, 2H), 3.61-3.53 (m, 10H), 3.29 (s, 3H), 3.17-3.09 (m, 2H), 3.09-2.97 (m, 4H), 2.10 (s, 3H), 1.41 (s, 2H), 1.35-1.23 (m, 4H), 1.20-1.10 (m, 4H), 1.10-0.98 (m, 2H). MS (ESI) m/e 804.3 (M+H)⁺.

### 1.6. Synthesis of 3-(1-{[3-(2-aminoethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-5-fluoro-3,4-dihydroisoquinolin-2(1H)-yl]pyridine-2-carboxylic acid (Compound W1.06)

### 1.6.1. 3-Cyanomethyl-4-fluorobenzoic acid methyl ester

To a solution of trimethylsilanecarbonitrile (1.49 mL) in tetrahydrofuran (2.5 mL) was added 1M tetrabutylammonium fluoride (11.13 mL) dropwise over 20 minutes. The solution was then stirred at room temperature for 30 minutes. Methyl 4-fluoro-3-(bromomethyl)benzoate (2.50 g) was dissolved in acetonitrile (12 mL) and was added to the first solution dropwise over 10 minutes. The solution was then heated to 80 °C for 60 minutes and cooled. The solution was concentrated under reduced pressure and was purified by flash column chromatography on silica gel, eluting with 20-30% ethyl acetate in heptanes. The solvent was evaporated under reduced pressure to provide the title compound.

### 1.6.2. 3-(2-Aminoethyl)-4-fluorobenzoic acid methyl ester

Example 1.6.1 (1.84 g) was dissolved in tetrahydrofuran (50 mL), and 1 M borane (in tetrahydrofuran, 11.9 mL) was added. The solution was stirred at room temperature for 16 hours and was slowly quenched with methanol. 4 M Aqueous hydrochloric acid (35 mL) was added, and the solution was stirred at room temperature for 16 hours. The mixture was concentrated under reduced pressure, and the pH was adjusted to between 11 and 12 using solid potassium carbonate. The solution was then extracted with dichloromethane (3x 100 mL). The organic extracts were combined and dried over anhydrous sodium sulfate. The solution was filtered and concentrated under reduced pressure, and the material was purified by flash column chromatography on silica gel, eluting with 10-20% methanol in dichloromethane. The solvent was evaporated under reduced pressure to provide the title compound. MS (ESI) m/e 198 (M+H)⁺.

### 1.6.3. 4-Fluoro-3-[2-(2,2,2-trifluoroacetylamino)ethyl]benzoic acid methyl ester

Example 1.6.2 (1.207 g) was dissolved in dichloromethane (40 mL), and N,N-diisopropylethylamine (1.3 mL) was added. Trifluoroacetic anhydride (1.0 mL) was then added dropwise. The solution was stirred for 15 minutes. Water (40 mL) was added, and the solution was diluted with ethyl acetate (100 mL). 1 M Aqueous hydrochloric acid was added (50 mL), and the organic layer was separated, washed with 1 M aqueous hydrochloric acid, and then washed with brine. The solution was dried on anhydrous sodium sulfate. After filtration, the solvent was evaporated under reduced pressure to provide the title compound.

### 1.6.4. 5-Fluoro-2-(2,2,2-trifluoroacetyl)-1,2,3,4-tetrahydroisoquinoline-8-carboxylic acid methyl ester

Example 1.6.3 (1.795 g) and paraformaldehyde (0.919 g) were placed in a flask and concentrated sulfuric acid (15 mL) was added. The solution was stirred at room temperature for one hour. Cold water (60 mL) was added, and the solution was extracted with ethyl acetate (2x 100 mL). The extracts were combined, washed with saturated aqueous sodium bicarbonate (100 mL) and water (100 mL), and dried over anhydrous sodium sulfate. The solution was filtered, concentrated under reduced pressure, and the material was purified by flash column chromatography on silica gel, eluting with 10-20% ethyl acetate in heptanes. The solvent was evaporated under reduced pressure to provide the title compound. MS (ESI) m/e 323 (M+NH₄)⁺.

### 1.6.5. 5-Fluoro-1,2,3,4-tetrahydroisoquinoline-8-carboxylic acid methyl ester

Example 1.6.4 (685 mg) was dissolved in methanol (6 mL) and tetrahydrofuran (6 mL). Water (3 mL) was added followed by potassium carbonate (372 mg). The reaction was stirred at room temperature for three hours, and then diluted with ethyl acetate (100 mL). The solution was washed with saturated aqueous sodium bicarbonate and dried on anhydrous sodium sulfate. The solvent was filtered and evaporated under reduced pressure to provide the title compound. MS (ESI) m/e 210 (M+H)⁺.

### 1.6.6. 2-(5-Bromo-6-tert-butoxycarbonylpyridin-2-yl)-5-fluoro-1,2,3,4-tetrahydroisoquinoline-8-carboxylic acid methyl ester

The title compound was prepared by substituting Example 1.6.5 for methyl 1,2,3,4-tetrahydroisoquinoline-8-carboxylate hydrochloride in 1.1.12. MS (ESI) m/e 465, 467 (M+H)⁺.

### 1.6.7. 2-[6-tert-Butoxycarbonyl-5-(4,4,5,5-tetramethyl-[1,3,2]dioxaborolan-2-yl)-pyridin-2-yl] -5-fluoro-1,2,3,4-tetrahydro-isoquinoline-8-carboxylic acid methyl ester

The title compound was prepared by substituting Example 1.6.6 for Example 1.1.12 in Example 1.1.13. MS (ESI) m/e 513 (M+H)⁺.

### 1.6.8. 2-((3-((4-iodo-5-methyl-1H-pyrazol-1-yl)methyl)-5,7-dimethyladamantan-1-yl)oxy)ethanamine

A solution of Example 1.1.7 (4.5 g) in 7N ammonium in methanol (15 mL) was stirred at 100°C for 20 minutes under microwave conditions (Biotage Initiator). The reaction mixture was concentrated under vacuum, and the residue was diluted with ethyl acetate (400 mL) and washed with aqueous NaHCO₃, water (60 mL) and brine (60 mL). The organic layer was dried with anhydrous Na₂SO₄, filtered and concentrated. The residue was used in the next reaction without further purification. MS (ESI) m/e 444.2 (M+H)⁺.

### 1.6.9. tert-butyl (2-((3-((4-iodo-5-methyl-1H-pyrazol-1-yl)methyl)-5,7-dimethyladamantan-1-yl)oxy)ethyl)carbamate

To a solution of Example 1.6.8 (4.4 g) in tetrahydrofuran (100 mL) was added di-t-butyl dicarbonate (2.6 g) and N,N-dimethyl-4-aminopyridine (100 mg). The mixture was stirred for 1.5 hours. The reaction mixture was then diluted with ethyl acetate (300 mL) and washed with aqueous NaHCO₃, water (60 mL) and brine (60 mL). After drying (anhydrous Na₂SO₄), the solution was filtered and concentrated and the residue was purified by silica gel column chromatography (20% ethyl acetate in dichloromethane) to give the title compound. MS (ESI) m/e 544.2 (M+H)⁺.

### 1.6.10. 2-(6-tert-Butoxycarbonyl-5-{1-[5-(2-tert-butoxycarbonylamino-ethoxy)-3,7-dimethyl-adamantan-1-ylmethyl]-5-methyl-1H-pyrazol-4-yl}-pyridin-2-yl)-5-fluoro-1,2,3,4-tetrahydro-isoquinoline-8-carboxylic acid methyl ester

The title compound was prepared by substituting Example 1.6.7 for Example 1.1.13 and Example 1.6.9 for Example 1.1.9 in Example 1.1.14. MS (ESI) m/e 802 (M+H)⁺.

### 1.6.11. 2-(6-tert-Butoxycarbonyl-5- {1- [5-(2-tert-butoxycarbonylamino-ethoxy)-3,7-dimethyl-adamantan-1-ylmethyl]-5-methyl-1H-pyrazol-4-yl}-pyridin-2-yl)-5-fluoro-1,2,3,4-tetrahydro-isoquinoline-8-carboxylic acid

The title compound was prepared by substituting Example 1.6.10 for Example 1.1.14 in Example 1.1.15. MS (ESI) m/e 788 (M+H)⁺.

### 1.6.12. 6-[8-(Benzothiazol-2-ylcarbamoyl)-5-fluoro-3,4-dihydro-1H-isoquinolin-2-yl]-3-{1-[5-(2-tert-butoxycarbonylamino-ethoxy)-3,7-dimethyl-adamantan-1-ylmethyl]-5-methyl-1H-pyrazol-4-yl}-pyridine-2-carboxylic acid tert-butyl ester

The title compound was prepared by substituting Example 1.6.11 for Example 1.1.15 in Example 1.1.16. MS (ESI) m/e 920 (M+H)⁺.

### 1.6.13. 3-(1-{[3-(2-aminoethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-5-fluoro-3,4-dihydroisoquinolin-2(1H)-yl]pyridine-2-carboxylic acid

The title compound was prepared by substituting Example 1.6.12 for Example 1.1.16 in Example 1.1.17. ¹H NMR (400MHz, dimethyl sulfoxide-*d*₆) δ ppm 12.88 (bs, 1H), 8.03 (d, 1H), 7.79 (d, 1H), 7.73 (m, 1H), 7.63 (m, 2H), 7.52 (d, 1H), 7.48 (t, 1H), 7.36 (t, 1H), 7.28 (dd, 2H), 7.04 (d, 1H), 5.02 (s, 2H), 3.95 (t, 2H), 3.83 (s, 2H), 3.49 (t, 2H), 2.90 (m, 4H), 2.11 (s, 3H), 1.41 (s, 2H), 1.35-1.23 (m, 4H), 1.19-0.99 (m, 6H), 0.87 (bs, 6H). MS (ESI) m/e 764 (M+H)⁺.

### 1.7 Synthesis of 3-(1-{[3-(2-aminoethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-6-fluoro-3,4-dihydroisoquinolin-2(1H)-yl]pyridine-2-carboxylic acid

### 1.7.1 (3-bromo-5-fluoro-phenyl)-acetonitrile

The title compound was prepared by substituting 1-bromo-3-(bromomethyl)-5-fluorobenzene for methyl 4-fluoro-3-(bromomethyl)benzoate in Example 1.6.1.

### 1.7.2 2-(3-bromo-5-fluoro-phenyl)-ethylamine

The title compound was prepared by substituting Example 1.7.1 for Example 1.6.1 in Example 1.6.2.

### 1.7.3 [2-(3-bromo-5-fluoro-phenyl)-ethyl]-carbamic acid tert-butyl ester

Example 1.7.2 (1.40 g) and N,N-dimethylpyridin-4-amine (0.078 g) were dissolved in acetonitrile (50 mL). Di-tert-butyl dicarbonate (1.54 g) was added. The solution was stirred at room temperature for 30 minutes. The solution was diluted with diethyl ether (150 mL), washed with 0.1 M aqueous HCl (25 mL) twice, washed with brine (50 mL), and dried on anhydrous sodium sulfate. The solution was filtered, concentrated under reduced pressure, and the crude material was purified by flash column chromatography on silica gel, eluting with 5-10% ethyl acetate in heptanes. The solvent was evaporated under reduced pressure to provide the title compound.

### 1.7.4 3-(2-tert-butoxycarbonylamino-ethyl)-5-fluoro-benzoic acid methyl ester

Example 1.7.3 (775 mg) and dichloro[1,1'-bis(diphenylphosphino)ferrocene]palladium(II) (36 mg) were added to a 50 mL pressure bottle. Methanol (10 mL) and trimethylamine (493 mg) were added. The solution was degassed and flushed with argon three times, followed by degassing and flushing with carbon monoxide. The reaction was heated to 100 °C for 16 hours under 60 psi of carbon monoxide. Additional dichloro[1,1'-bis(diphenylphosphino)ferrocene]palladium(II) (36 mg) was added and the degassing and flushing procedure was repeated. The reaction was heated to 100 °C for an additional 16 hours under 60 psi of carbon monoxide. The solvent was removed under reduced pressure, and the residue was purified by flash column chromatography on silica gel, eluting with 20-30% ethyl acetate in heptanes. The solvent was evaporated under reduced pressure to provide the title compound.

### 1.75 3-(2-amino-ethyl)-5-fluoro-benzoic acid methyl ester

Example 1.7.4 (292 mg) was dissolved in dichloromethane (3 mL). 2,2,2-Trifluoroacetic acid (1680 mg) was added, and the solution was stirred at room temperature for two hours. The solvent was removed under reduced pressure to provide the title compound which was used in the next step without further purification.

### 1.7.6 3-fluoro-5-[2-(2,2,2-trifluoro-acetylamino)-ethyl]-benzoic acid methyl ester

The title compound was prepared by substituting Example 1.7.5 for Example 1.6.2 in Example 1.6.3.

### 1.7.7 6-fluoro-2-(2,2,2-trifluoro-acetyl)-1,2,3,4-tetrahydro-isoquinoline-8-carboxylic acid methyl ester

The title compound was prepared by substituting Example 1.7.6 for Example 1.6.3 in Example 1.6.4.

### 1.7.8 6-fluoro-1,2,3,4-tetrahydro-isoquinoline-8-carboxylic acid methyl ester

The title compound was prepared by substituting Example 1.7.7 for Example 1.6.4 in Example 1.6.5.

### 1.7.9 2-(5-bromo-6-tert-butoxycarbonyl-pyridin-2-yl)-6-fluoro-1,2,3,4-tetrahydro-isoquinoline-8-carboxylic acid methyl ester

The title compound was prepared by substituting Example 1.7.8 for methyl 1,2,3,4-tetrahydroisoquinoline-8-carboxylate hydrochloride in Example 1.1.12. MS (ESI) m/e 464, 466 (M+H)⁺.

### 1.7.10 2-[6-tert-butoxycarbonyl-5-(4,4,5,5-tetramethyl-[1,3,2]dioxaborolan-2-yl)-pyridin-2-yl]-6-fluoro-1,2,3,4-tetrahydro-isoquinoline-8-carboxylic acid methyl ester

The title compound was prepared by substituting Example 1.7.9 for Example 1.1.12 in Example 1.1.13. MS (ESI) m/e 513 (M+H)⁺, 543 (M+MeOH-H)⁻.

### 1.7.11 {2-[5-(4-iodo-5-methyl-pyrazol-1-ylmethyl)-3,7-dimethyl-adamantan-1-yloxy]-ethyl}-di-tert-butyl iminodicarboxylate

Example 1.1.6 (5.000 g) was dissolved in dichloromethane (50 mL). Triethylamine (1.543 g) was added, and the solution was cooled on an ice bath. Methanesulfonyl chloride (1.691 g) was added dropwise. The solution was allowed to warm to room temperature and stir for 30 minutes. Saturated aqueous sodium bicarbonate solution (50 mL) was added. The layers were separated, and the organic layer was washed with brine (50 mL). The aqueous portions were then combined and back extracted with dichloromethane (50 mL). The organic portions were combined, dried over anhydrous sodium sulfate, filtered, and concentrated. The residue was dissolved in acetonitrile (50 mL). Di-tert-butyl iminodicarboxylate (2.689 g) and cesium carbonate (7.332 g) were added, and the solution was refluxed for 16 hours. The solution was cooled and added to diethyl ether (100 mL) and water (100 mL). The layers were separated. The organic portion was washed with brine (50 mL). The aqueous portions were then combined and back extracted with diethyl ether (100 mL). The organic portions were combined, dried over anhydrous sodium sulfate, filtered, and concentrated under reduced pressure. The material was purified by flash column chromatography on silica gel, eluting with 20% ethyl acetate in heptanes. The solvent was evaporated under reduced pressure to provide the title compound. MS (ESI) m/e 666 (M+Na)⁺.

### 1.7.12 methyl 2-(6-(tert-butoxycarbonyl)-5-(1-((3-(2-(di-(tert-butoxycarbonyl)amino)ethoxy)-5,7-dimethyladamantan-1-yl)methyl)-5-methyl-1H-pyrazol-4-yl)pyridin-2-yl)-6-fluoro-1,2,3,4-tetrahydroisoquinoline-8-carboxylate

The title compound was prepared by substituting Example 1.7.10 for Example 1.1.13 and Example 1.7.11 for Example 1.1.9 in Example 1.1.14. MS (ESI) m/e 902 (M+H)⁺.

### 1.7.13 2-(6-(tert-butoxycarbonyl)-5-(1-((3-(2-(di-(tert-butoxycarbonyl)amino)ethoxy)-5,7-dimethyladamantan-1-yl)methyl)-5-methyl-1H-pyrazol-4-yl)pyridin-2-yl)-6-fluoro-1,2,3,4-tetrahydroisoquinoline-8-carboxylic acid

The title compound was prepared by substituting Example 1.7.12 for Example 1.1.14 in Example 1.1.15. MS (ESI) m/e 888 (M+H)⁺, 886 (M-H)⁻.

### 1.7.14 tert-butyl 6-(8-(benzo[d]thiazol-2-ylcarbamoyl)-6-fluoro-3,4-dihydroisoquinolin-2(1H)-yl)-3-(1-((3-(2-(di-(tert-butoxycarbonyl)amino)ethoxy)-5,7-dimethyladamantan-1-yl)methyl)-5-methyl-1H-pyrazol-4-yl)picolinate

The title compound was prepared by substituting Example 1.7.13 for Example 1.1.15 in Example 1.1.16.

### 1.7.15 3-(1-{[3-(2-aminoethoxy)-5,7-dimethyitricyclo[3.3.1.1^{3,7}]dec-1-yl]methyl}-5-methyl-1H-pyrazol-4-yl)-6-[8-(1,3-benzothiazol-2-ylcarbamoyl)-6-fluoro-3,4-dihydroisoquinolin-2(1H)-yl]pyridine-2-carboxylic acid

The title compound was prepared by substituting Example 1.7.14 for Example 1.1.16 in Example 1.1.17. ¹H NMR (400 MHz, dimethyl sulfoxide-*d*₆) δ ppm 8.04 (d, 1H), 7.79 (d, 1H), 7.65 (bs, 3H), 7.50 (m, 2H), 7.40-7.29 (m, 3H), 6.98 (d, 1H), 4.91 (d, 2H), 3.88 (t, 2H), 3.83 (s, 2H), 3.02 (t, 2H), 2.89 (t, 4H), 2.10 (s, 3H), 1.44-1.20 (m, 6H), 1.19-1.00 (m, 6H), 0.86 (bs, 6 H). MS (ESI) m/e 764 (M+H)⁺, 762 (M-H)⁻.

### 1.8 Synthesis of 3-(1-{[3-(2-aminoethoxy)-5,7-dimethyitricyclo[3.3.1.1^{3,7}]dec-1-yl]methyl}-5-methyl-1H-pyrazol-4-yl)-6-[8-(1,3-benzothiazol-2-ylcarbamoyl)-7-fluoro-3,4-dihydroisoquinolin-2(1H)-yl]pyridine-2-carboxylic acid

### 1.8.1 [2-(3-bromo-4-fluoro-phenyl)-ethyl]-carbamic acid tert-butyl ester

The title compound was prepared by substituting 2-(3-bromo-4-fluorophenyl)ethanamine hydrochloride for Example 1.7.2 in Example 1.7.3.

### 1.8.2 5-(2-tert-butoxycarbonylamino-ethyl)-2-fluoro-benzoic acid methyl ester

The title compound was prepared by substituting Example 1.8.1 for Example 1.7.3 in Example 1.7.4. MS (ESI) m/e 315 (M+NH₄)⁺.

### 1.8.3 5-(2-amino-ethyl)-2-fluoro-benzoic acid methyl ester

The title compound was prepared by substituting Example 1.8.2 for Example 1.7.4 in Example 1.7.5.

### 1.8.4 2-fluoro-5-[2-(2,2,2-trifluoro-acetylamino)-ethyl]-benzoic acid methyl ester

The title compound was prepared by substituting Example 1.8.3 for Example 1.6.2 in Example 1.6.3.

### 1.8.5 7-fluoro-2-(2,2,2-trifluoro-acetyl)-1,2,3,4-tetrahydro-isoquinoline-8-carboxylic acid methyl ester

The title compound was prepared by substituting Example 1.8.4 for Example 1.6.3 in Example 1.6.4. MS (ESI) m/e 323 (M+NH₄)⁺.

### 1.8.6 7-fluoro-1,2,3,4-tetrahydro-isoquinoline-8-carboxylic acid methyl ester

The title compound was prepared by substituting Example 1.8.5 for Example 1.6.4 in Example 1.6.5. MS (ESI) m/e 210 (M+H)⁺, 208 (M-H)⁻.

### 1.8.7 2-(5-bromo-6-tert-butoxycarbonyl-pyridin-2-yl)-7-fluoro-1,2,3,4-tetrahydro-isoquinoline-8-carboxylic acid methyl ester

The title compound was prepared by substituting Example 1.8.6 for methyl 1,2,3,4-tetrahydroisoquinoline-8-carboxylate hydrochloride in Example 1.1.12. MS (ESI) m/e 465,467 (M+H)⁺.

### 1.8.8 2-[6-tert-butoxycarbonyl-5-(4,4,5,5-tetramethyl-[1,3,2]dioxaborolan-2-yl)-pyridin-2-yl]-7-fluoro-1,2,3,4-tetrahydro-isoquinoline-8-carboxylic acid methyl ester

The title compound was prepared by substituting Example 1.8.7 for Example 1.1.12 in Example 1.1.13. MS (ESI) m/e 513 (M+H)⁺, 543 (M+MeOH-H)⁻.

### 1.8.9 methyl 2-(6-(tert-butoxycarbonyl)-5-(1-((3-(2-(di-(tert-butoxycarbonyl)amino)ethoxy)-5,7-dimethyladamantan-1-yl)methyl)-5-methyl-1H-pyrazol-4-yl)pyridin-2-yl)-7-fluoro-1,2,3,4-tetrahydroisoquinoline-8-carboxylate

The title compound was prepared by substituting Example 1.8.8 for Example 1.1.13 and Example 1.7.11 for Example 1.1.9 in Example 1.1.14. MS (ESI) m/e 902 (M+H)⁺, 900 (M-H)⁻.

### 1.8.10 2-(6-(tert-butoxycarbonyl)-5-(1-((3-(2-(di-(tert-butoxycarbonyl)amino)ethoxy)-5,7-dimethyladamantan-1-yl)methyl)-5-methyl-1H-pyrazol-4-yl)pyridin-2-yl)-7-fluoro-1,2,3,4-tetrahydroisoquinoline-8-carboxylic acid

The title compound was prepared by substituting Example 1.8.9 for Example 1.1.14 in Example 1.1.15. MS (ESI) m/e 788 (M+H)⁺, 786 (M-H)⁻.

### 1.8.11 tert-butyl 6-(8-(benzo[d]thiazol-2-ylcarbamoyl)-7-fluoro-3,4-dihydroisoquinolin-2(1H)-yl)-3-(1-((3-(2-(di-(tert-butoxycarbonyl)amino)ethoxy)-5,7-dimethyladamantan-1-yl)methyl)-5-methyl-1H-pyrazol-4-yl)picolinate

The title compound was prepared by substituting Example 1.8.10 for Example 1.1.15 in Example 1.1.16.

### 1.8.12 3-(1-{[3-(2-aminoethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-7-fluoro-3,4-dihydroisoquinolin-2(1H)-yl]pyridine-2-carboxylic acid

The title compound was prepared by substituting Example 1.8.11 for Example 1.1.16 in Example 1.1.17. ¹H NMR (400 MHz, dimethyl sulfoxide-*d*₆) δ ppm 13.08 (bs, 1H), 11.41 (bs, 1H), 8.05 (d, 1H), 7.81 (d, 1H), 7.63 (m, 4H), 7.55-7.22 (m, 6H), 6.95 (d, 1H), 4.78 (s, 2H), 3.86 (m, 4H), 3.50 (m, 2H), 2.97 (m, 2H), 2.90 (m, 2H), 2.09 (s, 3H), 1.48-1.40 (m, 2H), 1.38-1.23 (m, 4H), 1.20-1.01 (m, 6H), 0.88 (bs, 6H). MS (ESI) m/e 764 (M+H)⁺, 762 (M-H)⁻.

### Example 2. Synthesis of Exemplary Synthons

This example provides synthetic methods for exemplary synthons that may be used to make ADCs.

### 2.1. Synthesis of N-[19-(2,5-dioxo-2,5-dihydro-1H-pyrrol-1-yl)-17-oxo-4,7,10,13-tetraoxa-16-azanonadecan-1-oyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl) carbamoyl}oxy)methyl]phenyl}-N⁵-carbamoyl-L-ornithinamide (Synthon E)

### 2.1.1. (S)-(9H-fluoren-9-yl)methyl(1-((4-(hydroxymethyl) phenyl)amino)-1-oxo-5-ureidopentan-2-yl)carbamate

(S)-2-((((9H-fluoren-9-yl)methoxy)carbonyl)amino)-5-ureidopentanoic acid (40 g) was dissolved in dichloromethane (1.3L). (4-Aminophenyl)methanol (13.01 g), 2-(3H-[1,2,3]triazolo[4,5-b]pyridin-3-yl)-1,1,3,3-tetramethylisouronium hexafluorophosphate(V) (42.1 g) and N,N-diisopropylethylamine (0.035 L) were added to the solution, and the resulting mixture was stirred at room temperature for 16 hours. The product was collected by filtration and rinsed with dichloromethane. The combined solids were dried under vacuum to yield the title compound, which was used in the next step without further purification. MS (ESI) m/e 503.3 (M+H)⁺.

### 2.1.2. (S)-2-amino-N-(4-(hydroxymethyl)phenyl)-5-ureidopentanamide

Example 2.1.1 (44 g) was dissolved in N,N-dimethylformamide (300 mL). The solution was treated with diethylamine (37.2 mL) and stirred for one hour at room temperature. The reaction mixture was filtered, and the solvent was concentrated under reduced pressure. The crude product was purified by basic alumina chromatography eluting with a gradient of 0-30% methanol in ethyl acetate to give the title compound. MS (ESI) m/e 281.2 (M+H)⁺.

### 2.1.3. tert-butyl ((S)-1-(((S)-1-((4-(hydroxymethyl)phenyl)amino)-1-oxo-5-ureidopentan-2-yl)amino)-3-methyl-1-oxobutan-2-yl)carbamate

(S)-2-(Tert-butoxycarbonylamino)-3-methylbutanoic acid (9.69 g) was dissolved in N,N-dimethylformamide (200 mL). To the solution was added 2-(3H-[1,2,3]triazolo[4,5-b]pyridin-3-yl)-1,1,3,3-tetramethylisouronium hexafluorophosphate(V) (18.65 g), and the reaction was stirred for one hour at room temperature. Example 2.1.2 (12.5 g) and N,N-diisopropylethylamine (15.58 mL) were added and the reaction mixture was stirred for 16 hours at room temperature. The solvent was concentrated under reduced pressure and the residue was purified by silica gel chromatography, eluting with 10% methanol in dichloromethane, to give the title compound. MS (ESI) m/e 480.2 (M+H)⁺.

### 2.1.4. (S)-2-((S)-2-amino-3-methylbutanamido)-N-(4-(hydroxymethyl)phenyl)-5-ureidopentanamide

Example 2.1.4 (31.8 g) was dissolved in dichloromethane (650 mL) and to the solution was added trifluoroacetic acid (4.85 mL). The reaction mixture was stirred for three hours at room temperature. The solvent was concentrated under reduced pressure to yield a mixture of the crude title compound and 4-((S)-2-((S)-2-amino-3-methylbutanamido)-5-ureidopentanamido)benzyl 2,2,2-trifluoroacetate. The crude material was dissolved in a 1:1 dioxane/water solution (300 mL) and to the solution was added sodium hydroxide (5.55 g). The mixture was stirred for three hours at room temperature. The solvent was concentrated under vacuum, and the crude product was purified by reverse phase HPLC using a CombiFlash system, eluting with a gradient of 5-60% acetonitrile in water containing 0.05% v/v ammonium hydroxide, to give the title compound. MS (ESI) m/e 380.2 (M+H)⁺.

### 2.1.5. 1-(3-(2,5-dioxo-2,5-dihydro-1H-pyrrol-1-yl)propanamido)-N-((S)-1-(((S)-1-((4-(hydroxymethyl)phenyl)amino)-1-oxo-5-ureidopentan-2-yl)amino)-3-methyl-1-oxobutan-2-yl)-3,6,9,12-tetraoxapentadecan-15-amide

To a solution of Example 2.1.4 (1.5 g) in N,N-dimethylformamide (50 mL) was added 2,5 -dioxopyrrolidin-1-yl 1-(2,5-dioxo-2,5-dihydro-1H-pyrrol-1-yl)-3-oxo-7,10,13,16-tetraoxa-4-azanonadecan-19-oate (2.03 g). The mixture was stirred at room temperature for three days. The crude material was added to a reverse phase column (C18, SF65-800g) and was eluted with 20-100% acetonitrile in water with 0.1% trifluoroacetic acid to afford the title compound. MS (ESI) m/e 778.3 (M+1)⁺.

### 2.1.6. 4-((2S,5S)-25-(2,5-dioxo-2,5-dihydro-1H-pyrrol-1-yl)-5-isopropyl-4,7,23-trioxo-2-(3-ureidopropyl)-10,13,16,19-tetraoxa-3,6,22-triazapentacosanamido)benzyl (4-nitrophenyl) carbonate

To a solution of Example 2.1.5 (2.605 g) and N,N-diisopropylamine (1.8 mL) in N,N-dimethylformamide (20 mL) was added bis(4-nitrophenyl) carbonate (1.23 g). The mixture was stirred at room temperature for 16 hours. The crude material was added to a reverse phase column (C18, SF65-800g) and was eluted with 20-100% acetonitrile in water with 0.1% trifluoroacetic acid to afford the title compound. MS (ESI) m/e 943.2 (M+1)⁺.

### 2.1.7. N-[19-(2,5-dioxo-2,5-dihydro-1H-pyrrol-1-yl)-17-oxo-4,7,10,13-tetraoxa-16-azanonadecan-1-oyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide

To a mixture of Example 2.1.6 (49.6 mg) and Example 1.1.17 (30 mg) in N,N-dimethylformamide (2 mL) at 0 °C was added N,N-diisopropylethylamine (0.018 mL). The reaction mixture was stirred at room temperature overnight, diluted with dimethyl sulfoxide, and purified by RP-HPLC using a Gilson system, eluting with 20-70% acetonitrile in 0.1% trifluoroacetic acid water solution to provide the title compound. MS (ESI) m/e 1563.4 (M+H)⁺.

### 2.2. Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide (Synthon D)

To a solution of 4-((S)-2-((S)-2-(6-(2,5-dioxo-2,5-dihydro-1H-pyrrol-1-yl)hexanamido)-3-methylbutanamido)-5-ureidopentanamido)benzyl 4-nitrophenyl carbonate (purchased from Synchem, 57 mg) and Example 1.1.17 (57 mg) in N,N-dimethylformamide (6 mL) was added N,N-diisopropylethylamine (0.5 mL). The mixture was stirred overnight and then concentrated under vacuum. The residue was diluted with methanol (3 mL) and acetic acid (0.3 mL) and purified by RP-HPLC (Gilson system, C18 column), eluting with 30-70% acetonitrile in water containing 0.1% trifluoroacetic acid. Lyophilization of the product fractions gave the title compound. ¹H NMR (300 MHz, dimethyl sulfoxide-d₆) δ ppm 12.86 (d, 1H), 9.98 (s, 1H), 7.96-8.10 (m, 2H), 7.74-7.83 (m, 2H), 7.54-7.64 (m, 3H), 7.31-7.52 (m, 6H), 7.24-7.29 (m, 3H), 6.99 (s, 2H), 6.94 (d, 1H), 4.96 (d, 4H), 4.33-4.43 (m, 2H), 4.12-4.24 (m, 2H), 3.22-3.42 (m, 7H), 2.77-3.07 (m, 7H), 1.86-2.32 (m, 7H), 0.92-1.70 (m, 22H), 0.72-0.89 (m, 13H). MS (ESI) m/e 1358.2 (M+H)⁺ .

### 2.3. Synthesis of N-[19-(2,5-dioxo-2,5-dihydro-1H-pyrrol-1-yl)-17-oxo-4,7,10,13-tetraoxa-16-azanonadecan-1-oyl]-L-alanyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-L-alaninamide (Synthon J)

### 2.3.1. (S)-2-((S)-2-((((9H-fluoren-9-yl)methoxy)carbonyl) amino)propanamido)propanoic acid

A solution of (S)-2,5-dioxopyrrolidin-1-yl 2-((((9H-fluoren-9-yl)methoxy)carbonyl)amino)propanoate (5 g) in 40 mL dimethoxyethane was added to a solution of L-alanine (1.145 g) and sodium bicarbonate (1.08 g) in water (40 mL). The reaction mixture was stirred at room temperature for 16 hours. Aqueous citric acid (15% v/v, 75 mL) was added to the reaction. The precipitate was filtered, washed with water (2 x 250 mL) and dried under vacuum. The solid was further triturated with diethyl ether (100 mL), filtered, and dried over sodium sulfate to yield the product, which was used in the next step without further purification. MS (ESI) m/e 383.0 (M+H)⁺.

### 2.3.2. (9H-fluoren-9-yl)methyl ((S)-1-(((S)-1-((4-(hydroxymethyl)phenyl)amino)-1-oxopropan-2-yl)amino)-1-oxopropan-2-yl)carbamate

N-Ethoxycarbonyl-2-ethoxy-1,2-dihydroquinoline (EEDQ) (6.21 g) was added to a solution of Example 2.3.1 (3.2 g) and 4-aminobenzyl alcohol (1.546 g) in 50 mL of 2:1 dichloromethane:methanol. The reaction was stirred at room temperature for 2 days. The solvent was concentrated under vacuum. The residue was triturated with 75 mL of ethyl acetate, and the solid was collected by filtration, and dried under vacuum to yield the title compound, which was used in the next step without further purification. MS (ESI) m/e 488.0 (M+H)⁺.

### 2.3.3. (S)-2-amino-N-((S)-1-((4-(hydroxymethyl)phenyl)amino)-1-oxopropan-2-yl)propanamide

Diethylamine (11.75 mL) was added to a solution of Example 2.3.2 (1.58 g) in N,N dimethylformamide (50 mL), and the reaction was allowed to stand at room temperature for 16 hours. The solvent was evaporated under vacuum. The residue was triturated with ethyl acetate (100 mL), and the product was collected by filtration and dried under vacuum to yield the title compound, which was used in the next step without further purification. MS (ESI) m/e 266.0 (M+H)⁺.

### 2.3.4. 1-(3-(2,5-dioxo-2,5-dihydro-1H-pyrrol-1-yl)propanamido)-N-((S)-1-(((S)-1-((4-(hydroxymethyl)phenyl)amino)-1-oxopropan-2-yl)amino)-1-oxopropan-2-yl)-3,6,9,12-tetraoxapentadecan-15-amide

Example 2.3.3 (1.033 g) was mixed with 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate (2 g) in N,N-dimethylformamide (19.5 mL) with 1% N,N-diisopropylethylamine for 16 hours. The crude reaction was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. MS (ESI) m/e 664.0 (M+H)⁺.

### 2.3.5. 4-((2S,5S)-25-(2,5-dioxo-2,5-dihydro-1H-pyrrol-1-yl)-2,5-dimethyl-4,7,23-trioxo-10,13,16,19-tetraoxa-3,6,22-triazapentacosanamido)benzyl (4-nitrophenyl) carbonate

Example 2.3.4 (1.5 g) was mixed with bis(4-nitrophenyl)carbonate (1.38 g) in N,N-dimethylformamide (11.3 mL) with 1% N,N-diisopropylethylamine. The reaction was stirred at room temperature for 16 hours. The crude reaction was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. MS (ESI) m/e 829.0 (M+H)⁺.

### 2.3.6. N-[19-(2,5-dioxo-2,5-dihydro-1H-pyrrol-1-yl)-17-oxo-4,7,10,13-tetraoxa-16-azanonadecan-1-oyl]-L-alanyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-L-alaninamide

The trifluoroacetic acid salt of Example 1.1.17 (15 mg) was mixed with Example 2.3.5 (21.3 mg) in N,N-dimethylformamide (1 mL) and N,N-diisopropylethylamine (0.006 mL). The reaction mixture was stirred at room temperature for one hour. The crude reaction was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. MS (ESI) m/e 1450.7 (M+H)⁺.

### 2.4. Synthesis of N-[6-(2,5-dioxo-2,5-dihydro-1H-pyrrol-1-yl)hexanoyl]-L-alanyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-L-alaninamide (Synthon K)

### 2.4.1. 6-(2,5-dioxo-2,5-dihydro-1H-pyrrol-1-yl)-N-((S)-1-(((S)-1-((4-(hydroxymethyl)phenyl)amino)-1-oxopropan-2-yl)amino)-1-oxopropan-2-yl)hexanamide

The title compound was prepared by substituting N-succinimidyl 6-maleimidohexanoate for 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate in Example 2.3.4. MS (ESI) m/e 640.8 (M+NH₄)⁺.

### 2.4.2. 4-((S)-2-((S)-2-(6-(2,5-dioxo-2,5-dihydro-1H-pyrrol-1-yl)hexanamido)propanamido)propanamido)benzyl(4-nitrophenyl)carbonate

The title compound was prepared by substituting Example 2.4.1 for Example 2.3.4 in Example 2.3.5.

### 2.4.3. N-[6-(2,5-dioxo-2,5-dihydro-1H-pyrrol-1-yl)hexanoyl]-L-alanyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-L-alaninamide

The title compound was prepared by substituting Example 2.4.2 for Example 2.3.5 in Example 2.3.6. ¹H NMR (400 MHz, dimethyl sulfoxide-d₆) δ ppm 9.56 (s, 1H), 7.98 (d, 1H), 7.76 (d, 1H), 7.71-7.52 (m, 3H), 7.51-7.21 (m, 4H), 6.97-6.84 (m, 1H), 4.98 (d, 2H), 4.42 (p, 1H), 4.27 (p, 1H), 3.89 (t, 1H), 3.80 (s, 2H), 3.43 (d, 19H), 3.03 (t, 7H), 2.87 (s, 2H), 2.32 (s, 1H), 2.11 (d, 3H), 1.52 (h, 2H), 1.41-0.94 (m, 12H), 0.84 (s, 3H). MS (ESI) m/e 1244.2 (M+H)⁺.

### 2.5. Synthesis of N-[6-(2,5-dioxo-2,5-dihydro-1H-pyrrol-1-yl)hexanoyl]-L-valyl-N-{4-[12-({(1s,3s)-3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]tricyclo[3.3.1.1^{3,7}]dec-1-yl}oxy)-4-methyl-3-oxo-2,7,10-trioxa-4-azadodec-1-yl]phenyl}-N⁵-carbamoyl- L-ornithinamide (Synthon L)

### 2.5.1. (3-bromoadamantan-1-yl)methanol

The title compound was prepared by substituting 3-bromoadamantane-1-carboxylic acid for Example 1.1.1 in Example 1.1.2.

### 2.5.2. 1-((3-bromoadamantan-1-yl)methyl)-1H-pyrazole

The title compound **was prepared** by substituting Example 2.5.1 for Example 1.1.2 in Example 1.1.3. MS (ESI) m/e 295.2 (M+H)⁺.

### 2.5.3. 2-(2-(2-((3-((1H-pyrazol-1-yl)methyl)adamantan-1-yl)oxy)ethoxy)ethoxy)ethanol

The title compound was prepared by substituting Example 2.5.2 for Example 1.1.3 and substituting silver sulfate for triethylamine in Example 1.2.1. MS (ESI) m/e 365.1 (M+H)⁺.

### 2.5.4. 2-(2-(2-((3-((5-methyl-1H-pyrazol-1-yl)methyl)adamantan-1-yl)oxy)ethoxy)ethoxy)ethanol

The title compound was prepared by substituting Example 2.5.3 for Example 1.2.1 in Example 1.2.2. MS (ESI) m/e 379.1 (M+H)⁺.

### 2.5.5. 2-(2-(2-((3-((4-iodo-5-methyl-1H-pyrazol-1-yl)methyl)adamantan-1-yl)oxy)ethoxy)ethoxy)ethanol

The title compound was prepared by substituting Example 2.5.4 for Example 1.2.2 in Example 1.2.3. MS (ESI) m/e 504.9 (M+H)⁺.

### 2.5.6. 2-(2-(2-((3-((4-iodo-5-methyl-1H-pyrazol-1-yl)methyl)adamantan-1-yl)oxy)ethoxy)ethoxy)-N-methylethanamine

The title compound was prepared by substituting Example 2.5.5 for Example 1.2.3 in Example 1.2.4. MS (ESI) m/e 518.4 (M+H)⁺.

### 2.5.7. tert-butyl (2-(2-(2-((3-((4-iodo-5-methyl-1H-pyrazol-1-yl)methyl)adamantan-1-yl)oxy)ethoxy)ethoxy)ethyl)(methyl)carbamate

The title compound was prepared by substituting Example 2.5.6 for Example 1.2.4 in Example 1.2.5. MS (ESI) m/e 617.9 (M+H)⁺.

### 2.5.8. tert-butyl methyl(2-(2-(2-((3-((5-methyl-4-(4,4,5,5-tetramethyl-1,3,2-dioxaborolan-2-yl)-1H-pyrazol-1-yl)methyl)adamantan-1-yl)oxy)ethoxy)ethoxy)ethyl)carbamate

The title compound was prepared by substituting Example 2.5.7 for Example 1.2.5 in Example 1.2.6. MS (ESI) m/e 618.2 (M+H)⁺.

### 2.5.9. tert-butyl 6-(8-(benzo[d]thiazol-2-ylcarbamoyl)-3,4-dihydroisoquinolin-2(1H)-yl)-3-(5-methyl-1-((3-((2,2,5-trimethyl-4-oxo-3,8,11-trioxa-5-azatridecan-13-yl)oxy)adamantan-1-yl)methyl)-1H-pyrazol-4-yl)picolinate

The title compound was prepared by substituting Example 2.5.8 for Example 1.2.6 in Example 1.2.10. MS (ESI) m/e 976.1 (M+H)⁺.

### 2.5.10. 6-(8-(benzo[d]thiazol-2-ylcarbamoyl)-3,4-dihydroisoquinolin-2(1H)-yl)-3-(5-methyl-1-(((1s,3s)-3-(2-(2-(2-(methylamino)ethoxy)ethoxy)ethoxy)adamantan-1-yl)methyl)-1H-pyrazol-4-yl)picolinic acid

The title compound was prepared by substituting Example 2.5.9 for Example 1.2.10 in Example 1.2.11. MS (ESI) m/e 820.3 (M+H)⁺.

### 2.5.11. N-[6-(2,5-dioxo-2,5-dihydro-1H-pyrrol-1-yl)hexanoyl]-L-valyl-N-{4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]tricyclo[3.3.1.1^{3,7}]dec-1-yl}oxy)-4-methyl-3-oxo-2,7,10-trioxa-4-azadodec-1-yl]phenyl}-N⁵-carbamoyl-L-ornithinamide

The title compound was prepared by substituting Example 2.5.10 for Example 1.1.17 in Example 2.2. ¹H NMR (400 MHz, dimethyl sulfoxide-d₆) δ ppm 9.96 (br.s, 1H), 7.96-8.12 (m, 2H), 7.73-7.83 (m, 2H), 7.29-7.66 (m, 9H), 7.17-7.30 (m, 3H), 6.89-7.01 (m, 2H), 4.86-5.01 (m, 4H), 4.28-4.45 (m, 1H), 4.12-4.21 (m, 1H), 3.69-3.92 (m, 3H), 3.27-3.62 (m, 9H), 2.78-3.06 (m, 7H), 2.01-2.23 (m, 7H), 1.87-2.01 (m, 1H), 1.54-1.72 (m, 4H), 1.01-1.54 (m, 22H), 0.72-0.89 (m, 6H). MS (ESI) m/e 1418.4 (M+H)⁺.

### 2.6. Synthesis of N-[19-(2,5-dioxo-2,5-dihydro-1H-pyrrol-1-yl)-17-oxo-4,7,10,13-tetraoxa-16-azanonadecan-1-oyl]-L-valyl-N-{4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]tricyclo[3.3.1.1^{3,7}]dec-1-yl}oxy)-4-methyl-3-oxo-2,7,10-trioxa-4-azadodec-1-yl]phenyl}-N⁵-carbamoyl-L-ornithinamide (Synthon M)

The title compound was prepared by substituting Example 2.5.10 for Example 1.1.17 in Example 2.1.7. ¹H NMR (500 MHz, dimethyl sulfoxide-d₆) δ ppm 9.97 (s, 1H), 8.07-8.13 (m, 1H), 7.97-8.05 (m, 2H), 7.86 (d, 1H), 7.78 (d, 1H), 7.55-7.63 (m, 3H), 7.40-7.51 (m, 3H), 7.32-7.38 (m, 2H), 7.25-7.30 (m, 2H), 6.98 (s, 1H), 6.93 (d, 1H), 4.91-5.01 (m, 4H), 4.31-4.41 (m, 1H), 4.17-4.24 (m, 1H), 3.83-3.91 (m, 2H), 3.76 (s, 2H), 3.30-3.62 (m, 21H), 3.10-3.17 (m, 1H), 2.89-3.05 (m, 4H), 2.81-2.88 (m, 3H), 2.42-2.47 (m, 1H), 2.27-2.40 (m, 3H), 2.04-2.15 (m, 5H), 1.91-2.00 (m, 1H), 1.30-1.72 (m, 16H), 0.76-0.88 (m, 6H). MS (ESI) m/e 1623.3 (M+H)⁺.

### 2.7. Synthesis of N-[6-(2,5-dioxo-2,5-dihydro-1H-pyrrol-1-yl)hexanoyl]-L-valyl-N-{4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-4-methyl-3-oxo-2,7,10-trioxa-4-azadodec-1-yl]phenyl}-N⁵-carbamoyl-L-ornithinamide (Synthon V)

The title compound was prepared by substituting Example 1.2.11 for Example 1.1.17 in Example 2.2. ¹H NMR (500 MHz, dimethyl sulfoxide-d₆) δ ppm 9.61 (s, 1H), 7.97 (d, 1H), 7.76 (d, 1H), 7.67 (d, 1H), 7.61 (d, 1H), 7.51-7.57 (m, 2H), 7.38-7.48 (m, 4H), 7.29-7.36 (m, 2H), 7.23-7.28 (m, 3H), 6.86-6.94 (m, 2H), 4.97 (d, 4H), 4.38-4.45 (m, 1H), 4.12-4.19 (m, 1H), 3.89 (t, 2H), 3.80 (s, 2H), 3.47-3.54 (m, 5H), 3.44 (s, 3H), 3.33-3.41 (m, 6H), 2.93-3.06 (m, 6H), 2.87 (s, 2H), 2.11-2.22 (m, 2H), 2.08 (s, 3H), 1.97-2.05 (m, 1H), 1.70-1.81 (m, 2H), 1.33-1.68 (m, 10H), 0.95-1.32 (m, 14H), 0.80-0.91 (m, 13H). MS (+ESI) m/e 1446.3 (M+H)⁺.

### 2.8. Synthesis of N-({2-[2-(2,5-dioxo-2,5-dihydro-1H-pyrrol-1-yl)ethoxy]ethoxy}acetyl)-L-valyl-N-{4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-4-methyl-3-oxo-2,7,10-trioxa-4-azadodec-1-yl]phenyl}-N⁵-carbamoyl-L-ornithinamide (Synthon DS)

### 2.8.1. (S)-2-((S)-2-(2-(2-(2-(2,5-dioxo-2,5-dihydro-1H-pyrrol-1-yl)ethoxy)ethoxy)acetamido)-3-methylbutanamido)-N-(4-(hydroxymethyl)phenyl)-5-ureidopentanamide

The title compound was prepared by substituting 2,5-dioxopyrrolidin-1-yl 2-(2-(2-(2,5-dioxo-2,5-dihydro-1H-pyrrol-1-yl)ethoxy)ethoxy)acetate for 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate in Example 2.1.5.

### 2.8.2. 4-((2S,5S)-14-(2,5-dioxo-2,5-dihydro-1H-pyrrol-1-yl)-5-isopropyl-4,7-dioxo-2-(3-ureidopropyl)-9,12-dioxa-3,6-diazatetradecanamido)benzyl (4-nitrophenyl) carbonate

The title compound was prepared by substituting Example 2.8.1 for Example 2.3.4 in Example 2.3.5.

### 2.8.3. N-({2-[2-(2,5-dioxo-2,5-dihydro-1H-pyrrol-1-yl)ethoxy]ethoxy}acetyl)-L-valyl-N-{4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-4-methyl-3-oxo-2,7,10-trioxa-4-azadodec-1-yl]phenyl}-N⁵-carbamoyl-L-ornithinamide

The title compound was prepared by substituting Example 1.2.11 for Example 1.1.17 and Example 2.8.2 for 4-((S)-2-((S)-2-(6-(2,5-dioxo-2,5-dihydro-1H-pyrrol-1-yl)hexanamido)-3-methylbutanamido)-5-ureidopentanamido)benzyl 4-nitrophenyl carbonate in Example 2.2. ¹H NMR (500 MHz, dimethyl sulfoxide-d₆) δ ppm 9.64 (s, 1H), 7.97 (d, 1H), 7.92 (d, 1H), 7.75 (d, 1H), 7.60 (d, 1H), 7.54 (d, 2H), 7.45 (d, 2H), 7.38-7.43 (m, 1H), 7.29-7.36 (m, 2H), 7.22-7.28 (m, 4H), 6.88-6.93 (m, 2H), 4.98 (d, 4H), 4.39-4.46 (m, 1H), 4.24-4.31 (m, 1H), 3.86-3.93 (m, 4H), 3.80 (s, 2H), 3.46-3.61 (m, 15H), 3.43-3.45 (m, 5H), 3.33-3.38 (m, 4H), 2.87 (s, 3H), 1.99-2.11 (m, 4H), 1.56-1.80 (m, 2H), 1.34-1.50 (m, 4H), 0.94-1.32 (m, 11H), 0.80-0.91 (m, 13H). MS (+ESI) m/e 1478.3 (M+H).

**2.9.** This paragraph is intentionally left blank.

### 2.10. Synthesis of N-[3-(2,5-dioxo-2,5-dihydro-1H-pyrrol-1-yl)propanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide (Synthon BG)

### 2.10.1. (S)-2-((S)-2-(3-(2,5-dioxo-2,5-dihydro-1H-pyrrol-1-yl)propanamido)-3-methylbutanamido)-N-(4-(hydroxymethyl)phenyl)-5-ureidopentanamide

Example 2.1.4 (3 g) and 2,5-dioxopyrrolidin-1-yl 3-(2,5-dioxo-2,5-dihydro-1H-pyrrol-1-yl)propanoate (1.789 g) were dissolved in methanol (30 mL) and stirred for three hours at room temperature. The solvent was concentrated under reduced pressure, and the residue was purified by silica gel chromatography, eluting with a gradient of 5-30% methanol in dichloromethane, to give the title compound. MS (ESI) m/e 531.0 (M+H)⁺.

### 2.10.2. 4-((S)-2-((S)-2-(3-(2,5-dioxo-2,5-dihydro-1H-pyrrol-1-yl)propanamido)-3-methylbutanamido)-5-ureidopentanamido)benzyl (4-nitrophenyl) carbonate

Bis(4-nitrophenyl) carbonate (2.293 g), N,N-diisopropylethylamine (1.317 mL) and Example 2.10.1 (2 g) were dissolved in N,N-dimethylformamide (30 mL) and stirred for 16 hours at room temperature. The solvent was concentrated under reduced pressure, and the residue was purified by silica gel chromatography, eluting with a gradient of 0-10% methanol in dichloromethane, to give the title compound. MS (ESI) m/e 696.9 (M+H)⁺.

### 2.10.3. N-[3-(2,5-dioxo-2,5-dihydro-1H-pyrrol-1-yl)propanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide

The title compound was prepared by substituting Example 2.10.2 for Example 2.9.4 in Example 2.9.5. ¹H NMR (400 MHz, dimethyl sulfoxide-d₆) δ ppm 12.86 (bs, 1H), 9.95 (s, 1H), 8.10 (d, 1H), 8.01 (dd, 2H), 7.79 (d, 1H), 7.65-7.56 (m, 3H), 7.55-7.40 (m, 3H), 7.40-7.33 (m, 2H), 7.35-7.24 (m, 3H), 6.99 (s, 2H), 6.95 (d, 1H), 4.42-4.28 (m, 1H), 4.15 (dd, 1H), 3.92-3.85 (m, 2H), 3.83-3.77 (m, 2H), 3.77-3.52 (m, 2H), 3.45-3.38 (m, 2H), 3.30-3.23 (m, 2H), 3.08-2.90 (m, 4H), 2.90-2.81 (m, 3H), 2.09 (s, 3H), 2.02-1.86 (m, 1H), 1.79-1.52 (m, 2H), 1.52-0.92 (m, 15H), 0.91-0.75 (m, 13H). MS (ESI) m/e 1316.1 (M+H)⁺.

**2.11.** This paragraph is intentionally left blank.

### 2.12. Synthesis of N-[3-(2,5-dioxo-2,5-dihydro-1H-pyrrol-1-yl)propanoyl]-L-alanyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-L-alaninamide (Synthon BI)

### 2.12.1. 3-(2,5-dioxo-2,5-dihydro-1H-pyrrol-1-yl)-N-((S)-1-(((S)-1-((4-(hydroxymethyl)phenyl)amino)-1-oxopropan-2-yl)amino)-1-oxopropan-2-yl)propanamide

A mixture of Example 2.3.3 (9 g) and 2,5-dioxopyrrolidin-1-yl 3-(2,5-dioxo-2,5-dihydro-1H-pyrrol-1-yl)propanoate (9.03 g) in N,N-dimethylformamide (50 mL) was stirred at room temperature for 16 hours. The reaction mixture was diluted with water. The aqueous layer was back extracted with methylene chloride (3 x 100 mL). The organic solvent was concentrated under vacuum. The resulting crude product was absorbed onto silica gel and purified by silica gel chromatography, eluting with 50:1 dichloromethane/methanol, to yield the title compound. MS (ESI) m/e 439.1 (M+Na)⁺.

### 2.12.2. 4-((S)-2-((S)-2-(3-(2,5-dioxo-2,5-dihydro-1H-pyrrol-1-yl)propanamido)propanamido)propanamido)benzyl (4-nitrophenyl) carbonate

The title compound was prepared by substituting Example 2.12.1 for Example 2.10.1 in Example 2.10.2. The product was purified by silica gel chromatography silica, eluting with 25% tetrahydrofuran /dichloromethane. MS (ESI) m/e 604.0 (M+H)⁺.

### 2.12.3. N-[3-(2,5-dioxo-2,5-dihydro-1H-pyrrol-1-yl)propanoyl]-L-alanyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-L-alaninamide

The title compound was prepared by substituting Example 2.12.2 for Example 2.9.4 in Example 2.9.5. ¹H NMR (400 MHz, dimethyl sulfoxide-d₆) δ ppm 9.51 (s, 1H), 7.97 (dd, 1H), 7.90-7.83 (m, 1H), 7.76 (d, 1H), 7.72-7.66 (m, 1H), 7.64-7.57 (m, 1H), 7.60-7.55 (m, 1H), 7.55 (s, 1H), 7.48-7.37 (m, 3H), 7.37-7.29 (m, 2H), 7.29-7.22 (m, 3H), 6.91 (d, 1H), 6.88 (s, 1H), 4.98 (s, 2H), 4.96 (bs, 2H), 4.40 (p, 1H), 4.24 (p, 1H), 3.89 (t, 2H), 3.79 (s, 2H), 3.64 (t, 2H), 3.44 (t, 2H), 3.29-3.14 (m, 2H), 3.02 (t, 2H), 2.86 (s, 3H), 2.08 (s, 3H), 1.36 (bs, 2H), 1.31 (d, 3H), 1.29-0.94 (m, 14H), 0.83 (s, 6H). MS (ESI) m/e 1202.1 (M+H)⁺.

**2.13.** This paragraph is intentionally left blank.

**2.14.** This paragraph is intentionally left blank.

**2.15.** This paragraph is intentionally left blank.

**2.16.** This paragraph is intentionally left blank.

### 2.17. Synthesis of N-[(2R)-4-(2,5-dioxo-2,5-dihydro-1H-pyrrol-l-yl)-2-sulfobutanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide (Synthon BO)

### 2.17.1. 3-(1-((3-(2-((((4-((S)-2-((S)-2-((((9H-fluoren-9-yl)methoxy)carbonyl)amino)-3-methylbutanamido)-5-ureidopentanamido)benzyl)oxy)carbonyl)(methyl)amino)ethoxy )-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate for Example 2.3.5 in Example 2.3.6. MS (ESI) m/e 1387.3 (M+H)⁺.

### 2.17.2. 3-(1-((3-(2-((((4-((S)-2-((S)-2-amino-3-methylbutanamido)-5-ureidopentanamido)benzyl)oxy)carbonyl)(methyl)amino)ethoxy )-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.17.1 (15 mg) was mixed with a solution of 30 % diethylamine in N,N-dimethylformamide (0.5 mL), and the reaction mixture was stirred at room temperature overnight. The crude reaction mixture was directly purified by reverse phase HPLC using a C18 column and a gradient of 10-100% acetonitrile in water containing 0.1% trifluoroacetic acid. The fractions containing the product were lyophilized to give the title compound as a trifluoroacetic acid salt. MS (ESI) m/e 1165.5 (M+H)⁺.

### 2.17.3. 4-(2,5-dioxo-2,5-dihydro-1H-pyrrol-1-yl)-1-((2,5-dioxopyrrolidin-1-yl)oxy)-1-oxobutane-2-sulfonate

In a 100 mL flask sparged with nitrogen, 1-carboxy-3-(2,5-dioxo-2,5-dihydro-1H-pyrrol-1-yl)propane-1-sulfonate was dissolved in dimethylacetamide (20 mL). To this solution N-hydroxysuccinimide (440 mg,) and 1-(3-dimethylaminopropyl)-3-ethylcarbodiimide hydrochloride (1000 mg) were added, and the reaction was stirred at room temperature under a nitrogen atmosphere for 16 hours. The solvent was concentrated under reduced pressure, and the residue was purified by silica gel chromatography, eluting with a gradient of 1-2% methanol in dichloromethane containing 0.1 % v/v acetic acid, to yield the title compound as a mixture of ∼ 80% activated ester and 20 % acid, which was used in the next step without further purification. MS (ESI) m/e 360.1 (M+H)⁺.

### 2.17.4. N-[(2R)-4-(2,5-dioxo-2,5-dihydro-1H-pyrrol-1-yl)-2-sulfobutanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide

The trifluoroacetic acid salt of Example 2.17.2 (6 mg) was mixed with Example 2.17.3 (16.85 mg) and N,N-diisopropylethylamine (0.025 mL) in N,N-dimethylformamide (0.500 mL), and the reaction mixture was stirred at room temperature overnight. The crude reaction mixture was purified by reverse phase HPLC using a Gilson system and a C18 25 x 100 mm column, eluting with 5-85% acetonitrile in water containing 0.1% v/v trifluoroacetic acid. The product fractions were lyophilized to give two diastereomers differing in the stereochemistry at the newly-added position deriving from racemic Example 2.17.3. The stereochemistry of the two products at that center was randomly assigned. MS (ESI) m/e 1408.5 (M-H)⁻.

### 2.18. Synthesis of N-[(2S)-4-(2,5-dioxo-2,5-dihydro-1H-pyrrol-1-yl)-2-sulfobutanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide (Synthon BP)

The title compound is the second diastereomer isolated during the preparation of Example 2.17.4 as described in Example 2.17.4. MS (ESI) m/e 1408.4 (M-H)⁻.

**2.19.** This paragraph is intentionally left blank.

**2.20.** This paragraph is intentionally left blank.

### 2.21. Synthesis of N-[6-(2,5-dioxo-2,5-dihydro-1H-pyrrol-1-yl)hexanoyl]-3-sulfo-L-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]phenyl}-L-alaninamide (Synthon IQ)

### 2.21.1. (S)-(9H-fluoren-9-yl)methyl (1-((4-(hydroxymethyl)phenyl)amino-1-oxopropan-2-yl)carbamate

To a solution of (S)-2-((((9H-fluoren-9-yl)methoxy)carbonyl)amino)propanoic acid (50 g) in methanol (400 mL) and dichloromethane (400 mL) was added (4-aminophenyl)methanol (23.73 g) and ethyl 2-ethoxyquinoline-1(2H)-carboxylate (79 g), and the reaction was stirred at room temperature overnight. The solvent was evaporated, and the residue was washed by dichloromethane to give the title compound.

### 2.21.2. (S)-2-amino-N-(4-(hydroxymethyl)phenyl)propanamide

To a solution of Example 2.21.1 (10 g) in N,N-dimethylformamide (100 mL) was added piperidine (40 mL), and the reaction was stirred for 2 hours. The solvent was evaporated, and the residue was dissolved in methanol. The solids were filtered off, and the filtrate was concentrated to give crude product.

### 2.21.3. (9H-fluoren-9-yl)methyl ((S)-1-(((S)-1-((4-(hydroxymethyl) phenyl)amino)-1-oxopropan-2-yl)amino)-3-methyl-1-oxobutan-2-yl)carbamate

To a solution of Example 2.21.2 (5 g) in N,N-dimethylformamide (100 mL) was added (S)-2-((((9H-fluoren-9-yl)methoxy)carbonyl)amino)-3-methylbutanoic acid (10.48 g) and 2-(1H-benzo[d][1,2,3]triazol-1-yl)-1,1,3,3-tetramethylisouronium hexafluorophosphate(V) (14.64 g), and the reaction was stirred overnight. The solvent was evaporated, the residue was washed with dichloromethane, and the solids were filtered to give the crude product.

### 2.21.4. (9H-fluoren-9-yl)methyl ((S)-3-methyl-1-(((S)-1-((4-((((4-nitrophenoxy)carbonyl)oxy)methyl)phenyl)amino)-1-oxopropan-2-yl)amino)-1-oxobutan-2-yl)carbamate

The title compound was prepared by substituting Example 2.21.3 for Example 2.10.1 in Example 2.10.2.

### 2.21.5. 3-(1-((3-(2-((((4-((S)-2-((S)-2-amino-3-methylbutanamido) propanamido)benzyl)oxy)carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of Example 1.3.7 (0.102 g), Example 2.21.4 (0.089 g) and N,N-diisopropylethylamine (0.104 mL) were stirred together in N,N-dimethylformamide (1 mL) at room temperature. After stirring overnight, diethylamine (0.062 mL) was added, and the reaction was stirred for an additional 2 hours. The reaction was diluted with water (1 mL), quenched with trifluoroacetic acid and was purified by Prep HPLC using a Gilson system, eluting with 10-85% acetonitrile in water containing 0.1% v/v trifluoroacetic acid. The desired fractions were combined and freeze-dried to provide the title compound.

### 2.21.6. 3-(1-((3-(2-((((4-((S)-2-((S)-2-((R)-2-amino-3-sulfopropanamido)-3-methylbutanamido)propanamido)benzyl)oxy) carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of (R)-2-((((9H-fluoren-9-yl)methoxy)carbonyl)amino)-3-sulfopropanoic acid (0.028 g) and 2-(3H-[1,2,3]triazolo[4,5-b]pyridin-3-yl)-1,1,3,3-tetramethylisouronium hexafluorophosphate(V) (0.027 g) in N,N-dimethylformamide (1 mL) was added N,N-diisopropylethylamine (0.042 mL), and the reaction was stirred for 5 minutes. The mixture was added to Example 2.21.5 (0.050 g), and the mixture was stirred for 1 hour. Diethylamine (0.049 mL) was then added to the reaction and stirring was continued for an additional 1 hour. The reaction was diluted with N,N-dimethylformamide (1 mL) and water (0.5 mL), quenched with trifluoroacetic acid and purified by reverse-phase HPLC using a Gilson system, eluting with 10-88% acetonitrile in water containing 0.1% v/v trifluoroacetic acid. The desired fractions were combined and freeze-dried to provide the title compound. MS (ESI) m/e 1214.4 (M-H)⁻.

### 2.21.7. N-[6-(2,5-dioxo-2,5-dihydro-1H-pyrrol-1-yl)hexanoyl]-3-sulfo-L-alanyl-L-valyl-N-{4-[({[2-({3-[(4-{6- [8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy) ethyl]carbamoyl}oxy)methyl]phenyl}-L-alaninamide

To a solution of Example 2.21.6 (0.030 g) and 2,5-dioxopyrrolidin-1-yl 6-(2,5-dioxo-2,5-dihydro-1H-pyrrol-1-yl)hexanoate (8.34 mg) in N,N-dimethylformamide (0.5 mL) was added N,N-diisopropylethylamine (0.020 mL), and the reaction was stirred for 1 hour. The reaction was diluted with N,N-dimethylformamide (1 mL) and water (0.5 mL) and was purified by prep HPLC using a Gilson system, eluting with 10-85% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 9.41 (s, 1H), 8.26 (d, 1H), 8.11-7.95 (m, 3H), 7.79 (d, 1H), 7.68 (d, 2H), 7.61 (d, 1H), 7.57-7.27 (m, 6H), 7.24 (d, 2H), 7.12 (t, 1H), 7.02-6.90 (m, 3H), 4.94 (d, 4H), 4.67 (td, 2H), 4.34-4.22 (m, 2H), 4.04-3.94 (m, 2H), 3.88 (t, 2H), 3.82 (s, 2H), 3.42-3.27 (m, 4H), 3.11-2.96 (m, 5H), 2.84 (dd, 1H), 2.30-1.98 (m, 6H), 1.56-1.41 (m, 4H), 1.41-0.79 (m, 28H). MS (ESI) m/e 1409.1 (M+H)⁺.

### 2.22. Synthesis of 4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon DB)

### 2.22.1. (E)-tert-butyldimethyl((3-(4,4,5,5-tetramethyl-1,3,2-dioxaborolan-2-yl)allyl)oxy)silane

To a flask charged with tert-butyldimethyl(prop-2-yn-1-yloxy)silane (5 g) and dichloromethane (14.7 mL) under a nitrogen atmosphere was added dropwise 4,4,5,5-tetramethyl-1,3,2-dioxaborolane (3.94 g). The mixture was stirred at room temperature for one minute then transferred via cannula to a nitrogen-sparged flask containing Cp₂ZrClH (chloridobis(η5-cyclopentadienyl)hydridozirconium, Schwartz's Reagent) (379 mg). The resulting reaction mixture was stirred at room temperature for 16 hours. The mixture was carefully quenched with water (15 mL), and then extracted with diethyl ether (3x 30 mL). The combined organic phases were washed with water (15 mL), dried over MgSO₄, filtered, concentrated, and purified by silica gel chromatography, eluting with a gradient from 0-8% ethyl acetate in heptanes, to give the title compound. MS (ESI) m/z 316.0 (M+NH₄)⁺.

### 2.22.2. (2S,3R,4S,5S,6S)-2-(4-bromo-2-nitrophenoxy)-6-(methoxycarbonyl)tetrahydro-2H-pyran-3,4,5-triyl triacetate

(2R,3R,4S,5S,6S)-2-Bromo-6-(methoxycarbonyl)tetrahydro-2H-pyran-3,4,5-triyl triacetate (5 g) was dissolved in acetonitrile (100 mL). Ag₂O (2.92 g) was added to the solution, and the reaction was stirred for 5 minutes at room temperature. 4-Bromo-2-nitrophenol (2.74 g) was added, and the reaction mixture was stirred at room temperature for 4 hours. The silver salt residue was filtered through diatomaceous earth, and the filtrate was concentrated under reduced pressure. The residue was purified by silica gel chromatography, eluting with a gradient of 10-70% ethyl acetate in heptanes, to give the title compound. MS (ESI+) m/z 550.9 (M+NH₄)⁺.

### 2.22.3. (2S,3R,4S,5S,6S)-2-(4-((E)-3-((tertbutyldimethylsilyl)oxy)prop-1-en-1-yl)-2-nitrophenoxy)-6-(methoxycarbonyl)tetrahydro-2H-pyran-3,4,5-triyl triacetate

Example 2.22.2 (1 g), sodium carbonate (0.595 g), tris(dibenzylideneacetone)dipalladium (0.086 g), and 1,3,5,7-tetramethyl-6-phenyl-2,4,8-trioxa-6-phosphaadamantane (0.055 g) were combined in a 3-neck 50-mL round bottom flask equipped with a reflux condenser, and the system was degassed with nitrogen. Separately, a solution of Example 2.22.1 (0.726 g) in tetrahydrofuran (15 mL) was degassed with nitrogen for 30 minutes. The latter solution was transferred via cannula into the flask containing the solid reagents, followed by addition of degassed water (3 mL) via syringe. The reaction was heated to 60 °C for two hours. The reaction mixture was partitioned between ethyl acetate (3x 30 mL) and water (30 mL). The combined organic phases were dried (Na₂SO₄), filtered, and concentrated. The residue was purified by silica gel chromatography, eluting with a gradient from 0-35% ethyl acetate in heptanes, to provide the title compound. MS (ESI+) m/z 643.1 (M+NH₄)⁺.

### 2.22.4. (2S,3R,4S,5S,6S)-2-(2-amino-4-((E)-3-hydroxyprop-1-en-1-yl)phenoxy)-6-(methoxycarbonyl)tetrahydro-2H-pyran-3,4,5-triyl triacetate

A 500-mL three-neck, nitrogen-flushed flask equipped with a pressure-equalizing addition funnel was charged with zinc dust (8.77 g). A degassed solution of Example 2.22.3 (8.39 g) in tetrahydrofuran (67 mL) was added via cannula. The resulting suspension was chilled in an ice bath, and 6N HCl (22.3 mL) was added dropwise via the addition funnel at such a rate that the internal temperature of the reaction did not exceed 35 °C. After the addition was complete, the reaction was stirred for two hours at room temperature, and filtered through a pad of diatomaceous earth, rinsing with water and ethyl acetate. The filtrate was treated with saturated aqueous NaHCO₃ solution until the water layer was no longer acidic, and the mixture was filtered to remove the resulting solids. The filtrate was transferred to a separatory funnel, and the layers were separated. The aqueous layer was extracted with ethyl acetate (3x 75 mL), and the combined organic layers were washed with water (100 mL), dried over Na₂SO₄, filtered, and concentrated. The residue was triturated with diethyl ether and the solid collected by filtration to provide the title compound. MS (ESI+) m/z 482.0 (M+H)⁺.

### 2.22.5. (9H-fluoren-9-yl)methyl (3-chloro-3-oxopropyl)carbamate

To a solution of 3-((((9H-fluoren-9-yl)methoxy)carbonyl)amino)propanoic acid (5.0 g) in dichloromethane (53.5 mL) was added sulfurous dichloride (0.703 mL). The mixture was stirred at 60 °C for one hour. The mixture was cooled and concentrated to give the title compound, which was used in the next step without further purification.

### 2.22.6. (2S,3R,4S,5S,6S)-2-(2-(3-((((9H-fluoren-9-yl)methoxy)carbonyl)amino)propanamido)-4-((E)-3-hydroxyprop-1-en-1-yl)phenoxy)-6-(methoxycarbonyl)tetrahydro-2H-pyran-3,4,5-triyl triacetate

Example 2.22.4 (6.78 g) was dissolved in dichloromethane (50 mL), and the solution was chilled to 0 °C in an ice bath. N,N-Diisopropylethylamine (3.64 g) was added, followed by dropwise addition of a solution of Example 2.22.5 (4.88 g) in dichloromethane (50 mL). The reaction was stirred for 16 hours allowing the ice bath to come to room temperature. Saturated aqueous NaHCO₃ solution (100 mL) was added, and the layers were separated. The aqueous layer was further extracted with dichloromethane (2 x 50 mL). The extracts were dried over Na₂SO₄, filtered, concentrated and purified by silica gel chromatography, eluting with a gradient of 5-95% ethyl acetate/heptane, to give an inseparable mixture of starting aniline and desired product. The mixture was partitioned between 1N aqueous HCl (40 mL) and a 1:1 mixture of diethyl ether and ethyl acetate (40 mL), and then the aqueous phase was further extracted with ethyl acetate (2x 25 mL). The organic phases were combined, washed with water (2x 25 mL), dried over Na₂SO₄, filtered, and concentrated to give the title compound. MS (ESI+) m/z 774.9 (M+H)⁺.

### 2.22.7. (2S,3R,4S,5S,6S)-2-(2-(3-((((9H-fluoren-9-yl)methoxy)carbonyl)amino)propanamido)-4-((E)-3-(((4-nitrophenoxy)carbonyl)oxy)prop-1-en-1-yl)phenoxy)-6-(methoxycarbonyl)tetrahydro-2H-pyran-3,4,5-triyl triacetate

Example 2.22.6 (3.57 g) was dissolved in dichloromethane (45 mL) and bis(4-nitrophenyl)carbonate (2.80 g) was added, followed by dropwise addition of N,N-diisopropylethylamine (0.896 g). The reaction mixture was stirred at room temperature for two hours. Silica gel (20 g) was added to the reaction solution, and the mixture was concentrated to dryness under reduced pressure, keeping the bath temperature at or below 25 °C. The silica residue was loaded atop a column, and the product was purified by silica gel chromatography, eluting with a gradient from 0-100% ethyl acetate-heptane, providing partially purified product which was contaminated with nitrophenol. The material was triturated with methyl tert-butyl ether (250 mL), and the resulting slurry was allowed to sit for 1 hour. The product was collected by filtration. Three successive crops were collected in a similar fashion to give the title compound. MS (ESI+) m/z 939.8 (M+H)⁺.

### 2.22.8. 3-(1-((3-(2-(((((E)-3-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)phenyl)allyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a cold (0 °C) solution of the trifluoroacetic acid salt of Example 1.1.17 (77 mg) and Example 2.22.7 (83 mg) in N,N-dimethylformamide (3.5 mL) was added N,N-diisopropylethylamine (0.074 mL). The reaction was slowly warmed to room temperature and stirred for 16 hours. The reaction was quenched by the addition of water and ethyl acetate. The layers were separated, and the aqueous was extracted twice with additional ethyl acetate. The combined organics were dried with anhydrous sodium sulfate, filtered and concentrated under reduced pressure to yield the title compound, which was used in the subsequent step without further purification.

### 2.22.9. 3-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To an ambient solution of Example 2.22.8 (137 mg) in methanol (3 mL) was added 2M lithium hydroxide solution (0.66 mL). The reaction mixture was stirred for two hours at 35 °C and quenched by the addition of acetic acid (0.18 mL). The reaction was concentrated to dryness, and the residue was diluted with methanol. The crude product was purified by reverse phase HPLC using a Gilson system and a C18 25 x 100 mm column, eluting with 20-75% acetonitrile in water containing 0.1% v/v trifluoroacetic acid. The product fractions were lyophilized to give the title compound as a trifluoroacetic acid salt. MS (ESI) m/e 1220.3 (M+Na)⁺.

### 2.22.10.4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5, 7-dimethyltricyclo[3.3.1.1^{3,7}]dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

To a solution of the trifluoroacetic acid salt of Example 2.22.9 (41.9 mg) in N,N-dimethylformamide (1 mL) were added N-succinimidyl 6-maleimidohexanoate (9.84 mg) and N,N-diisopropylethylamine (0.010 mL), and the reaction was stirred at room temperature for 16 hours. The crude reaction was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. ¹H NMR (500 MHz, dimethyl sulfoxide-d₆) δ ppm 12.86 (bs, 2H), 9.03 (s, 1H), 8.25 (bs, 1H), 8.03 (d, 1H), 7.97-7.85 (m, 1H), 7.79 (d, 1H), 7.64-7.59 (m, 1H), 7.56-7.39 (m, 3H), 7.40-7.32 (m, 2H), 7.28 (s, 1H), 7.14-7.06 (m, 1H), 7.04 (d, 1H), 6.98 (s, 2H), 6.95 (d, 1H), 6.60-6.52 (m, 1H), 6.22-6.12 (m, 1H), 4.95 (bs, 2H), 4.90-4.75 (m, 1H), 4.63 (d, 2H), 4.24-4.05 (m, 1H), 4.08-3.62 (m, 8H), 3.50-3.24 (m, 10H), 3.04-2.97 (m, 2H), 2.92-2.82 (m, 3H), 2.11-2.06 (m, 3H), 2.03 (t, J = 7.4 Hz, 2H), 1.53-1.39 (m, 4H), 1.41-0.73 (m, 23H). MS (ESI) m/e 1413.3 (M+Na)⁺.

### 2.23. Synthesis of 4-{(1E)-3-[({2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}carbamoyl)oxy]prop-1-en-1-yl}-2-({N-[3-(2,5-dioxo-2,5-dihydro-1H-pyrrol-1-yl)propanoyl]-beta-alanyl}amino)phenyl beta-D-glucopyranosiduronic acid (Synthon DM)

### 2.23.1. 3-(1-((3-(2-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)amino)ethoxy)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a cold (0 °C) solution of Example 2.22.7 (94 mg) and Example 1.4.10 (90 mg) was added N,N-diisopropylamine (0.054 mL). The reaction was slowly warmed to room temperature and stirred overnight. The reaction was quenched by the addition of water and ethyl acetate. The layers were separated, and the aqueous layer was extracted twice with additional ethyl acetate. The combined organics were dried with anhydrous sodium sulfate, filtered and concentrated under reduced pressure. The crude material was dissolved in tetrahydrofuran/methanol/H₂O (2:1:1, 8 mL), to which was added lithium hydroxide monohydrate (40 mg). The reaction mixture was stirred overnight. The mixture was concentrated under vacuum, acidified with trifluoroacetic acid and dissolved in dimethyl sulfoxide/methanol. The solution was purified by reverse phase HPLC using a Gilson system and a C18 column, eluting with 10-85% acetonitrile in 0.1% trifluoroacetic acid in water, to give the title compound. MS (ESI) m/e 1228.1 (M+H)⁺.

### 2.23.2. 4-{(IE)-3-[({2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}carbamoyl)oxy]prop-1-en-1-yl}-2-({N-[3-(2,5-dioxo-2,5-dihydro-1H-pyrrol-1-yl)propanoyl]-beta-alanyl}amino)phenyl beta-D-glucopyranosiduronic acid

To a solution of Example 2.23.1 (20 mg) and 2,5-dioxopyrrolidin-1-yl 3-(2,5-dioxo-2,5-dihydro-1H-pyrrol-1-yl)propanoate (5.5 mg) in N,N-dimethylformamide (2 mL) was added N,N-diisopropylethylamine (0.054 mL). The reaction was stirred overnight. The reaction mixture was diluted with methanol (2 mL) and acidified with trifluoroacetic acid. The solution was purified by reverse phase HPLC using a Gilson system and a C18 column, eluting with 10-85% acetonitrile in 0.1% trifluoroacetic acid in water, to give the title compound. ¹H NMR (400 MHz, dimethyl sulfoxide-d₆) δ ppm 12.85 (s, 1H), 9.03 (s, 1H), 8.24 (s, 1H), 7.95-8.11 (m, 2H), 7.79 (d, 1H), 7.61 (d, 1H), 7.32-7.52 (m, 5H), 7.28 (s, 1H), 7.02-7.23 (m, 3H), 6.91-6.96 (m, 3H), 6.57 (d, 1H), 6.05-6.24 (m, 1H), 4.95 (s, 2H), 4.87 (d, 1H), 4.59 (d, 2H), 3.78-3.95 (m, 4H), 3.13 (q, 2H), 3.01 (t, 2H), 2.51-2.57 (m, 2H), 2.27-2.39 (m, 3H), 2.11 (s, 3H), 0.92-1.43 (m, 16H), 0.83 (s, 6H). MS (ESI) m/e 1379.2 (M+H)⁺.

### 2.24. Synthesis of 4-{(1E)-3-[({2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}carbamoyl)oxy]prop-1-en-1-yl}-2-({N-[6-(2,5-dioxo-2,5-dihydro-1H-pyrrol-1-yl)hexanoyl]-beta-alanyl}amino)phenylbeta-D-glucopyranosiduronic acid (Synthon DL)

To a solution of Example 2.23.1 (20 mg) and 2,5-dioxopyrrolidin-1-yl 6-(2,5-dioxo-2,5-dihydro-1H-pyrrol-1-yl)hexanoate (6.5 mg) in N,N-dimethylformamide (2 mL) was added N,N-diisopropylethylamine (0.054 mL). The reaction mixture was stirred overnight. The reaction mixture was diluted with methanol (2 mL) and acidified with trifluoroacetic acid. The mixture was purified by reverse phase HPLC using a Gilson system and a C18 column, eluting with 10-85% acetonitrile in 0.1% trifluoroacetic acid in water, to give the title compound. ¹H NMR (400 MHz, dimethyl sulfoxide-d₆) δ ppm 12.85 (s, 1H), 9.03 (s, 1H), 8.24 (s, 1H), 8.03 (d, 1H), 7.87 (t, 1H), 7.78 (s, 1H), 7.61 (d, 1H), 7.32-7.55 (m, 5H), 6.90-7.19 (m, 5H), 6.56 (d, 1H), 6.08-6.24 (m, 1H), 4.91-4.93 (m, 1H), 4.86 (s, 1H), 4.59 (d, 2H), 3.27-3.46 (m, 14H), 3.13 (q, 3H), 2.96-3.02 (m, 2H), 2.50-2.59 (m, 3H), 2.09 (s, 3H), 2.00-2.05 (m, 3H), 0.94-1.54 (m, 20H), 0.83 (s, 6H). MS (ESI) m/e 1421.2 (M+H)⁺.

### 2.25. Synthesis of 4-[(1E)-14-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-6-methyl-5-oxo-4,9,12-trioxa-6-azatetradec-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon DR)

### 2.25.1. 3-(1-((3-(((E)-14-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)phenyl)-9-methyl-10-oxo-3,6,11-trioxa-9-azatetradec-13-en-1-yl)oxy)-5,7-dimethyladamantan-1-yl)methyl)-5-methyl-1H-pyrazol-4-yl)-6-(8-(benzo [d]thiazol-2-ylcarbamoyl)-3,4-dihydroisoquinolin-2(1H)-yl)picolinic acid

To a cold (0 °C) solution of Example 2.22.7 (90 mg) and Example 1.2.11 (92 mg) was added N,N-diisopropylamine (0.050 mL). The ice bath was removed, and the reaction was stirred overnight. The reaction was quenched by the addition of water and ethyl acetate. The layers were separated, and the aqueous was extracted twice with additional ethyl acetate. The combined organics were dried with anhydrous sodium sulfate, filtered and concentrated under reduced pressure to provide the title compound, which was used in the subsequent step without further purification. MS (ESI) m/e 1648.2 (M+H)⁺.

### 2.25.2. 3-(1-((3-(((E)-14-(3-(3-aminopropanamido)-4-(((2S,3R,4S,5S,6S)-6-carboxy-3,4,5-trihydroxytetrahydro-2H-pyran-2-yl)oxy)phenyl)-9-methyl-10-oxo-3,6,11-trioxa-9-azatetradec-13-en-1-yl)oxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a cold (0 °C) solution of Example 2.25.1 (158 mg) in methanol (2.0 mL) was added 2M aqueous lithium hydroxide solution (0.783 mL). The reaction was stirred for 4 hours and quenched by the addition of acetic acid (0.1 mL). The reaction was concentrated to dryness, and the residue was chromatographed using a Biotage Isolera One system and a reverse-phase C18 40g column, eluting with 10-85% acetonitrile in 0.1% trifluoroacetic acid in water. The fractions containing the product were lyophilized to give the title compound as a solid. MS (ESI) m/e 1286.2 (M+H)⁺.

### 2.25.3. 4-[(1E)-14-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-6-methyl-5-oxo-4,9,12-trioxa-6-azatetradec-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

To an ambient solution of Example 2.25.2 (9.03 mg) in N,N-dimethylformamide (1.0 mL) was added 2,5-dioxopyrrolidin-1-yl 6-(2,5-dioxo-2,5-dihydro-1H-pyrrol-1-yl)hexanoate (4 mg) and N,N-diisopropylamine (0.020 mL), and the reaction was stirred overnight. The reaction was diluted with dimethyl sulfoxide and methanol and purified by RP-HPLC on a Biotage Isolera chromatography unit (40g C18 column), eluting with gradient of 10 to 75% acetonitrile in water containing 0.1% v/v trifluoroacetic acid. The fractions containing the product were concentrated by lyophilization to yield the title compound as a solid. ¹H NMR (400MHz, dimethyl sulfoxide-d₆) δ ppm 12.85 (s, 1H), 8.04 (d, 1H), 7.99 (t, 1H), 7.79 (d, 1H), 7.60 (d, 1H), 7.53-7.41 (m, 3H), 7.40-7.32 (m, 2H), 7.28 (s, 1H), 6.99 (s, 2H), 6.98-6.92 (m, 1H), 4.95 (bs, 2H), 3.92-3.85 (m, 1H), 3.81 (s, 2H), 3.63-3.55 (m, 4H), 3.55-3.31 (m, 28H), 3.18-3.10 (m, 2H), 3.05-2.98 (m, 2H), 2.97 (s, 2H), 2.80 (s, 2H), 2.59-2.50 (m, 1H), 2.32 (t, 2H), 2.10 (s, 3H), 1.39-1.34 (m, 2H), 1.31-1.18 (m, 4H), 1.20-0.92 (m, 6H), 0.84 (s, 6H). MS (ESI) m/e 1479.3 (M+H)⁺.

### 2.26. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-[2-(2-{[6-(2,5-dioxo-2,5-dihydro-1H-pyrrol-1-yl)hexanoyl]amino}ethoxy)ethoxy]phenyl beta-D-glucopyranosiduronic acid (Synthon DZ)

### 2.26.1. (2S,3R,4S,5S,6S)-2-(4-formyl-3-hydroxyphenoxy)-6-(methoxycarbonyl)tetrahydro-2H-pyran-3,4,5-triyl triacetate

To a solution of 2,4-dihydroxybenzaldehyde (15 g) and (2S,3R,4S,5S,6S)-2-bromo-6-(methoxycarbonyl)tetrahydro-2H-pyran-3,4,5-triyl triacetate (10 g) in acetonitrile was added silver carbonate (10 g), and the reaction was heated to 40 °C. After stirring for 4 hours, the reaction was cooled, filtered and concentrated. The crude product was suspended in dichloromethane and filtered through diatomaceous earth and concentrated. The residue was purified by silica gel chromatography, eluting with a gradient of 10-100% ethyl acetate in heptane, to give the title compound.

### 2.26.2. (2S,3R,4S,5S,6S)-2-(3-hydroxy-4-(hydroxymethyl)phenoxy)-6-(methoxycarbonyl)tetrahydro-2H-pyran-3,4,5-triyl triacetate

A solution of Example 2.26.1 (16.12 g) in tetrahydrofuran (200 mL) and methanol (200 mL) was cooled to 0 °C and sodium borohydride (1.476 g) was added portionwise. The reaction was stirred for 20 minutes, then quenched with a 1:1 mixture of water: saturated sodium bicarbonate solution (400 mL). The resulting solids were filtered off and rinsed with ethyl acetate. The phases were separated and the aqueous layer extracted four times with ethyl acetate. The combined organic layers were dried over magnesium sulfate, filtered, and concentrated. The crude product was purified via silica gel chromatography, eluting with a gradient of 10-100% ethyl acetate in heptane, to give the title compound. MS (ESI) m/e 473.9 (M+NH₄)⁺.

### 2.26.3. (2S,3R,4S,5S,6S)-2-(4-(((tert-butyldimethylsilyl)oxy)methyl)-3-hydroxyphenoxy)-6-(methoxycarbonyl)tetrahydro-2H-pyran-3,4,5-triyl triacetate

To Example 2.26.2 (7.66 g) and *tert*-butyldimethylsilyl chloride (2.78 g) in dichloromethane (168 mL) at -5 °C was added imidazole (2.63 g), and the reaction mixture was stirred overnight allowing the internal temperature of the reaction to warm to 12 °C. The reaction mixture was poured into saturated aqueous ammonium chloride solution and extracted four times with dichloromethane. The combined organics were washed with brine, dried over magnesium sulfate, filtered, and concentrated. The crude product was purified via silica gel chromatography, eluting with a gradient of 10-100% ethyl acetate in heptane, to give the title compound. MS (ESI) m/e 593.0 (M+Na)⁺.

### 2.26.4. (2S,3R,4S,5S,6S)-2-(3-(2-(2-((((9H-fluoren-9-yl)methoxy)carbonyl)amino)ethoxy)ethoxy)-4-(((tert-butyldimethylsilyl)oxy)methyl)phenoxy)-6-(methoxycarbonyl)tetrahydro-2H-pyran-3,4,5-triyl triacetate

Example 2.26.3 (5.03 g) and triphenylphosphine (4.62 g) in toluene (88 mL) was added di-*tert*-butyl-azodicarboxylate (4.06 g), and the reaction mixture was stirred for 30 minutes. (9H-Fluoren-9-yl)methyl (2-(2-hydroxyethoxy)ethyl)carbamate was added, and the reaction was stirred for an additional 1.5 hours. The reaction was loaded directly onto silica gel, eluting with a gradient of 10-100% ethyl acetate in heptane, to give the title compound.

### 2.26.5. (2S,3R,4S,5S,6S)-2-(3-(2-(2-((((9H-fluoren-9-yl)methoxy)carbonyl)amino)ethoxy)ethoxy)-4-(hydroxymethyl)phenoxy)-6-(methoxycarbonyl)tetrahydro-2H-pyran-3,4,5-triyl triacetate

Example 2.26.4 (4.29 g) was stirred in a 3:1:1 solution of acetic acid:water:tetrahydrofuran (100 mL) overnight. The reaction mixture was poured into saturated aqueous sodium bicarbonate and extracted with ethyl acetate. The organic layer was dried over magnesium sulfate, filtered and concentrated. The crude product was purified via silica gel chromatography, eluting with a gradient of 10-100% ethyl acetate in heptane, to give the title compound.

### 2.26.6. (2S,3R,4S,5S,6S)-2-(3-(2-(2-((((9H-fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyl triacetate

To a solution of Example 2.26.5 (0.595 g) and bis(4-nitrophenyl)carbonate (0.492 g) in N,N-dimethylformamide (4 mL) was added N,N-diisopropylamine (0.212 mL). After 1.5 hours the reaction was concentrated under high vacuum. The residue was purified by silica gel chromatography, eluting with a gradient of 10-100% ethyl acetate in heptane, to give the title compound. MS (ESI) m/e 922.9 (M+Na)⁺.

### 2.26.7. 3-(1-((3-(2-((((2-(2-(2-((((9H-fluoren-9-yl)methoxy)carbonyl)amino)ethoxy)ethoxy)-4-(((2S,3R,4S,5S,6S)-3,4,5-triacetoxy-6-(methoxycarbonyl)tetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.1.17 (0.106 g) and Example 2.26.6 (0.130 g) in N,N-dimethylformamide (1.5 mL) was added N,N-diisopropylamine (0.049 mL). After 6 hours, additional N,N-diisopropylamine (0.025 mL) was added, and the reaction was stirred overnight. The reaction was diluted with ethyl acetate (50 mL) and washed with water (10 mL) followed by four times with brine (15 mL). The organic layer was dried over magnesium sulfate, filtered, and concentrated to give the title compound, which was used in the next step without further purification.

### 2.26.8. 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo [d]thiazol-2-ylcarbam oyl)-3,4-dihydroisoquinolin-2(1H)-yl)picolinic acid

A suspension of Example 2.26.7 (0.215 g) in methanol (2 mL) was treated with 2.0M aqueous lithium hydroxide (1 mL). After stirring for 1 hour, the reaction was quenched by the addition of acetic acid (0.119 mL). The resulting suspension was diluted with dimethyl sulfoxide (1 mL) and was purified by prep HPLC using a Gilson system, eluting with 10-85% acetonitrile in water containing 0.1% v/v trifluoroacetic acid. The desired fractions were combined and freeze-dried to provide the title compound.

### 2.26.9. 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-[2-(2-{[6-(2,5-dioxo-2,5-dihydro-1H-pyrrol-1-yl)hexanoyl]amino}ethoxy)ethoxy]phenyl beta-D-glucopyranosiduronic acid

To a solution of Example 2.26.8 (0.050 g) in N,N-dimethylformamide (1 mL) was added N,N-diisopropylamine (0.037 mL) followed by 2,5-dioxopyrrolidin-1-yl 6-(2,5-dioxo-2,5-dihydro-1H-pyrrol-1-yl)hexanoate (0.017 g), and the reaction was stirred at room temperature. After stirring for 1 hour the reaction was diluted with water and was purified by reverse phase HPLC using a Gilson system, eluting with 10-85% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.86 (s, 1H), 8.03 (d, 1H), 7.82-7.77 (m, 2H), 7.62 (d, 1H), 7.53-7.41 (m, 3H), 7.40-7.33 (m, 2H), 7.28 (s, 1H), 7.19 (d, 1H), 6.98 (s, 2H), 6.95 (d, 1H), 6.66 (s, 1H), 6.60 (d, 1H), 5.06 (t, 1H), 5.00-4.93 (m, 4H), 4.18-4.04 (m, 2H), 3.95-3.85 (m, 2H), 3.85-3.77 (m, 2H), 3.71 (t, 2H), 3.41-3.30 (m, 4H), 3.30-3.23 (m, 4H), 3.19 (q, 2H), 3.01 (t, 2H), 2.85 (d, 3H), 2.09 (s, 3H), 2.02 (t, 2H), 1.53-1.40 (m, 4H), 1.40-0.78 (m, 24H). MS (ESI) m/e 1380.5 (M-H)⁻.

### 2.27. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid (Synthon EA)

To a solution of Example 2.26.8 (0.031 g) in N,N-dimethylformamide (1 mL) was added N,N-diisopropylamine (0.023 mL) followed by 2,5-dioxopyrrolidin-1-yl 3-(2,5-dioxo-2,5-dihydro-1H-pyrrol-1-yl)propanoate (9 mg), and the reaction was stirred at room temperature. After stirring for 1 hour, the reaction was diluted with water and was purified by prep HPLC using a Gilson system, eluting with 10-85% acetonitrile in water containing 0.1% v/v trifluoroacetic acid. The desired fractions were combined and freeze-dried to provide the title compound. ¹H NMR (400 MHz, dimethyl sulfoxide-d₆) δ ppm 12.84 (s, 1H), 8.03 (d, 1H), 8.00 (t, 1H), 7.79 (d, 1H), 7.61 (d, 1H), 7.54-7.41 (m, 3H), 7.40-7.32 (m, 2H), 7.28 (s, 1H), 7.19 (d, 1H), 6.97 (s, 2H), 6.95 (d, 1H), 6.66 (s, 1H), 6.60 (d, 1H), 5.11-5.02 (m, 1H), 4.96 (s, 4H), 4.18-4.02 (m, 2H), 3.96-3.84 (m, 2H), 3.80 (s, 2H), 3.71 (t, 2H), 3.43-3.22 (m, 12H), 3.17 (q, 2H), 3.01 (t, 2H), 2.85 (d, 3H), 2.33 (t, 2H), 2.09 (s, 3H), 1.44-0.76 (m, 18H). MS (ESI) m/e 1338.5 (M-H)⁻.

### 2.28. Synthesis of 6-[8-(1,3-benzothiazol-2-ylcarbamoyl)-3,4-dihydroisoquinolin-2(1H)-yl]-3-{1-[(3-{2-[({[3-({N-[6-(2,5-dioxo-2,5-dihydro-1H-pyrrol-1-yl)hexanoyl]-beta-alanyl}amino)-4-(beta-D-galactopyranosyloxy)benzyl]oxy}carbonyl)(methyl)amino]ethoxy}-5,7-dimethyltricyclo [3.3.1.1^{3,7}]dec-1-yl)methyl]-5-methyl-1H-pyrazol-4-yl}pyridine-2-carboxylic acid(Synthon EO)

### 2.28.1. (2R,3S,4S,5R,6S)-2-(acetoxymethyl)-6-bromotetrahydro-2H-pyran-3,4,5-triyl triacetate

A dry 100 mL round bottom flask was nitrogen-sparged and charged with (2S,3R,4S,5S,6R)-6-(acetoxymethyl)tetrahydro-2H-pyran-2,3,4,5-tetrayl tetraacetate (5 g) and capped with a rubber septum under nitrogen atmosphere. Hydrogen bromide solution in glacial acetic acid (33% wt, 11.06 mL) was added, and the reaction was stirred at room temperature for two hours. The reaction mixture was diluted with dichloromethane (75 mL) and poured into 250 mL ice cold water. The layers were separated, and the organic layer was further washed with ice cold water (3 x 100 mL) and saturated aqueous sodium bicarbonate solution (100 mL). The organic layer was dried over MgSO₄, filtered and concentrated under reduced pressure. The residual acetic acid was removed by azeotroping it from toluene (3x 50) mL. The solvent was concentrated under reduced pressure to yield the title compound, which was used in the next step without further purification. MS (ESI) m/e 429.8 (M+NH₄)⁺.

### 2.28.2. (2R,3S,4S,5R,6S)-2-(acetoxymethyl)-6-(4-formyl-2-nitrophenoxy)tetrahydro-2H-pyran-3,4,5-triyl triacetate

Example 2.28.1 (5.13 g) was dissolved in acetonitrile (100 mL). Silver(I) oxide (2.89 g) was added, and the reaction was stirred for 20 minutes. 4-Hydroxy-3-nitrobenzaldehyde (2.085 g) was added, and the reaction mixture was stirred at room temperature for four hours and then vacuum filtered through a Millipore 0.22 µm filter to remove the silver salts. The solvent was concentrated under reduced pressure to yield the title compound, which was used in the next step without further purification. MS (ESI) m/e 514.9 (M+NH₄)⁺.

### 2.28.3. (2R,3S,4S,5R,6S)-2-(acetoxymethyl)-6-(4-(hydroxymethyl)-2-nitrophenoxy)tetrahydro-2H-pyran-3,4,5-triyl triacetate

A dry 1L round bottom flask nitrogen-sparged was charged with a finely ground powder of Example 2.28.2 (5.0 g,) and was kept under a nitrogen atmosphere. Tetrahydrofuran (70 mL) was added, and the solution was sonicated for two minutes to yield a suspension. Methanol (140 mL) was added, and the suspension was sonicated for another 3 minutes. The suspension was set on an ice bath and stirred for 20 minutes under a nitrogen atmosphere to reach equilibrium (0 °C). Sodium borohydride (0.380 g) was added portion wise over 20 minutes, and the cold (0 °C) reaction was stirred for 30 minutes. Ethyl acetate (200 mL) was added to the reaction mixture, and the reaction was quenched while on the ice bath with addition of 300 mL saturated ammonium chloride solution, followed by 200 mL water. The reaction mixture was extracted with ethyl acetate (3x 300 mL), washed with brine (300 mL), dried over MgSO₄, and filtered, and the solvent was concentrated under reduced pressure to yield the title compound. MS (ESI) m/e 516.9 (M+NH₄)⁺.

### 2.28.4. (2R,3S,4S,5R,6S)-2-(acetoxymethyl)-6-(2-amino-4-(hydroxymethyl)phenoxy)tetrahydro-2H-pyran-3,4,5-triyl triacetate

The title compound was prepared by substituting Example 2.28.3 for Example 2.22.2 in Example 2.22.3 and eliminating the trituration step. The product was used in the next step without further purification. MS (ESI) m/e 469.9 (M+H)⁺.

### 2.28.5. (2S,3R,4S,5S,6R)-2-(2-(3-((((9H-fluoren-9-yl)methoxy)carbonyl)amino)propanamido)-4-(hydroxymethyl)phenoxy)-6-(acetoxymethyl)tetrahydro-2H-pyran-3,4,5-triyl triacetate

The title compound was prepared by substituting Example 2.28.4 for Example 2.22.3 in Example 2.22.5. The reaction was quenched by partitioning between dichloromethane and water. The layers were separated, and the aqueous was extracted twice with ethyl acetate. The combined organic layers were washed with IN aqueous hydrochloric acid and brine, dried over Na₂SO₄, filtered, and concentrated under reduce pressure. The product was purified by silica gel chromatography, eluting with a gradient of 10-100% ethyl acetate in heptane, to yield the title compound. MS (ESI) m/e 762.9 (M+H)⁺.

### 2.28.6. (2S,3R,4S,5S,6R)-2-(2-(3-((((9H-fluoren-9-yl)methoxy)carbonyl)amino)propanamido)-4-((((4-nitrophenoxy)carbonyl)oxy)methyl)phenoxy)-6-(acetoxymethyl)tetrahydro-2H-pyran-3,4,5-triyl triacetate

To an ambient solution of Example 2.28.5 (3.2g) and bis(4-nitrophenyl)carbonate (1.914 g) in N,N-dimethylformamide (20 mL) was added N,N-diisopropylethylamine (1.10 mL,) dropwise. The reaction was stirred for 1.5 hours at room temperature. The solvent was concentrated under reduced pressure. The crude product was purified by silica gel chromatography, eluting with a gradient of 10-100% ethyl acetate in heptanes, to give the title compound. MS (ESI) m/e 927.8 (M+H), 950.1 (M+Na)⁺.

### 2.28.7. 3-(1-((3-(2-((((3-(3-((((9H-fluoren-9-yl)methoxy)carbonyl)amino)propanamido)-4-(((2S,3R,4S,5S,6R)-3,4,5-triacetoxy-6-(acetoxymethyl)tetrahydro-2H-pyran-2-yl)oxy)benzyl)oxy)carbonyl)(methyl)amino)ethoxy)-5, 7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 2.28.6 for Example 2.22.7 in Example 2.22.8. MS (ESI) m/e 1548.3 (M+H)⁺.

### 2.28.8. 3-(1-((3-(2-((((3-(3-aminopropanamido)-4-(((2S,3R,4S,5R,6R)-3,4,5-trihydroxy-6-(hydroxymethyl)tetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 2.28.7 for Example 2.22.7 in Example 2.22.8. MS (ESI) m/e 1158.3 (M+H)⁺.

### 2.28.9. 6-[8-(1,3-benzothiazol-2-ylcarbamoyl)-3,4-dihydroisoquinolin-2(1H)-yl]-3-{1-[(3-{2-[({[3-({N-[6-(2,5-dioxo-2,5-dihydro-1H-pyrrol-1-yl)hexanoyl]-beta-alanyl}amino)-4-(beta-D-galactopyranosyloxy)benzyl]oxy}carbonyl)(methyl)amino]ethox y}-5,7-dimethyltricyclo[3.3.1.1^{3,7}] dec-1-yl)methyl]-5-methyl-1H-pyrazol-4-yl}pyridine-2-carboxylic acid

The title compound was prepared by substituting Example 2.28.8 for Example 2.22.8 in Example 2.22.9. ¹HNMR (400 MHz, dimethyl sulfoxide-d₆) δ ppm 12.85 (bs, 1H), 9.13 (bs, 1H), 8.19 (bs, 1H), 8.03 (d, 1H), 7.88 (d, 1H), 7.79 (d, 1H), 7.62 (d, 1H), 7.55-7.39 (m, 3H), 7.41-7.30 (m, 2H), 7.28 (s, 1H), 7.14 (d, 1H), 7.05-6.88 (m, 4H), 4.96 (bs, 4H), 3.57-3.48 (m, 1H), 3.49-3.09 (m, 11H), 3.08-2.57 (m, 7H), 2.33 (d, 1H), 2.14-1.97 (m, 6H), 1.55-0.90 (m, 20H), 0.86-0.79 (m, 6H). MS (ESI) m/e 1351.3 (M+H)⁺.

### 2.29. Synthesis of 2-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-5-[2-(2-{[6-(2,5-dioxo-2,5-dihydro-1H-pyrrol-1-yl)hexanoyl]amino}ethoxy)ethoxy]phenyl beta-D-glucopyranosiduronic acid (Synthon FB)

### 2.29.1. 4-(2-(2-bromoethoxy)ethoxy)-2-hydroxybenzaldehyde

A solution of 2,4-dihydroxybenzaldehyde (1.0 g), 1-bromo-2-(2-bromoethoxy)ethane (3.4 g) and potassium carbonate (1.0 g) in acetonitrile (30 mL) was heated to 75 °C for 2 days. The reaction was cooled, diluted with ethyl acetate (100 mL), washed with water (50 mL) and brine (50 mL), dried over magnesium sulfate, filtered and concentrated. Purification of the residue by silica gel chromatography, eluting with a gradient of 5-30% ethyl acetate in heptane, provided the title compound. MS (ELSD) m/e 290.4 (M+H)⁺.

### 2.29.2. 4-(2-(2-azidoethoxy)ethoxy)-2-hydroxybenzaldehyde

To a solution of Example 2.29.1 (1.26 g) in N,N-dimethylformamide (10 mL) was added sodium azide (0.43 g), and the reaction was stirred at room temperature overnight. The reaction was diluted with diethyl ether (100 mL), washed with water (50 mL) and brine (50 mL), dried over magnesium sulfate, filtered, and concentrated. Purification of the residue by silica gel chromatography, eluting with a gradient of 5-30% ethyl acetate in heptane, gave the title compound. MS (ELSD) m/e 251.4 (M+H)⁺.

### 2.29.3. (2S,3R,4S,5S,6S)-2-(5-(2-(2-azidoethoxy)ethoxy)-2-formylphenoxy)-6-(methoxycarbonyl)tetrahydro-2H-pyran-3,4,5-triyl triacetate

A solution of Example 2.29.2 (0.84 g), (3R,4S,5S,6S)-2-bromo-6-(methoxycarbonyl)tetrahydro-2H-pyran-3,4,5-triyl triacetate (1.99 g) and silver (I) oxide (1.16 g) were stirred together in acetonitrile (15 mL). After stirring overnight, the reaction was diluted with dichloromethane (20 mL). Diatomaceous earth was added, and the reaction filtered and concentrated. Purification of the residue by silica gel chromatography, eluting with a gradient of 5-75% ethyl acetate in heptane, gave the title compound.

### 2.29.4. (2S,3R,4S,5S,6S)-2-(5-(2-(2-azidoethoxy)ethoxy)-2-(hydroxymethyl)phenoxy)-6-(methoxycarbonyl)tetrahydro-2H-pyran-3,4,5-triyl triacetate

A solution of Example 2.9.3 (0.695 g) in methanol (5 mL) and tetrahydrofuran (2 mL) was cooled to 0 °C. Sodium borohydride (0.023 g) was added, and the reaction was warmed to room temperature. After stirring for a total of 1 hour, the reaction was poured into a mixture of ethyl acetate (75 mL) and water (25 mL), and saturated aqueous sodium bicarbonate (10 mL) was added. The organic layer was separated, washed with brine (50 mL), dried over magnesium sulfate, filtered, and concentrated. Purification of the residue by silica gel chromatography, eluting with a gradient of 5-85% ethyl acetate in heptane, gave the title compound. MS (ELSD) m/e 551.8 (M-H₂O)⁻.

### 2.29.5. (2S,3R,4S,5S,6S)-2-(5-(2-(2-aminoethoxy)ethoxy)-2-(hydroxymethyl)phenoxy)-6-(methoxycarbonyl)tetrahydro-2H-pyran-3,4,5-triyl triacetate

To Example 2.29.4 (0.465 g) in tetrahydrofuran (20 mL) was added 5% Pd/C (0.1 g) in a 50 mL pressure bottle, and the mixture was shaken for 16 hours under 30 psi hydrogen. The reaction was filtered and concentrated to give the title compound, which was used without further purification. MS (ELSD) m/e 544.1 (M+H)⁺.

### 2.29.6. (2S,3R,4S,5S,6S)-2-(5-(2-(2-((((9H-fluoren-9-yl)methoxy)carbonyl)amino)ethoxy)ethoxy)-2-(hydroxymethyl)phenoxy)-6-(methoxycarbonyl)tetrahydro-2H-pyran-3,4,5-triyl triacetate

A solution of Example 2.29.5 (0.443 g) in dichloromethane (8 mL) was cooled to 0°C, then N,N-diisopropylamine (0.214 mL) and (9H-fluoren-9-yl)methyl carbonochloridate (0.190 g) were added. After 1 hour, the reaction was concentrated. Purification of the residue by silica gel chromatography, eluting with a gradient of 5-95% ethyl acetate in heptane, gave the title compound. MS (ELSD) m/e 748.15 (M-OH)⁻.

### 2.29.7. (2S,3R,4S,5S,6S)-2-(5-(2-(2-((((9H-fluoren-9-yl)methoxy)carbonyl)amino)ethoxy)ethoxy)-2-((((4-nitrophenoxy)carbonyl)oxy)methyl)phenoxy)-6-(methoxycarbonyl)tetrahydro-2H-pyran-3,4,5-triyl triacetate

To a solution of Example 2.29.6 (0.444 g) in N,N-dimethylformamide (5 mL) was added N,N-diisopropylamine (0.152 mL) and bis(4-nitrophenyl) carbonate (0.353 g), and the reaction was stirred at room temperature. After 5 hours, the reaction was concentrated. Purification of the residue by silica gel chromatography, eluting with a gradient of 5-90% ethyl acetate in heptane, gave the title compound.

### 2.29.8. 3-(1-((3-(2-((((4-(2-(2-((((9H-fluoren-9-yl)methoxy)carbonyl)amino)ethoxy)ethoxy)-2-(((2S,3R,4S,5S,6S)-3,4,5-triacetoxy-6-(methoxycarbonyl)tetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.1.17 (0.117 g) and Example 2.29.7 (0.143 g) in N,N-dimethylformamide (1.5 m) was added N,N-diisopropylamine (0.134 mL), and the reaction was stirred overnight. The reaction was diluted with ethyl acetate (75 mL) then washed with water (20 mL), followed by brine (4x 20 mL). The organic layer was dried over magnesium sulfate, filtered and concentrated to give the title compound, which was used without further purification.

### 2.29.9. 3-(1-((3-(2-((((4-(2-(2-aminoethoxy)ethoxy)-2-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A suspension of Example 2.29.8 (0.205 g) in methanol (2 mL) was treated with a solution of lithium hydroxide hydrate (0.083 g) in water (1 mL). After stirring for 1 hour, the reaction was quenched by the addition of acetic acid (0.113 mL), diluted with dimethyl sulfoxide, and purified by prep HPLC using a Gilson system eluting with 10-85% acetonitrile in water containing 0.1% v/v trifluoroacetic acid. The desired fractions were combined and freeze-dried to provide the title compound.

### 2.29.10.2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-5-[2-(2-{[6-(2,5-dioxo-2,5-dihydro-1H-pyrrol-1-yl)hexanoyl]amino}ethoxy)ethoxy]phenyl beta-D-glucopyranosiduronic acid

To a solution of Example 2.29.9 (0.080 g) in N,N-dimethylformamide (1 mL) was added N,N-diisopropylamine (0.054 mL) followed by 2,5-dioxopyrrolidin-1-yl 6-(2,5-dioxo-2,5-dihydro-1H-pyrrol-1-yl)hexanoate (0.025 g), and the reaction was stirred at room temperature. After stirring for 1 hour, the reaction was diluted with water (0.5 mL) and purified by prep HPLC (Gilson system), eluting with 10-85% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.86 (s, 1H), 8.03 (d, 1H), 7.86-7.81 (m, 1H), 7.79 (d, 1H), 7.62 (d, 1H), 7.52-7.41 (m, 3H), 7.39-7.32 (m, 2H), 7.28 (s, 1H), 7.19 (d, 1H), 6.99 (s, 2H), 6.95 (d, 1H), 6.68 (d, 1H), 6.59 (d, 1H), 5.09-4.99 (m, 3H), 4.96 (s, 2H), 4.05 (s, 2H), 3.94 (d, 1H), 3.88 (t, 2H), 3.81 (d, 2H), 3.47-3.24 (m, 15H), 3.19 (q, 2H), 3.01 (t, 2H), 2.86 (d, 3H), 2.09 (s, 3H), 2.03 (t, 2H), 1.51-1.41 (m, 4H), 1.41-0.78 (m, 18H), MS (ESI) m/e 1382.2 (M+H)⁺.

### 2.30. Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]-5-[2-(2- {[3-(2,5-dioxo-2,5-dihydro-1H-pyrrol-1-yl)propanoyl]amino}ethoxy)ethoxy]phenyl beta-D-glucopyranosiduronic acid (Synthon KX)

### 2.30.1. 3-(1-((3-(2-((((4-(2-(2-aminoethoxy)ethoxy)-2-(((2S,3R,4S,5S,6S)-6-carboxy-3,4,5-trihydroxytetrahydro-2H-pyran-2-yl)oxy)benzyl)oxy)carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.3.7 (0.071 g) and Example 2.29.7 (0.077 g) in N,N-dimethylformamide (0.5 mL) was added N,N-diisopropylamine (0.072 mL), and the reaction was stirred for 3 hours. The reaction was concentrated, and the resulting oil was dissolved in tetrahydrofuran (0.5 mL) and methanol (0.5 mL) and treated with lithium hydroxide monohydrate (0.052 g) solution in water (0.5 mL). After stirring for 1 hour, the reaction was diluted with N,N-dimethylformamide (1 mL) and purified by prep HPLC using a Gilson system, eluting with 10-75% acetonitrile in water containing 0.1% v/v trifluoroacetic acid. The desired fractions were combined and freeze-dried to provide the title compound. MS (ESI) m/e 1175.2 (M+H)⁺.

### 2.30.2. 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]-5-[2-(2-{[3-(2,5-dioxo-2,5-dihydro-1H-pyrrol-1-yl)propanoyl]amino}ethoxy)ethoxy]phenyl beta-D-glucopyranosiduronic acid

To a solution of Example 2.30.1 (0.055 g) and 2,5-dioxopyrrolidin-1-yl 3-(2,5-dioxo-2,5-dihydro-1H-pyrrol-1-yl)propanoate (0.012 g) in N,N-dimethylformamide (0.5 mL) was added N,N-diisopropylamine (0.022 mL), and the reaction was stirred at room temperature. After stirring for 1 hour, the reaction was diluted with a 1:1 solution of N,N-dimethylformamide and water (2 mL) and purified by prep HPLC using a Gilson system eluting with 10-85% acetonitrile in water containing 0.1% v/v trifluoroacetic acid. The desired fractions were combined and freeze-dried to provide the title compound. ¹H NMK (400 MHz, dimethyl sulfoxide-*d*₆) δ ppm 12.85 (s, 1H), 8.07 - 8.00 (m, 2H), 7.79 (d, 1H), 7.62 (d, 1H), 7.55 - 7.41 (m, 3H), 7.40 - 7.32 (m, 2H), 7.28 (s, 1H), 7.20 (d, 1H), 7.11 (t, 1H), 6.98 (s, 2H), 6.95 (d, 1H), 6.66 (s, 1H), 6.60 (dd, 1H), 5.04 (d, 1H), 5.00 (s, 2H), 4.96 (s, 2H), 4.10 - 4.03 (m, 2H), 3.95 (d, 2H), 3.88 (t, 2H), 3.70 (t, 2H), 3.59 (t, 2H), 3.46 - 3.38 (m, 4H), 3.36 - 3.25 (m, 4H), 3.17 (q, 2H), 3.08 - 2.98 (m, 4H), 2.33 (t, 2H), 2.10 (s, 3H), 1.37 (s, 2H), 1.25 (q, 4H), 1.18 - 0.93 (m, 6H), 0.84 (s, 6H), MS (ESI) m/e 1325.9 (M+H)⁺.

### 2.31. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-(3-{[6-(2,5-dioxo-2,5-dihydro-1H-pyrrol-1-yl)hexanoyl]amino}propoxy)phenyl beta-D-glucopyranosiduronic acid (Synthon FF)

### 2.31.1. (2S,3R,4S,5S,6S)-2-(3-(3-((((9H-fluoren-9-yl)methoxy)carbonyl)amino)propoxy)-4-formylphenoxy)-6-(methoxycarbonyl)tetrahydro-2H-pyran-3,4,5-triyl triacetate

To a solution of (9H-fluoren-9-yl)methyl (3-hydroxypropyl)carbamate (0.245 g) and triphenylphosphine (0.216 g) in tetrahydrofuran (2 mL) at 0 °C was added diisopropyl azodicarboxylate (0.160 mL) dropwise. After stirring for 15 minutes, Example 2.26.1 (0.250 g) was added, the ice bath was removed, and the reaction was allowed to warm to room temperature. After 2 hours, the reaction was concentrated. Purification of the residue by silica gel chromatography, eluting with a gradient of 5-70% ethyl acetate in heptane, gave the title compound. MS (APCI) m/e 512.0 (M-FMOC)⁻.

### 2.31.2. (2S,3R,4S,5S,6S)-2-(3-(3-((((9H-fluoren-9-yl)methoxy)carbonyl)amino)propoxy)-4-(hydroxymethyl)phenoxy)-6-(methoxycarbonyl)tetrahydro-2H-pyran-3,4,5-triyl triacetate

To a suspension of Example 2.31.1 (0.233 g) in methanol (3 mL) and tetrahydrofuran (1 mL) was added sodium borohydride (6 mg). After 30 minutes, the reaction was poured into ethyl acetate (50 mL) and water (25 mL) followed by the addition of saturated aqueous sodium bicarbonate solution (5 mL). The organic layer was separated, washed with brine (25 mL), dried over magnesium sulfate, filtered, and concentrated. Purification of the residue by silica gel chromatography, eluting with a gradient of 5-80% ethyl acetate in heptane, gave the title compound. MS (APCI) m/e 718.1 (M-OH)⁻.

### 2.31.3. (2S,3R,4S,5S,6S)-2-(3-(3-((((9H-fluoren-9-yl)methoxy)carbonyl)amino)propoxy)-4-((((4-nitrophenoxy)carbonyl)oxy)methyl)phenoxy)-6-(methoxycarbonyl)tetrahydro-2H-pyran-3,4,5-triyl triacetate

To a solution of Example 2.31.2 (0.140 g) and bis(4-nitrophenyl) carbonate (0.116 g) in N,N-dimethylformamide (1 mL) was added N,N-diisopropylamine (0.050 mL). After 1.5 hours, the reaction was concentrated under high vacuum. Purification of the residue by silica gel chromatography, eluting with a gradient of 10-70% ethyl acetate in heptane, gave the title compound.

### 2.31.4. 3-(1-((3-(2-((((2-(3-((((9H-fluoren-9-yl)methoxy)carbonyl)amino)propoxy)-4-(((2S,3R,4S,5S,6S)-3,4,5-triacetoxy-6-(methoxycarbonyl)tetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.1.17 (0.065 g) and Example 2.31.3 (0.067 g) in N,N-dimethylformamide (0.75 mL) was added N,N-diisopropylamine (0.065 mL). After 6 hours, additional N,N-diisopropylamine (0.025 mL) was added, and the reaction mixture was stirred overnight. The reaction was diluted with ethyl acetate (50 mL) and washed with water (20 mL) followed by brine (20 mL). The ethyl acetate layer was dried over magnesium sulfate, filtered, and concentrated to give the title compound, which was used in the next step without further purification.

### 2.31.5. 3-(1-((3-(2-((((2-(3-aminopropoxy)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.31.4 (0.064 g) was dissolved in methanol (0.75 mL) and treated with lithium hydroxide monohydrate (0.031 g) as a solution in water (0.75 mL). After stirring for 2 hours, the reaction was diluted with N,N-dimethylformamide (1 mL) and quenched with trifluoroacetic acid (0.057 mL). The solution was purified by prep HPLC using a Gilson system, eluting with 10-85% acetonitrile in water containing 0.1% v/v trifluoroacetic acid. The desired fractions were combined and freeze-dried to provide the title compound.

### 2.31.6. 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-(3-{[6-(2,5-dioxo-2,5-dihydro-1H-pyrrol-1-yl)hexanoyl] amino}propoxy)phenyl beta-D-glucopyranosiduronic acid

To a solution of Example 2.31.5 (0.020 g) and 2,5-dioxopyrrolidin-1-yl 6-(2,5-dioxo-2,5-dihydro-1H-pyrrol-1-yl)hexanoate (5.8 mg) in N,N-dimethylformamide (0.5 mL) was added N,N-diisopropylamine (0.014 mL). After stirring for 2 hours, the reaction was diluted with N,N-dimethylformamide (1.5 mL) and water (0.5 mL). The solution was purified by prep HPLC using a Gilson system, eluting with 10-75% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.83 (s, 1H), 8.03 (d, 1H), 7.83 (t, 1H), 7.79 (d, 1H), 7.62 (d, 1H), 7.54-7.42 (m, 3H), 7.37 (d, 1H), 7.34 (d, 1H), 7.28 (s, 1H), 7.19 (d, 1H), 6.98 (s, 2H), 6.95 (d, 1H), 6.64 (d, 1H), 6.59 (d, 1H), 5.05 (t, 1H), 4.96 (d, 4H), 4.02-3.94 (m, 2H), 3.88 (t, 2H), 3.46-3.22 (m, 14H), 3.18 (q, 2H), 3.01 (t, 2H), 2.85 (d, 3H), 2.09 (s, 3H), 2.02 (t, 2H), 1.81 (p, 2H), 1.54-1.41 (m, 4H), 1.41-0.78 (m, 18H). MS (ESI) m/e 1350.5 (M-H)⁻.

### 2.32. Synthesis of 1-O-({4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-[2-(2-{[6-(2,5-dioxo-2,5-dihydro-1H-pyrrol-1-yl)hexanoyl]amino}ethoxy)ethoxy]phenyl}carbamoyl)-beta-D-glucopyranuronic acid (Synthon FU)

### 2.32.1. 2-amino-5-(hydroxymethyl)phenol

Diisobutylaluminum hydride (1M in dichloromethane, 120 mL) was added to methyl 4-amino-3-hydroxybenzoate (10 g) in 50 mL dichloromethane at -78°C over 5 minutes, and the solution was allowed to warm to 0 °C. The reaction mixture was stirred 2 hours. Another 60 mL of diisobutylaluminum hydride (1M in dichloromethane) was added, and the reaction was stirred at 0 °C for one hour more. Methanol (40 mL) was carefully added. Saturated sodium potassium tartrate solution (100 mL) was added, and the mixture was stirred overnight. The mixture was extracted twice with ethyl acetate, the combined extracts were concentrated to a volume of roughly 100 mL, and the mixture was filtered. The solid was collected, and the solution was concentrated to a very small volume and filtered. The combined solids were dried to give the title compound.

### 2.32.2. 2-(2-azidoethoxy)ethyl 4-methylbenzenesulfonate

To an ambient solution of 2-(2-azidoethoxy)ethanol (4.85 g), triethylamine (5.16 mL), and N,N-dimethylpyridin-4-amine (0.226 g) in dichloromethane (123 mL) was added 4-methylbenzene-1-sulfonyl chloride (7.05 g). The reaction was stirred overnight and quenched by the addition of dichloromethane and saturated aqueous ammonium chloride solution. The layers were separated, and the organic layer was washed twice with brine. The organic layer was dried with anhydrous sodium sulfate, filtered and concentrated under reduced pressure to provide the title compound, which was used in the subsequent reaction without further purification. MS (ESI) m/e 302.9 (M+NH₄)⁺.

### 2.32.3. (4-amino-3-(2-(2-azidoethoxy)ethoxy)phenyl)methanol

To an ambient solution of Example 2.32.1 (0.488 g) in N,N-dimethylformamide (11.68 mL) was added sodium hydride (0.140 g). The mixture was stirred for 0.5 hours, and Example 2.32.2 (1.0 g) was added as a solution in N,N-dimethylformamide (2.0 mL). The reaction was heated to 50 °C overnight. The reaction mixture was quenched by the addition of water and ethyl acetate. The layers were separated, and the aqueous layer was extracted twice with ethyl acetate. The combined organics were dried with anhydrous sodium sulfate, filtered and concentrated under reduced pressure. The residue was purified by silica gel chromatography, eluting with a gradient of 25-100% ethyl acetate, to give the title compound. MS (ESI) m/e 253.1 (M+H)⁺.

### 2.32.4. 2-(2-(2-azidoethoxy)ethoxy)-4-(((tert-butyldimethylsilyl)oxy)methyl)aniline

To an ambient solution of Example 2.32.3 (440 mg) and imidazole (178 mg) in tetrahydrofuran (10.6 mL) was added tert-butyldimethylchlorosilane (289 mg). The reaction mixture was stirred for 16 hours and quenched by the addition of ethyl acetate (30 mL) and saturated aqueous sodium bicarbonate (20 mL). The layers were separated, and the aqueous was extracted twice with ethyl acetate. The combined organics were dried with anhydrous sodium sulfate, filtered and concentrated under reduced pressure. The residue was purified by silica gel chromatography, eluting with a gradient of 0 to 50% ethyl acetate in heptanes, to give the title compound. MS (ESI) m/e 366.9 (M+H).

### 2.32.5. (2S,3R,4S,5S,6S)-2-(((2-(2-(2-azidoethoxy)ethoxy)-4-(((tert-butyldimethylsilyl)oxy)methyl)phenyl)carbamoyl)oxy)-6-(methoxycarbonyl)tetrahydro-2H-pyran-3,4,5-triyl triacetate

Example 2.32.4 (410 mg) was dried overnight in a 50 mL dry round-bottom flask under high vacuum. To a cold (0 °C bath temperature) solution of Example 2.32.4 (410 mg) and triethylamine (0.234 mL) in toluene (18 mL) was added phosgene (0.798 mL, 1M in dichloromethane). The reaction was slowly warmed to room temperature and stirred for one hour. The reaction was cooled (0 °C bath temperature), and a solution of (3R,4S,5S,6S)-2-hydroxy-6-(methoxycarbonyl)tetrahydro-2H-pyran-3,4,5-triyl triacetate (411 mg) and triethylamine (0.35 mL) in toluene (5 mL) was added. The reaction was warmed to room temperature and heated to 50 °C for 2 hours. The reaction was quenched by the addition of saturated aqueous bicarbonate solution and ethyl acetate. The layers were separated, and the aqueous layer was extracted twice with ethyl acetate. The combined organic layers were dried with anhydrous sodium sulfate, filtered and concentrated under reduced pressure. The residue was purified by silica gel chromatography, eluting with a gradient of 0-40% ethyl acetate in heptane, to give the title compound. MS (ESI) m/e 743.9 (M+NH₄)⁺.

### 2.32.6. (2S,3R,4S,5S,6S)-2-(((2-(2-(2-azidoethoxy)ethoxy)-4-(hydroxymethyl)phenyl)carbamoyl)oxy)-6-(methoxycarbonyl)tetrahydro-2H-pyran-3,4,5-triyl triacetate

To a solution of Example 2.32.5 (700 mg) in methanol (5 mL) was added a solution of p-toluenesulfonic acid monohydrate (18.32 mg) in methanol (2 mL). The reaction was stirred at room temperature for 1 hour. The reaction was quenched by the addition of saturated aqueous sodium bicarbonate solution and dichloromethane. The layers were separated, and the aqueous layer was extracted with additional dichloromethane. The combined organics were dried over MgSO₄ and filtered, and the solvent was evaporated under reduced pressure to yield the title compound, which was used in the subsequent step without further purification. MS (ESI) m/e 629.8 (M+ NH₄)⁺.

### 2.32.7. (2S,3R,4S,5S,6S)-2-(((2-(2-(2-azidoethoxy)ethoxy)-4-((((4-nitrophenoxy)carbonyl)oxy )methyl)phenyl)carbamoyl)oxy)-6-(methoxycarbonyl)tetrahydro-2H-pyran-3,4,5-triyl triacetate

N,N-Diisopropylethylamine (0.227 mL) was added dropwise to an ambient solution of Example 2.32.6 (530 mg) and bis(4-nitrophenyl)carbonate (395 mg) in N,N-dimethylformamide (4.3 mL). The reaction mixture was stirred at ambient temperature for 1.5 hours. The solvent was concentrated under reduced pressure. The residue was purified by silica gel chromatography, eluting with a gradient of 0-50% ethyl acetate in heptanes to give the title compound. MS (ESI) m/e 794.9 (M+NH₄)⁺.

### 2.32.8. 3-(1-((3-(2-((((3-(2-(2-azidoethoxy)ethoxy)-4-(((((2S,3R,4S,5S,6S)-3,4,5-triacetoxy-6-(methoxycarbonyl)tetrahydro-2H-pyran-2-yl)oxy)carbonyl)amino)benzyl)oxy)carbonyl)(methyl)amino)eth oxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a cold (0 °C) solution of the trifluoroacetic acid salt of Example 1.1.17 (111 mg) and Example 2.32.7 (98.5 mg) in N,N-dimethylformamide (3.5 mL) was added N,N-diisopropylethylamine (0.066 mL). The reaction was slowly warmed to room temperature and stirred for 16 hours. The reaction was quenched by the addition of water and ethyl acetate. The layers were separated, and the aqueous layer was extracted twice with ethyl acetate. The combined organics were dried with anhydrous sodium sulfate, filtered and concentrated under reduced pressure to yield the title compound, which was used in the subsequent step without further purification. MS (ESI) m/e 1398.2 (M+H)⁺.

### 2.32.9. 3-(1-((3-(2-((((3-(2-(2-azidoethoxy)ethoxy)-4-(((((2S,3R,4S,5S,6S)-6-carboxy-3,4,5-trihydroxytetrahydro-2H-pyran-2-yl)oxy)carbonyl)amino)benzyl)oxy)carbonyl)(methyl)amino)eth oxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a cold (0 °C) solution of Example 2.32.8 (150 mg) in methanol (3.0 mL) was added 2M lithium hydroxide solution (0.804 mL). The reaction was stirred for 1 hour and was quenched by the addition of acetic acid (0.123 mL) while still at 0 °C. The crude reaction solution was purified by reverse phase HPLC using a Gilson system with a C18 column, eluting with a gradient of 10-100% acetonitrile in water containing 0.1% v/v trifluoroacetic acid. The fractions containing the product were lyophilized to give the title compound. MS (ESI) m/e 1258.2 (M+H)⁺.

### 2.32.10.3-(1-((3-(2-((((3-(2-(2-aminoethoxy)ethoxy)-4-(((((2S,3R,4S,5S,6S)-6-carboxy-3,4,5-trihydroxytetrahydro-2H-pyran-2-yl)oxy)carbonyl)amino)benzyl)oxy)carbonyl)(methyl)amino)eth oxy)-5,7-dimethyladamantan-1-yl)methyl)-5-methyl-1H-pyrazol-4-yl)-6-(8-(benzo [d] thiazol-2-ylcarbam oyl)-3,4-dihydroisoquinolin-2(1H)-yl)picolinic acid

To a solution of Example 2.32.9 (45 mg) dissolved in 2:1 tetrahydrofuran:water (0.3 mL) was added a solution oftris(2-carboxyethyl))phosphine hydrochloride (51.3 mg in 0.2 mL water). The reaction was stirred at room temperature for 16 hours. The solvent was partially concentrated under reduced pressure to remove most of the tetrahydrofuran. The crude reaction was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound as a trifluoroacetic acid salt. MS (ESI) m/e 1232.3 (M+H)⁺.

### 2.32.11.1-O-({4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-[2-(2-{[6-(2,5-dioxo-2,5-dihydro-1H-pyrrol-1-yl)hexanoyl]amino}ethoxy)ethoxy]phenyl}carbamoyl)-beta-D-glucopyranuronic acid

To a solution of the trifluoroacetic acid salt of Example 2.32.10 (15 mg) in 1 mL N,N-dimethylformamide were added 2,5-dioxopyrrolidin-1-yl 6-(2,5-dioxo-2,5-dihydro-1H-pyrrol-1-yl)hexanoate (4.12 mg) and N,N-diisopropylethylamine (0.010 mL), and the reaction was stirred at room temperature for 16 hours. The crude reaction mixture was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. ¹H NMR (500 MHz, dimethyl sulfoxide-d₆) δ ppm 12.84 (s, 1H),8.58 (d, 1H), 8.03 (d, 1H), 7.79 (t, 2H),7.68 (s, 1H), 7.61 (d, 1H), 7.40-7.54 (m, 3H), 7.36 (q, 2H),7.27 (s, 1H), 7.05 (s, 1H), 6.97 (s, 2H), 6.93 (t, 2H), 5.41(d, Hz, 1H), 5.38 (d, 1H), 5.27 (d, 1H),4.85-5.07 (m, 4H), 4.11 (t, 2H), 3.87 (t, 2H), 3.80(s, 2H), 3.71-3.77 (m, 3H), 3.46 (s, 3H), 3.22 (d, 2H), 3.00(t, 2H), 2.86 (d, 3H), 2.08 (s, 3H), 2.01 (t, 2H), 1.44 (dd, 4H), 1.34 (d, 2H), 0.89-1.29(m, 16H), 0.82 (d, 7H), 3.51-3.66 (m, 3H). MS (ESI) m/e 1447.2 (M+Na)⁺.

### 2.33. Synthesis of 6-[8-(1,3-benzothiazol-2-ylcarbamoyl)-3,4-dihydroisoquinolin-2(1H)-yl]-3-(1-{[3-(2-{[({3-[(N-{[2-({N-[19-(2,5-dioxo-2,5-dihydro-1H-pyrrol-1-yl)-17-oxo-4,7,10,13-tetraoxa-16-azanonadecan-1-oyl]-3-sulfo-D-alanyl}amino)ethoxy]acetyl}-beta-alanyl)amino]-4-(beta-D-galactopyranosyloxy)benzyl}oxy)carbonyl] (methyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon GH)

### 2.33.1. (R)-28-(2,5-dioxo-2,5-dihydro-1H-pyrrol-1-yl)-7,10,26-trioxo-8-(sulfomethyl)-3,13,16,19,22-pentaoxa-6,9,25-triazaoctacosan-1-oic acid

The title compound was synthesized using solid phase peptide synthesis as described herein. 2-(2-((((9H-Fluoren-9-yl)methoxy)carbonyl)amino)ethoxy)acetic acid (1543 mg) was dissolved in 10 mL dioxane, and the solvent was concentrated under reduced pressure. (The procedure was repeated twice). The material was lyophilized overnight. The dioxane-dried amino acid was dissolved in 20 mL sieve-dried dichloromethane to which was added N,N-diisopropylethylamine (4.07 mL). The solution was added to a 2-chlorotrityl solid support resin (8000 mg), which was previously washed (twice) with sieve-dried dichloromethane. The mixture of resin and amino acid was shaken at ambient temperature for 4 hours, drained, washed with 17:2:1 dichloromethane:methanol:N,N-diisopropylethylamine, and washed three times with N,N-dimethylformamide. The mixture was then washed three more times, alternating between sieve-dried dichloromethane and methanol. The loaded resin was dried in a vacuum oven at 40 °C. The resin loading was determined by quantitative Fmoc-loading test measuring absorbance at 301 nm of a solution obtained by deprotecting a known amount of resin by treatment with 20% piperidine in N,N-dimethylformamide. All Fmoc deprotection steps were performed by treatment of the resin with 20% piperidine in N,N-dimethylformamide for 20 minutes followed by a washing step with N,N-dimethylformamide. Coupling of the amino acids (R)-2-((((9H-fluoren-9-yl)methoxy)carbonyl)amino)-3-sulfopropanoic acid and subsequently 1-(2,5-dioxo-2,5-dihydro-1H-pyrrol-1-yl)-3-oxo-7,10,13,16-tetraoxa-4-azanonadecan-19-oic acid was done by activation of 4 equivalents of amino acid with 4 equivalents of ((1H-benzo[d][1,2,3]triazol-1-yl)oxy)tri(pyrrolidin-1-yl)phosphonium hexafluorophosphate(V) and 8 equivalents of N,N-diidopropylethylamine in N,N-dimethylformamide for one minute followed by incubation with the resin for one hour. The title compound was cleaved from the resin by treatment with 5 % trifluoroacetic acid in dichloromethane for 30 minutes. The resin was filtered, and the filtrate was concentrated under reduced pressure to yield the title compound which was used in the next step without further purification. MS (ESI) m/e 669.0 (M+H)⁺.

### 2.33.2. 6-[8-(1,3-benzothiazol-2-ylcarbamoyl)-3,4-dihydroisoquinolin-2(1H)-yl]-3-(1-{ [3-(2-{[({3-[(N-{[2-({N-[19-(2,5-dioxo-2,5-dihydro-1H-pyrrol-1-yl)-17-oxo-4,7,10,13-tetraoxa-16-azanonadecan-1-oyl]-3-sulfo-D-alanyl}amino)ethoxy]acetyl}-beta-alanyl)amino]-4-(beta-D-galactopyranosyloxy)benzyl}oxy)carbonyl](methyl)amino}ethox y)-5,7-dimethyltricyclo[3.3.1.1^{3,7}]dec-1-yl]methyl}-5-methyl-1H-pyrazol-4-yl)pyridine-2-carboxylic acid

Example 2.33.1 (5.09 mg) was mixed with 2-(3H-[1,2,3]triazolo[4,5-b]pyridin-3-yl)-1,1,3,3-tetramethylisouronium hexafluorophosphate(V) (2.63 mg,) and N,N-diisopropylethylamine (0.004 mL) in 1 mL N,N-dimethylformamide and stirred for two minutes. Example 2.28.8 (8.8 mg) was added, and the reaction mixture was stirred at room temperature for 1.5 hours. The crude reaction mixture was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. MS (ESI) m/e 1806.5 (M-H)⁻.

### 2.34. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-[3-({N-[6-(2,5-dioxo-2,5-dihydro-1H-pyrrol-1-yl)hexanoyl]-3-sulfo-L-alanyl}amino)propoxy]phenyl beta-D-glucopyranosiduronic acid (Synthon FX)

### 2.34.1. 3-(1-((3-(2-((((2-(3-((R)-2-amino-3-sulfopropanamido)propoxy)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of (R)-2-((((9H-fluoren-9-yl)methoxy)carbonyl)amino)-3-sulfopropanoic acid (0.019 g) and 2-(3H-[1,2,3]triazolo[4,5-b]pyridin-3-yl)-1,1,3,3-tetramethylisouronium hexafluorophosphate(V) (0.019 g) in N,N-dimethylformamide (0.5 mL) was added N,N-diisopropylamine (7.82 µl). After stirring for 2 minutes, the reaction was added to a solution of Example 2.31.5 (0.057 g) and N,N-diisopropylamine (0.031 mL) in N,N-dimethylformamide (0.5 mL) at room temperature and stirred for 3 hours. Diethylamine (0.023 mL) was added to the reaction and stirring was continued for an additional 2 hours. The reaction was diluted with water (1 mL), quenched with trifluoroacetic acid (0.034 mL), and the solution was purified by prep HPLC using a Gilson system, eluting with 10-85% acetonitrile in water containing 0.1% v/v trifluoroacetic acid. The desired fractions were combined and freeze-dried to provide the title compound. MS (ESI) m/e 1310.1 (M+H)⁺.

### 2.34.2. 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl] -5,7-dimethyltricyclo[3.3.1.1^{3,7}]dec-1-yl}oxy)ethyl](methyl)carbamoyl}oxy)methyl]-3-[3-({N-[6-(2,5-dioxo-2,5-dihydro-1H-pyrrol-1-yl)hexanoyl]-3-sulfo-L-alanyl}amino)propoxy]phenyl beta-D-glucopyranosiduronic acid

To a solution of Example 2.34.1 (0.0277 g) and 2,5-dioxopyrrolidin-1-yl 6-(2,5-dioxo-2,5-dihydro-1H-pyrrol-1-yl)hexanoate (7.82 mg) in N,N-dimethylformamide (0.5 mL) was added N,N-diisopropylamine (0.018 mL) and the reaction was stirred at room temperature. The reaction was purified by prep HPLC using a Gilson system eluting with 10-85% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.81 (s, 1H), 8.02 (d, 1H), 7.89-7.81 (m, 2H), 7.78 (d, 1H), 7.60 (d, 1H), 7.53-7.40 (m, 3H), 7.39-7.31 (m, 2H), 7.29 (s, 1H), 7.16 (d, 1H), 6.98-6.92 (m, 3H), 6.63 (s, 1H), 6.56 (d, 1H), 5.08-4.99 (m, 1H), 4.95 (s, 4H), 4.28 (q, 2H), 3.90-3.85 (m, 4H), 3.48-3.06 (m, 12H), 3.00 (t, 2H), 2.88-2.64 (m, 8H), 2.08 (s, 3H), 2.04 (t, 2H), 1.80 (p, 2H), 1.51-1.39 (m, 4H), 1.39-0.75 (m, 18H). MS (ESI) m/e 1501.4 (M-H)⁻.

### 2.35. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.1^{3,7}]dec-1-yl}oxy)ethyl](methyl)carbamoyl}oxy)methyl]-2-({N-[6-(2,5-dioxo-2,5-dihydro-1H-pyrrol-1-yl)hexanoyl]-beta-alanyl}amino)phenyl beta-D-glucopyranosiduronic acid (Synthon H)

### 2.35.1. (2S,3R,4S,5S,6S)-2-(4-formyl-2-nitrophenoxy)-6-(methoxycarbonyl)tetrahydro-2H-pyran-3,4,5-triyl triacetate

To a solution of (2R,3R,4S,5S,6S)-2-bromo-6-(methoxycarbonyl)tetrahydro-2H- pyran-3,4,5-triyl triacetate (4 g) in acetonitrile (100 mL)) was added silver(I) oxide (10.04 g) and 4-hydroxy-3- nitrobenzaldehyde (1.683 g). The reaction mixture was stirred for 4 hours at room temperature and filtered. The filtrate was concentrated, and the residue was purified by silica gel chromatography, eluting with 5-50% ethyl acetate in heptanes, to provide the title compound. MS (ESI) m/e (M+18)⁺.

### 2.35.2. (2S,3R,4S,5S,6S)-2-(4-(hydroxymethyl)-2-nitrophenoxy)-6-(methoxycarbonyl)tetrahydro-2H-pyran-3,4,5-triyl triacetate

To a solution of Example 2.35.1 (6 g) in a mixture of chloroform (75 mL) and isopropanol (18.75 mL) was added 0.87 g of silica gel. The resulting mixture was cooled to 0 °C, NaBH₄ (0.470 g) was added, and the resulting suspension was stirred at 0 °C for 45 minutes. The reaction mixture was diluted with dichloromethane (100 mL) and filtered through diatomaceous earth. The filtrate was washed with water and brine and concentrated to give the crude product, which was used without further purification. MS (ESI) m/e (M+NH₄)⁺:

### 2.35.3. (2S,3R,4S,5S,6S)-2-(2-amino-4-(hydroxymethyl)phenoxy)-6-(methoxycarbonyl)tetrahydro-2H-pyran-3,4,5-triyl triacetate

A stirred solution of Example 2.35.2 (7 g) in ethyl acetate (81 mL) was hydrogenated at 20 °C under 1 atmosphere H₂, using 10% Pd/C (1.535 g) as a catalyst for 12 hours. The reaction mixture was filtered through diatomaceous earth, and the solvent was evaporated under reduced pressure. The residue was purified by silica gel chromatography, eluting with 95/5 dichloromethane/methanol, to give the title compound.

### 2.35.4. 3-((((9H-fluoren-9-yl)methoxy)carbonyl)amino)propanoic acid

3-Aminopropanoic acid (4.99 g) was dissolved in 10% aqueous Na₂CO₃ solution (120 mL) in a 500 mL flask and cooled with an ice bath. To the resulting solution, (9H-fluoren-9-yl)methyl carbonochloridate (14.5 g) in 1,4-dioxane (100 mL) was gradually added. The reaction mixture was stirred at room temperature for 4 hours, and water (800 mL) was then added. The aqueous phase layer was separated from the reaction mixture and washed with diethyl ether (3 x 750 mL). The aqueous layer was acidified with 2N HCl aqueous solution to a pH value of 2 and extracted with ethyl acetate (3 x 750 mL). The organic layers were combined and concentrated to obtain crude product. The crude product was recrystallized in a mixed solvent of ethyl acetate: hexane 1:2 (300 mL) to give the title compound.

### 2.35.5. (9H-fluoren-9-yl)methyl (3-chloro-3-oxopropyl)carbamate

To a solution of Example 2.35.4 in dichloromethane (160 mL) was added sulfurous dichloride (50 mL). The mixture was stirred at 60 °C for 1 hour. The mixture was cooled and concentrated to give the title compound.

### 2.35.6. (2S,3R,4S,5S,6S)-2-(2-(3-((((9H-fluoren-9-yl)methoxy)carbonyl)amino)propanamido)-4-(hydroxymethyl)phenoxy)-6-(methoxycarbonyl)tetrahydro-2H-pyran-3,4,5-triyl triacetate

To a solution of Example 2.35.3 (6 g) in dichloromethane (480 mL) was added N,N-diisopropylethylamine (4.60 mL). Example 2.35.5 (5.34 g) was added, and the mixture was stirred at room temperature for 30 minutes. The mixture was poured into saturated aqueous sodium bicarbonate and was extracted with ethyl acetate. The combined extracts were washed with water and brine and were dried over sodium sulfate. Filtration and concentration gave a residue that was purified via radial chromatography, using 0-100% ethyl acetate in petroleum ether as mobile phase, to give the title compound.

### 2.35.7. (2S,3R,4S,5S,6S)-2-(2-(3-((((9H-fluoren-9-yl)methoxy)carbonyl)amino)propanamido)-4-((((4-nitrophenoxy)carbonyl)oxy)methyl)phenoxy)-6-(methoxycarbonyl)tetrahydro-2H-pyran-3,4,5-triyl triacetate

To a mixture of Example 2.35.6 (5.1 g) in N,N-dimethylformamide (200 mL) was added bis(4-nitrophenyl) carbonate (4.14 g) and N,N-diisopropylethylamine (1.784 mL). The mixture was stirred for 16 hours at room temperature and concentrated under reduced pressure. The crude material was dissolved in dichloromethane and aspirated directly onto a 1 mm radial Chromatotron plate and eluted with 50-100% ethyl acetate in hexanes to give the title compound. MS (ESI) m/e (M+H)⁺.

### 2.35.8. 3-(1-((3-(2-((((3-(3-aminopropanamido)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.1.17 (325 mg) and Example 2.35.7 (382 mg) in N,N-dimethylformamide (9 mL) at 0 °C was added N,N-diisopropylamine (49.1 mg). The reaction mixture was stirred at 0 °C for 5 hours, and acetic acid (22.8 mg) was added. The resulting mixture was diluted with ethyl acetate and washed with water and brine. The organic layer was dried over Na₂SO₄, filtered and concentrated. The residue was dissolved in a mixture of tetrahydrofuran (10 mL) and methanol (5 mL). To this solution at 0 °C was added 1 M aqueous lithium hydroxide solution (3.8 mL). The resulting mixture was stirred at 0 °C for 1 hour, acidified with acetic acid and concentrated. The concentrate was lyophilized to provide a powder. The powder was dissolved in N,N-dimethylformamide (10 mL), cooled in an ice-bath, and piperidine (1 mL) at 0 °C was added. The mixture was stirred at 0 °C for 15 minutes and 1.5 mL of acetic acid was added. The solution was purified by reverse-phase HPLC using a Gilson system, eluting with 30-80% acetonitrile in water containing 0.1% v/v trifluoroacetic acid, to provide the title compound. MS (ESI) m/e 1172.2 (M+H)⁺.

### 2.35.9. 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.1^{3,7}]dec-1-yl}oxy)ethyl](methyl)carbamoyl}oxy)methyl]-2-({N-[6-(2,5-dioxo-2,5-dihydro-1H-pyrrol-1-yl)hexanoyl]-beta-alanyl}amino)phenyl beta-D-glucopyranosiduronic acid

To Example 2.35.8 (200 mg) in N,N-dimethylformamide (5 mL) at 0 °C was added 2,5-dioxopyrrolidin-1-yl 6-(2,5-dioxo-2,5-dihydro-1H-pyrrol-1-yl)hexanoate (105 mg) and N,N-diisopropylethylamine (0.12 mL). The mixture was stirred at 0 °C for 15 minutes, warmed to room temperature and purified by reverse-phase HPLC on a Gilson system using a 100g C18 column, eluting with 30-80% acetonitrile in water containing 0.1% v/v trifluoroacetic acid, to provide the title compound. ¹H NMK (500 MHz, dimethyl sulfoxide-d₆) δ ppm 12.85 (s, 2H) 9.07 (s, 1H) 8.18 (s, 1H) 8.03 (d, 1H) 7.87 (t, 1H) 7.79 (d, 1H) 7.61 (d, 1H) 7.41-7.53 (m, 3H) 7.36 (q, 2H) 7.28 (s, 1H) 7.03-7.09 (m, 1H) 6.96-7.03 (m, 3H) 6.94 (d, 1H) 4.95 (s, 4H) 4.82 (t, 1H) 3.88 (t, 3H) 3.80 (d, 2H) 3.01 (t, 2H) 2.86 (d, 3H) 2.54 (t, 2H) 2.08 (s, 3H) 2.03 (t, 2H) 1.40-1.53 (m, 4H) 1.34 (d, 2H) 0.90-1.28 (m, 12H) 0.82 (d, 6H). MS (ESI) m/e 1365.3 (M+H)⁺.

### 2.36. Synthesis of 4-[({[2-({3-[(4-{6-[8-(1,3-benzothiazol-2-yl carbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-({N-[19-(2,5-dioxo-2,5-dihydro-1H-pyrrol-1-yl)-17-oxo-4,7,10,13-tetraoxa-16-azanonadecan-1-oyl]-beta-alanyl}amino)phenyl beta-D-glucopyranosiduronic acid (Synthon I)

The title compound was prepared using the procedure in Example 2.35.9, replacing 2,5-dioxopyrrolidin-1-yl 6-(2,5-dioxo-2,5-dihydro-1H-pyrrol-1-yl)hexanoate with 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate. ¹H NMR (500 MHz, dimethyl sulfoxide-d₆) δ ppm 8.95 (s, 1H) 8.16 (s, 1H) 7.99 (d, 1H) 7.57-7.81 (m, 4H) 7.38-7.50 (m, 3H) 7.34 (q, 2H) 7.27 (s, 1H) 7.10 (d, 1H) 7.00 (d, 1H) 6.88-6.95 (m, 2H) 4.97 (d, 4H) 4.76 (d, 2H) 3.89 (t, 2H) 3.84 (d, 2H) 3.80 (s, 2H) 3.57-3.63 (m, 4H) 3.44-3.50 (m, 4H) 3.32-3.43 (m, 6H) 3.29 (t, 2H) 3.16 (q, 2H) 3.02 (t, 2H) 2.87 (s, 3H) 2.52-2.60 (m, 2H) 2.29-2.39 (m, 3H) 2.09 (s, 3H) 1.37 (s, 2H) 1.20-1.29 (m, 4H) 1.06-1.18 (m, 4H) 0.92-1.05 (m, 2H) 0.83 (s, 6H). MS (ESI) m/e 1568.6 (M-H)⁻.

### 2.37. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-({N-[4-(2,5-dioxo-2,5-dihydro-1H-pyrrol-1-yl)butanoyl]-beta-alanyl}amino)phenyl beta-D-glucopyranosiduronic acid (Synthon KQ)

The title compound was prepared using the procedure in Example 2.35.9, replacing 2,5-dioxopyrrolidin-1-yl 6-(2,5-dioxo-2,5-dihydro-1H-pyrrol-1-yl)hexanoate with 2,5-dioxopyrrolidin-1-yl 4-(2,5-dioxo-2,5-dihydro-1H-pyrrol-1-yl)butanoate. ¹H NMR (500 MHz, dimethyl sulfoxide-d₆) δ ppm 12.86 (s, 3H) 9.08 (s, 2H) 8.17 (s, 1H) 8.03 (d, 1H) 7.89 (t, 1H) 7.79 (d, 1H) 7.61 (d, 1H) 7.46-7.53 (m, 1H) 7.41-7.46 (m, 1H) 7.31-7.40 (m, 1H) 7.28 (s, 1H) 7.03-7.10 (m, 1H) 6.91-7.03 (m, 2H) 4.69-5.08 (m, 4H) 3.83-3.95 (m, 2H) 3.74-3.83 (m, 2H) 3.21-3.47 (m, 12H) 2.95-3.08 (m, 1H) 2.86 (d, 2H) 1.98-2.12 (m, 3H) 1.62-1.79 (m, 2H) 0.90-1.43 (m, 8H) 0.82 (d, 3H). MS (ESI) m/e 1337.2 (M+H)⁺.

### 2.38. Synthesis of 4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-4-methyl-3-oxo-2,7,10-trioxa-4-azadodec-1-yl]-2-1[N-(12-[2-(2,5-dioxo-2,5-dihydro-1H-pyrrol-1-yl)ethoxy]ethoxy}acetyl)-beta-alanyl]amino}phenyl beta-D-glucopyranosiduronic acid (Synthon KP)

### 2.38.1. 3-(1-((-((1-(3-(3-aminopropanamido)-4-(((2S,3R,4S,5S,6S)-6-carboxy-3,4,5-trihydroxytetrahydro-2H-pyran-2-yl)oxy)phenyl)-4-methyl-3-oxo-2,7,10-trioxa-4-azadodecan-12-yl)oxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 1.2.11 for Example 1.1.17 in Example 2.35.8.

### 2.38.2. 4-[12-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)-4-methyl-3-oxo-2,7,0-trioxa-4-azadodec-1-yl]-2-{[N-({2-[2-(2,5-dioxo-2,5-dihydro-1H-pyrrol-1-yl)ethoxy]ethoxy}acetyl)-beta-alanyl]amino}phenyl beta-D-glucopyranosiduronic acid

The title compound was prepared by substituting Example 2.38.1 for Example 2.35.8 and 2,5-dioxopyrrolidin-1-yl 2-(2-(2-(2,5-dioxo-2,5-dihydro-1H-pyrrol-1-yl)ethoxy)ethoxy)acetate for 2,5-dioxopyrrolidin-1-yl 6-(2,5-dioxo-2,5-dihydro-1H-pyrrol-1-yl)hexanoate in Example 2.35.9. ¹H NMR (500 MHz, dimethyl sulfoxide-d₆) δ ppm 8.92 (s, 1H), 8.12-8.15 (m, 1H), 7.97 (d, 1H), 7.76 (d, 1H), 7.61 (d, 1H), 7.28-7.49 (m, 6H), 7.25 (s, 1H), 7.09 (d, 1H), 6.97-7.02 (m, 1H), 6.88-6.94 (m, 2H), 4.97 (d, 4H), 4.75 (d, 1H), 3.76-3.93 (m, 9H), 3.47-3.60 (m, 16H), 3.32-3.47 (m, 15H), 2.88 (s, 3H), 2.59 (t, 2H), 2.08 (s, 3H), 1.38 (s, 2H), 0.93-1.32 (m, 11H), 0.84 (s, 6H). MS (ESI) m/e 1485.2 (M+H)⁺.

### 2.39. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-[(N-{6-[(ethenylsulfonyl)amino]hexanoyl}-beta-alanyl)amino]phenyl beta-D-glucopyranosiduronic acid (Synthon HA)

### 2.39.1. methyl 6-(vinylsulfonamido)hexanoate

To a solution of 6-methoxy-6-oxohexan-l-aminium chloride (0.3 g) and triethylamine (1.15 mL) in dichloromethane at 0 °C was dropwise added ethenesulfonyl chloride (0.209 g). The reaction mixture was warmed to room temperature and stirred for 1 hour. The mixture was diluted with dichloromethane and washed with brine. The organic layer was dried over sodium sulfate, filtered, and concentrated to provide the title compound. MS (ESI) m/e 471.0 (2M+H)⁺.

### 2.39.2. 6-(vinylsulfonamido)hexanoic acid

A solution of Example 2.39.1 (80 mg) and lithium hydroxide monohydrate (81 mg) in a mixture of tetrahydrofuran (1 mL) and water (1 mL) was stirred for 2 hours, then diluted with water (20 mL), and washed with diethyl ether (10 mL). The aqueous layer was acidified to pH 4 with IN aqueous HCl and extracted with dichloromethane (3x 10 mL). The organic layer was washed with brine (5 mL), dried over sodium sulfate, filtered and concentrated to provide the title compound.

### 2.39.3. 2,5-dioxopyrrolidin-1-yl 6-(vinylsulfonamido)hexanoate

A mixture of Example 2.39.2 (25 mg), 1-ethyl-3-[3-(dimethylamino)propyl]-carbodiimide hydrochloride (43.3 mg) and 1-hydroxypyrrolidine-2,5-dione (15.6 mg) in dichloromethane (8 mL) was stirred overnight, washed with saturated aqueous ammonium chloride solution and brine, and concentrated to provide the title compound.

### 2.39.4. 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-[(N-{6-[(ethenylsulfonyl)amino]hexanoyl}-beta-alanyl)amino]phenyl beta-D-glucopyranosiduronic acid

The title compound was prepared using the procedure in Example 2.35.9, replacing 2,5-dioxopyrrolidin-1-yl 6-(2,5-dioxo-2,5-dihydro-1H-pyrrol-1-yl)hexanoate with Example 2.39.3. ¹H NMR (500 MHz, dimethyl sulfoxide-d₆) δ ppm 12.85 (s, 2H) 9.07 (s, 1H) 8.18 (s, 1H) 8.03 (d, 1H) 7.87 (t, 1H) 7.79 (d, 1H) 7.61 (d, 1H) 7.41-7.53 (m, 3H) 7.33-7.39 (m, 2H) 7.28 (s, 1H) 7.17 (t, 1H) 7.04-7.08 (m, 1H) 6.98-7.03 (m, 1H) 6.95 (d, 1H) 6.65 (dd, 1H) 5.91-6.04 (m, 2H) 4.96 (s, 4H) 4.82 (s, 1H) 3.22-3.48 (m, 11H) 3.01 (t, 2H) 2.86 (d, 3H) 2.73-2.80 (m, 2H) 2.51-2.57 (m, 2H) 1.99-2.12 (m, 5H) 1.29-1.52 (m, 6H) 0.90-1.29 (m, 12H) 0.82 (d, 6H). MS (ESI) m/e 1375.3 (M+H)⁺.

### 2.40. Synthesis of 4-[({[2-({3-[(4-{6-[8-(1,3-benzothiazol-2-ylcarbarmoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-({N-[6-(ethenylsulfonyl)hexanoyl]-beta-alanyl}amino)phenyl beta-D-glucopyranosiduronic acid (Synthon HB)

### 2.40.1. ethyl 6-((2-hydroxyethyl)thio)hexanoate

A mixture of ethyl 6-bromohexanoate (3 g), 2-mercaptoethanol (0.947 mL) and K₂CO₃ (12 g) in ethanol (100 mL) was stirred overnight and filtered. The filtrate was concentrated. The residue was dissolved in dichloromethane (100 mL) and washed with water and brine. The organic layer was dried over Na₂SO₄, filtered, and concentrated to provide the title compound.

### 2.40.2. 6-((2-hydroxyethyl)thio)hexanoic acid

The title compound was prepared using the procedure in Example 2.39.2, replacing Example 2.39.2 with Example 2.40.1. MS (ESI) m/e 175.1 (M-H₂O).

### 2.40.3. 6-((2-hydroxyethyl)sulfonyl)hexanoic acid

To a stirred solution of Example 2.40.2 (4 g) in a mixture of water (40 mL) and 1,4-dioxane (160 mL) was added Oxone® (38.4 g). The mixture was stirred overnight. The mixture was filtered and the filtrate was concentrated. The residual aqueous layer was extracted with dichloromethane. The extracts were combined and dried over Na₂SO₄, filtered, and concentrated to provide the title compound.

### 2.40.4. 6-(vinylsulfonyl)hexanoic acid

To a stirred solution of Example 2.40.3 (1 g) in dichloromethane (10 mL) under argon was added triethylamine (2.8 mL), followed by the addition of methanesulfonyl chloride (1.1 mL) at 0 °C. The mixture was stirred overnight and washed with water and brine. The organic layer was dried over sodium sulfate, filtered and concentrated to provide the title compound.

### 2.40.5. 2,5-dioxopyrrolidin-1-yl 6-(vinylsulfonyl)hexanoate

To a stirred solution of Example 2.40.4 (0.88 g) in dichloromethane (10 mL) was added 1-hydroxypyrrolidine-2,5-dione (0.54 g) and N,N'-methanediylidenedicyclohexanamine (0.92 g). The mixture was stirred overnight and filtered. The filtrate was concentrated and purified by flash chromatography, eluting with 10-25% ethyl acetate in petroleum to provide the title compound. MS (ESI) m/e 304.1 (M+H)⁺.

### 2.40.6. 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-({N-[6-(ethenylsulfonyl)hexanoyl]-beta-alanyl}amino)phenyl beta-D-glucopyranosiduronic acid

The title compound was prepared using the procedure in Example 2.35.9, replacing 2,5-dioxopyrrolidin-1-yl 6-(2,5-dioxo-2,5-dihydro-1H-pyrrol-1-yl)hexanoate with Example 2.40.5. ¹H NMR (500 MHz, dimethyl sulfoxide-d₆) δ ppm 12.84 (s, 2H) 9.07 (s, 1H) 8.18 (s, 1H) 8.03 (d, 1H) 7.89 (t, 1H) 7.79 (d, 1H) 7.61 (d, 1H) 7.41-7.53 (m, 3H) 7.32-7.40 (m, 2H) 7.28 (s, 1H) 7.04-7.11 (m, 1H) 6.98-7.03 (m, 1H) 6.88-6.97 (m, 2H) 6.17-6.26 (m, 2H) 4.95 (s, 4H) 4.82 (s, 1H) 3.74-3.99 (m, 8H) 3.41-3.46 (m, 8H) 3.24-3.41 (m, 8H) 2.97-3.08 (m, 4H) 2.86 (d, 3H) 2.54 (t, 2H) 2.00-2.13 (m, 5H) 1.43-1.64 (m, 4H) 0.89-1.40 (m, 15H) 0.82 (d, 6H). MS (ESI) m/e 1360.2 (M+H)⁺.

### 2.41. Synthesis of 4-[({[2-({3-[(4-{6-[8-(1,3-benzothiazol-2-ylcarbamoyl)-5-fluoro-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]-3-[2-(2-{[3-(2,5-dioxo-2,5-dihydro-1H-pyrrolyl)propanoyl]amino}ethoxy)ethoxy]phenyl beta-D-glucopyranosiduronic acid (Synthon LB)

### 2.41.1. 3-(1-((3-(2-((((2-(2-(2-((((9H-fluoren-9-yl)methoxy)carbonyl)amino)ethoxy)ethoxy)-4-(((2S,3R,4S,5S,6S)-3,4,5-triacetoxy-6-(methoxycarbonyl)tetrahydro-2H-pyran-2-yl)oxy)benzyl)oxy)carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-5-fluoro-3,4-dihydroisoquinolin-2(1H)-yl)picolinic acid

The title compound was prepared by substituting Example 1.6.13 for Example 1.1.17 in Example 2.26.7.

### 2.41.2. 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-5-fluoro-3,4-dihydroisoquinolin-2(1H)-yl)picolinic acid

The title compound was prepared by substituting Example 2.41.1 for Example 2.26.7 in Example 2.26.8. MS (ESI) m/e 1193 (M+H)⁺, 1191 (M-H)⁻.

### 2.41.3. 4-[({[2-({3-[(4-{6-[8-(1,3-benzothiazol-2-ylcarbamoyl)-5-fluoro-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid

The title compound was prepared by substituting Example 2.41.2 for Example 2.26.8 in Example 2.27. ¹H NMR (400MHz, dimethyl sulfoxide-*d*₆) δ ppm 12.88 (bs, 1H), 8.03 (d, 1H), 8.02 (t, 1H), 7.78 (d, 1H), 7.73 (1H), 7.53 (d, 1H), 7.47 (td, 1H), 7.35 (td, 1H), 7.29 (s, 1H), 7.26 (t, 1H), 7.26 (t, 1H), 7.19 (d, 1H), 7.02 (d, 1H), 6.98 (s, 1H), 6.65 (d, 1H), 6.59 (dd, 1H), 5.07 (d, 1H), 5.01 (s, 1H), 4.92 (1H), 4.08 (m, 2H), 3.94 (t, 2H), 3.90 (d, 2H), 3.87 (s, 2H), 3.70 (m, 6H), 3.60 (m, 6H), 3.44 (t, 2H), 3.39 (t, 2H), 3.32 (t, 1H), 3.28 (dd, 1H), 3.17 (q, 2H), 3.03 (q, 2H), 2.92 (t, 2H), 2.33 (t, 2H), 2.10 (s, 3H), 1.37 (s, 2H), 1.25 (q, 4H), 1.11 (q, 4H), 1.00 (dd, 2H), 0.83 (s, 6H). MS (ESI) m/e 1366 (M+Na)⁺, 1342 (M-H)⁻.

### 2.42. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-{2-[2-({N-[3-(2,5-dioxo-2,5-dihydro-1H-pyrrol-1-yl)propanoyl]-3-sulfo-L-alanyl}amino)ethoxy]ethoxy}phenyl beta-D-glucopyranosiduronic acid (Synthon NF)

### 2.42.1. (2S,3R,4S,5S,6S)-2-(4-formyl-3-hydroxyphenoxy)-6-(methoxycarbonyl)tetrahydro-2H-pyran-3,4,5-triyl triacetate

2,4-Dihydroxybenzaldehyde (15 g) and (2S,3R,4S,5S,6S)-2-bromo-6-(methoxycarbonyl)tetrahydro-2H-pyran-3,4,5-triyl triacetate (10 g) were dissolved in acetonitrile followed by the addition of silver carbonate (10 g) and the reaction was heated to 49°C. After stirring for 4 hours, the reaction was cooled, filtered and concentrated. The crude title compound was suspended in dichloromethane and was filtered through diatomaceous earth and concentrated. The residue was purified by silica gel chromatography eluting with 1-100% ethyl acetate/heptane to provide the title compound.

### 2.42.2. (2S,3R,4S,5S,6S)-2-(3-hydroxy-4-(hydroxymethyl)phenoxy)-6-(methoxycarbonyl)tetrahydro-2H-pyran-3,4,5-triyl triacetate

A solution of Example 2.42.1 (16.12 g) in tetrahydrofuran (200 mL) and methanol (200 mL) was cooled to 0°C and sodium borohydride (1.476 g) was added portionwise. The reaction was stirred for 20 minutes and was quenched with a 1:1 mixture of water:aqueous saturated sodium bicarbonate solution (400 mL). The resulting solids were filtered off and rinsed with ethyl acetate. The phases were separated and the aqueous layer was extracted four times with ethyl acetate. The combined organic layers were dried over magnesium sulfate, filtered, and concentrated. The crude title compound was purified via silica gel chromatography eluting with 1-100% ethyl acetate/heptanes to provide the title compound. MS (ESI) m/e 473.9 (M+NH₄)⁺.

### 2.42.3. (2S,3R,4S,5S,6S)-2-(4-(((tert-butyldimethylsilyl)oxy)methyl)-3-hydroxyphenoxy)-6-(methoxycarbonyl)tetrahydro-2H-pyran-3,4,5-triyl triacetate

To Example 2.42.2 (7.66 g) and *tert*-butyldimethylsilyl chloride (2.78 g) in dichloromethane (168 mL) at -5°C was added imidazole (2.63 g) and the reaction was stirred overnight allowing the internal temperature of the reaction to warm to 12°C. The reaction mixture was poured into saturated aqueous ammonium chloride and extracted four times with dichloromethane. The combined organics were washed with brine, dried over magnesium sulfate, filtered and concentrated. The crude title compound was purified via silica gel chromatography eluting with 1-50% ethyl acetate/heptanes to provide the title compound. MS (ESI) m/e 593.0 (M+Na)⁺.

### 2.42.4. (2S,3R,4S,5S,6S)-2-(3-(2-(2-((((9H-fluoren-9-yl)methoxy)carbonyl)amino)ethoxy)ethoxy)-4-(((tert-butyldimethylsilyl)oxy)methyl)phenoxy)-6-(methoxycarbonyl)tetrahydro-2H-pyran-3,4,5-triyl triacetate

To Example 2.42.3 (5.03 g) and triphenylphosphine (4.62 g) in toluene (88 mL) was added di-*tert*-butyl-azodicarboxylate (4.06 g) and the reaction was stirred for 30 minutes. (9H-Fluoren-9-yl)methyl (2-(2-hydroxyethoxy)ethyl)carbamate was added and the reaction was stirred for an addition 1.5 hours. The reaction was loaded directly onto silica gel and was eluted with 1-50% ethyl acetate/heptanes to provide the title compound.

### 2.42.5. (2S,3R,4S,5S,6S)-2-(3-(2-(2-((((9H-fluoren-9-yl)methoxy)carbonyl)amino)ethoxy)ethoxy)-4-(hydroxymethyl)phenoxy)-6-(methoxycarbonyl)tetrahydro-2H-pyran-3,4,5-triyl triacetate

Example 2.42.4 (4.29 g) was stirred in a 3:1:1 solution of acetic acid:water:tetrahydrofuran (100 mL) overnight. The reaction was poured into saturated aqueous sodium bicarbonate and extracted with ethyl acetate. The organic layer was dried over magnesium sulfate, filtered and concentrated. The crude title compound was purified via silica gel chromatography eluting with 1-50% ethyl acetate/heptanes to provide the title compound.

### 2.42.6. (2S,3R,4S,5S,6S)-2-(3-(2-(2-((((9H-fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyl triacetate

To a solution of Example 2.42.5 (0.595 g) and bis(4-nitrophenyl) carbonate (0.492 g) in N,N-dimethylformamide (4 mL) was added N-ethyl-N-isopropylpropan-2-amine (0.212 mL). After 1.5 hours, the reaction was concentrated under high vacuum. The reaction was loaded directly onto silica gel and eluted using 1-50% ethyl acetate/heptanes to provide the title compound. MS (ESI) m/e 922.9 (M+Na)⁺.

### 2.42.7. 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 1.1.17 (92 mg) was dissolved in dimethylformamide (0.6 mL). Example 2.42.6 (129 mg) and N-ethyl-N-isopropylpropan-2-amine (0.18 mL) were added. The reaction was stirred at room temperature for one hour. The reaction was then concentrated and the residue was dissolved in tetrahydrofuran (0.6 mL) and methanol (0.6 mL). Aqueous LiOH (1.94*N*, 0.55 mL) was added and the mixture stirred at room temperature for one hour. Purification by reverse phase chromatography (C18 column), eluting with 10-90% acetonitrile in 0.1% TFA water, provided the title compound as a trifluoroacetic acid salt. MS (ESI) m/e 1187.4 (M-H)⁻.

### 2.42.8. 3-(1-((3-(2-((((2-(2-(2-((R)-2-amino-3-sulfopropanamido)ethoxy)ethoxy)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 2.26.8 for Example 2.31.5 in Example 2.34.1. MS (ESI) m/e 1338.4 (M-H)⁻.

### 2.42.9. 6-(8-(benzo[d]thiazol-2-ylcarbamoyl)-3,4-dihydroisoquinolin-2(1H)-yl)-3-(1-((3-(2-((((4-(((2S,3R,4S,5S,6S)-6-carboxy-3,4,5-trihydroxytetrahydro-2H-pyran-2-yl)oxy)-2-(2-(2-((R)-2-(3-(2,5-dioxo-2,5-dihydro-1H-pyrrol-1-yl)propanamido)-3-sulfopropanamido)ethoxy)ethoxy)benzyl)oxy)carbonyl)(methyl) amino)ethoxy)-5,7-dimethyladamantan-1-yl)methyl)-5-methyl-1H-pyrazol-4-yl)picolinic acid

The title compound was prepared by substituting Example 2.42.2 for Example 2.34.1 and 2,5-dioxopyrrolidin-1-yl 3-(2,5-dioxo-2,5-dihydro-1H-pyrrol-1-yl)propanoate for 2,5-dioxopyrrolidin-1-yl 6-(2,5-dioxo-2,5-dihydro-1H-pyrrol-1-yl)hexanoate in Example 2.34.2. ¹H NMR (400 MHz, dimethyl sulfoxide-d₆) δ ppm 8.06 (d, 1H), 8.02 (d, 1H), 7.80 (m, 2H), 7.61 (d,1H), 7.52 (d, 1H), 7.45 (m, 2H), 7.36 (m, 2H), 7.30 (s, 1H), 7.18 (d, 1H), 6.97 (s, 2H), 6.96 (m,2H), 6.66 (d, 1H), 6.58 (dd, 1H), 5.06 (br m, 1H), 4.96 (s, 4H), 4.31 (m, 1H), 4.09 (m, 2H), 3.88 (m, 3H), 3.80 (m, 2H), 3.71 (m, 2H), 3.59 (t, 2H), 3.44 (m, 6H), 3.28 (m, 4H), 3.19 (m, 2H), 3.01 (m, 2H), 2.82 (br m, 3H), 2.72 (m, 1H), 2.33 (m, 2H), 2.09 (s, 3H), 1.33 (br m, 2H), 1.28-0.90 (m, 10H), 0.84, 0.81 (both s, total 6H). MS (ESI-) m/e 1489.5 (M-1).

### 2.43. Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-{2-[2-({N-[6-(2,5-dioxo-2,5-dihydro-1H-pyrrol-1-yl)hexanoyl]-3-sulfo-L-alanyl}amino)ethoxy]ethoxy}phenyl beta-D-glucopyranosiduronic acid (Synthon NG)

The title compound was prepared by substituting Example 2.42.1 for Example 2.34.1 in Example 2.34.2. ¹H NMR (400 MHz, dimethyl sulfoxide-d₆) δ ppm 8.02 (d, 1H), 7.87 (d, 1H), 7.80 (m, 2H), 7.61 (d, 1H), 7.52 (d, 1H), 7.45 (m, 2H), 7.36 (m, 2H), 7.30 (s, 1H), 7.18 (d, 1H), 6.97 (s, 2H), 6.96 (m,2H), 6.66 (d, 1H), 6.58 (dd, 1H), 5.06 (br m, 1H), 4.96 (s, 4H), 4.31 (m, 1H), 4.09 (m, 2H), 3.88 (m, 3H), 3.80 (m, 2H), 3.71 (m, 2H), 3.59 (t, 2H), 3.44 (m, 6H), 3.28 (m, 4H), 3.19 (m, 2H), 3.01 (m, 2H), 2.82 (br m, 3H), 2.72 (m, 1H), 2.09 (s, 3H), 2.05 (t, 2H), 1.46 (br m, 4H), 1.33 (br m, 2H), 1.28-0.90 (m, 12H), 0.84, 0.81 (both s, total 6H). MS (ESI-) m/e 1531.5 (M-1).

### 2.44. Synthesis of 6-[8-(1,3-benzothiazol-2-ylcarbamoyl)-3,4-dihydroisoquinolin-2(1H)-yl]-3-{1-[(3-{[22-(2,5-dioxo-2,5-dihydro-1H-pyrrol-1-yl)-3-methyl-4,20-dioxo-7,10,13,16-tetraoxa-3,19-diazadocos-1-yl]oxy}-5,7-dimethyltricyclo[3.3.1.1^{3,7}]dec-1-yl)methyl]-5-methyl-1H-pyrazol-4-yl}pyridine-2-carboxylic acid (Synthon AS)

To a solution of Example 1.1.17 (56.9 mg) and N,N-diisopropylethylamine (0.065 mL) in N,N-dimethylformamide (1.0 mL) was added 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate (50 mg). The reaction was stirred overnight, and the solution was purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (400 MHz dimethyl sulfoxide-*d*₆) δ ppm 12.85 (s, 1H), 8.08-7.95 (m, 1H), 7.79 (d, 1H), 7.62 (d, 1H), 7.55-7.40 (m, 3H), 7.40-7.32 (m, 2H), 7.28 (s, 1H), 7.01-6.89 (m, 3H), 4.95 (s, 2H), 3.89 (s, 2H), 3.81 (s, 2H), 3.55-3.25 (m, 23H), 3.14 (d, 2H), 2.97 (t, 4H), 2.76 (d, 2H), 2.57 (s, 1H), 2.31 (d, 1H), 2.09 (s, 3H), 1.35 (s, 2H), 1.30-0.93 (m, 12H), 0.85 (d, 6H). MS (ESI) m/e 1180.3 (M+Na)⁺.

### 2.45. Synthesis of 6-[8-(1,3-benzothiazol-2-ylcarbamoyl)-3,4-dihydroisoquinolin-2(1H)-yl]-3-{1-[(3-{[28-(2,5-dioxo-2,5-dihydro-1H-pyrrol-1-yl)-9-methyl-10,26-dioxo-3,6,13,16,19,22-hexaoxa-9,25-diazaoctacos-1-yl]oxy}-5,7-dimethyltricyclo[3.3.1.1^{3,7}]dec-1-yl)methyl]-5-methyl-1H-pyrazol-4-yl}pyridine-2-carboxylic acid (Synthon AT)

To a solution of Example 1.2.11 (50 mg) and N,N-diisopropylethylamine (0.051 mL) in N,N-dimethylformamide (1.0 mL) was added 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate (39 mg). The reaction was stirred overnight and purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (400 MHz dimethyl sulfoxide-*d*₆) δ ppm 12.85 (s, 1H), 8.04 (d, 1H), 7.99 (t, 1H), 7.79 (d, 1H), 7.60 (d, 1H), 7.53-7.41 (m, 3H), 7.40-7.32 (m, 2H), 7.28 (s, 1H), 6.99 (s, 2H), 6.98-6.92 (m, 1H), 4.95 (bs, 2H), 3.92-3.85 (m, 1H), 3.81 (s, 2H), 3.63-3.55 (m, 4H), 3.55-3.31 (m, 28H), 3.18-3.10 (m, 2H), 3.05-2.98 (m, 2H), 2.97 (s, 2H), 2.80 (s, 2H), 2.59-2.50 (m, 1H), 2.32 (t, 2H), 2.10 (s, 3H), 1.39-1.34 (m, 2H), 1.31-1.18 (m, 4H), 1.20-0.92 (m, 6H), 0.84 (s, 6H). MS (ESI) m/e 1268.4 (M+Na)⁺.

### 2.46. Synthesis of 6-[8-(1,3-benzothiazol-2-ylcarbamoyl)-3,4-dihydroisoquinolin-2(1H)-yl]-3-{1-[(3-{2-[2-(2-{[6-(2,5-dioxo-2,5-dihydro-1H-pyrrol-1-yl)hexanoyl](methyl)amino}ethoxy)ethoxy]ethoxy}-5,7-dimethyltricyclo[3.3.1.1^{3,7}]dec-1-yl)methyl]-5-methyl-1H-pyrazol-4-yl}pyridine-2-carboxylic acid (Synthon AU)

To a solution of Example 1.2.11 (50 mg) and N,N-diisopropylethylamine (0.051 mL) in N,N-dimethylformamide (1.0 mL) was added 2,5-dioxopyrrolidin-1-yl 6-(2,5-dioxo-2,5-dihydro-1H-pyrrol-1-yl)hexanoate (18 mg). The reaction was stirred overnight and purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.92-12.82 (m, 1H), 8.03 (d, 1H), 7.79 (d, 1H), 7.62 (d, 1H), 7.53-7.41 (m, 3H), 7.40-7.32 (m, 2H), 7.28 (s, 1H), 7.01-6.97 (m, 2H), 6.98-6.92 (m, 1H), 4.95 (bs, 2H), 4.04-3.84 (m, 3H), 3.86-3.75 (m, 3H), 3.49-3.32 (m, 10H), 3.01 (s, 2H), 2.95 (s, 2H), 2.79 (s, 2H), 2.31-2.19 (m, 2H), 2.10 (s, 3H), 1.52-1.40 (m, 4H), 1.36 (s, 2H), 1.31-0.94 (m, 14H), 0.84 (s, 6H). MS (ESI) m/e 1041.3 (M+H)⁺.

### 2.47. Synthesis of 6-[8-(1,3-benzothiazol-2-ylcarbamoyl)-3,4-dihydroisoquinolin-2(1H)-yl]-3-(1-{[3-(2-{[4-(2,5-dioxo-2,5-dihydro-1H-pyrrol-1-yl)-2-sulfobutanoyl](methyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon BK)

### 2.47.1. 4-(2,5-dioxo-2,5-dihydro-1H-pyrrol-1-yl)-1-((2,5-dioxopyrrolidin-1-yl)oxy)-1-oxobutane-2-sulfonate

In a 100 mL flask sparged with nitrogen, 1-carboxy-3-(2,5-dioxo-2,5-dihydro-1H-pyrrol-1-yl)propane-1-sulfonate was dissolved in dimethylacetamide (20 mL). To this solution N-hydroxysuccinimide (440 mg,) and 1-(3-dimethylaminopropyl)-3-ethylcarbodiimide hydrochloride (1000 mg) were added, and the reaction was stirred at room temperature under a nitrogen atmosphere for 16 hours. The solvent was concentrated under reduced pressure, and the residue was purified by silica gel chromatography running a gradient of 1-2% methanol in dichloromethane with 0.1 % acetic acid v/v included in the solvents to yield the title compound as a mixture of ∼ 80% activated ester and 20 % acid, which was used in the next step without further purification. MS (ESI) m/e 360.1 (M+H)⁺.

### 2.47.2. 6-[8-(1,3-benzothiazol-2-ylcarbamoyl)-3,4-dihydroisoquinolin-2(1H)-yl]-3-(1-{[3-(2-{[4-(2,5-dioxo-2,5-dihydro-1H-pyrrol-1-yl)-2-sulfobutanoyl] (methyl)amino}ethoxy)-5,7-dimethyltricyclo[3.3.1.1^{3,7}]dec-1-yl]methyl}-5-methyl-1H-pyrazol-4-yl)pyridine-2-carboxylic acid

To a solution of Example 1.1.17 (5 mg) and Example 2.47.1 (20.55 mg) in N,N-dimethylformamide (0.25 mL) was added N,N-diisopropylethylamine (0.002 mL) and the reaction was stirred at room temperature for 16 hours. The crude reaction mixture was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. ¹H NMR (400 MHz, dimethyl sulfoxide-*d*₆) δ ppm 8.01-7.95 (m, 1H), 7.76 (d, 1H), 7.60 (dd, 1H), 7.49-7.37 (m, 3H), 7.37-7.29 (m, 2H), 7.28-7.22 (m, 1H), 6.92 (d, 1H), 6.85 (s, 1H), 4.96 (bs, 2H), 3.89 (t, 2H), 3.80 (s, 2H), 3.35 (bs, 5H), 3.08-2.96 (m, 3H), 2.97-2.74 (m, 2H), 2.21 (bs, 1H), 2.08 (s, 4H), 1.42-1.38 (m, 2H), 1.31-1.23 (m, 4H), 1.23-1.01 (m, 6H), 0.97 (d, 1H), 0.89-0.79 (m, 6H). MS (ESI) m/e 1005.2 (M+H)⁺.

### 2.48. Synthesis of 6-[8-(1,3-benzothiazol-2-ylcarbamoyl)-3,4-dihydroisoquinolin-2(1H)-yl]-3-{1-[(3-{[34-(2,5-dioxo-2,5-dihydro-1H-pyrrol-1-yl)-3-methyl-4,32-dioxo-7,10,13,16,19,22,25,28-octaoxa-3,31-diazatetratriacont-1-yl]oxy}-5,7-dimethyltricyclo[3.3.1.1^{3,7}]dec-1-yl)methyl]-5-methyl-1H-pyrazol-4-yl}pyridine-2-carboxylic acid (Synthon BQ)

The title compound was prepared as described in Example 2.44, replacing 2,5-dioxopyrrolidin-1-yl 1-(2,5 -dioxo-2,5 -dihydro-1H-pyrrol-1-yl)-3 -oxo-7,10,13,16-tetraoxa-4-azanonadecan-19-oate with 2,5-dioxopyrrolidin-1-yl 1-(2,5-dioxo-2,5-dihydro-1H-pyrrol-1-yl)-3-oxo-7,10,13,16,19,22,25,28-octaoxa-4-azahentriacontan-31-oate (MAL-dPEG8-NHS-Ester). MS (ESI) m/e 1334.3 (M+H)⁺.

### 2.49. Synthesis of 6-[8-(1,3-benzothiazol-2-ylcarbamoyl)-3,4-dihydroisoquinolin-2(1H)-yl]-3-{1-[(3-{[28-(2,5-dioxo-2,5-dihydro-1H-pyrrol-1-yl)-3-methyl-4,26-dioxo-7,10,13,16,19,22-hexaoxa-3,25-diazaoctacos-1-yl]oxy}-5,7-dimethyltricyclo[3.3.1.1^{3,7}]dec-1-yl)methyl]-5-methyl-1H-pyrazol-4-yl}pyridine-2-carboxylic acid (Synthon BR)

The title compound was prepared as described in Example 2.44, replacing 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate with 2,5-dioxopyrrolidin-1-yl 1-(2,5-dioxo-2,5-dihydro-1H-pyrrol-1-yl)-3-oxo-7,10,13,16,19,22-hexaoxa-4-azapentacosan-25-oate (MAL-dPEG6-NHS-Ester). MS (ESI) m/e 1246.3 (M+H)⁺.

### 2.50 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.1^{3,7}]dec-1-yl}oxy)ethyl](methyl)carbamoyl}oxy)methyl]-5-{2-[2-({N-[6-(2,5-dioxo-2,5-dihydro-1H-pyrrol-1-yl)hexanoyl]-3-sulfo-L-alanyl}amino)ethoxy]ethoxy}phenyl beta-D-glucopyranosiduronic acid

### 2.50.1 3-(1-((3-(2-((((4-(2-(2-aminoethoxy)ethoxy)-2-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 1.1.17 for Example 1.3.7 in Example 2.30.1. MS (ESI) m/e 1189.5 (M+H)⁺.

### 2.50.2 3-(1-((3-(2-((((4-(2-(2-((R)-2-amino-3-sulfopropanamido)ethoxy)ethoxy)-2-(((2S,3R,4S,5S,6S)-6-carboxy-3,4,5-trihydroxytetrahydro-2H--pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 2.50.1 for Example 2.31.5 in Example 2.34.1. MS (ESI) m/e 1339.5 (M+H)⁺.

### 2.50.3 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-5-{2-[2-({N-[6-(2,5-dioxo-2,5-dihydro-1H-pyrrol-1-yl)hexanoyl]-3-sulfo-L-alanyl}amino)ethoxy]ethoxy}phenyl beta-D-glucopyranosiduronic acid

The title compound was prepared by substituting Example 2.50.2 for Example 2.34.1 in Example 2.34.2. ¹H NMR (500 MHz, dimethyl sulfoxide-*d*₆) δ ppm 12.83 (s, 2H); 8.01 (dd, 1H), 7.86 (d, 1H), 7.80 - 7.71 (m, 2H), 7.60 (dd, 1H), 7.52 - 7.26 (m, 7H), 7.16 (d, 1H), 6.94 (d, 3H), 6.69 (d, 1H), 6.61 - 6.53 (m, 1H), 5.09 - 4.91 (m, 5H), 3.46 - 3.08 (m, 14H), 2.99 (t, 2H), 2.88 - 2.63 (m, 5H), 2.13 - 1.94 (m, 5H), 1.52 - 0.73 (m, 27H). MS (ESI) m/e 1531.4 (M-H)⁻.

### 2.51 Synthesis of N²-[6-(2,5-dioxo-2,5-dihydro-1H-pyrrol-1-yl)hexanoyl]-N⁶-(37-oxo-2,5,8,11,14,17,20,23,26,29,32,35-dodecaoxaheptatriacontan-37-yl)-L-lysyl-L-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]phenyl}-L-alaninamide

### 2.51.1 (S)-6-((((9H-fluoren-9-yl)methoxy)carbonyl)amino)-2-((tert-butoxycarbonyl)amino)hexanoic acid

To a cold (ice bath) solution of (S)-6-amino-2-((tert-butoxycarbonyl)amino)hexanoic acid (8.5 g) in a mixture of 5% aqueous NaHCO₃ solution (300 mL) and 1,4-dioxane (40 mL) was added dropwise a solution of (9H-fluoren-9-yl)methyl pyrrolidin-1-yl carbonate (11.7 g) in 1,4-dioxane (40 mL). The reaction mixture was allowed to warm to room temperature and was stirred for 24 hours. Three additional vials were set up as described above. After the reactions were complete, the four reaction mixtures were combined, and the organic solvent was removed under vacuum. The aqueous layer was acidified to pH 3 with aqueous hydrochloric acid solution (1N) and then extracted with ethyl acetate (3 × 500 mL). The combined organic layers were washed with brine, dried over magnesium sulfate, filtered, and concentrated under vacuum to give a crude compound, which was recrystallized from methyl tert-butyl ether to afford the title compound. ¹H NMR (400MHz, chloroform-*d*) δ 11.05 (br. s., 1H), 7.76 (d, 2H), 7.59 (d, 2H), 7.45 - 7.27 (m, 4H), 6.52 - 6.17 (m, 1H), 5.16 - 4.87 (m, 1H), 4.54 - 4.17 (m, 4H), 3.26 - 2.98 (m, 2H), 1.76 - 1.64 (m, 1H), 1.62 - 1.31 (m, 14H).

### 2.51.2 tert-butyl 17-hydroxy-3,6,9,12,15-pentaoxaheptadecan-1-oate

To a solution of 3,6,9,12-tetraoxatetradecane-1,14-diol (40 g) in toluene (800 mL) was added portion-wise potassium tert-butoxide (20.7 g). The mixture was stirred at room temperature for 30 minutes. Tert-butyl 2-bromoacetate (36 g) was added dropwise to the mixture. The reaction was stirred at room temperature for 16 hours. Two additional vials were set up as described above. After the reactions were complete, the three reaction mixtures were combined. Water (500 mL) was added to the combined mixture, and the volume was concentrated to 1 liter. The mixture was extracted with dichloromethane and was washed with aqueous IN potassium tert-butoxide solution (1 L). The organic layer was dried over anhydrous sodium sulfate, filtered and concentrated under reduced pressure. The residue was purified by silica gel column chromatography, eluting with dichloromethane: methanol 50:1, to obtain the title compound. ¹H NMR (400MHz, chloroform-*d*) δ 4.01 (s, 2H), 3.75 - 3.58 (m, 21H), 1.46 (s, 9H).

### 2.51.3 tert-butyl 17-(tosyloxy)-3,6,9,12,15-pentaoxaheptadecan-1-oate

To a solution of Example 2.51.2 (30 g) in dichloromethane (500 mL) was added dropwise a solution of 4-methylbenzene-1-sulfonyl chloride (19.5 g) and triethylamine (10.3 g) in dichloromethane (500 mL) at 0 °C under a nitrogen atmosphere. The mixture was stirred at room temperature for 18 hours and was poured into water (100 mL). The solution was extracted with dichloromethane (3 × 150 mL), and the organic layer was washed with hydrochloric acid (6N, 15 mL) then NaHCO₃ (5% aqueous solution, 15 mL) followed by water (20 mL). The organic layer was dried over anhydrous sodium sulfate, filtered and concentrated to obtain a residue, which was purified by silica gel column chromatography, eluting with petroleum ether:ethyl acetate 10:1 to dichloromethane: methanol 5:1, to obtain the title compound. ¹H NMR (400 MHz, chloroform-*d*) δ 7.79 (d, 2H), 7.34 (d, 2H), 4.18 - 4.13 (m, 2H), 4.01 (s, 2H), 3.72 - 3.56 (m, 18H), 2.44 (s, 3H), 1.47 (s, 9H).

### 2.51.4 2,5,8,11,14,17,20,23,26,29,32,35-dodecaoxaheptatriacontan-37-oic acid

To a solution of Example 2.51.3 (16 g) in tetrahydrofuran (300 mL) was added sodium hydride (1.6 g) at 0 °C. The mixture was stirred at room temperature for 4 hours. A solution of 2,5,8,11,14,17-hexaoxanonadecan-19-ol (32.8 g) in tetrahydrofuran (300 mL) was added dropwise at room temperature to the reaction mixture. The resulted reaction mixture was stirred at room temperature for 16 hours, and water (20 mL) was added. The mixture was stirred at room temperature for another 3 hours to complete the tert-butyl ester hydrolysis. The final reaction mixture was concentrated under reduced pressure to remove the organic solvent. The aqueous residue was extracted with dichloromethane (2 × 150 mL). The aqueous layer was acidified to pH 3 and then extracted with ethyl acetate (2 × 150 mL). Finally, the aqueous layer was concentrated to obtain crude product, which was purified by silica gel column chromatography, eluting with a gradient of petroleum ether:ethyl acetate 1:1 to dichloromethane:methanol 5:1, to obtain the title compound. ¹H NMR (400MHz, chloroform-d) δ 4.19 (s, 2H), 3.80 - 3.75 (m, 2H), 3.73 - 3.62 (m, 40H), 3.57 (dd, 2H), 3.40 (s, 3H)

### 2.51.5 (43S,46S)-43-((tert-butoxycarbonyl)amino)-46-methyl-37,44-dioxo-2,5,8,11,14,17,20,23,26,29,32,35-dodecaoxa-38,45-diazaheptatetracontan-47-oic acid

Example 2.51.5 was synthesized using standard Fmoc solid phase peptide synthesis procedures and a 2-chlorotrytil resin. Specifically, 2-chlorotrytil resin (12 g), (S)-2-((((9H-fluoren-9-yl)methoxy)carbonyl)amino)propanoic acid (10 g) and N,N-diisopropylethylamine (44.9 mL) in anhydrous, sieve-dried dichloromethane (100 mL) was shaken at 14 °C for 24 hours. The mixture was filtered, and the cake was washed with dichloromethane (3 × 500 mL), N,N-dimethylformamide (2 × 250 mL) and methanol (2 × 250 mL) (5 minutes each step). To the above resin was added 20% piperidine/N,N-dimethylformamide (100 mL) to remove the Fmoc group. The mixture was bubbled with nitrogen gas for 15 minutes and filtered. The resin was washed with 20% piperidine/N,N-dimethylformamide (100 mL) another five times (5 minutes each washing step), and washed with N,N-dimethylformamide (5 × 100 mL) to give the deprotected, L-Ala loaded resin.

To a solution of Example 2.51.1 (9.0 g) in N,N-dimethylformamide (50 mL) was added hydroxybenzotriazole (3.5 g), 2-(6-chloro-1H-benzotriazole-1-yl)-1,1,3,3-tetramethylaminium hexafluorophosphate (9.3 g) and N,N-diisopropylethylamine (8.4 mL). The mixture was stirred at 20 °C for 30 minutes. The above mixture was added to the L-Ala loaded resin and mixed by bubbling with nitrogen gas at room temperature for 90 minutes. The mixture was filtered, and the resin was washed with N,N-dimethylformamide (5 minutes each step). To the above resin was added approximately 20% piperidine/ N,N-dimethylformamide (100 mL) to remove the Fmoc group. The mixture was bubbled with nitrogen gas for 15 minutes and filtered. The resin was washed with 20% piperidine/N,N-dimethylformamide (100 mL x 5) and N,N-dimethylformamide (100 mL x 5) (5 minutes each washing step).

To a solution of Example 2.51.4 (11.0 g) in N,N-dimethylformamide (50 mL) was added hydroxybenzotriazole (3.5 g), 2-(6-chloro-1H-benzotriazole-1-yl)-1,1,3,3-tetramethylaminium hexafluorophosphate (9.3 g) and N,N-diisopropylethylamine (8.4 mL), and the mixture was added to the resin and mixed by bubbling with nitrogen gas at room temperature for 3 hours. The mixture was filtered and the residue was washed with N,N-dimethylformamide (5 × 100 mL), dichloromethane (8 × 100 mL) (5 minutes each step).

To the final resin was added 1% trifluoroacetic acid/dichloromethane (100 mL) and mixed by bubbling with nitrogen gas for 5 minutes. The mixture was filtered, and the filtrate was collected. The cleavage operation was repeated four times. The combined filtrate was brought to pH 7 with NaHCO₃ and washed with water. The organic layer was dried over anhydrous sodium sulfate, filtered and concentrated to obtain the title compound. ¹H NMR (400MHz, methanol-*d₄*) δ 4.44 - 4.33 (m, 1H), 4.08 - 4.00 (m, 1H), 3.98 (s, 2H), 3.77 - 3.57 (m, 42H), 3.57 - 3.51 (m, 2H), 3.36 (s, 3H), 3.25 (t, 2H), 1.77 (br. s., 1H), 1.70 - 1.51 (m, 4H), 1.44 (s, 9H), 1.42 - 1.39 (m, 3H).

### 2.51.6 3-(1-((3-(2-((((4-((S)-2-((S)-2-amino-3-methylbutanamido)propanamido)benzyl)oxy)carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of the trifluoroacetic acid salt of Example 1.3.7 (0.102 g), Example 2.21.4 (0.089 g) and N,N-diisopropylethylamine (0.104 mL) were stirred in N,N-dimethylformamide (1 mL) at room temperature for 16 hours. Diethylamine (0.062 mL) was added, and the reaction was stirred for 2 hours at room temperature. The reaction was diluted with water (1 mL), quenched with trifluoroacetic acid (0.050 mL) and purified by reverse-phase HPLC using a Gilson system and a C18 column, eluting with 5-85% acetonitrile in water containing 0.1% v/v trifluoroacetic acid. The product fractions were lyophilized to give the title compound. MS (LC-MS) m/e 1066.5 (M+H)⁺.

### 2.51.7 6-(8-(benzo[d]thiazol-2-ylcarbamoyl)-3,4-dihydroisoquinolin-2(1H)-yl)-3-(1-((3-(2-((((4-((43S,46S,49S,52S)-43-((tert-butoxycarbonyl)amino)-49-isopropyl-46,52-dimethyl-37,44,47,50-tetraoxo-2,5,8,11,14,17,20,23,26,29,32,35-dodecaoxa-38,45,48,51-tetraazatripentacontanamido)benzyl)oxy)carbonyl)amino)ethoxy)-5,7-dimethyladamantan-1-yl)methyl)-5-methyl-1H-pyrazol-4-yl)picolinic acid

Example 2.51.5 (16.68 mg), was mixed with 1-[bis(dimethylamino)methylene]-1H-1,2,3-triazolo[4,5-b]pyridinium 3-oxid hexafluorophosphate (7.25 mg) and N,N-diisopropylethylamine (0.015 mL) in N-methylpyrrolidone (1 mL) for 10 minutes and was added to a solution of Example 2.51.6 (25 mg) and N,N-diisopropylethylamine (0.015 mL) in N-methylpyrrolidinone (1.5 mL). The reaction mixture was stirred at room temperature for two hours. The reaction mixture was purified by reverse-phase HPLC using a Gilson system and a C18 column, eluting with 5-85% acetonitrile in water containing 0.1% v/v trifluoroacetic acid. The product fractions were lyophilized to give the title compound. MS (ESI) m/e 961.33 (2M+H)²⁺.

### 2.51.8 3-(1-((3-(2-((((4-((43S,46S,49S,52S)-43-amino-49-isopropyl-46,52-dimethyl-37,44,47,50-tetraoxo-2,5,8,11,14,17,20,23,26,29,32,35-dodecaoxa-38,45,48,51-tetraazatripentacontanamido)benzyl)oxy)carbonyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.51.7 (25 mg) was treated with 1 mL trifluoroacetic acid for 5 minutes. The solvent was removed by a gentle flow of nitrogen. The residue was lyophilized from 1:1 acetonitrile: water to give the title compound, which was used in the next step without further purification. MS (LC-MS) m/e 1822.0 (M+H)⁺.

### 2.51.9 N²-[6-(2,5-dioxo-2,5-dihydro-1H-pyrrol-1-yl)hexanoyl]-N⁶-(37-oxo-2,5,8,11,14,17,20,23,26,29,32,35-dodecaoxaheptatriacontan-37-yl)-L-lysyl-L-alanyl-L-valyl-N-{4-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]phenyl}-L-alaninamide

To a solution of Example 2.51.8, (23 mg), N-succinimidyl 6-maleimidohexanoate (4.40 mg) and hydroxybenzotriazole (0.321 mg) in N-methylpyrrolidone (1.5 mL) was added N,N-diisopropylethylamine (8.28 µL). The reaction mixture was stirred for 16 hours at room temperature. The reaction mixture was purified by reverse-phase HPLC using a Gilson system and a C18 column, eluting with 5-85% acetonitrile in water containing 0.1% v/v trifluoroacetic acid. The product fractions were lyophilized to give the title compound. ¹H NMR (400 MHz, dimethyl sulfoxide-*d*₆) δ ppm 7.76 (dq, 3H), 7.64 - 7.51 (m, 5H), 7.45 (dd, 4H), 7.35 (td, Hz, 3H), 4.97 (d, 5H), 3.95 - 3.79 (m, 8H), 3.57 (d, 46H), 3.50 - 3.30 (m, 14H), 1.58 - 0.82 (m, 59H). MS (LC-MS) m/e 1007.8 (2M+H)²⁺.

### 2.52 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-5-[2-(2-{[3-(2,5-dioxo-2,5-dihydro-1H-pyrrol-1-yl)propanoyl]amino}ethoxy)ethoxy]phenyl beta-D-glucopyranosiduronic acid

The title compound was prepared by substituting Example 2.50.1 for Example 2.30.1 in Example 2.30.2. ¹H NMR (500 MHz, dimethyl sulfoxide-*d*₆) δ ppm 12.87 (s, 2H); 8.06 - 7.98 (m, 1H), 7.78 (d, 1H), 7.61 (dd, 1H), 7.52 - 7.41 (m, 2H), 7.39 - 7.26 (m, 2H), 7.18 (d, 1H), 7.01 - 6.91 (m, 2H), 6.68 (d, 1H), 6.59 (d, 1H), 5.08 - 4.98 (m, 2H), 4.95 (s, 1H), 3.59 (t, 1H), 3.46 - 3.36 (m, 3H), 3.34 - 3.22 (m, 2H), 3.16 (q, 1H), 3.01 (t, 1H), 2.85 (d, 2H), 2.32 (t, 1H), 2.09 (s, 2H), 1.44 - 0.71 (m, 10H). MS (ESI) m/e 1338.4 (M-H)⁻.

### 2.53 Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-3-[3-({N-[3-(2,5-dioxo-2,5-dihydro-H-pyrrol-1-yl)propanoyl]-3-sulfo-L-alanyl}amino)propoxy]phenyl beta-D-glucopyranosiduronic acid

The title compound was prepared as described in Example 2.34.2, substituting 2,5-dioxopyrrolidin-1-yl 3-(2,5-dioxo-2,5-dihydro-1H-pyrrol-1-yl)propanoate for 2,5-dioxopyrrolidin-1-yl 6-(2,5-dioxo-2,5-dihydro-1H-pyrrol-1-yl)hexanoate and N-methyl-2-pyrrolidone for N,N-dimethylformamide. MS (ESI) m/e 1458.0 (M-H)⁻.

### Example 3. Synthesis of Examplary Bcl-xL Inhibitory ADCs

Exemplary ADCs were synthesized using one of four exemplary methods, described below. Table 1 correlates which method was used to synthesize each exemplary ADC.

**Method A.** A solution of TCEP (10 mM, 0.017 mL) was added to a solution of antibody (10 mg/mL, 1 mL) preheated to 37 °C. The reaction mixture was kept at 37 °C for 1 hour. The solution of reduced antibody was added to a solution of linker-warhead payload (3.3 mM, 0.160 mL in DMSO) and gently mixed for 30 minutes. The reaction solution was loaded onto a desalting column (PD10, washed with DPBS 3x before use), followed by DPBS (1.6 mL) and eluted with additional DPBS (3 mL). The purified ADC solution was filtered through a 0.2 micron, low protein-binding 13 mm syringe-filter and stored at 4 °C.

**Method B.** A solution of TCEP (10 mM, 0.017 mL) was added to the solution of antibody (10 mg/mL, 1 mL) preheated to 37 °C. The reaction mixture was kept at 37 °C for 1 hour. The solution of reduced antibody was adjusted to pH=8 by adding boric buffer (0.05 mL, 0.5 M, pH8), added to a solution of linker-warhead payload (3.3 mM, 0.160 mL in DMSO) and gently mixed for 4 hours. The reaction solution was loaded onto a desalting column (PD10, washed with DPBS 3x before use), followed by DPBS (1.6 mL) and eluted with additional DPBS (3 mL). The purified ADC solution was filtered through a 0.2 micron, low protein-binding 13 mm syringe-filter and stored at 4 °C.

**Method C.** Conjugations were performed using a PerkinElmer Janus (part AJL8M01) robotic liquid handling system equipped with an 1235/96 tip ModuLar Dispense Technology (MDT), disposable head (part 70243540) containing a gripper arm (part 7400358), and an 8-tip Varispan pipetting arm (part 7002357) on an expanded deck. The PerkinElmer Janus system was controlled using the WinPREP version 4.8.3.315 Software.

A Pall Filter plate 5052 was prewet with 100 µL 1x DPBS using the MDT. Vacuum was applied to the filter plate for 10 seconds and was followed by a 5 second vent to remove DPBS from filter plate. A 50% slurry of Protein A resin (GE MabSelect Sure) in DPBS was poured into an 8 well reservoir equipped with a magnetic ball, and the resin was mixed by passing a traveling magnet underneath the reservoir plate. The 8 tip Varispan arm, equipped with 1mL conductive tips, was used to aspirate the resin (250 µL) and transfer to a 96-well filter plate. A vacuum was applied for 2 cycles to remove most of the buffer. Using the MDT, 150 µL of 1xPBS was aspirated and dispensed to the 96-well filter plate holding the resin. A vacuum was applied, removing the buffer from the resin. The rinse/vacuum cycle was repeated 3 times. A 2 mL, 96-well collection plate was mounted on the Janus deck, and the MDT transferred 450 µL of 5x DPBS to the collection plate for later use. Reduced antibody (2 mg) as a solution in (200 µL) DPBS was prepared as described above for Conditions A and preloaded into a 96 well plate. The solutions of reduced antibody were transferred to the filter plate wells containing the resin, and the mixture was mixed with the MDT by repeated aspiration/dispensation of a 100 µL volume within the well for 45 seconds per cycle. The aspiration/dispensation cycle was repeated for a total of 5 times over the course of 5 minutes. A vacuum was applied to the filter plate for 2 cycles, thereby removing excess antibody. The MDT tips were rinsed with water for 5 cycles (200 µL, 1 mL total volume). The MDT aspirated and dispensed 150 µL of DPBS to the filter plate wells containing resin -bound antibody, and a vacuum was applied for two cycles. The wash and vacuum sequence was repeated two more times. After the last vacuum cycle, 100 µL of 1x DPBS was dispensed to the wells containing the resin-bound antibody. The MDT then collected 30 µL each of 3.3 mM dimethyl sulfoxide solutions of synthons plated in a 96-well format and dispensed it to the filter plate containing resin-bound antibody in DPBS. The wells containing the conjugation mixture were mixed with the MDT by repeated aspiration/dispensation of a 100 µL volume within the well for 45 seconds per cycle. The aspiration/dispensation sequence was repeated for a total of 5 times over the course of 5 minutes. A vacuum was applied for 2 cycles to remove excess synthon to waste. The MDT tips were rinsed with water for 5 cycles (200 µL, 1 mL total volume). The MDT aspirated and dispensed DPBS (150 µL) to the conjugation mixture, and a vacuum was applied for two cycles. The wash and vacuum sequence was repeated two more times. The MDT gripper then moved the filter plate and collar to a holding station. The MDT placed the 2 mL collection plate containing 450 µL of 10x DPBS inside the vacuum manifold. The MDT reassembled the vacuum manifold by placement of the filter plate and collar. The MDT tips were rinsed with water for 5 cycles (200 µL, 1 mL total volume). The MDT aspirated and dispensed 100 µL of IgG Elution Buffer 3.75 (Pierce) to the conjugation mixture. After one minute, a vacuum was applied for 2 cycles, and the eluent was captured in the receiving plate containing 450 µL of 5x DPBS. The aspiration/dispensation sequence was repeated 3 additional times to deliver ADCsamples with concentrations in the range of 1.5-2.5 mg/mL at pH 7.4 in DPBS.

**Method D.** Conjugations were performed using a PerkinElmer Janus (part AJL8M01) robotic liquid handling system equipped with an 1235/96 tip ModuLar Dispense Technology (MDT), disposable head (part 70243540) containing a gripper arm (part 7400358), and an 8-tip Varispan pipetting arm (part 7002357) on an expanded deck. The PerkinElmer Janus system was controlled using the WinPREP version 4.8.3.315 Software.

A Pall Filter plate 5052 was prewet with 100 µL 1x DPBS using the MDT. Vacuum was applied to the filter plate for 10 seconds and was followed by a 5 second vent to remove DPBS from filter plate. A 50% slurry of Protein A resin (GE MabSelect Sure) in DPBS was poured into an 8-well reservoir equipped with a magnetic ball, and the resin was mixed by passing a traveling magnet underneath the reservoir plate. The 8 tip Varispan arm, equipped with 1mL conductive tips, was used to aspirate the resin (250 µL) and transfer to a 96-well filter plate. A vacuum was applied to the filter plate for 2 cycles to remove most of the buffer. The MDT aspirated and dispensed 150 µL of DPBS to the filter plate wells containing the resin. The wash and vacuum sequence was repeated two more times. A 2 mL, 96-well collection plate was mounted on the Janus deck, and the MDT transferred 450 µL of 5x DPBS to the collection plate for later use. Reduced antibody (2 mg) as a solution in (200 µL) DPBS was prepared as described above for Conditions A and dispensed into the 96-well plate. The MDT then collected 30 µL each of 3.3 mM dimethyl sulfoxide solutions of synthons plated in a 96-well format and dispensed it to the plate loaded with reduced antibody in DPBS. The mixture was mixed with the MDT by twice repeated aspiration/dispensation of a 100 µL volume within the well. After five minutes, the conjugation reaction mixture (230 µL) was transferred to the 96-well filter plate containing the resin. The wells containing the conjugation mixture and resin were mixed with the MDT by repeated aspiration/dispensation of a 100 µL volume within the well for 45 seconds per cycle. The aspiration/dispensation sequence was repeated for a total of 5 times over the course of 5 minutes. A vacuum was applied for 2 cycles to remove excess synthon and protein to waste. The MDT tips were rinsed with water for 5 cycles (200 µL, 1 mL total volume). The MDT aspirated and dispensed DPBS (150 µL) to the conjugation mixture, and a vacuum was applied for two cycles. The wash and vacuum sequence was repeated two more times. The MDT gripper then moved the filter plate and collar to a holding station. The MDT placed the 2 mL collection plate containing 450 µL of 10x DPBS inside the vacuum manifold. The MDT reassembled the vacuum manifold by placement of the filter plate and collar. The MDT tips were rinsed with water for 5 cycles (200 µL, 1 mL total volume). The MDT aspirated and dispensed 100 µL of IgG Elution Buffer 3.75 (P) to the conjugation mixture. After one minute, a vacuum was applied for 2 cycles, and the eluent was captured in the receiving plate containing 450 µL of 5x DPBS. The aspiration/dispensation sequence was repeated 3 additional times to deliver ADCsamples with concentrations in the range of 1.5-2.5 mg/mL at pH 7.4 in DPBS.

**Method E.** A solution of TCEP (10 mM, 0.017 mL) was added to the solution of antibody (10 mg/mL, 1 mL) at room temperature. The reaction mixture was heated to 37 °C for 75 minutes. The solution of reduced antibody cooled to room temperature and was added to a solution of synthon (10 mM, 0.040 mL in DMSO) followed by addition of boric buffer (0.1 mL, 1M, pH 8). The reaction solution was let to stand for 3 days at room temperature, loaded onto a desalting column (PD10, washed with DPBS 3x5mL before use), followed by DPBS (1.6 mL) and eluted with additional DPBS (3 mL). The purified ADC solution was filtered through a 0.2 micron, low protein-binding 13 mm syringe-filter and stored at 4 C.

Table 1, below, indicates which exemplary ADCs were synthesized via which exemplary method. The monoclonal antibody to EpCAM referred to as EpCAM(ING-1) is described in Studnicka et al., 1994, Protein Engineering, 7:805-814 and Ammons et al., 2003, Neoplasia 5:146-154. The NCAM-1 antibody referred to as N901 was described in Roguska et al., 1994, Proc Natl Acad Sci USA 91:969-973. The EGFR antibody referred to as AB033 is described in WO 2009/134776 (*see* page 120).

| **Table 1** | | |
|---|---|---|
| **Synthetic Methods Used to Synthesize Exemplary ADCs** | | |
| **Appln Ex. No.** | **ADC** | **Method** |
| 3.1 | AB033-E | A |
| 3.2 | AB033-D | A |
| 3.3 | AB033-J | A |
| 3.4 | AB033-K | A |
| 3.5 | AB033-NG | D |
| 3.6 | AB033-M | A |
| 3.7 | AB033-V | A |
| 3.8 | AB033-DS | A |
| 3.10 | AB033-BG | A |
| 3.12 | AB033-BI | A |
| 3.17 | AB033-BO | A |
| 3.18 | AB033-BP | A |
| 3.21 | AB033-IQ | A |
| 3.22 | AB033-DB | A |
| 3.23 | AB033-DM | A |
| 3.24 | AB033-DL | A |
| 3.25 | AB033-DR | A |
| 3.26 | AB033-DZ | A |
| 3.27 | AB033-EA | A |
| 3.28 | AB033-EO | A |
| 3.29 | AB033-FB | A |
| 3.30 | AB033-KX | A |
| 3.31 | AB033-FF | A |
| 3.32 | AB033-FU | A |
| 3.33 | AB033-LB | A |
| 3.34 | AB033-FX | A |
| 3.35 | AB033-H | A |
| 3.36 | AB033-I | A |
| 3.37 | AB033-HB | A |
| 3.38 | AB033-KQ | D |
| 3.39 | AB033-KP | D |
| 3.40 | AB033-HA | B |
| 3.41 | AB033-NF | D |
| 3.42 | AB033-NG | D |
| 3.43 | EpCAM(ING-1)-EA | A |
| 3.44 | EpCAM(ING-1)-FX | A |
| 3.46 | EpCAM(ING-1)-DB | A |
| 3.55 | N901-D | A |
| 3.56 | N901-DB | A |
| 3.57 | NCAM1-H (N901-H) | A |
| 3.58 | AB033-AS | A |
| 3.59 | AB033-AT | A |
| 3.60 | AB033-AU | A |
| 3.61 | AB033-BK | A |
| 3.62 | AB033-BQ | A |
| 3.63 | AB033-BR | A |
| 3.64 | AB033-OI | A |
| 3.65 | AB033-NX | A |
| 3.66 | AB033-OJ | A |
| 3.67 | AB033-XY | E |

### Example 4. Exemplary Bcl-xL inhibitors Bind Bcl-xL

The ability of the exemplary Bcl-xL inhibitors of Examples 1.1, 1.2, 1.3, 1.4, 1.5 1.6, 1.7, and 1.8 (compounds W1.01-W1.08 respectively) to bind Bcl-xL was demonstrated using the Time Resolved-Fluorescence Resonance Energy Transfer (TR-FRET) Assay. Tb-anti-GST antibody was purchased from Invitrogen (Catalog No. PV4216).

### 4.1. Probe Synthesis

### 4.1.1. Reagents

All reagents were used as obtained from the vendor unless otherwise specified. Peptide synthesis reagents including diisopropylethylamine (DIEA), dichloromethane (DCM), N-methylpyrrolidone (NMP), 2-(1H-benzotriazole-1-yl)-1,1,3,3-tetramethyluronium hexafluorophosphate (HBTU), N-hydroxybenzotriazole (HOBt) and piperidine were obtained from Applied Biosystems, Inc. (ABI), Foster City, CA or American Bioanalytical, Natick, MA.

Preloaded 9-Fluorenylmethyloxycarbonyl (Fmoc) amino acid cartridges (Fmoc-Ala-OH, Fmoc-Cys(Trt)-OH, Fmoc-Asp(tBu)-OH, Fmoc-Glu(tBu)-OH, Fmoc-Phe-OH, Fmoc-Gly-OH, Fmoc-His(Trt)-OH, Fmoc-Ile-OH, Fmoc-Leu-OH, Fmoc-Lys(Boc)-OH, Fmoc-Met-OH, Fmoc-Asn(Trt)-OH, Fmoc-Pro-OH, Fmor-Gln(Trt)-OH, Fmoc-Arg(Pbf)-OH, Fmoc-Ser(tBu)-OH, Fmoc-Thr(tBu)-OH, Fmoc-Val-OH, Fmoc-Trp(Boc)-OH, Fmoc-Tyr(tBu)-OH) were obtained from ABI or Anaspec, San Jose, CA.

The peptide synthesis resin (Fmoc-Rink amide MBHA resin) and Fmoc-Lys(Mtt)-OH were obtained from Novabiochem, San Diego, CA.

Single-isomer 6-carboxyfluorescein succinimidyl ester (6-FAM-NHS) was obtained from Anaspec.

Trifluoroacetic acid (TFA) was obtained from Oakwood Products, West Columbia, SC.

Thioanisole, phenol, triisopropylsilane (TIS), 3,6-dioxa-1,8-octanedithiol (DODT) and isopropanol were obtained from Aldrich Chemical Co., Milwaukee, WI.

Matrix-assisted laser desorption ionization mass-spectra (MALDI-MS) were recorded on an Applied Biosystems Voyager DE-PRO MS).

Electrospray mass-spectra (ESI-MS) were recorded on Finnigan SSQ7000 (Finnigan Corp., San Jose, CA) in both positive and negative ion mode.

### 4.1.2. General Procedure For Solid-Phase Peptide Synthesis (SPPS)

Peptides were synthesized with, at most, 250 µmol preloaded Wang resin/vessel on an ABI 433A peptide synthesizer using 250 µmol scale Fastmoc™ coupling cycles. Preloaded cartridges containing 1 mmol standard Fmoc-amino acids, except for the position of attachment of the fluorophore, where 1 mmol Fmoc-Lys(Mtt)-OH was placed in the cartridge, were used with conductivity feedback monitoring. N-terminal acetylation was accomplished by using 1 mmol acetic acid in a cartridge under standard coupling conditions.

### 4.1.3. Removal Of 4-Methyltrityl (Mtt) From Lysine

The resin from the synthesizer was washed thrice with dichloromethane and kept wet. 150 mL of 95:4:1 dichloromethane:triisopropylsilane:trifluoroacetic acid was flowed through the resin bed over 30 minutes. The mixture turned deep yellow then faded to pale yellow. 100 mL of DMF was flowed through the bed over 15 minutes. The resin was then washed thrice with DMF and filtered. Ninhydrin tests showed a strong signal for primary amine.

### 4.1.4. Resin Labeling With 6-Carboxyfluorescein-NHS (6-FAM-NHS)

The resin was treated with 2 equivalents 6-FAM-NHS in 1% DIEA/DMF and stirred or shaken at ambient temperature overnight. When complete, the resin was drained, washed thrice with DMF, thrice with (1 × dichloromethane and 1× methanol) and dried to provide an orange resin that was negative by ninhydrin test.

### 4.1.5. General Procedure For Cleavage And Deprotection Of Resin-Bound Peptide

Peptides were cleaved from the resin by shaking for 3 hours at ambient temperature in a cleavage cocktail consisting of 80% TFA, 5% water, 5% thioanisole, 5% phenol, 2.5% TIS, and 2.5% EDT (1 mL/0.1 g resin). The resin was removed by filtration and rinsing twice with TFA. The TFA was evaporated from the filtrates, and product was precipitated with ether (10 mL/0.1 g resin), recovered by centrifugation, washed twice with ether (10 mL/0.1 g resin) and dried to give the crude peptide.

### 4.1.6. General Procedure For Purification Of Peptides

The crude peptides were purified on a Gilson preparative HPLC system running Unipoint® analysis software (Gilson, Inc., Middleton, WI) on a radial compression column containing two 25 × 100 mm segments packed with Delta-Pak™ C18 15 µm particles with 100 Å pore size and eluted with one of the gradient methods listed below. One to two milliliters of crude peptide solution (10 mg/mL in 90% DMSO/water) was purified per injection. The peaks containing the product(s) from each run were pooled and lyophilized. All preparative runs were run at 20 mL/min with eluents as buffer A: 0.1% TFA-water and buffer B: acetonitrile.

### 4.1.7. General Procedure For Analytical HPLC

Analytical HPLC was performed on a Hewlett-Packard 1200 series system with a diode-array detector and a Hewlett-Packard 1046A fluorescence detector running HPLC 3D ChemStation software version A.03.04 (Hewlett-Packard. Palo Alto, CA) on a 4.6 × 250 mm YMC column packed with ODS-AQ 5 µm particles with a 120 Å pore size and eluted with one of the gradient methods listed below after preequilibrating at the starting conditions for 7 minutes. Eluents were buffer A: 0.1% TFA-water and buffer B: acetonitrile. The flow rate for all gradients was 1 mL/min.

### 4.1.8. Synthesis of Probe F-Bak

Peptide probe F-bak, which binds Bcl-xL, was synthesized as described below. Probe F-Bak is acetylated at the N-terminus, amidated at the C-terminus and has the amino acid sequence GQVGRQLAIIGDKINR. It is fluoresceinated at the lysine residue (K) with 6-FAM. Probe F-Bak can be abbreviated as follows: acetyl-GQVGRQLAIIGDK(6-FAM)INR-NH₂.

To make probe F-Bak, Fmoc-Rink amide MBHA resin was extended using the general peptide synthesis procedure to provide the protected resin-bound peptide (1.020 g). The Mtt group was removed, labeled with 6-FAM-NHS and cleaved and deprotected as described hereinabove to provide the crude product as an orange solid (0.37 g). This product was purified by RP-HPLC. Fractions across the main peak were tested by analytical RP-HPLC, and the pure fractions were isolated and lyophilized, with the major peak providing the title compound (0.0802 g) as a yellow solid; MALDI-MS m/z = 2137.1 [(M+H)⁺].

### 4.1.9. Alternative Synthesis of Peptide Probe F-Bak

In an alternative method, the protected peptide was assembled on 0.25 mmol Fmoc-Rink amide MBHA resin (Novabiochem) on an Applied Biosystems 433A automated peptide synthesizer running Fastmoc™ coupling cycles using pre-loaded 1 mmol amino acid cartridges, except for the fluorescein(6-FAM)-labeled lysine, where 1 mmol Fmoc-Lys(4-methyltrityl) was weighed into the cartridge. The N-terminal acetyl group was incorporated by putting 1 mmol acetic acid in a cartridge and coupling as described hereinabove. Selective removal of the 4-methyltrityl group was accomplished with a solution of 95:4:1 DCM:TIS:TFA (v/v/v) flowed through the resin over 15 minutes, followed by quenching with a flow of dimethylformamide. Single-isomer 6-carboxyfluorescein-NHS was reacted with the lysine side-chain in 1% DIEA in DMF and confirmed complete by ninhydrin testing. The peptide was cleaved from the resin and side-chains deprotected by treating with 80:5:5:5:2.5:2.5 TFA/water/phenol/ thioanisole/triisopropylsilane: 3,6-dioxa-1,8-octanedithiol (v/v/v/v/v/v), and the crude peptide was recovered by precipitation with diethyl ether. The crude peptide was purified by reverse-phase high-performance liquid chromatography, and its purity and identity were confirmed by analytical reverse-phase high-performance liquid chromatography and matrix-assisted laser-desorption mass-spectrometry (m/z = 2137.1 ((M+H)⁺).

### 4.2. Time Resolved-Fluorescence Resonance Energy Transfer (TR-FRET) Assay

The ability of exemplary Bcl-xL inhibitors W1.01-W1.08 to compete with probe F-Bak for binding Bcl-xL was demonstrated using a Time Resolved Fluorescence Resonance Energy Transfer (TR-FRET) binding assay.

### 4.2.1. Method

For the assay, test compounds were serially diluted in DMSO starting at 50 µM (2x starting concentration; 10% DMSO) and 10 µL transferred into a 384-well plate. 10 µL of a protein/probe/antibody mix was then added to each well at final concentrations listed below:

| | | |
|---|---|---|
| Protein: | GST-Bcl-xL | 1 nM |
| Antibody | Tb-anti-GST | 1 nM |
| Probe: | F-Bak | 100 nM |

The samples were then mixed on a shaker for 1 minute and incubated for an additional 2 hours at room temperature. For each assay plate, a probe/antibody and protein/antibody/probe mixture were included as a negative and a positive control, respectively. Fluorescence was measured on the Envision (Perkin Elmer) using a 340/35 nm excitation filter and 520/525 (F-Bak) and 495/510 nm (Tb-labeled anti-his antibody) emission filters. Dissociation constants (Kᵢ) were determined using Wang's equation (Wang, 1995, FEBS Lett. 360:111-114). The TR-FRET assay can be performed in the presence of varying concentrations of human serum (HS) or fetal bovine serum (FBS). Compounds were tested both without HS and in the presence of 1% HS.

### 4.2.2. Results

The results of binding assays (Kᵢ in nanomolar) are provided in Table 2, below:

| **Table 2** | | |
|---|---|---|
| **TR-FRET Bcl-xL Binding Data** | | |
| **Appln Ex. No.** | **Bcl-xL Binding Kᵢ (nM)** | **Bcl-xL Binding Kᵢ (nM, 1% HS)** |
| 1.1 | 0.0029 | 0.39 |
| 1.2 | 0.04 | 0.51 |
| 1.3 | < 0.001 | 0.097 |
| 1.4 | 0.01 | 0.24 |
| 1.5 | < 0.001 | 0.078 |
| 1.6 | 0.019 | 0.62 |
| 1.7 | 0.011 | 0.094 |
| 1.8 | 0.1 | 17 |

### Example 5. Exemplary Bcl-xL Inhibitors Inhibit Bcl-xL in Molt-4 Cell Viability Assays

The ability of exemplary Bcl-xL inhibitors can be determined in cell-based killing assays using a variety of cell lines and mouse tumor models. For example, their activity on cell viability can be assessed on a panel of cultured tumorigenic and non-tumorigenic cell lines, as well as primary mouse or human cell populations. Bcl-xL inhibitory activity of exemplary Bcl-xL inhibitors was confirmed in a cell viability assay with Molt-4 cells.

### 5.1. Method

In one exemplary set of conditions, Molt-4 (ATCC, Manassas, VA) human acute lymphoblastic leukemia cells were plated 12,500 cells per well in 384-well tissue culture plates (Corning, Corning, NY) in a total volume of 25 µL tissue culture medium supplemented with 10% human serum (Sigma-Aldrich, St. Louis, MO) and treated with a 3-fold serial dilution of the compounds of interest from 10 µM to 0.0005 µM. Each concentration was tested in duplicate at least 3 separate times. The number of viable cells following 48 hours of compound treatment was determined using the CellTiter-Glo® Luminescent Cell Viability Assay according to the manufacturer's recommendations (Promega Corp., Madison, WI). Compounds were tested in the presence of 10% HS.

### 5.2. Results

The results of a Molt-4 cell viability assay (EC₅₀ in nanomolar) carried out in the presence of 10% HS for exemplary Bcl-xL inhibitors of Examples 1.1-1.6 (compounds W1.01-W1.08, respectively) are provided in Table 3, below.

| **Table 3** | | | |
|---|---|---|---|
| **Bcl-xL Inhibitor In Vitro Data** | | | |
| **Appln Ex. No.** | **Bcl-xL Binding Kᵢ (nM)** | **Bcl-xL Binding Kᵢ (nM, 1% HS)** | **Molt-4 Viability EC₅₀ (nM, 10% HS)** |
| 1.1 | 0.0029 | 0.39 | 7.8 |
| 1.2 | 0.04 | 0.51 | 5.4 |
| 1.3 | < 0.001 | 0.097 | 6.6 |
| 1.4 | 0.01 | 0.24 | 12.1 |
| 1.5 | < 0.001 | 0.078 | 1.85 |
| 1.6 | 0.019 | 0.62 | 47 |
| 1.7 | 0.011 | 0.094 | 4.0 |
| 1.8 | 0.1 | 17 | NT |

| | | | |
|---|---|---|---|
| **NT = not tested** | | | |

### Example 6. DAR and Aggregation of Exemplary ADCs

The DAR and percentage aggregation of exemplary ADCs synthesized as described in Example 3, above, were determined by LC-MS and size exclusion chromatography (SEC), respectively.

### 6.1. LC-MS General Methodology

LC-MS analysis was performed using an Agilent 1100 HPLC system interfaced to an Agilent LC/MSD TOF 6220 ESI mass spectrometer. The ADC was reduced with 5 mM (final concentration) Bond-Breaker® TCEP solution (Thermo Scientific, Rockford, IL), loaded onto a Protein Micro-trap (Michrom Bioresorces, Auburn, CA) desalting cartridge, and eluted with a gradient of 10% B to 75% B in 0.2 minutes at ambient temperature. Mobile phase A was H20 with 0.1% formic acid (FA), mobile phase B was acetonitrile with 0.1% FA, and the flow rate was 0.2 ml/min. Electrospray-ionization time-of-flight mass spectra of the co-eluting light and heavy chains were acquired using Agilent MassHunter(TM) acquisition software. The extracted intensity vs. m/z spectrum was deconvoluted using the Maximum Entropy feature of MassHunter software to determine the mass of each reduced antibody fragment. DAR was calculated from the deconvoluted spectrum by summing intensities of the naked and modified peaks for the light chain and heavy chain, normalized by multiplying intensity by the number of drugs attached. The summed, normalized intensities were divided by the sum of the intensities, and the summing results for two light chains and two heavy chains produced a final average DAR value for the full ADC.

### 6.2. Size Exclusion Chromatography General Methodology

Size exclusion chromatography was performed using a Shodex KW802.5 column in 0.2M potassium phosphate pH 6.2 with 0.25 mM potassium chloride and 15% IPA at a flow rate of 0.75 ml/min. The peak area absorbance at 280 nm was determined for each of the high molecular weight and monomeric eluents by integration of the area under the curve. The % aggregate fraction of the conjugate sample was determined by dividing the peak area absorbance at 280 nM for the high molecular weight eluent by the sum of the peak area absorbances at 280 nM of the high molecular weight and monomeric eluents multiplied by 100%.

### 6.3. Results

The average DAR values determined by the above LC-MS method and the % aggregate fraction for the exemplary ADCs are reported in Table 4. ADCs comprising the monoclonal antibody ING-1 (*see* Studnicka et al., 1994, Protein Engineering, 7:805-814 and Ammons et al., 2003, Neoplasia 5:146-154),which targets EpCAM, were evaluated in the assay. The EGFR targeting antibody AB033 is described in WO 2009/134776. The monoclonal antibody N901 (targeting NCAM-1) is described in Roguska et al., 1994, Proc Natl Acad Sci USA 91:969-973.

| **Table 4** | | | |
|---|---|---|---|
| **ADC Analytical Characterization** | | | |
| **Appln Ex. No.** | **ADC Code** | **% Agg (by SEC)** | **DAR (by MS)** |
| 3.1 | AB033-E | 48 | 4 |
| 3.2 | AB033-D | 45 | 4 |
| 3.3 | AB033-J | 28 | 4 |
| 3.4 | AB033-K | 28 | 4 |
| 3.5 | AB033-NG | 2.5 | 3.0 |
| 3.6 | AB033-M | 9.2 | 0.2 |
| 3.7 | AB033-V | 7 | 2.1 |
| 3.8 | AB033-DS | 40 | 2.7 |
| 3.10 | AB033-BG | 11 | 0.7 |
| 3.12 | AB033-BI | 27 | 2.2 |
| 3.17 | AB033-BO | 11 | 1.3 |
| 3.18 | AB033-BP | 10 | 1.2 |
| 3.21 | AB033-IQ | 34 | 3 |
| 3.22 | AB033-DB | 10.6 | 3.6 |
| 3.23 | AB033-DM | 19 | 3.8 |
| 3.24 | AB033-DL | 11 | 4.3 |
| 3.25 | AB033-DR | 8 | 3.9 |
| 3.26 | AB033-DZ | 2.8 | 3.4 |
| 3.27 | AB033-EA | 3.6 | 3.6 |
| 3.28 | AB033-EO | 24 | 4 |
| 3.29 | AB033-FB | 3 | 2.9 |
| 3.30 | AB033-KX | 11 | 3.92 |
| 3.31 | AB033-FF | 4.6 | 2.4 |
| 3.32 | AB033-FU | 10 | 3.68 |
| 3.33 | AB033-LB | 13.1 | 3.96 |
| 3.34 | AB033-FX | 2.3 | 2.83 |
| 3.35 | AB033-H | 4.2 | 3.9 |
| 3.36 | AB033-I | 1.5 | 4 |
| 3.37 | AB033-HB | 7.7 | 1.73 |
| 3.38 | AB033-KQ | 15.8 | 4.1 |
| 3.39 | AB033-KP | 12.5 | 1.9 |
| 3.40 | AB033-HA | 27.4 | 2.9 |
| 3.41 | AB033-NF | 3.6 | 3.3 |
| 3.42 | AB033-NG | 2.5 | 3 |
| 3.43 | EpCAM(ING-1)-EA | 2.90 | 2.6 |
| 3.44 | EpCAM(ING-1)-FX | 1.30 | 2.2 |
| 3.46 | EpCAM(ING-1)-DB | 2.66 | 2.7 |
| 3.55 | N901-D | 53 | 4.1 |
| 3.56 | N901-DB | 2.54 | 3.2 |
| 3.57 | N901-H | 1.8 | 3 |
| 3.58 | AB033-AS | 4.8 | 1.6 |
| 3.59 | AB033-AT | 9 | 1.9 |
| 3.60 | AB033-AU | 3 | 3.5 |
| 3.61 | AB033-BK | 5 | 3 |
| 3.62 | AB033-BQ | 9.8 | 2.9 |
| 3.63 | AB033-BR | 13.5 | 2.8 |
| 3.64 | AB033-OI | 3.1 | 2.73 |
| 3.65 | AB033-NX | 24.80 | 2.35 |
| 3.66 | AB033-OJ | 8.9 | 2.46 |
| 3.67 | AB033-XY | 1.4 | 2 |

### Example 7. EGFR-Targeted ADCs Inhibit the Growth of Cancer Cells In Vitro

Certain exemplary ADCs comprising antibody AB033 was evaluated. Antibody AB033 targets human EGFR. The variable heavy and light chain sequences of antibody AB033 are described in WO 2009/134776 (*see* page 120). The ability of antibody AB033 to inhibit the growth of cancer cells was demonstrated with *mcl-1-*/*-* mouse embryonic fibroblast (MEF) cells. *Mcl-1*^{*-*/*-*} MEFs are dependent upon Bcl-xL for survival (Lessene et al., 2013, Nature Chemical Biology 9:390-397). To evaluate the efficacy of exemplary AB033-targeted Bcl-xL-ADCs, human EGFR was over-expressed in *mel-1*^{*-*/*-*} MEFs.

### 7.1. Method

Retroviral supernatants were produced through transfection of the GP2-293 packaging cell line (Clontech) with the retroviral construct pLVC-IRES-Hygro (Clontech) containing huEGFR sequence or the empty vector utilizing FuGENE 6 transfection reagent (Roche Molecular Biochemicals, Mannheim, Germany). After 48 hours of culture, virus-containing supernatant was harvested and applied to *mcl-1*^{*-*/*-*} MEFs in 75 cm² culture flasks (0.5x10⁶ per flask) for a further 48 hours in the presence of polybrene (8 µg/ml; Sigma). *Mcl-1*^{*-*/*-*} MEFs were washed and selected after 3 days with 250 µg/ml hygromycin B (Invitrogen) in the full complement of media. The expression of huEGFR was confirmed by flow cytometry and compared to the parental cell line or those transfected with the empty vector.

*Mcl-1*^{*-*/*-*} MEFs expressing huEGFR or the pLVX empty vector (Vct Ctrl) were treated with AB033-targeted Bcl-xL-ADCs, AB033 alone or MSL109-targeted Bcl-xL-ADCs for 96 hours in DMEM containing 10% FBS. For the assay, the cells were plated at 250 cells per well in 384-well tissue culture plates (Corning, Corning, NY) in a total volume of 25 µL of assay media (DMEM and 10% HI FBS). The plated cells were treated with a 4-fold serial dilution of the Antibody Drug Conjugates of interest from 1 µM to 1 pM dispensed by an Echo 550 Acoustic Liquid Handler (Labcyte). Each concentration was tested in twelve replicates for the *Mcl-1*^{*-*/*-*} MEF huEGFR cell line and in six replicates for the *Mcl-1*^{*-*/*-*} MEF vector cell line. The fraction of viable cells following 96 hours of Antibody Drug Conjugate treatment at 37 °C and 5% CO₂ was determined using the CellTiter-Glo® Luminescent Cell Viability Assay according to the manufacturer's recommendations (Promega Corp., Madison, WI). The plates were read in a Perkin Elmer Envision using a Luminescence protocol with 0.5 sec integration time. The replicate values for each dilution point were averaged and the EC₅₀ values for the Antibody Drug Conjugates were generated by fitting the data with GraphPad Prism 5 (GraphPad Software, Inc.) to a sigmoidal curve model using linear regression, Y=((Bottom-Top)/(1+((x/K)ⁿ)))+Top, where Y is the measured response, x is the compound concentration, n is the Hill Slope and K is the EC₅₀ and Bottom and Top are the lower and higher asymptotes respectively. Visual inspection of curves was used to verify curve fit results.. *Mcl-1*^{*-*/*-*} MEFs were obtained from David C. S. Huang of the Walter and Eliza Hall Institute of Medical Research.

### 7.2. Results

Cell viability assay results (EC₅₀ in nanomolar) for representative Examples are provided below in Table 5.

| **Table 5** | | |
|---|---|---|
| ***In Vitro* Cell Viability Efficacy of Exemplary EGFR-Targeted ADCs** | | |
| **Appln Ex. No.** | **ADC Code** | **huEGFR⁺ *mcl-1*^{*-*/*-*} MEF ECso (nM)** |
| 3.1 | AB033-E | NT |
| 3.2 | AB033-D | 0.23 |
| 3.3 | AB033-J | NT |
| 3.4 | AB033-K | NT |
| 3.5 | AB033-NG | 25 |
| 3.6 | AB033-M | NT |
| 3.7 | AB033-V | 0.63 |
| 3.8 | AB033-DS | 1.7 |
| 3.10 | AB033-BG | NT |
| 3.12 | AB033-BI | NT |
| 3.17 | AB033-BO | NT |
| 3.18 | AB033-BP | NT |
| 3.21 | AB033-IQ | 3.53 |
| 3.22 | AB033-DB | 0.3 |
| 3.23 | AB033-DM | NT |
| 3.24 | AB033-DL | NT |
| 3.25 | AB033-DR | 0.11 |
| 3.26 | AB033-DZ | 0.2 |
| 3.27 | AB033-EA | 5.8 |
| 3.28 | AB033-EO | 0.9 |
| 3.29 | AB033-FB | 3.9 |
| 3.30 | AB033-KX | 31.1 |
| 3.31 | AB033-FF | 4.1 |
| 3.32 | AB033-FU | 3.21 |
| 3.33 | AB033-LB | 22.1 |
| 3.34 | AB033-FX | 1.65 |
| 3.35 | AB033-H | 2.5 |
| 3.36 | AB033-I | 1.04 |
| 3.37 | AB033-HB | 0.54 |
| 3.38 | AB033-KQ | 18.25 |
| 3.39 | AB033-KP | 133.1 |
| 3.40 | AB033-HA | 0.56 |
| 3.41 | AB033-NF | 27.4 |
| 3.42 | AB033-NG | 25.0 |
| 3.58 | AB033-AS | 8.11 |
| 3.59 | AB033-AT | 0.32 |
| 3.60 | AB033-AU | 0.93 |
| 3.61 | AB033-BK | NT |
| 3.62 | AB033-BQ | NT |
| 3.63 | AB033-BR | NT |
| 3.64 | AB033-OI | 28 |
| 3.65 | AB033-NX | 63 |
| 3.66 | AB033-OJ | 42 |
| 3.67 | AB033-XY | NT |

| | | |
|---|---|---|
| NT = not tested | | |

Cell viability assay results (EC₅₀ in nanomolar) for representative Examples 3.28, 3.29 and 3.35 against the *Mcl-1*^{*-*/*-*} MEF vector cell line are 67 nM, 69 nM and 249 nM, respectively.

### Example 8. EpCAM-, and NCAM1-Targeted Antibody Drug Conjugates Inhibit the Growth of Cancer Cells In Vitro

The ability of certain exemplary ADCs comprising an antibody that targets human cell adhesion molecule (EpCAM) to inhibit Bcl-xL and induce apoptosis was demonstrated NCC38 cells, a human breast cancer cell line expressing endogenous EpCAM protein. Cytotoxicity of certain exemplary ADCs targeted to human neural cell adhesion molecule NCAM1 was demonstrated in NCI-H146 cells, a human small cell lung cancer line that expresses endogenous NCAM-1.

### 8.1. Method

For the assay, both HCC38 and NCI-H146 cell lines were cultured in RPMI 1640 media (Invitrogen, #11995) containing 10% FBS. Prior to the assay, cells were resuspended to 4x10⁴ cells/mL in culture media and then added to the 96-well tissue culture plates at 75 µL cells/well for a final concentration of 3,000 cells/well. The assay plates were then incubated at 37 °C with 5% CO₂ for overnight. On the following day, EpCAM, NCAM-1 or negative control (MSL109) ADCs were serially diluted in culture media and were added to the assay plates at 25 µl/well. The assay plates were then incubated at 37 °C with 5% CO₂ for 72 hours. Cell viability was measured by the CellTiter-Glo® Luminescent Cell Viability Assay Kit (Promega, #G7573).

### 8.2. Results

Data was analyzed using Graphpad Prism software. The IC₅₀ values (the concentration of ADC to achieve 50% of the maximum growth inhibition of the cells) are reported in Tables 6 and 7, respectively.

As shown in Table 6, EpCAM-targeted ADCs potently killed HCC38 breast cancer cells (IC₅₀ ≤ 0.4 nM) while the negative control ADC MSL109-DB showed weak activity. As shown in Table 7, NCAM1-DB and NCAM1-H ADCs showed specific activity toward NCI-146 small cell lung cancer cells (IC₅₀ ∼ 20 nM).

| **Table 6** | | |
|---|---|---|
| **EpCAM ADCs Inhibit the Growth of HCC38 Breast Cancer Cells** | | |
| **Appln Ex. No.** | **ADC Code** | **HCC38 Cells IC₅₀ (nM)** |
| 3.46 | EpCAM(ING-1)-DB | 0.09 |
| | MSL109-DB | 8.69 |

| **Table 7** | | |
|---|---|---|
| **NCAM1 ADCs Inhibit the Growth of NCI-H146 Small Cell Lung Cancer Cells** | | |
| **Appln Ex. No.** | **ADC Code** | **NCI-H146 Cells IC₅₀ (nM)** |
| 3.56 | N901-DB | 28.8 |
| 3.57 | N901-H | 21.6 |
| | MSL109-DB | 17.36 |
| | MSL109-H | 197.75 |

### Example 9. Bcl-xL Inhibitory Antibody Drug Conjugates (ADCs) Targeting EGFR, Alone and In Combination with Docetaxel Inhibit the Growth of Non Small Cell Lung Cancer (NSCLC) Xenografts In Vivo

The efficacy and selectivity of exemplary Bcl-xL inhibitory ADCs (also referred to herein as Bcl-xLi ADCs) targeting tumor associated antigens (TAAs) such as EGFR are demonstrated in the two NSCLC xenograft models, NCI-H1650 and EBC-1. As revealed by immunohistochemistry, membrane expression of the antigens on the xenografted tumors is illustrated in Table 8, below.

| **Table 8** | | |
|---|---|---|
| **Expression of EGFR and EpCAM on the surface of xenografted tumor cells** | | |
| **Cell line \ Antigen** | **EGFR** | **EpCAM** |
| NCI-H1650 | +++ | +/+++ |
| NCI-EBC-1 | ++ | +/++ |

### Example 10. EGFR-Targeting Bcl-xL Antibody Drug Conjugates Inhibit the Growth of NSCLC Tumor Cells In Vivo

The ability of certain exemplary EGFR-targeted ADCs to selectively inhibit the growth of EGFR-expressing tumor cells in mice was demonstrated in xenograft models derived from human NSCLC cell lines, NCI-H1650 and EBC-1.

### 10.1. Method

The NSCLC cell line NCI-H1650 was purchased from the American Type Culture Collection (ATCC, Manassas, VA). The cells were cultured as monolayers in RPMI 1640 culture medium (Invitrogen, Carlsbad, CA) that was supplemented with Fetal Bovine Serum (FBS, Hyclone, Logan, UT). The squamous lung carcinoma cell line, EBC-1 was obtained from Japanese Collection of Research Biosources (JCRB, Osaka, Japan). The cells were cultured as monolayers in MEM culture medium (Invitrogen, Carlsbad, CA) that was supplemented with 10% Fetal Bovine Serum (FBS, Hyclone, Logan, UT). To generate xenografts, 5^{∗}10⁶ viable cells were inoculated subcutaneously into the right flank of immune deficient female SCID/bg mice (Charles River Laboratories, Wilmington, MA). The injection volume was 0.2 ml and composed of a 1:1 mixture of S-MEM and Matrigel (BD, Franklin Lakes, NJ). Tumors were size matched at approximately 200 mm³. Antibodies and conjugates were formulated in phosphate buffered saline (PBS) and injected intraperitoneally. Injection volume did not exceed 400 µl. Therapy began within 24 hours after size matching of the tumors. Mice weighed approximately 25 g at the onset of therapy. Tumor volume was estimated two to three times weekly. Measurements of the length (L) and width (W) of the tumor were taken via electronic caliper and the volume was calculated according to the following equation: V = L x W²/2. Mice were euthanized when tumor volume reached 3,000 mm³ or skin ulcerations occurred. Eight to ten mice were housed per cage. Food and water were available ad libitum. Mice were acclimated to the animal facilities for a period of at least one week prior to commencement of experiments. Animals were tested in the light phase of a 12-hour light: 12-hour dark schedule (lights on at 06:00 hours). All experiments were conducted in compliance with AbbVie's Institutional Animal Care and Use Committee and the National Institutes of Health Guide for Care and Use of Laboratory Animals guidelines in a facility accredited by the Association for the Assessment and Accreditation of Laboratory Animal Care.

### 10.2. Result description and analysis

To refer to efficacy of therapeutic agents, parameters of amplitude (TGIₘₐₓ), durability (TGD) and response frequency (CR, PR, OR) of therapeutic response are used.

TGIₘₐₓ is the maximum tumor growth inhibition during the experiment. Tumor growth inhibition is calculated by 100^{∗}(1-Tᵥ/Cᵥ) where Tᵥ and Cᵥ are the mean tumor volumes of the treated and control groups, respectively.

TGD or tumor growth delay is the extended time of a treated tumor needed to reach a volume of 1 cm³ relative to the control group. TGD is calculated by 100^{∗}(Tₜ/Cₜ-1) where Tₜ and Cₜ are the median time periods to reach 1 cm³ of the treated and control groups, respectively.

Distribution of the response amplitude in a specific group is given by the frequency of complete responders (CR), partial responders (PR), and overall responders (OR). CR is the percentage of mice within a group with a tumor burden of 25 mm³ for at least three measurements. PR is the percentage of mice within a group with a tumor burden larger than 25 mm³ but less than one-half of the volume at onset of treatment for at least three measurements. OR is the sum of CR and PR.

The 2-tailed Student's test and Kaplan-Meier log-rank test were used to determine significance of the difference in TGIₘₐₓ and TGD, respectively.

### 10.3. Growth inhibition of the lung adenocarcinoma Model NCI-H1650 (hereafter called H1650)

### 10.3.1. EGFR-Targeting ADCs

For the EGFR-targeted ADCs, the synthons of Example 2.2 (synthon D), Example 2.35 (synthon H) and Example 2.36 (synthon I) were conjugated to the EGFR-targeting antibody AB033 as described in Examples 3.2, 3.35 and 3.36, respectively, yielding ADCs AB033-D, AB033-H and AB033-I, respectively. Conjugates of synthon H, DB or I and the cytomegalovirus (CMV) targeting antibody MSL109 (MSL109-H, MSL109-DB or MSL-109-I) were used as a passive targeting control. These conjugates are hereafter also referred to as 'non-targeting' ADCs because the carrier antibody does not recognize a tumor associated antigen. MSL109 is described in Drobyski et al., 1991, Transplantation 51:1190-1196 and U.S. Patent No. 5,750,106. An antibody that targets tetanus toxoid (antibody AB095) was used as a control for the effect of administering IgG. *See* Larrick et al., 1992, Immunological Reviews 69-85.

### 10.3.2. Results with EGFR-Targeted ADCs

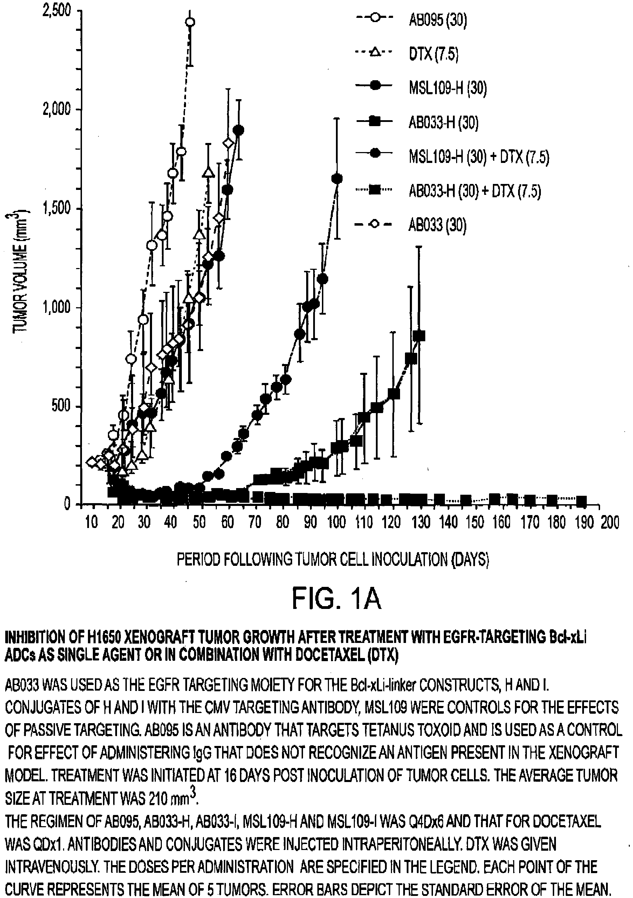

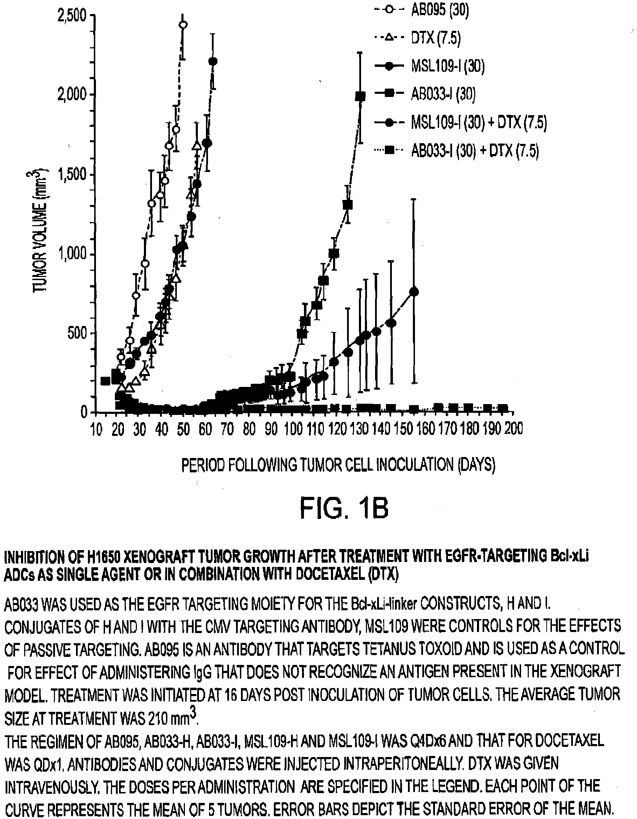

The efficacy and selectivity of inhibition of H1650 xenografts growth with EGFR-targeted ADCs is illustrated by **FIG. 1** and Table 9, below. In **FIG. 1****,** the amounts given per administration are specified in the legend. Treatment was initiated at 16 days post inoculation of tumor cells. The average tumor size at treatment was 210 mm³. The regimen for AB095, AB033-H, AB033-I, MSL109-H and MSLI09-1 was Q4Dx6. The standard-of-care chemotherapeutic agent docetaxel (DTX) was used at a dose of 7.5 mg/kg (QDx1) either as a single agent or in combination with ADCs. Antibodies and conjugates were injected intraperitoneally. DTX was given intravenously. Each point of the curve represents the mean of 5 tumors. Error bars depict the standard error of the mean.

Administered as a small molecule, the Bcl-xL inhibitor A-1331852 (Leverson et al., 2015, Sci. Transl. Med. 7:279ra40) inhibited xenograft growth significantly as demonstrated by TGIₘₐₓ of 57% and TGD of 78% (Table 9). Given as single agents, EGFR targeting Bcl-xLi ADCs (30 mg/kg, Q4Dx6) inhibited tumor growth (TGIₘₐₓ) approximately 1.5-fold more than the non-targeted ADC MSL109 H and the naked anti-EGFR antibody AB033. The growth inhibition and durability of the response of DTX treatment was enhanced by the non-targeting ADCs MSL109-H and MSL109-I. EGFR targeting ADCs AB033-H and AB033-I, when added to DTX, caused a more durable tumor regression than the addition of the non-targeting ADCs (Table 9 and **FIG. 1**).

| **Table 9** | | | | | | |
|---|---|---|---|---|---|---|
| **Inhibition of H1650 xenograft tumor growth after treatment with EGFR-targeting Bcl-xLi ADCs as single agent or in combination with docetaxel (DTX)** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 30/IP/Q4Dx6 | 0 | 0 | 0 | 0 | 0 |
| AB033 | 30/IP/Q4Dx6 | 62* | 117* | 0 | 0 | 0 |
| DTX | 7.5/IV/QDx1 | 73* | 94* | 0 | 0 | 0 |
| A-1331852 | 25/PO/QDx14 | 57* | 78* | 0 | 0 | 0 |
| A-1331852 | 25/PO/QDx14 | 95*^{¥} | 289*^{¥} | 0 | 100 | 100 |
| + DTX | +7.5/IV/QDx1 | | | | | |
| MSLI09-H^{†} | 30/IP/Q4Dx6 | 64* | 117* | 0 | 0 | 0 |
| MSL109-H^{†} | 30/IP/Q4Dx6 | 97* | 367*^{¥} | 0 | 100 | 100 |
| + DTX | + 7.5/IV/QDx1 | | | | | |
| MSL109-I^{†} | 30/IP/Q4Dx6 | 63* | 78* | 0 | 0 | 0 |
| MSL109-I^{†} | 30/IP/Q4Dx6 | 99*^{¥} | 900*^{¥} | 100 | 0 | 100 |
| + DTX | + 7.5/IV/QDx1 | | | | | |
| AB033-H | 30/IP/Q4Dx6 | 99* | 622* | 40 | 60 | 100 |
| AB033-H | 30/IP/Q4Dx6 | 99* | >900*^{¥} | 100 | 0 | 100 |
| + DTX | + 7.5/IV/QDx1 | | | | | |
| AB033-I | 30/IP/Q4Dx6 | 99* | 478* | 80 | 20 | 100 |
| AB033-I | 30/IP/Q4Dx6 | 100*^{¥} | >900*^{¥} | 100 | 0 | 100 |
| + DTX | + 7.5/IV/QDx1 | | | | | |
| ** IgG1 mAb | | | | | | |
| ^{†} Non-targeting antibody | | | | | | |
| ^{[a]} dose is given in mg/kg/day | | | | | | |
| * = p < 0.05 as compared to control treatment (AB095) | | | | | | |
| ^{¥} = p < 0.05 as compared to the most active partner in a drug combination | | | | | | |

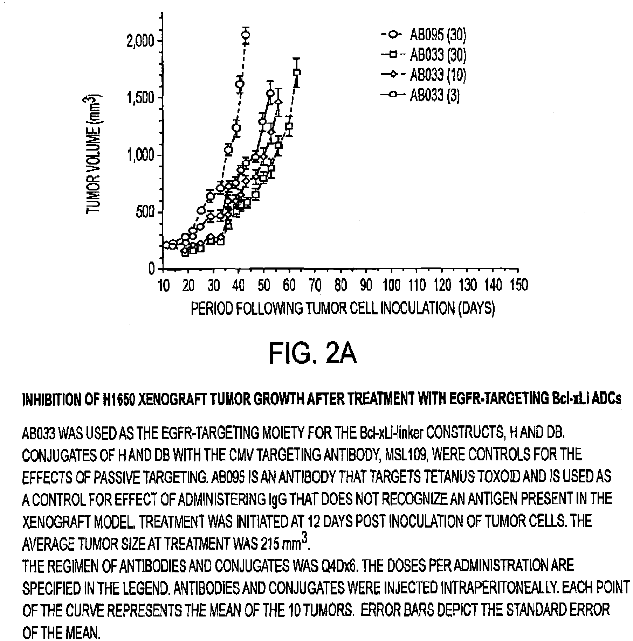

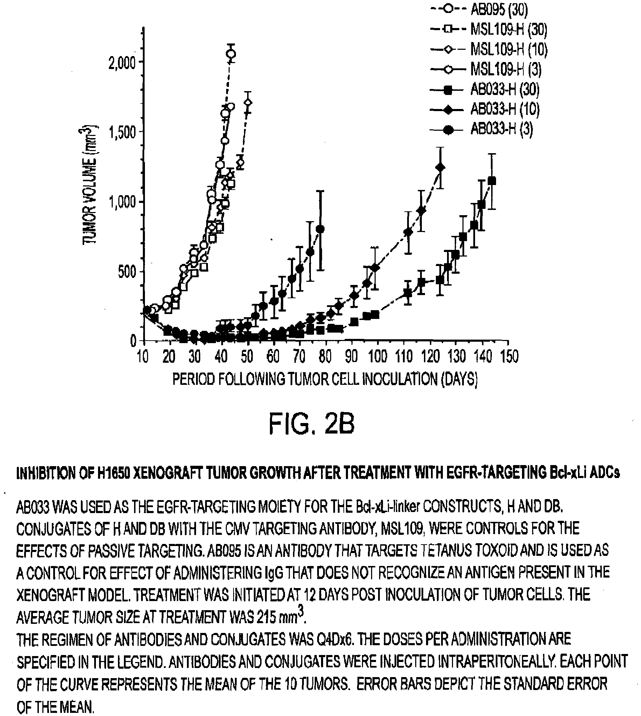

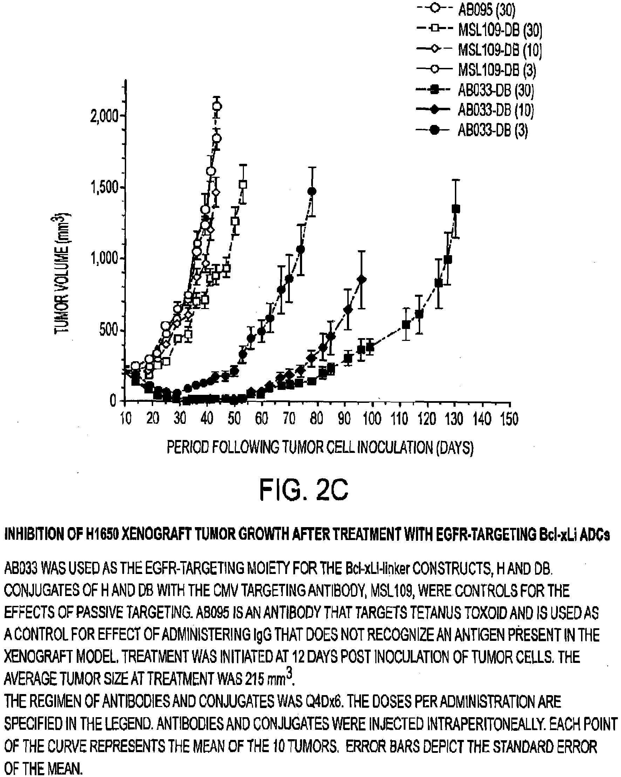

The previous experiment showed that growth of H1650 was significantly inhibited by EGFR-targeting Bcl-xL inhibitory ADCs. This model was therefore fit to further explore minimal efficacious doses of these conjugates and to identify additional eficacious Bcl-xL inhibitors. Table 10 and **FIG. 2** illustrate the dose-response relationship of EGFR-targeting Bcl-xLi ADCs, AB033-H and AB033-DB. Treatment was initiated at 12 days post inoculation of tumor cells. The average tumor size at treatment was 215 mm³. The regimen for antibodies and conjugates was Q4Dx6. The amounts given per administration are specified in the legend. Antibodies and conjugates were injected intraperitoneally. Each point of the curve in **FIG. 2** represents the mean of 10 tumors. Error bars depict the standard error of the mean.

Although the carrier antibody AB033 inhibits tumor growth, the Bcl-xL inhibitor-linker moiety of the ADC is mainly responsible for the efficacy of the conjugate. The durability of inhibition with AB033 is dose-responsive and lies between a TGD of 44 and 80 % at doses of 3 and 30 mg/kg at a Q4Dx6 regimen. This efficacy is considerably lower as compared to that of AB033-H and AB033-DB. The lowest TGD of these conjugates (AB033-DB) was 160 % at 3 mg/kg. Moreover, treatment with AB033 does not result in a measurable response rate while the Bcl-xL ADCs, even at 3 mg/kg, induce at minimum a 90% overall response rate. The efficacy of the EGFR-targeting conjugates is also unlikely caused by effects of passive targeting because the use of MSL109 as a carrier antibody yielded negligable growth inhibition in the absence of any response rate.

| **Table 10** | | | | | | |
|---|---|---|---|---|---|---|
| **Inhibition of H1650 xenograft tumor growth after treatment with various doses of EGFR-targeting Bcl-xLi ADCs.** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 30/IP/Q4Dx6 | 0 | 0 | 0 | 0 | 0 |
| AB033 | 30/IP/Q4Dx6 | 71* | 80* | 0 | 0 | 0 |
| AB033 | 10/IP/Q4Dx6 | 62* | 56* | 0 | 0 | 0 |
| AB033 | 3/IP/Q4Dx6 | 54* | 44* | 0 | 0 | 0 |
| MSL109-H^{†} | 30/IP/Q4Dx6 | 45* | 24* | 0 | 0 | 0 |
| MSL109-H^{†} | 10/IP/Q4Dx6 | 43* | 20* | 0 | 0 | 0 |
| MSL109-H^{†} | 3/IP/Q4Dx6 | 18* | 0 | 0 | 0 | 0 |
| AB033-H | 30/IP/Q4Dx6 | 99* | 432* | 100 | 0 | 100 |
| AB033-H | 10/IP/Q4Dx6 | 100* | 352* | 100 | 0 | 100 |
| AB033-H | 3/IP/Q4Dx6 | 98* | 196* | 70 | 30 | 100 |
| MSL109-DB^{†} | 30/IP/Q4Dx6 | 57* | 56* | 0 | 0 | 0 |
| MSL109-DB^{†} | 10/IP/Q4Dx6 | 29* | 20* | 0 | 0 | 0 |
| MSL109-DB^{†} | 3/IP/Q4Dx6 | 17* | 0 | 0 | 0 | 0 |
| AB033-DB | 30/IP/Q4Dx6 | 99* | 364* | 100 | 0 | 100 |
| AB033-DB | 10/IP/Q4Dx6 | 99* | 246* | 100 | 0 | 100 |
| AB033-DB | 3/IP/Q4Dx6 | 92* | 160* | 90 | 0 | 90 |
| ** IgG1 mAb | | | | | | |
| ^{†} Non-targeting antibody | | | | | | |
| ^{[a]} dose is given in mg/kg/day | | | | | | |
| * = p < 0.05 as compared to control treatment (AB095) | | | | | | |

The inhibition of H1650 xenograft growth by a single dose of various Bcl-xL inhibitors linked to AB033 is summarized in Table 11. Treatment was initiated at 11 days post inoculation of tumor cells. The average tumor size at treatment was 216 mm³. The regimen for antibodies and conjugates was QDx1. Ten mg/kg was given as a single dose. Antibody and conjugates were intraperitoneally administered. Each treatment group consisted of 8 mice.

The EGFR targeting Bcl-xLi ADCs were consistently more efficacious than the control conjugate. Despite the administration of a single dose, the targeting ADCs induced durable responses as shown by TGDs ranging from 133 to >507%.

| **Table 11** | | | | | | |
|---|---|---|---|---|---|---|
| **Inhibition of H1650 xenograft tumor growth after treatment with a single dose of EGFR-targeting Bcl-xLi ADC** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 10/IP/QDx1 | 0 | 0 | 0 | 0 | 0 |
| MSL109-H^{†} | 10/IP/QDx1 | 20 | 7* | 0 | 0 | 0 |
| AB033-H | 10/IP/QDx1 | 89* | >507* | 13 | 63 | 88 |
| AB033-DB | 10/IP/QDx1 | 75* | 133* | 13 | 13 | 25 |
| AB033-FB | 10/IP/QDx1 | 99* | 280* | 13 | 88 | 100 |

| **Table 11** | | | | | | |
|---|---|---|---|---|---|---|
| **Inhibition of H1650 xenograft tumor growth after treatment with a single dose of EGFR-targeting Bcl-xLi ADC** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB033-FU | 10/IP/QDx1 | 90* | 230* | 0 | 75 | 75 |
| AB033-FX | 10/IP/QDx1 | 98* | 483* | 100 | 0 | 100 |
| ** IgG1 mAb | | | | | | |
| ^{†} Non-targeting antibody | | | | | | |
| ^{[a]} dose is given in mg/kg/day | | | | | | |
| * = p < 0.05 as compared to control treatment (AB095) | | | | | | |

The inhibition of H1650 xenograft growth by two additional Bcl-xL inhibitors linked to AB033 was evaluated in a separate experiment (Table 12). Treatment was initiated at 12 days post inoculation of tumor cells. The average tumor size at treatment was 213 mm³. The regimen for antibodies and conjugates was QDx1 administered at a dose of 10 mg/kg. Antibody and conjugates were intraperitoneally administered. Each treatment group consisted of 8 mice.

| **Table 12** | | | | | | |
|---|---|---|---|---|---|---|
| **Inhibition of H1650 xenograft tumor growth after treatment with a single dose of EGFR-targeting Bcl-xLi ADC** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 10/IP/QDx1 | 0 | 0 | 0 | 0 | 0 |
| AB033-DB | 10/IP/QDx1 | 97* | >500* | 0 | 100 | 100 |
| AB033-KX | 10/IP/QDx1 | 96* | >500* | 100 | 0 | 100 |
| AB033-LB | 10/IP/QDx1 | 98* | >500* | 100 | 0 | 100 |
| ** IgG1 mAb | | | | | | |
| ^{[a]} dose is given in mg/kg/day | | | | | | |
| * = p < 0.05 as compared to control treatment (AB095) | | | | | | |

### 10.4. Growth inhibition of the Squamous NSCLC model EBC-1

### 10.4.1. Method for EGFR-Targeted ADCs

General methods of generation of xenografts, monitoring of tumor growth and data analysis are provided in Example 2. For the EGFR-targeted ADCs, the synthons of Example 2.2 (synthon D), Example 2.35 (synthon H) and Example 2.36 (synthon I) were conjugated to the EGFR-targeting antibody AB033 as described in Examples 3.2, 3.35 and 3.36, respectively, yielding ADCs AB033-D, AB033-H and AB033-I, respectively. A conjugate of synthon H and the CMV targeting antibody MSL109 (MSL109-H) was used as a passive targeting control. An antibody that targets tetanus toxoid (antibody AB095) was used as a control for the effect of administering IgG.

### 10.4.2. Results with EGFR-Targeted ADCs

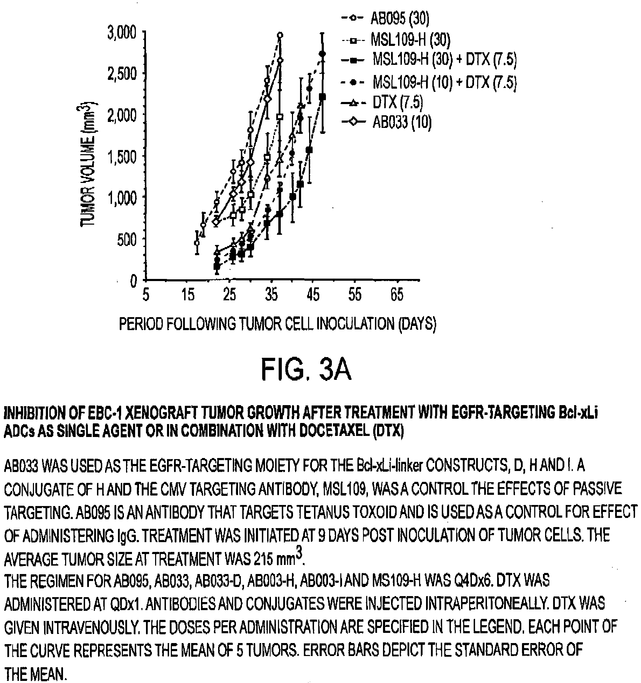

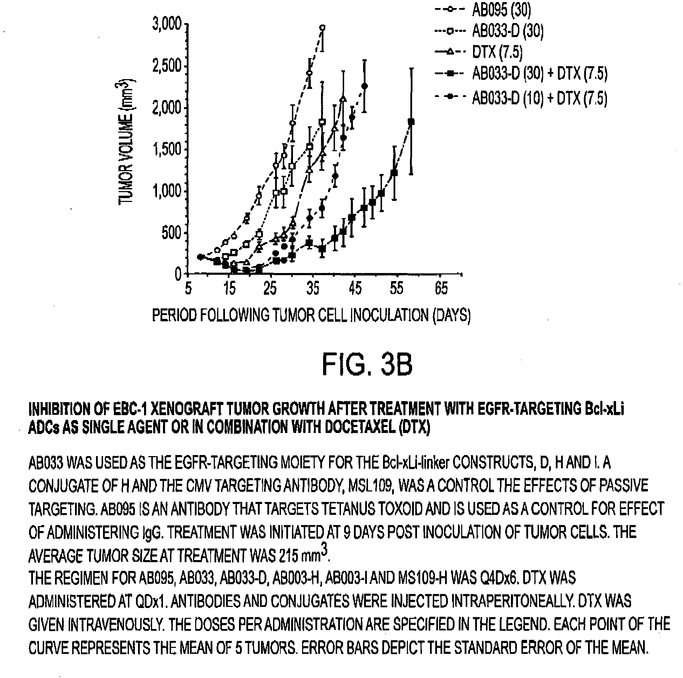

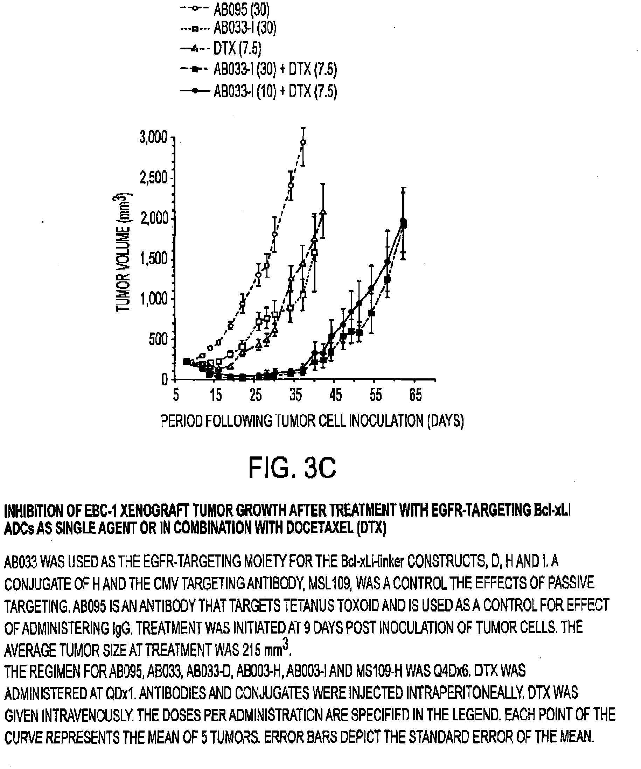

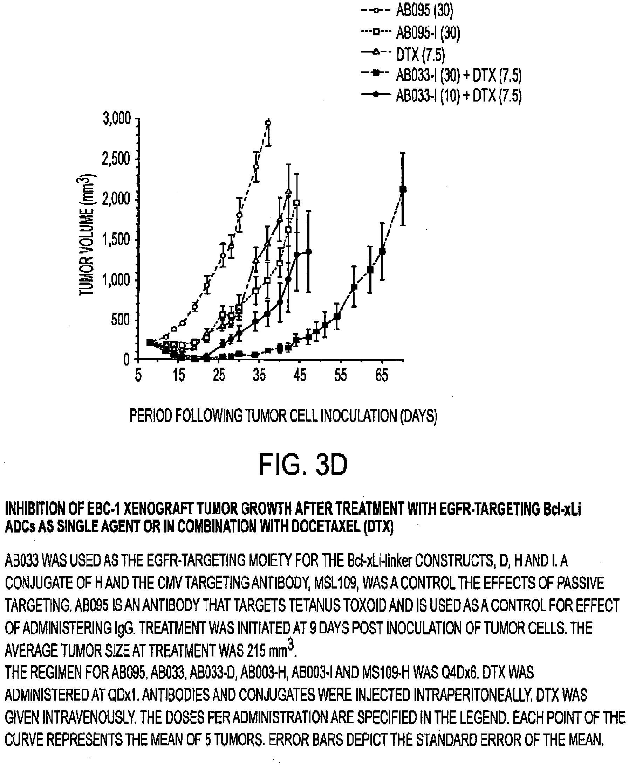

The therapeutic efficacy of EGFR-targeting ADCs was also observed when xenograft models of the squamous carcinoma EBC-1 were treated. The results of this experiment with the EGFR-targeted ADCs are shown in **Fig. 3** and Table 13, below. In **Fig. 3****,** the doses per administration are specified in the legend. Each point of the curve represents the mean of 5 tumors. Error bars depict the standard error of the mean. Treatment was initiated at 9 days post inoculation of tumor cells. The average tumor size at treatment was 215 mm³. The regimen for AB095, AB033, AB033-D, AB033-H, AB033-I and MSL109-H was Q4Dx6. DTX was administered at QDx1. Antibodies and conjugates were injected intraperitoneally. DTX was given intravenously.

The CMV-targeting ADC MSL109-H, at a dose of 30 mg/kg, inhibited tumor growth by 43%, thus showing anti-tumor activity that is associated with passive targeting. The standard-of-care chemotherapeutic agent docetaxel (DTX) was used at a dose of 7.5 mg/kg either as a single agent or in combination with ADCs throughout the experiment. As a single agent, DTX caused a TGI of 76%. In combination with DTX, MSL109-H (at 30 mg/kg) increased the TGI from 76 to 83 % and the TGD from 44 to 89% (Table 13, **FIG. 3A**). The efficacy achieved with passive targeting is inferior to the efficacy seen with the ADCs that use the EGFR targeting antibody, AB033. The TGIₘₐₓ of EGFR-targeted ADC AB033-H at 30 mg/kg is 64% (**FIG. 3C**). Addition of AB033-H (30 mg/kg) to DTX increased the TGIₘₐₓ from 76 to 98% and the TGD from 44 to 178%. Administration of AB033-I caused a maximum tumor growth inhibition of 66% (**FIG. 3D**). When given in combination, this conjugate boosted the TGIₘₐₓ and TGD of DTX to 98 and 178%, respectively. AB033-D was marginally efficacious when given as a single agent (TGIₘₐₓ = 49%). Nonetheless addition of this conjugate to DTX increased the TGIₘₐₓ and TGD of DTX to 94 and 156%, respectively (Table 13, **FIG. 3B**). Of note, the EGFR targeting conjugates given at 10 mg/kg also increased the efficacy of DTX (Table 13). The therapeutic benefit of adding a targeted Bcl-xLi ADC to DTX is increase of durability rather than enhancement of the amplitude of the response.

| **Table 13** | | | | | | |
|---|---|---|---|---|---|---|
| **Inhibition of EBC-1 xenograft tumor growth after treatment with EGFR-targeting Bcl-xLi ADCs as single agent or in combination with docetaxel (DTX)** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 30/IP/Q4Dx6 | 0 | 0 | 0 | 0 | 0 |
| AB033 | 30/IP/Q4Dx6 | 26* | 0 | 0 | 0 | 0 |
| DTX | 7.5/IV/QDx1 | 76* | 44* | 0 | 0 | 0 |
| MSL109-H^{†} | 30/IP/Q4Dx6 | 43* | 44* | 0 | 0 | 0 |
| MSL109-H^{†} | 30/IP/Q4Dx6 | | | | | |
| + DTX | + 7.5/IV/QDx1 | 83*^{¥} | 89* | 0 | 20 | 20 |
| MSL109-H^{†} | 10/IP/Q4Dx6 | | | | | |
| + DTX | + 7.5/IV/QDx1 | 75*^{¥} | 61* | 0 | 0 | 0 |
| AB033-D | 30/IP/Q4Dx6 | 49* | 22* | 0 | 0 | 0 |
| AB033-D | 30/IP/Q4Dx6 | | | | | |
| + DTX | + 7.5/IV/QDx1 | 94*^{¥} | 156*^{¥} | 0 | 100 | 100 |
| AB033-D | 10/IP/Q4Dx6 | | | | | |
| + DTX | + 7.5/IV/QDx1 | 91*^{¥} | 78*^{¥} | 0 | 60 | 60 |
| AB033-H | 30/IP/Q4Dx6 | 64* | 89* | 0 | 0 | 0 |
| AB033-H | 30/IP/Q4Dx6 | | | | | |
| + DTX | + 7.5/IV/QDx1 | 98*^{¥} | 178*^{¥} | 80 | 20 | 100 |
| AB033-H | 10/IP/Q4Dx6 | | | | | |
| + DTX | + 7.5/IV/QDx1 | 97*^{¥} | 156*^{¥} | 60 | 40 | 100 |
| AB033-I | 30/IP/Q4Dx6 | 70* | 61* | 0 | 0 | 0 |
| AB033-I | 30/IP/Q4Dx6 | | | | | |
| + DTX | + 7.5/IV/QDx1 | 98*^{¥} | 178*^{¥} | 60 | 40 | 100 |
| AB033-I | 10/IP/Q4Dx6 | | | | | |
| + DTX | + 7.5/IV/QDx1 | 95*^{¥} | 100*^{¥} | 0 | 100 | 100 |
| ** IgG1 mAb | | | | | | |
| ^{†} Non-targeting ADC | | | | | | |
| ^{[a]} dose is given in mg/kg/day | | | | | | |
| * = p < 0.05 as compared to control treatment (AB095) | | | | | | |
| ^{¥} = p < 0.05 as compared to the most active partner in a drug combination | | | | | | |

### Example 11. Bcl-xL Inhibitory ADCs Targeting NCAM-1, Alone and In Combination with a Bcl-2-Selective Inhibitor, Inhibit the Growth of small Cell Lung Cancer (SCLC) Tumors In Vivo

The efficacy of exemplary Bcl-xLi ADCs targeting NCAM1, both alone and in combination with compound ABT-199, a small molecule selective inhibitor of Bcl-2, in inhibiting the growth of tumor cells as in two small cell lung cancer (SCLC) xenograft models; NCI-H146 and NCI-H1963.FP5.

### 11.1. Methods

The SCLC cell line, NCI-H146 (hereafter called H146) was purchased from the American Type Culture Collection (ATCC, Manassas, VA). The cells were cultured as monolayers in RPMI-1640 culture medium (Invitrogen, Carlsbad, CA) that was supplemented with 10 % Fetal Bovine Serum (FBS, Hyclone, Logan, UT). The SCLC carcinoma cell line, NCI-H1963.FP5 (hereafter called H1963.FP5) was derived from NCI-H1963 (ATCC) following 5 sequential passages in the flank of immunocompromised mice. The cells were cultured as monolayers in RPMI-1640 culture medium (Invitrogen, Carlsbad, CA) that was supplemented with Fetal Bovine Serum (FBS, Hyclone, Logan, UT). To generate xenografts, 5^{∗}10⁶ viable cells were inoculated subcutaneously into the right flank of immune deficient female SCID/bg mice (Charles River Laboratories, Wilmington, MA). The injection volume was 0.2 ml and composed of a 1:1 mixture of S-MEM and Matrigel (BD, Franklin Lakes, NJ). Tumors were size matched at approximately 200 mm³. Antibodies and conjugates were formulated in phosphate buffered saline (PBS) and injected intraperitoneally. Injection volume did not exceed 400 µl. Therapy began within 24 hours after size matching of the tumors. Mice weighed approximately 25 g at the onset of therapy. Tumor volume determinations and animal husbandry were executed as described in example 10.

For the assay, NCAM1-targeted ADC α-NCAM1-H (also referred to as N901-H) was used. The ADCs evaluated comprised the monoclonal antibody NCAM-1 (N901) (*see* Roguska et al., 1994, Proc Natl Acad Sci USA 91:969-973). The targeted tumor associated antigen NCAM1 is expressed on the surface of H146 and H1963.FP5 cells. As for the prior experiments, antibody AB095 was used as a negative control. Bcl-xLi ADCs were administered as single agents and in combination with ABT-199.

### 11.2. Results

### 11.2.1. Efficacy of NCAM-1-targeting ADCs on growth of H146 SCLC xenografts

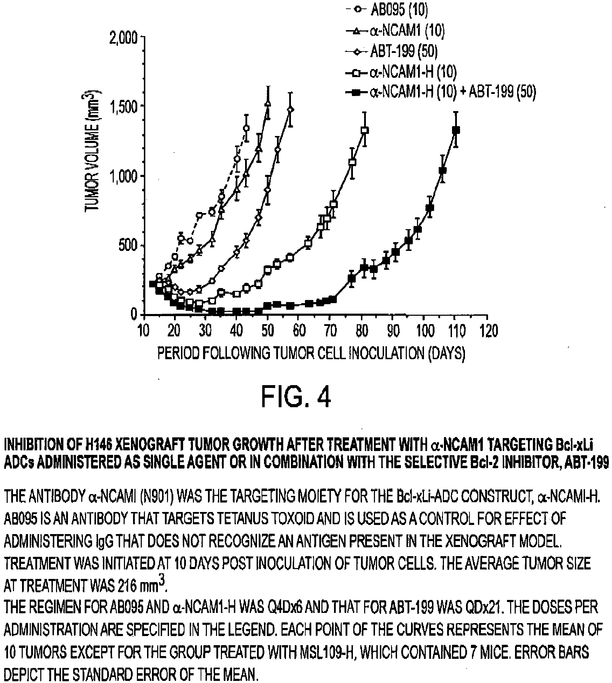

Treatment was initiated at 10 days post inoculation of tumor cells. The average tumor size at treatment was 216 mm³. The regimen for AB095 and α-NCAM-1-H was Q4Dx6 and that for ABT-199 was QDx21.The results of the experiment are shown in FIG. 4 and Table 14. Bcl-xL ADCs were administered as single agents and in combination with ABT-199. Antibodies and conjugates were injected intraperitoneally. ABT-199 was given orally. In **FIG.4****,** the amounts given per administration are specified in the legend. Each point of the curves represents the mean of 10 tumors except for the groups treated with MSL109-H, which contained 7 mice. Error bars depict the standard error of the mean.

The NCAM1-targeted Bcl-xLi ADC inhibited tumor growth when administered as a single agent. Targeted ADC α-NCAM1-H inhibited tumor growth by 88% and increased the time to reach a volume of 1 cm³ by 144% (**FIG.4****,** Table 14). The efficacy of the NCAM1-H ADC was selective as its efficacy was significantly higher than that of the naked antibody α-NCAM1 or the non-targeted control MSL109-H (Table 14). In addition, a significant additive effect was noted when treatment with α-NCAM1-H was combined with administration of a selective Bcl-2 inhibitor, ABT-199. The TGI, TGD and complete response rates were increased from 88, 144 and 0% to 98, 204 and 80 %, respectively.

| **Table 14** | | | | | | |
|---|---|---|---|---|---|---|
| **Inhibition of H146 xenograft tumor growth after treatment with α-NCAM1 targeting Bcl-xLi ADCs administered as single agent or in combination with the selective Bcl-2 inhibitor, ABT-199** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 10/IP/Q4Dx6 | 0 | 0 | 0 | 0 | 0 |
| MSLI09-H^{†} | 10/IP/Q4Dx6 | 56* | 48* | 0 | 0 | 0 |
| α-NCAM1 | 10/IP/Q4Dx6 | 35* | 11* | 0 | 0 | 0 |

| **Table 14** | | | | | | |
|---|---|---|---|---|---|---|
| **Inhibition of H146 xenograft tumor growth after treatment with α-NCAM1 targeting Bcl-xLi ADCs administered as single agent or in combination with the selective Bcl-2 inhibitor, ABT-199** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| ABT-199 | 50/PO/QDx21 | 75* | 48* | 0 | 0 | 0 |
| α-NCAM1-H | 10/IP/Q4Dx6 | 88* | 144* | 0 | 50 | 50 |
| α-NCAM1-H | 10/IP/Q4Dx6 | | | | | |
| + ABT-199 | + 50/PO/QDx21 | 98*^{¥} | 244*^{¥} | 80 | 20 | 100 |
| ** IgG1 mAb | | | | | | |
| ^{†} Non-targeting ADC | | | | | | |
| ^{[a]} dose is given in mg/kg/day | | | | | | |
| * = p < 0.05 as compared to control treatment (AB095) | | | | | | |
| ^{¥} = p < 0.05 as compared to the most active partner in a drug combination | | | | | | |

### 11.2.2. Efficacy of NCAM-1-targeting ADCs on growth of H1963.FP5 SCLC xenografts

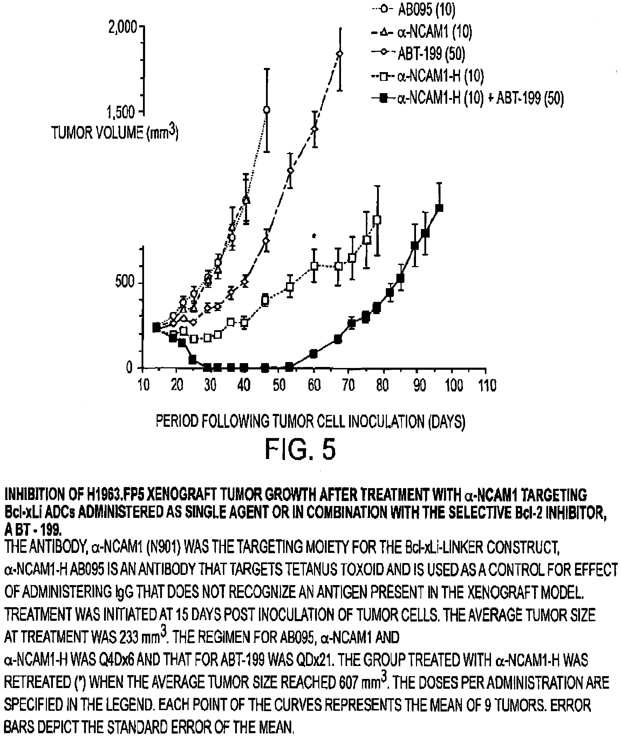

Additional xenograft experiments were performed with H1963 xenografts. These xenografts express NCAM1. Treatment was initiated at 15 days post inoculation of tumor cells. The average tumor size at treatment was 233 mm³. The regimen for AB095 and α-NCAM1-H was Q4Dx6 and that for ABT-199 was QDx21. The results of the experiment are shown in **FIG. 5** and Table 15. Bcl-xLi ADCs were administered as single agents and in combination with ABT-199. Antibodies and conjugates were injected intraperitoneally. ABT-199 was given orally. In **FIG.5****,** the doses per administration are specified in the legend. Each point of the curves represents the mean of 9 tumors except for the group treated with α MSL109-H that contained 5 mice. Error bars depict the standard error of the mean.

### 11.2.3. Results

The results of the H1963.FP5 xenograft experiment are provided Table 15, below. The conjugate targeting NCAM1 caused inhibition of tumor growth when administered as a single agent. α-NCAM1-H (N901-H) inhibited tumor growth by 74 % (**FIG. 5****,** Table 15). The efficacy of α-NCAM1-H (also referred to as N901-H) was selective as the efficacy was significantly higher than that of either naked antibody or the non-targeted control MSL109-H (Table 15). Similar to the efficacy observed in the H146 xenografts, a significant additive effect was noted when treatment with α-NCAM1-H was combined with administration of a selective Bcl-2 inhibitor, ABT-199.

| **Table 15** | | | | | | |
|---|---|---|---|---|---|---|
| **Inhibition of H1963.FP3 xenograft tumor growth after treatment with α-NCAM1 targeting Bcl-xLi ADCs administered as single agent or in combination with the selective Bcl-2 inhibitor, ABT-199** | | | | | | |
| | | **Growth Inhibition** | | **Response Frequency** | | |
| **Treatment** | **Dose^{[a]}/route/regimen** | **TGIₘₐₓ (%)** | **TGD (%)** | **CR (%)** | **PR (%)** | **OR (%)** |
| AB095** | 10/IP/Q4Dx6 | 0 | 0 | 0 | 0 | 0 |
| MSLI09-H^{†} | 10/IP/Q4Dx6 | 55* | 22* | 0 | 0 | 0 |
| α-NCAM1 | 10/IP/Q4Dx6 | 18 | 0 | 0 | 0 | 0 |
| ABT-199 | 50/PO/QDx21 | 50* | 22* | 0 | 0 | 0 |
| α-NCAM1-H | 10/IP/Q4Dx6 | 74* | 117* | 0 | 0 | 0 |
| α-NCAM1-H | 10/IP/Q4Dx6 | | | | | |
| + ABT-199 | + 50/PO/QDx21 | 100*^{¥} | 178*^{¥} | 100 | 0 | 0 |
| ** IgG1 mAb | | | | | | |
| ^{†} Non-targeting ADC | | | | | | |
| ^{[a]} dose is given in mg/kg/day | | | | | | |
| * = p < 0.05 as compared to control treatment (AB095) | | | | | | |
| ^{¥} = p < 0.05 as compared to the most active partner in a drug combination | | | | | | |

### Example 12. Bcl-xL Antibody-Drug Conjugates Mitigate Systemic Toxicity

### 12.1. Circumvention of thrombocytopenia

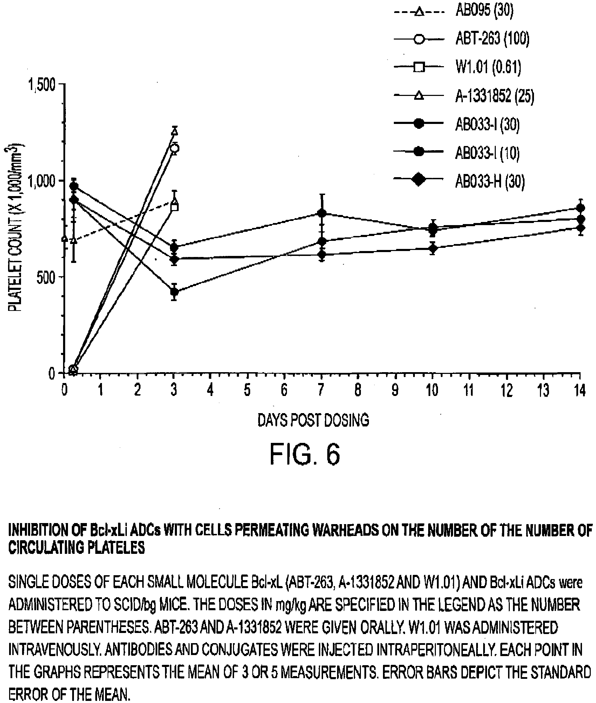

Administration of Bcl-xLi ADCs as antibody drug conjugates can possibly circumvent the systemic toxicity of the small molecule via selective targeting of the tumor. In this manner, the ADC can bypass systemic toxicity and allow tumor-specific efficacy via two possible mechanisms. First, for ADCs with a cell membrane permeating Bcl-xL inhibitor, the binding to the carrier antibody can limit systemic exposure to the small molecule. Second, the ADC can drive the internalization of a non-permeating Bcl-xL inhibitor and thus selectively affect tumor cells that carry the targeted antigen. Evidence of the first mechanism is presented in **FIG. 6****.**

### 12.1.1. Method & Results

The influence of two Bcl-xL inhibitory ADCs (Bcl-xLi ADCs) on the number of circulating platelets in mice was tested following a single intraperitoneal injection (the inhibitory ADCs comprises of anti-EGFR antibody AB033 and are designated AB033-H and AB033-I). The anti-tetanus toxoid antibody AB095 was used as a negative control. Navitoclax (ABT-263, a dual Bcl-2 and Bcl-xL inhibitor), A-1331852 (selective Bcl-xL inhibitor, Leverson et al., 2015, Sci. Transl. Med. 7:279ra40.) and the unconjugated Bcl-xL inhibitor caused thrombocytopenia which was maximal at 6 hours following injection of the compounds. A dose of 0.61 mg/kg, which is the equivalent amount of Bcl-xL inhibitor found in Bcl-xL ADC at 30 mg/kg, decreased the platelet number 100-fold from a normal count of approximately 6^{∗}10⁵ /mm³ to 6^{∗}10³ /mm³.

In contrast, none of the Bcl-xL inbitory ADCs caused a meaningful reduction of the platelets 6 hours after administration (Table 16) or at any time point during an observation period of 14 days. The latter observation renders induction of thrombocytopenia caused by slow release of the inhibitor from the ADCs is unlikely.

| **Table 16** | | | |
|---|---|---|---|
| **Influence of Bcl-xLi ADCs with cell permeating Bcl-xL inhibitors on the number of circulating platelets** | | | |
| **Compound** | **Dose (mg/kg)** | **Lowest thrombocyte count** | **Time to lowest count (hours)** |
| None | | 594 | 0 |
| AB095 | 30 | 539 | 6 |
| ABT-263 | 100 | 10 | 6 |
| W1.01 | 0.61 | 6 | 6 |
| A-1331852 | 25 | 9 | 6 |
| AB033-I | 30 | 335 | 72 |
| AB033-I | 10 | 567 | 72 |
| AB033-H | 30 | 521 | 72 |
| Platelet count is presented as 1/10³ of the platelet#/mm³ | | | |

While various specific embodiments have been illustrated and described, it will be appreciated that various changes can be made without departing from the spirit and scope of the disclosure.

Further, the invention provides the following embodiments:
Embodiment 1. An antibody drug conjugate (ADC) comprising a drug linked to an antibody by way of a linker, wherein the drug is a Bcl-xL inhibitor according to structural formula (IIa): 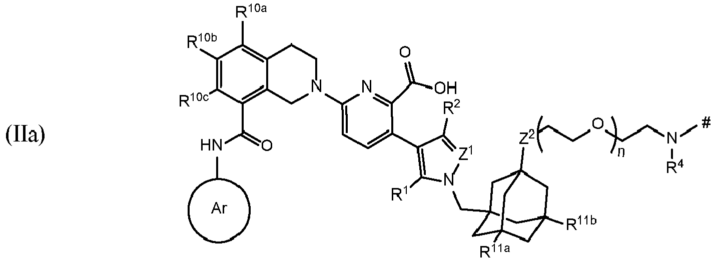 or pharmaceutically acceptable salts thereof, wherein:
   **Ar** is selected from 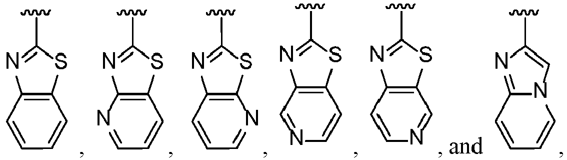 which is optionally substituted with one or more substituents independently selected from halo, cyano, methyl, and halomethyl;
   **Z¹** is selected from N, CH and C-CN;
   **Z²** is selected from NH, CH₂, O, S, S(O), and S(O₂);
   **R¹** is selected from methyl, chloro, and cyano;
   **R²** is selected from hydrogen, methyl, chloro, and cyano;
   **R⁴** is hydrogen, C₁₋₄ alkanyl, C₂₋₄ alkenyl, C₂₋₄ alkynyl, C₁₋₄ haloalkyl or C₁₋₄ hydroxyalkyl, wherein the R⁴ C₁₋₄ alkanyl, C₂₋₄ alkenyl, C₂₋₄ alkynyl, C₁₋₄ haloalkyl and C₁₋₄ hydroxyalkyl are optionally substituted with one or more substituents independently selected from OCH₃, OCH₂CH₂OCH₃, and OCH₂CH₂NHCH₃;
   **R^{10a,} R^{10b},** and **R^{10c}** are each, independently of one another, selected from hydrogen, halo, C₁₋₆ alkanyl, C₂₋₆ alkenyl, C₂₋₆ alkynyl, and C₁₋₆ haloalkyl;
   **R^{11a}** and **R^{11b}** are each, independently of one another, selected from hydrogen, methyl, ethyl, halomethyl, hydroxyl, methoxy, halo, CN and SCH₃;
   **n** is 0, 1, 2 or 3; and
   # represents the point of attachment to linker L.
2. The ADC of **embodiment 1,** or a pharmaceutically acceptable salt thereof, which has a drug-to-antibody ratio of 1-10.
3. The ADC of **embodiment 1,** or a pharmaceutically acceptable salt thereof, in which the linker is cleavable by a lysosomal enzyme.
4. The ADC of **embodiment 3,** or a pharmaceutically acceptable salt thereof, in which the lysosomal enzyme is Cathepsin B.
5. The ADC of **embodiment 3,** or a pharmaceutically acceptable salt thereof, in which the linker comprises a segment according to structural formula (IVa), (IVb), or (IVc): 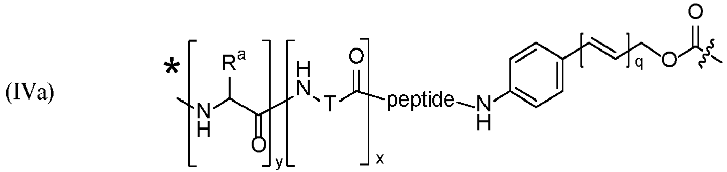 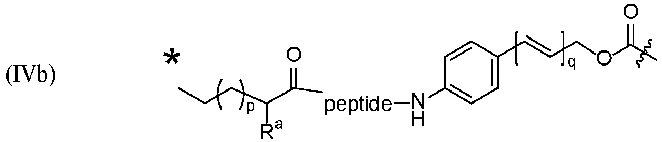 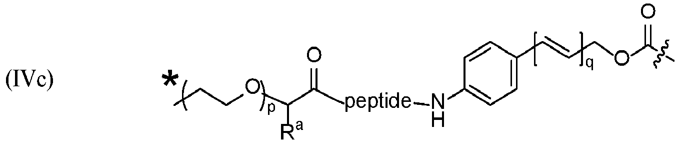 or a salt thereof, wherein:
   **peptide** represents a peptide (illustrated N→C, wherein peptide includes the amino and carboxy "termini") a cleavable by a lysosomal enzyme;
   **T** represents a polymer comprising one or more ethylene glycol units or an alkylene chain, or combinations thereof;
   **R^{a}** is selected from hydrogen, alkyl, sulfonate and methyl sulfonate;
   **p** is an integer ranging from 0 to 5;
   **q** is 0 or 1;
   **x** is 0 or 1;
   **y** is 0 or 1;
   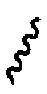 represents the point of attachment of the linker to the Bcl-xL inhibitor; and
   * represents the point of attachment to the remainder of the linker.
6. The ADC of **embodiment 5,** or a pharmaceutically acceptable salt thereof, in which **peptide** is selected from the group consisting of: Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; Cit-Asp; Ala-Val; Val-Ala; Phe-Lys; Lys-Phe; Val-Lys; Lys-Val; Ala-Lys; Lys-Ala; Phe-Cit; Cit-Phe; Leu-Cit; Cit-Leu; Ile-Cit; Cit-Ile; Phe-Arg; Arg-Phe; Cit-Trp; and Trp-Cit, or salts thereof.
7. The ADC of **embodiment 3,** or a pharmaceutically acceptable salt thereof, in which the lysosomal enzyme is β-glucuronidase or β-galactosidase.
8. The ADC of **embodiment 7,** or a pharmaceutically acceptable salt thereof, in which the linker comprises a segment according to structural formula (Va), (Vb), (Vc), or (Vd): 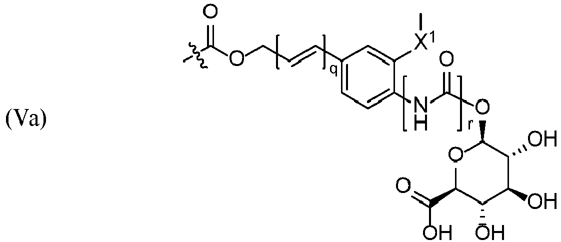 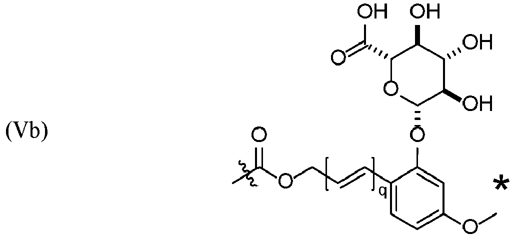 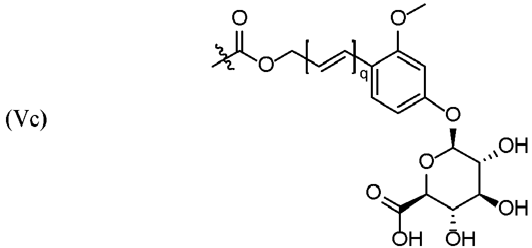 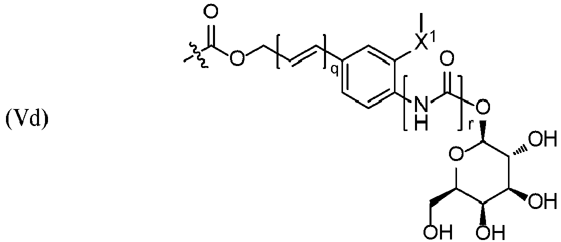 or a salt thereof, wherein:
   **q** is 0 or 1;
   **r** is 0 or 1;
   **X¹** is O or NH;
   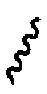 represents the point of attachment of the linker to the drug; and
   * represents the point of attachment to the remainder of the linker.
9. The ADC of **embodiment 1,** or a pharmaceutically acceptable salt thereof, in which the linker comprises a polyethylene glycol segment having from 1 to 6 ethylene glycol units.
10. The ADC of **embodiment 1,** or a pharmaceutically acceptable salt thereof, in which the antibody binds a cell surface receptor or a tumor associated antigen expressed on a tumor cell.
11. The ADC of **embodiment 10,** or a pharmaceutically acceptable salt thereof, in which the antibody binds one of the cell surface receptors or tumor associated antigens selected from EGFR, EpCAM and NCAM1.
12. The ADC of **embodiment 11,** or a pharmaceutically acceptable salt thereof, in which the antibody binds EGFR, EpCAM or NCAM1.
13. The ADC, or a pharmaceutically acceptable salt thereof, of **embodiment 11** in which the antibody binds EpCAM.
14. The ADC of **embodiment 1,** or a pharmaceutically acceptable salt thereof, which is a compound according to structural formula (I): 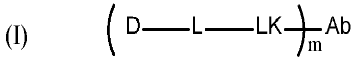 or a salt thereof, wherein:
   **D** is the drug;
   **L** is the linker;
   **Ab** is the antibody;
   **LK** represents a covalent linkage linking linker L to antibody Ab; and
   **m** is an integer ranging from 1 to 20.
15. The ADC, or a pharmaceutically acceptable salt thereof, of **embodiment 14** in which **m** is an integer ranging from 1-8.
16. The ADC of **embodiment 14,** or a pharmaceutically acceptable salt thereof, in which **m** is 2, 3 or 4.
17. The ADC of **embodiment 14,** or a pharmaceutically acceptable salt thereof, in which **Ar** is selected from 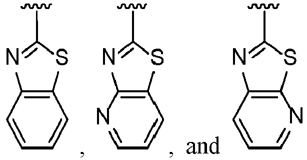 and is optionally substituted with one or more substituents independently selected from halo, cyano, methyl, and halomethyl.
18. The ADC of **embodiment 14,** or a pharmaceutically acceptable salt thereof, in which **Ar** is 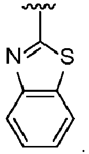
19. The ADC of **embodiment 14,** or a pharmaceutically acceptable salt thereof, in which **Z¹** is N.
20. The ADC of **embodiment 14,** or a pharmaceutically acceptable salt thereof, in which **Z¹** is CH.
21. The ADC of **embodiment 14,** or a pharmaceutically acceptable salt thereof, in which **Z²** is O.
22. The ADC of **embodiment 14,** or a pharmaceutically acceptable salt thereof, in which **R¹** is selected from methyl and chloro.
23. The ADC of **embodiment 14,** or a pharmaceutically acceptable salt thereof, in which **R²** is selected from hydrogen and methyl.
24. The ADC of **embodiment 14,** or a pharmaceutically acceptable salt thereof, in which **R²** is hydrogen.
25. The ADC of **embodiment 14,** or a pharmaceutically acceptable salt thereof, in which **R^{10a}** is halo and **R^{10b}** and **R^{10c}** are each hydrogen.
26. The ADC of **embodiment 14,** or a pharmaceutically acceptable salt thereof, in which **R^{10a}** is fluoro and **R^{10b}** and **R^{10c}** are each hydrogen.
27. The ADC of **embodiment 14,** or a pharmaceutically acceptable salt thereof, in which **R^{10a}, R^{10b}** and **R^{10c}** are each hydrogen.
28. The ADC of **embodiment 14,** or a pharmaceutically acceptable salt thereof, in which **R^{11a}** and **R^{11b}** are the same.
29. The ADC of **embodiment 14,** or a pharmaceutically acceptable salt thereof, in which **R¹¹³** and **R^{11b}** are each methyl.
30. The ADC of **embodiment 14,** or a pharmaceutically acceptable salt thereof, in which ***n*** is 0 or 1.
31. The ADC of **embodiment 14** in which **D** is selected from the group consisting of W1.01, W1.02, W1.03, W1.04, W1.05, W1.06, W1.07, and W1.08 and pharmaceutically acceptable salts thereof.
32. The ADC of **embodiment 14** in which linker L is selected from the group consisting of IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c.2, V1d.1-V1d.3, and pharmaceutically acceptable salts thereof.
33. The ADC of **embodiment 14,** or a pharmaceutically acceptable salt thereof, in which **LK** is a linkage formed with an amino group on antibody **Ab.**
34. The ADC of **embodiment 33,** or a pharmaceutically acceptable salt thereof, in which **LK** is an amide or a thiourea.
35. The ADC of **embodiment 14,** or a pharmaceutically acceptable salt thereof, in which **LK** is a linkage formed with a sulfhydryl group on antibody **Ab.**
36. The ADC of **embodiment 35,** or a pharmaceutically acceptable salt thereof, in which **LK** is a thioether.
37. The ADC of **embodiment 14,** or a pharmaceutically acceptable salt thereof, in which antibody **Ab** binds EGFR or NCAM1.
38. The ADC of **embodiment 14,** or a pharmaceutically acceptable salt thereof, in which antibody **Ab** binds EGFR.
39. The ADC of **embodiment 14** in which:
   **D** is selected from the group consisting of W1.01, W1.02, W1.03, W1.04, W1.05, W1.06, W1.07, and W1.08 and pharmaceutically acceptable salts thereof;
   **L** is selected from the group consisting of linkers IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c.2, V1d.1-V1d.3, and salts thereof;
   **LK** is selected from the group consisting of amide, thiourea and thioether; and
   **m** is an integer ranging from 1 to 8.
40. The ADC of **embodiment 39,** or a pharmaceutically acceptable salt thereof, in which the **Ab** binds EGFR or NCAM1.
41. A composition comprising an ADC according to **any one of embodiments 1-40** and a carrier, excipient and/or diluent.
42. The composition of **embodiment 41** which is formulated for pharmaceutical use in humans.
43. The composition of **embodiment 42** which is in unit dosage form.
44. A synthon according to structural formula D-L-R*^{x}*, or a pharmaceutically acceptable salt thereof, wherein:
   **D** is a drug;
   **L** is a linker; and
   **R*^{x}*** is a moiety comprising a functional group capable of covalently linking the synthon to an antibody,
   and further wherein drug **D** is a Bcl-xL inhibitor according to structural formula : 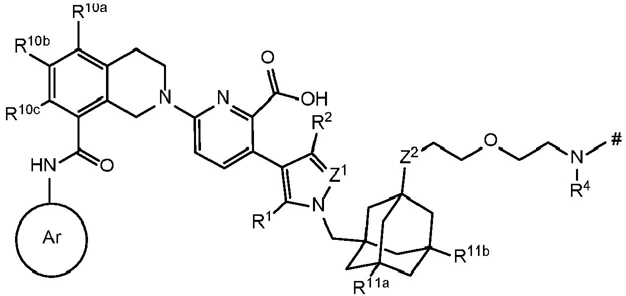
   or pharmaceutically acceptable salts thereof, wherein:
   **Ar** is selected from 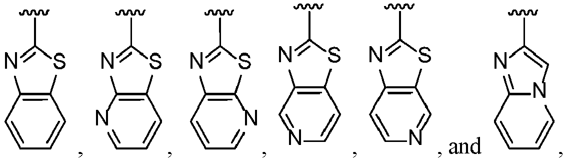 which is optionally substituted with one or more substituents independently selected from halo, cyano, methyl, and halomethyl;
   **Z¹** is selected from N, CH and C-CN;
   **Z²** is selected from NH, CH₂, O, S, S(O), and S(O₂);
   **R¹** is selected from methyl, chloro, and cyano;
   **R²** is selected from hydrogen, methyl, chloro, and cyano;
   **R⁴** is hydrogen, C₁₋₄ alkanyl, C₂₋₄ alkenyl, C₂₋₄ alkynyl, C₁₋₄ haloalkyl or C₁₋₄ hydroxyalkyl, wherein the R⁴ C₁₋₄ alkanyl, C₂₋₄ alkenyl, C₂₋₄ alkynyl, C₁₋₄ haloalkyl and C₁₋₄ hydroxyalkyl are optionally substituted with one or more substituents independently selected from OCH₃, OCH₂CH₂OCH₃, and OCH₂CH₂NHCH₃;
   **R^{10a}, R^{10b}** and **R^{10c}** are each, independently of one another, selected from hydrogen, halo, C₁₋₆ alkanyl, C₂₋₆ alkenyl, C₂₋₆ alkynyl, and C₁₋₆ haloalkyl;
   **R^{11a}** and **R^{11b}** are each, independently of one another, selected from hydrogen, methyl, ethyl, halomethyl, hydroxyl, methoxy, halo, CN and SCH₃;
   **n** is 0, 1, 2 or 3; and
   # represents the point of attachment to linker L.
45. The synthon of **embodiment 44,** or pharmaceutically acceptable salts thereof, in which the linker is cleavable by a lysosomal enzyme.
46. The synthon of **embodiment 45,** or pharmaceutically acceptable salts thereof, in which the lysosomal enzyme is Cathepsin B.
47. The synthon of **embodiment 44,** or pharmaceutically acceptable salts thereof, in which the linker comprises a segment according to structural formula (IVa), (IVb), or (IVc): 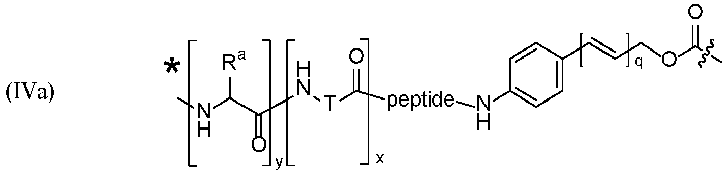 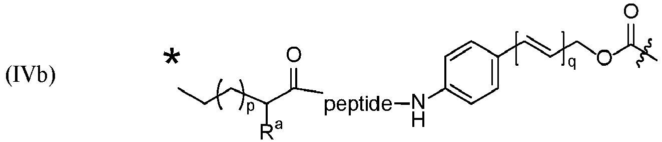 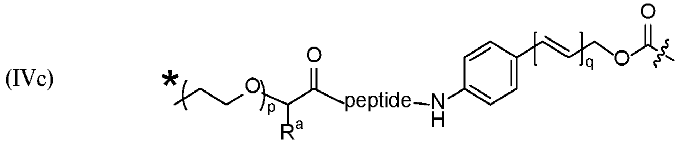 wherein:
   **peptide** represents a peptide (illustrated N→C, wherein peptide includes the amino and carboxy "termini") a cleavable by a lysosomal enzyme;
   **T** represents a polymer comprising one or more ethylene glycol units or an alkylene chain, or combinations thereof;
   **R^{a}** is selected from hydrogen, alkyl, sulfonate and methyl sulfonate;
   **p** is an integer ranging from 0 to 5;
   **q** is 0 or 1;
   **x** is 0 or 1;
   **y** is 0 or 1;
   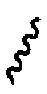 represents the point of attachment of the linker to the Bcl-xL inhibitor; and
   * represents the point of attachment to the remainder of the linker.
48. The synthon of **embodiment 47,** or pharmaceutically acceptable salts thereof, in which **peptide** is selected from the group consisting of Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; Cit-Asp; Ala-Val; Val-Ala; Phe-Lys; Lys-Phe; Val-Lys; Lys-Val; Ala-Lys; Lys-Ala; Phe-Cit; Cit-Phe; Leu-Cit; Cit-Leu; Ile-Cit; Cit-Ile; Phe-Arg; Arg-Phe; Cit-Trp; and Trp-Cit.
49. The synthon of **embodiment 44,** or pharmaceutically acceptable salts thereof, in which the lysosomal enzyme is β-glucuronidase or β-galactosidase.
50. The synthon of **embodiment 49** in which the linker comprises a segment according to structural formula (Va), (Vb) or (Vc): 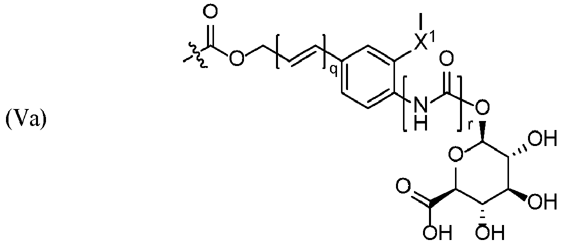 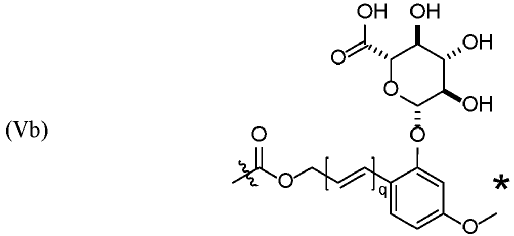 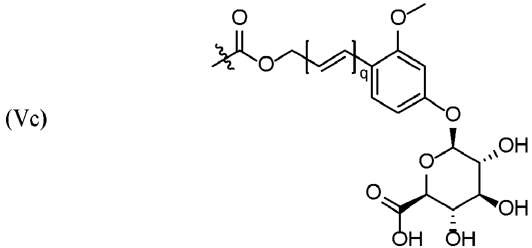 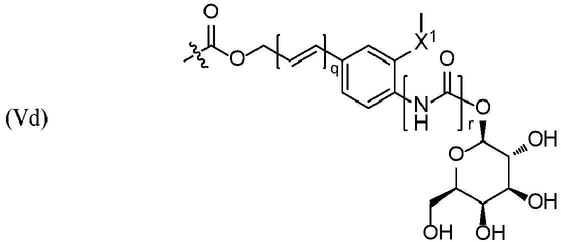 or pharmaceutically acceptable salts thereof, wherein:
   **q** is 0 or 1;
   **r** is 0 or 1;
   **X¹** is O or NH;
   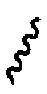 represents the point of attachment of the linker to the drug; and
   * represents the point of attachment to the remainder of the linker.
51. The synthon of **embodiment 44,** or pharmaceutically acceptable salts thereof, in which the linker comprises a polyethylene glycol segment having from 1 to 6 ethylene glycol units.
52. The synthon of **embodiment 44,** or pharmaceutically acceptable salts thereof, in which **Ar** is selected from 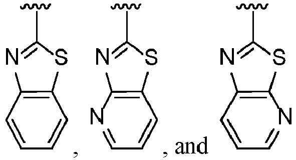 and is optionally substituted with one or more substituents independently selected from halo, cyano, methyl, and halomethyl.
53. The synthon of **embodiment 44,** or pharmaceutically acceptable salts thereof, in which **Ar** is 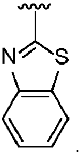
54. The synthon of **embodiment 44,** or pharmaceutically acceptable salts thereof, in which **Z¹** is N.
55. The synthon of **embodiment 44,** or pharmaceutically acceptable salts thereof, in which **Z¹** is CH.
56. The synthon of **embodiment 44,** or pharmaceutically acceptable salts thereof, in which **Z²** is O.
57. The synthon of **embodiment 44,** or pharmaceutically acceptable salts thereof, in which **R¹** is selected from methyl and chloro.
58. The synthon of **embodiment 44,** or pharmaceutically acceptable salts thereof, in which **R²** is selected from hydrogen and methyl.
59. The synthon of **embodiment 44,** or pharmaceutically acceptable salts thereof, in which **R²** is hydrogen.
60. The synthon of **embodiment 44,** or pharmaceutically acceptable salts thereof, in which **R^{10a}** is halo and **R^{10b}** and **R^{10c}** are each hydrogen.
61. The synthon of **embodiment 44,** or pharmaceutically acceptable salts thereof, in which **R^{10a}** is fluoro and **R^{10b}** and **R^{10c}** are each hydrogen.
62. The synthon of **embodiment 44,** or pharmaceutically acceptable salts thereof, in which **R^{10a}, R^{10b}** and **R^{10c}** are each hydrogen.
63. The synthon of **embodiment 44,** or pharmaceutically acceptable salts thereof, in which **R^{11a}** and **R^{11b}** are the same.
64. The synthon of **embodiment 44,** or pharmaceutically acceptable salts thereof, in which **R^{11a}** and **R^{11b}** are each methyl.
65. The synthon of **embodiment 44,** or pharmaceutically acceptable salts thereof, in which ***n*** is 0 or 1.
66. The synthon of **embodiment 44** in which **D** is selected from the group consisting of W1.01, W1.02, W1.03, W1.04, W1.05, W1.06, W1.07, and W1.08, and pharmaceutically acceptable salts thereof.
67. The synthon of **embodiment 44,** and pharmaceutically acceptable salts thereof, in which linker L is selected from the group consisting of linkers IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c.2, V1d.1-V1d.3, and pharmaceutically acceptable salts thereof.
68. The synthon of **embodiment 44,** and pharmaceutically acceptable salts thereof, in which **R***^{x}* comprises a functional group capable of linking the synthon to an amino group on an antibody.
69. The synthon of **embodiment 68,** and pharmaceutically acceptable salts thereof, in which **R***^{x}* comprises an NHS-ester or an isothiocyanate.
70. The synthon of **embodiment 66,** and pharmaceutically acceptable salts thereof, in which **R***^{x}* comprises a functional group capable of linking the synthon to a sulfhydryl group on an antibody.
71. The synthon of **embodiment 70,** and pharmaceutically acceptable salts thereof, in which **R***^{x}* comprises a haloacetyl or a maleimide.
72. The synthon of **embodiment 66,** and pharmaceutically acceptable salts thereof, in which:
   **D** is selected from the group consisting of W1.01, W1.02, W1.03, W1.04, W1.05, W1.06, W1.07, and W1.08, and pharmaceutically acceptable salts thereof;
   **L** is selected from the group consisting of linkers IVa.1-IVa.7, IVb.1-IVb.15, IVc.1-IVc.2, Va.1-Va.12, Vb.1-Vb.4, Vc.1-Vc.9, Vd.1-Vd.2, VIa.1, V1c.1-V1c.2, V1d.1-V1d.3, and salts thereof; and
   **R***^{x}* comprises a functional group selected from the group consisting of NHS-ester, isothiocyanate, haloacetyl and maleimide.
73. An ADC formed by contacting an antibody that binds a cell surface receptor or tumor associated antigen expressed on a tumor cell with a synthon according to **any one of embodiments 44-72** under conditions in which the synthon covalently links to the antibody.
74. The ADC of **embodiment 73** in which the contacting step is carried out under conditions such that the ADC has a DAR of 2, 3 or 4.
75. A composition comprising an ADC according to **embodiment 73 or 74** and an excipient, carrier and/or diluent.
76. The composition of **embodiment 75** which is formulated for pharmaceutical use in humans.
77. The composition of **embodiment 76** which is in unit dosage form.
78. A method of making an ADC, comprising contacting a synthon according to **any one of embodiments 63-69** under conditions in which the synthon covalently links to the antibody.
79. A method of inhibiting Bcl-xL activity in a cell that expresses Bcl-xL, comprising contacting the cell with an ADC according to **any one of embodiments 1-40 and 73-74** that is capable of binding the cell, under conditions in which the ADC binds the cell.
80. A method of inducing apoptosis in a cell which expresses Bcl-xL, comprising contacting the cell with an ADC according to **any one of embodiments 1-40 and 73-74** that is capable of binding the cell, under conditions in which the ADC binds the cell.
81. A method of treating a disease involving dysregulated intrinsic apoptosis, comprising administering to a subject having a disease involving dysregulated apotosis an amount of an ADC according **to any one of embodiments 1-40 and 73-74** effective to provide therapeutic benefit, wherein the antibody of the ADC binds a cell surface receptor on a cell whose intrinsic apoptosis is dysregulated.
82. A method of treating cancer, comprising administering to a subject having cancer an ADC according to **any one of embodiments 1-40 and 73-74** that is capable of binding a cell surface receptor or a tumor associated antigen expressed on the surface of the cancer cells, in an amount effective to provide therapeutic benefit.
83. The method of **embodiment 74** in which the ADC is administered as monotherapy.
84. The method of **embodiment 74** in which the cancer being treated is a tumorigenic cancer.
85. The method of **embodiment 74** in which the ADC is administered adjunctive to another chemotherapeutic agent radiation therapy.
86. The method of **embodiment 85** in which the ADC is administered concurrently with the initiation of the chemotherapy and/or radiation therapy.
87. The method of **embodiment 85** in which the ADC is administered prior to initiating the chemotherapy and/or radiation therapy.
88. The method of **any one of embodiments 85-87** in which the ADC is administered in an amount effective to sensitize the tumor cells to standard chemotherapy and/or radiation therapy.
89. A method of sensitizing a tumor to standard cytotoxic agents and/or radiation, comprising contacting the tumor with an ADC according to **any one of embodiments 1-40 and 73-**74 that is capable of binding the tumor, in an amount effective to sensitize the tumor cell to a standard cytotoxic agent and/or radiation.
90. The method of **embodiment 89** in which the tumor has become resistant to treatment with standard cytotoxic agents and/or radiation.
91. The method of **embodiment 89** in which the tumor has not been previously exposed to standard cytotoxic agents and/or radiation therapy.
92. The synthon selected from the group consisting of synthon examples 2.1, 2.2, 2.3, 2.4, 2.5, 2.6, 2.7, 2.8, 2.10, 2.12, 2.17, 2.18, 2.21, 2.22, 2.23, 2.24, 2.25, 2.26, 2.27, 2.28, 2.29, 2.30, 2.31, 2.32, 2.33, 2.34, 2.35, 2.36, 2.37, 2.38, 2.39, 2.40, 2.41, 2.42, 2.43, 2.44, 2.45, 2.46, 2.47, 2.48, 2.49, 2.50, 2.51, 2.52, 2.53, and pharmaceutically acceptable salts thereof.
93. An antibody drug conjugate (ADC), or a pharmaceutically acceptable salt thereof, comprising a synthon according to **embodiment 92** conjugated to an antibody.

## Claims

1. An antibody drug conjugate (ADC), formed by contacting an antibody that binds a cell surface receptor or tumor associated antigen expressed on a tumor cell with a synthon according to structural formula D-L-R*^{x}*, or a pharmaceutically acceptable salt thereof, wherein:
D is a drug;
L is a linker; and
R*^{x}* is a moiety comprising a functional group capable of covalently linking the synthon to an antibody,
and further wherein drug D is a Bcl-xL inhibitor according to structural formula: 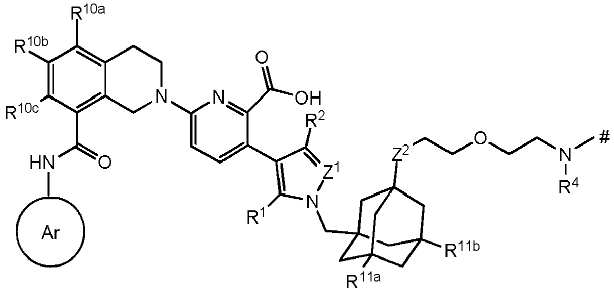
or pharmaceutically acceptable salts thereof, wherein:
Ar is selected from 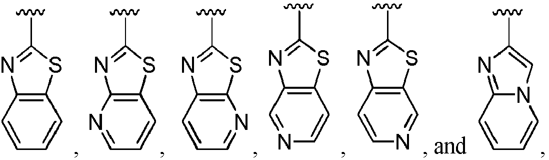 which is optionally substituted with one or more substituents independently selected from halo, cyano, methyl, and halomethyl;
Z¹ is selected from N, CH and C-CN;
Z² is selected from NH, CH₂, O, S, S(O), and S(O₂);
R¹ is selected from methyl, chloro, and cyano;
R² is selected from hydrogen, methyl, chloro, and cyano;
R⁴ is hydrogen, C₁₋₄ alkanyl, C₂₋₄ alkenyl, C₂₋₄ alkynyl, C₁₋₄ haloalkyl or C₁₋₄ hydroxyalkyl, wherein the R⁴ C₁₋₄ alkanyl, C₂₋₄ alkenyl, C₂₋₄ alkynyl, C₁₋₄ haloalkyl and C₁₋₄ hydroxyalkyl are optionally substituted with one or more substituents independently selected from OCH₃, OCH₂CH₂OCH₃, and OCH₂CH₂NHCH₃;
R^{10a}, R^{10b}, and R^{10c} are each, independently of one another, selected from hydrogen, halo, C₁₋₆ alkanyl, C₂₋₆ alkenyl, C₂₋₆ alkynyl, and C₁₋₆ haloalkyl;
R^{11a} and R^{11b} are each, independently of one another, selected from hydrogen, methyl, ethyl, halomethyl, hydroxyl, methoxy, halo, CN and SCH₃;
n is 0, 1, 2 or 3; and
# represents the point of attachment to linker L;
under conditions in which the synthon covalently links to an antibody.

2. The antibody drug conjugate of claim 1, wherein R^{11a} and R^{11b} are the same.

3. The antibody drug conjugate of claim 2, in which R^{11a} and R^{11b} are each methyl.

4. The antibody drug conjugate of any of claims 1 to 3, in which n is 0 or 1.

5. The antibody drug conjugate of any of claims 1 to 4, in which D is selected from the group consisting of W1.01, W1.02, W1.03, W1.04, W1.05, W1.06, W1.07, and W1.08, and pharmaceutically acceptable salts thereof.

6. The antibody drug conjugate of any of claims 1 to 5, in which linker L is selected from the group consisting of linkers IVa.1-IVa.7, IVb.1-IVb. 15, IVc.1-IVc.2, Va. 1-Va. 12, Vb.1-Vb.4, Vc.1-Vc.9, Vd.1-Vd.2, VIa.1, V1c.1-V1c.2, V1d.1-V1d.3, and pharmaceutically acceptable salts thereof.

7. The antibody drug conjugate of any of claims 1 to 6, in which R*^{x}* comprises a functional group capable of linking the synthon to an amino group on an antibody.

8. The antibody drug conjugate of claim 7, in which R*^{x}* comprises an NHS-ester or an isothiocyanate.


SEQUENCE LISTING

<110> ABBVIE INC.

<120> ANTIBODY DRUG CONJUGATES WITH CELL PERMEABLE BCL-XL INHIBITORS

<130> ABV12127WO01

<140> PCT/US2015/064686
<141> 2015-12-09

<150> 62/089,766
<151> 2014-12-09

<160> 3

<170> PatentIn version 3.5

<210> 1
<211> 4
<212> PRT
<213> Artificial Sequence

<220>
<221> source
<223> /note="Description of Artificial Sequence: Synthetic
      peptide"

<400> 1
Gly Phe Leu Gly
1

<210> 2
<211> 4
<212> PRT
<213> Artificial Sequence

<220>
<221> source
<223> /note="Description of Artificial Sequence: Synthetic
      peptide"

<400> 2
Ala Leu Ala Leu
1

<210> 3
<211> 16
<212> PRT
<213> Artificial Sequence

<220>
<221> source
<223> /note="Description of Artificial Sequence: Synthetic
      peptide probe F-bak"

<220>
<221> source
<223> /note="N-term acetylated"

<220>
<221> MOD_RES
<222> (13)..(13)
<223> Lys(6-FAM)

<220>
<221> source
<223> /note="C-term amidated"

<400> 3
Gly Gln Val Gly Arg Gln Leu Ala Ile Ile Gly Asp Lys Ile Asn Arg
1               5                   10                  15